# EUROPEAN PATENT APPLICATION

(11) **EP 4 215 524 A1**
(43) Date of publication of application: **26.07.2023**
(21) Application number: 21868667.3
(22) Date of filing: 16.09.2021
(51) Int. Cl.: C07D 401/02, C07D 471/00, A61K 31/53, A61K 31/438, A61P 5/14, A61P 1/16, A61P 9/10, A61P 9/12, A61P 3/06, A61P 3/04, A61P 3/10, A61P 35/00

(54) **COMPOUND SERVING AS THYROID HORMONE BETA RECEPTOR AGONIST AND USES OF COMPOUND**

(30) Priority: 17.09.2020 CN 202010977443; 03.12.2020 CN 202011413890
(71) Applicant: Sunshine Lake Pharma Co., Ltd., Dongguan, Guangdong 523000 (CN)
(72) Inventor: GU, Zheng, Dongguan, Guangdong 523871 (CN); LI, Jianhao, Dongguan, Guangdong 523871 (CN); DENG, Jianchao, Dongguan, Guangdong 523871 (CN); LI, Yongxiang, Dongguan, Guangdong 523871 (CN); QIN, Haoxiong, Dongguan, Guangdong 523871 (CN); CHEN, Daoqian, Dongguan, Guangdong 523871 (CN); ZHENG, Xiaomin, Dongguan, Guangdong 523871 (CN); YU, Jianghong, Dongguan, Guangdong 523871 (CN); ZHAO, Huantian, Dongguan, Guangdong 523871 (CN)
(74) Representative: Hoffmann Eitle
(86) International application number: PCT/CN2021/118638
(87) International publication number: WO 2022/057845

(57) **Abstract**

Provided are a compound serving as a thyroid hormone β receptor agonist and uses of the compound, also provided is a pharmaceutical composition comprising the compound. The compound or the pharmaceutical composition is applicable in preparing a medicament for preventing, treating, or alleviating thyroid hormone β receptor agonist-modulated diseases, and specifically applicable in preparing a medicament for treating nonalcoholic fatty liver disease.

## Description

### FIELD OF THE INVENTION

The invention belongs to the pharmaceutical field. It specifically relates to a compound as a thyroid hormone β receptor agonist and use thereof, and further relates to a pharmaceutical composition containing the compound. The present invention further relates to the use of the compound and pharmaceutical composition in the manufacture of a medicament for preventing, treating or alleviating diseases regulated by thyroid hormone β receptors, especially in the manufacture of a medicament for treating non-alcoholic fatty liver disease.

### BACKGROUND ART

Thyroid hormone (TH) plays an extremely important role in growth, differentiation, development and maintenance of metabolic balance. TH is synthesized by the thyroid and secreted into the circulatory system in two major forms, triiodothyronine (T3) and tetraiodothyronine (T4). Although T4 is the predominant form secreted by the thyroid, T3 is the more physiologically active form. T4 is converted to T3 by tissue-specific deiodinases, which are present in all tissues but mainly in the liver and kidney.

The physiological action of TH is mainly carried out through the thyroid hormone receptor (TR). TR is a member of the nuclear receptor superfamily, a transcription factor induced by ligand T3, and plays a central role in mediating the action of ligand T3. TR is mainly located in the nucleus, forms a heterodimer with retinoid X receptor (RXR) and other nuclear receptors, and binds to the thyroid hormone response element (TRE) in the promoter region of the target gene, thereby regulating gene transcription. There are two subtypes of TR: TRα and TRβ. TRα can be divided into TRα1 and TRα2, and TRβ can be further divided into TRβ1 and TRβ2. Among them, only TRα1, TRβ1 and TRβ2 can bind to the ligand T3. TRα mainly regulates heart rate, and TRβ plays a key role in controlling hepatic cholesterol metabolism and inhibiting thyroid-stimulating hormone (TSH) release, which may be related to the high expression of TRβ in the liver and pituitary gland.

TH has some therapeutic benefit if side effects can be minimized or eliminated (Paul M. Yen et. al. Physiological Reviews, Vol. 81(3): pp. 1097-1126 (2001); Paul Webb et. al. Expert Opin. Investig. Drugs, Vol. 13(5): pp. 489-500 (2004)). For example, TH increases metabolic rate, oxygen consumption, and heat production, thereby reducing body weight. Reducing body weight will have a beneficial effect on obese patients by improving obesity-related co-morbidities and may also have a beneficial effect on glycemic control in obese patients with type 2 diabetes.

TH also lowers serum low-density lipoprotein (LDL) (Eugene Morkin et. al. Journal of Molecular and Cellular Cardiology, Vol. 37: pp. 1137-1146 (2004)). Hyperthyroidism has been found to be associated with low total serum cholesterol due to TH increasing hepatic LDL receptor expression and stimulating the metabolism of cholesterol to bile acids (JJ. Abrams et. al. J. Lipid Res., Vol. 22: pp. 323-38 (1981)). Hypothyroidism is associated with hypercholesterolemia, and TH replacement therapy has been reported to lower total cholesterol (M. Aviram et. al. Clin. Biochem., Vol. 15: pp. 62-66 (1982); JJ. Abrams et. al. J. Lipid Res., Vol. 22: pp. 323-38 (1981)). In animal models, TH has been shown to have beneficial effects in increasing HDL cholesterol and increasing the conversion of LDL to HDL by increasing the expression of apo A-1 (one of the main apolipoproteins of HDL) (Gene C. Ness et. al. Biochemical Pharmacology, Vol. 56: pp. 121-129 (1998); GJ. Grover et. al. Endocrinology, Vol. 145: pp. 1656-1661 (2004); GJ. Grover et. al. Proc. Natl. Acad. Sci. USA, Vol. 100: pp. 10067-10072 (2003)). The incidence of atherosclerotic vascular diseases is directly related to the level of LDL cholesterol. Through the regulation of LDL and HDL, TH may also reduce the risk of atherosclerosis and other cardiovascular diseases. Additionally, there is evidence that TH reduces lipoprotein(a), an important risk factor that is elevated in atherosclerotic patients (Paul Webb et. al. Expert Opin. Investig. Drugs, Vol. 13(5): pp. 489-500 (2004); de Bruin et. al. J. Clin. Endo. Metab., Vol. 76: pp. 121-126 (1993)).

TH is also a key signal for oligodendrocyte differentiation and myelination during development and stimulates remyelination in adult models of multiple sclerosis (MS) (Calza et al., Brain Res Revs 48:339-346, 2005). However, due to the limited therapeutic window to achieve remyelination while avoiding the cardiotoxicity and bone demineralization associated with chronic hyperthyroidism, TH cannot be used in long-term courses. Some TH analogs can activate TH-responsive genes while avoiding TH-related disadvantages by exploiting the molecular and physiological features of TH receptors (Malm et. al. Mini Rev Med Chem 7:79-86, 2007).

In addition, non-alcoholic fatty liver disease (NAFLD) is also closely related to TH. On the one hand, NAFLD affects the transformation and inactivation of TH, which can lead to a decrease in the level of serum TH; on the other hand, the decrease in the level of TH further causes lipid metabolism disorder and glucose metabolism disorder, and participates in the occurrence of NAFLD. Studies have shown that fatty liver formation in rats was induced by a choline-methionine-deficient diet, and the reversal of fatty liver can be observed after feeding T3 (PerraA, et al. Faseb, 2008, 22 (8): 2981).

However, endogenous TH is non-selective and has side effects, such as hyperthyroidism, especially related to cardiovascular toxicity. Therefore, the development of TH analogues (such as thyroid hormone β receptor agonists) that avoid the adverse effects of hyperthyroidism while maintaining the beneficial effects of TH will open new avenues for the treatment of patients with diseases such as obesity, hyperlipidemia, hypercholesterolemia, diabetes, hepatic steatosis, nonalcoholic fatty liver disease, atherosclerosis, cardiovascular disease, hypothyroidism, thyroid cancer, thyroid disease, and related conditions and diseases.

### SUMMARY

The present invention provides a class of compounds with good agonistic activity on thyroid hormone β receptors. Such compounds and their pharmaceutical compositions can be used in the manufacture of a medicament for preventing, treating or alleviating nonalcoholic fatty liver diseases, liver fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer in patients.

In one aspect, the present invention provides a compound having Formula (I) or a stereoisomer, a geometric isomer, a tautomer, an *N*-oxide, a solvate, a metabolite, a pharmaceutically acceptable salt or a prodrug thereof, 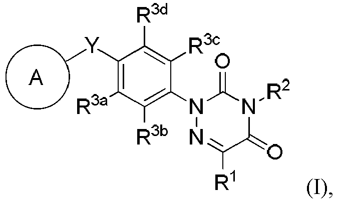 wherein,
Y is -O-, -S-, -NR⁰-, -C(=O)-, C₁₋₆ alkylene, C₂₋₆ alkenylene, C₂₋₆ alkynylene, -NR°C(=O)- or -C(=O)NR⁰-; wherein the Y is optionally substituted with 1, 2 or 3 R^{x};
R⁰ is H, deuterium, C₁₋₆ alkyl, C₁₋₆ haloalkyl, hydroxy C₁₋₆ alkyl, amino C₁₋₆ alkyl or cyano C₁₋₆ alkyl;
each of R^{3a}, R^{3b}, R^{3c} and R^{3d} is independently H, deuterium, F, Cl, Br, I, -CN, -NO₂, -COOH, -OH, -NH₂, -SH, C₁₋₆ alkyl, C₁₋₆ alkoxy, C₁₋₆ alkylthio, C₁₋₆ alkylamino, C₁₋₆ haloalkyl, C₁₋₆ haloalkoxy, hydroxy C₁₋₆ alkyl, amino C₁₋₆ alkyl or cyano C₁₋₆ alkyl;
R¹ is H, deuterium, F, Cl, Br, I, -CN, -NO₂, -COOH, -OH, -NH₂, -SH, C₁₋₆ alkyl, C₂₋₆ alkenyl, C₂₋₆ alkynyl, -C(=O)-C₁₋₆ alkoxy, -C(=O)-C₁₋₆ alkyl, -C(=O)-C₁₋₆ alkylamino, -C(=O)NH₂, -S(=O)₂-C₁₋₆ alkyl, -S(=O)₂-C₁₋₆ alkylamino, -S(=O)₂NH₂, C₁₋₆ alkylamino, C₁₋₆ alkoxy, C₁₋₆ haloalkyl, C₁₋₆ haloalkoxy, hydroxy C₁₋₆ alkyl, amino C₁₋₆ alkyl, carboxy C₁₋₆ alkyl or cyano C₁₋₆ alkyl;
R² is H, deuterium, C₁₋₆ alkyl, C₂₋₆ alkenyl, C₂₋₆ alkynyl, C₃₋₆ cycloalkyl, heterocyclyl consisting of 5-6 atoms, C₆₋₁₀ aryl or heteroaryl consisting of 5-6 atoms;
ring A is 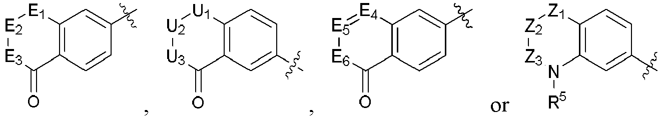 ; wherein, ring A is optionally substituted with 1, 2 or 3 R^{y};
each of E₁, U₁ and Z₁ is independently -(CR^{4a}R^{4b})_{q}-, -C(=O)-, -O-, -S-, -S(=O)-, -S(=O)₂- or -NR^{a}-;
each of E₂, U₂ and Z₂ is independently -CR^{4c}R^{4d}-, -C(=O)-, -O-, -S-, -S(=O)-, -S(=O)₂- or -NR^{b}-;
each of E₃, E₆, U₃ and Z₃ is independently -CR^{4e}R^{4f}-, -C(=O)-, -O-, -S-, -S(=O)-, -S(=O)₂- or -NR^{c}-;
E₄ is -CR^{4g}= or -N=; E₅ is -CR^{4h}= or -N=;
q is 0, 1, 2 or 3;
each R^{a}, R^{b}, R^{c} and R⁵ is independently H, deuterium, C₁₋₆ alkyl, C₂₋₆ alkenyl, C₂₋₆ alkynyl, C₁₋₆ haloalkyl, C₃₋₆ cycloalkyl, heterocyclyl consisting of 5-6 atoms, C₆₋₁₀ aryl or heteroaryl consisting of 5-6 atoms, wherein each R^{a}, R^{b}, R^{c} and R⁵ is independently and optionally substituted with 1, 2 or 3 R^{y1};
each R^{4a}, R^{4b}, R^{4c}, R^{4a}, R^{4e}, R^{4f}, R^{4g} and R^{4h} is independently H, deuterium, F, Cl, Br, I, -CN, -NO₂, -COOH, -OH, -NH₂, -SH, C₁₋₆ alkyl, C₂₋₆ alkenyl, C₂₋₆ alkynyl, C₁₋₆ alkoxy, C₁₋₆ alkylamino, C₁₋₆ haloalkyl, C₁₋₆ haloalkoxy, C₃₋₆ cycloalkyl, C₃₋₆ cycloalkyl-C₁₋₄ alkylene, heterocyclyl consisting of 5-6 atoms, (heterocyclyl consisting of 5-6 atoms)-C₁₋₄ alkylene, C₆₋₁₀ aryl, C₆₋₁₀ aryl-C₁₋₄ alkylene, heteroaryl consisting of 5-6 atoms or (heteroaryl consisting of 5-6 atoms)-C₁₋₄ alkylene, wherein each R^{4a}, R^{4b}, R^{4c}, R^{4a}, R^{4e}, R^{4f}, R^{4g} and R^{4h} is independently and optionally substituted with 1, 2 or 3 R^{y2};
or R^{4a} and R^{4b}, together with the carbon atoms to which they are attached, form a C₃₋₈ carbon ring or a heterocyclyl consisting of 5-6 atoms, or R^{4c} and R^{4d}, together with the carbon atoms to which they are attached, form a C₃₋₈ carbon ring or a heterocyclyl consisting of 5-6 atoms, or R^{4e} and R^{4f}, together with the carbon atoms to which they are attached, form a C₃₋₈ carbon ring or a heterocyclyl consisting of 5-6 atoms, wherein each C₃₋₈ carbon ring and heterocyclyl consisting of 5-6 atoms is independently unsubstituted or substituted with 1, 2 or 3 R^{y3};
each R^{x} is independently deuterium, F, Cl, Br, I, -CN, -OH, -NH₂, C₁₋₆ alkyl, C₁₋₆ haloalkyl, C₁₋₆ haloalkoxy, C₁₋₆ alkoxy or C₁₋₆ alkylamino;
each R^{y} is independently deuterium, F, Cl, Br, I, -CN, -OH, -NH₂, C₁₋₆ alkyl, C₁₋₆ haloalkyl, C₁₋₆ haloalkoxy, C₁₋₆ alkoxy or C₁₋₆ alkylamino; or two R^{y} linked on adjacent atoms, together with the atoms to which they are attached, form a C₃₋₈ carbon ring or a heterocyclyl consisting of 5-6 atoms, wherein each C₃₋₈ carbon ring and heterocyclyl consisting of 5-6 atoms is independently unsubstituted or substituted with 1, 2 or 3 R^{y4};
each R^{y1} is independently deuterium, F, Cl, Br, I, -CN, -OH, -NH₂, -SH, oxo, -OC(=O)-C₁₋₆ alkyl, -C(=O)-C₁₋₆ alkoxy, -C(=O)-C₁₋₆ alkyl, -C(=O)-C₁₋₆ alkylamino, -C(=O)NH₂, -S(=O)₂-C₁₋₆ alkyl, -S(=O)₂-C₁₋₆ alkylamino, -S(=O)₂NH₂, C₁₋₆ alkyl, C₁₋₆ haloalkyl, C₁₋₆ haloalkoxy, C₁₋₆ alkoxy, C₁₋₆ alkylthio, C₁₋₆ alkylamino, C₃₋₆ cycloalkyl, heterocyclyl consisting of 5-6 atoms, C₆₋₁₀ aryl or heteroaryl consisting of 5-6 atoms, wherein each R^{y1} is optionally substituted with 1, 2 or 3 R^{z};
each R^{z}, R^{y2}, R^{y3} and R^{y4} is independently deuterium, F, Cl, Br, I, -CN, -OH, -NH₂, -COOH, C₁₋₆ alkyl, C₁₋₆ haloalkyl, C₁₋₆ haloalkoxy, C₁₋₆ alkoxy, C₁₋₆ alkylthio or C₁₋₆ alkylamino.

In some embodiments, each of R^{3a}, R^{3b}, R^{3c} and R^{3d} is independently H, deuterium, F, Cl, Br, I, -CN, -NO₂, -COOH, -OH, -NH₂, -SH, methyl, ethyl, n-propyl, isopropyl, methoxy, ethoxy, methylthio, methylamino, -CF₃, -CHF₂, -CH₂F, -CH₂CF₃, -CH₂CHF₂, -OCF₃, -OCHF₂, -OCH₂F, hydroxymethyl, aminomethyl or cyanomethyl.

In some embodiments, R¹ is H, deuterium, F, Cl, Br, I, -CN, -NO₂, -COOH, -OH, -NH₂, -SH, methyl, ethyl, n-propyl, isopropyl, -CH=CH₂, -CH₂CH=CH₂, -CH=CHCH₃, -C≡CH, -C(=O)-OCH₃, -C(=O)-OCH₂CH₃, -C(=O)-OCH(CH₃)₂, -C(=O)-OCH₂CH₂CH₃, -C(=O)-O(CH₂)₃CH₃, -C(=O)-OCH₂CH(CH₃)₂, -C(=O)-CH₃, -C(=O)-CH₂CH₃, -C(=O)-NHCH₃, -C(=O)-N(CH₃)₂, -C(=O)NH₂, -S(=O)₂-CH₃, -S(=O)₂-CH₂CH₃, -S(=O)₂-NHCH₃, -S(=O)₂NH₂, methylamino, ethylamino, methoxy, ethoxy, -CF₃, -CHF₂, -CH₂F, -CH₂CF₃, -CH₂CHF₂, -OCF₃, -OCHF₂, -OCH₂F, hydroxymethyl, aminomethyl, carboxymethyl or cyanomethyl.

In some embodiments, each R^{a}, R^{b}, R^{c} and R⁵ is independently H, deuterium, C₁₋₄ alkyl, C₂₋₄ alkenyl, C₂₋₄ alkynyl, C₁₋₄ haloalkyl, C₃₋₆ cycloalkyl, heterocyclyl consisting of 5-6 atoms, C₆₋₁₀ aryl or heteroaryl consisting of 5-6 atoms, wherein each R^{a}, R^{b}, R^{c} and R⁵ is optionally substituted with 1, 2 or 3 R^{y1}; wherein R^{y1} has the meaning described in the present invention.

In some embodiments, each R^{a}, R^{b}, R^{c} and R⁵ is independently H, deuterium, methyl, ethyl, *n*-propyl, isopropyl, *n*-butyl, *tert*-butyl, -CH=CH₂, -CH₂CH=CH₂, -CH=CHCH₃, -C≡CH, -CF₃, -CHF₂, -CH₂F, -CH₂CF₃, -CH₂CHF₂, cyclopropyl, cyclobutyl, cyclopentyl, cyclohexyl, pyrrolidinyl, pyrazolidinyl, tetrahydrofuranyl, tetrahydrothiophenyl, tetrahydropyranyl, piperidinyl, morpholinyl, thiomorpholinyl, piperazinyl, phenyl, furanyl, thienyl, imidazolyl, pyrimidinyl, pyridyl, pyrrolyl, pyrazinyl, thiazolyl or oxazolyl, wherein each R^{a}, R^{b}, R^{c} and R⁵ is independently and optionally substituted with 1, 2 or 3 R^{y1}; wherein R^{y1} has the meaning described in the present invention.

In some embodiments, each R^{y1} is independently deuterium, F, Cl, Br, I, -CN, -OH, -NH₂, -SH, oxo, -OC(=O)-C₁₋₄ alkyl, -C(=O)-C₁₋₄ alkoxy, -C(=O)-C₁₋₄ alkyl, -C(=O)-C₁₋₄ alkylamino, -C(=O)NH₂, -S(=O)₂-C₁₋₄ alkyl, -S(=O)₂-C₁₋₄ alkylamino, -S(=O)₂NH₂, C₁₋₄ alkyl, C₁₋₄ haloalkyl, C₁₋₄ haloalkoxy, C₁₋₄ alkoxy, C₁₋₄ alkylthio, C₁₋₄ alkylamino, C₃₋₆ cycloalkyl, heterocyclyl consisting of 5-6 atoms, C₆₋₁₀ aryl or heteroaryl consisting of 5-6 atoms, wherein each R^{y1} is independently and optionally substituted with 1, 2 or 3 R^{z}; wherein R^{z} has the meaning described in the present invention.

In some embodiments, each R^{y1} is independently deuterium, F, Cl, Br, I, -CN, -OH, -NH₂, -SH, oxo, -OC(=O)-methyl, -OC(=O)-ethyl, -OC(=O)-*n*-propyl, -OC(=O)-isopropyl, -OC(=O)-*n*-butyl, -OC(=O)-*tert*-butyl, -OC(=O)-isobutyl, -C(=O)O-methyl, -C(=O)O-ethyl, -C(=O)O-*n*-propyl, -C(=O)O-isopropyl, -C(=O)O-*n*-butyl, -C(=O)O-*tert*-butyl, -C(=O)O-isobutyl, -C(=O)-methyl, -C(=O)-ethyl, -C(=O)-*n*-propyl, -C(=O)-isopropyl, -C (=O)-*n*-butyl, -C(=O)-*tert*-butyl, -C(=O)-isobutyl, -C(=O)-methylamino, -C(=O)-ethylamino, -C(=O)NH₂, -S(=O)₂-C₁₋₃ alkyl, -S(=O)₂-C₁₋₃ alkylamino, -S(=O)₂NH₂, methyl, ethyl, *n*-propyl, isopropyl, *tert*-butyl, -CF₃, -CHF₂, -CH₂F, -CH₂CF₃, -CH₂CHF₂, -OCF₃, -OCHF₂, -OCH₂F, -OCH₂CF₃, -OCH₂CHF₂, -OCHFCH₃, methoxy, ethoxy, *n*-propoxy, isopropoxy, methylthio, ethylthio, methylamino, ethylamino, cyclopropyl, cyclobutyl, cyclopentyl, cyclohexyl, pyrrolidinyl, pyrazolidinyl, tetrahydrofuryl, tetrahydrothiophenyl, tetrahydropyranyl, piperidinyl, morpholinyl, thiomorpholinyl, piperazinyl, phenyl, furyl, thienyl, imidazolyl, pyrimidinyl, pyridinyl, pyrrolyl, pyridazinyl, pyrazinyl, thiazolyl or oxazolyl, wherein each R^{y1} is independently and optionally substituted with 1, 2 or 3 R^{z}; wherein R^{z} has the meaning described in the present invention.

In some embodiments, each R^{4a}, R^{4b}, R^{4c}, R^{4a}, R^{4e}, R^{4f}, R^{4g} and R^{4h} is independently H, deuterium, F, Cl, Br, I, -CN, -NO₂, -COOH, -OH, -NH₂, -SH, methyl, ethyl, *n*-propyl, isopropyl, *n*-butyl, *tert*-butyl, -CH=CH₂, -CH₂CH=CH₂, -CH=CHCH₃, -C≡CH, methoxy, ethoxy, methylamino, ethylamino, -CF₃, -CHF₂, -CH₂F, -CH₂CF₃, -CH₂CHF₂, -OCF₃, -OCHF₂, -OCH₂F, cyclopropyl, cyclobutyl, cyclopentyl, cyclohexyl, cyclopropyl-CH₂-, cyclobutyl-CH₂-, cyclopentyl-CH₂-, cyclohexyl-CH₂-, pyrrolidinyl, pyrazolidinyl, tetrahydrofuranyl, tetrahydrothienyl, piperidinyl, morpholinyl, thiomorpholinyl, piperazinyl, pyrrolidinyl-CH₂-, pyrazolidinyl-CH₂-, tetrahydrofuranyl-CH₂-, tetrahydrothiophenyl-CH₂-, piperidinyl-CH₂-, morpholinyl-CH₂-, thiomorpholinyl-CH₂-, piperazinyl-CH₂-, phenyl, phenyl-CH₂-, phenyl-CH₂CH₂-, furanyl, thienyl, imidazolyl, pyrimidinyl, pyridyl, pyrrolyl, pyridazinyl, pyrazinyl, thiazolyl, oxazolyl, furyl-CH₂-, thienyl-CH₂-, imidazolyl-CH₂-, pyrimidinyl-CH₂-, pyridyl-CH₂- or pyrrolyl-CH₂-, wherein each R^{4a}, R^{4b}, R^{4c}, R^{4a}, R^{4e}, R^{4f}, R^{4g} and R^{4h} is independently and optionally substituted with 1, 2 or 3 R^{y2};

or R^{4a} and R^{4b}, together with the carbon atoms to which they are attached, form a C₃₋₆ carbon ring or a heterocyclyl consisting of 5-6 atoms, or R^{4c} and R^{4d}, together with the carbon atoms to which they are attached, form a C₃₋₆ carbon ring or a heterocyclyl consisting of 5-6 atoms, or R^{4e} and R^{4f}, together with the carbon atoms to which they are attached, form a C₃₋₆ carbon ring or a heterocyclyl consisting of 5-6 atoms, wherein each C₃₋₆ carbon ring and heterocyclyl consisting of 5-6 atoms is independently unsubstituted or substituted with 1, 2 or 3 R^{y3}; wherein R^{y2} and R^{y3} have the meanings described in the present invention.

In some embodiments, each R^{z}, R^{y2}, R^{y3} and R^{y4} is independently deuterium, F, Cl, Br, I, -CN, -OH, -NH₂, -COOH, methyl, ethyl, n-propyl, isopropyl, -CF₃, -CHF₂, -CH₂F, -OCF₃, -OCHF₂, -OCH₂F, methoxy, ethoxy or methylamino.

In another aspect, the present invention relates to a pharmaceutical composition comprising the compound of the present invention, optionally, further comprising any one of pharmaceutically acceptable carriers, excipients, adjuvants, and vehicles or any combination thereof.

In one aspect, the present invention relates to use of the compound of the present invention or the pharmaceutical composition of the present invention in the manufacture of a medicament for stimulating thyroid hormone receptors; or for preventing, treating or alleviating diseases regulated by thyroid hormone receptors.

In one aspect, the present invention relates to a method of stimulating thyroid hormone receptors, or preventing, treating or alleviating diseases regulated by thyroid hormone receptors in a subject comprising administering to the subject a therapeutically effective amount of the compound of the present invention or the pharmaceutical composition of the present invention.

In one aspect, the present invention relates to the compound of the present invention or the pharmaceutical composition of the present invention for use in stimulating thyroid hormone receptors; or in preventing, treating or alleviating diseases regulated by thyroid hormone receptors in a subject.

In some embodiments, the thyroid hormone receptor of the present invention is a thyroid hormone β receptor.

In some embodiments, the diseases regulated by thyroid hormone receptors in the present invention are neurodegenerative diseases, nonalcoholic fatty liver diseases, idiopathic pulmonary fibrosis (IPF), atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer.

In another aspect, the present invention relates to use of the compound of the present invention or the pharmaceutical composition of the present invention in the manufacture of a medicament for preventing, treating or alleviating the following diseases: neurodegenerative diseases, nonalcoholic fatty liver diseases, liver fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer.

In another aspect, the present invention relates to a method of preventing, treating or alleviating the following diseases: neurodegenerative diseases, nonalcoholic fatty liver diseases, liver fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer in a subject comprising administering to the subject a therapeutically effective amount of the compound of the present invention or the pharmaceutical composition of the present invention.

In another aspect, the present invention relates to the compound of the present invention or the pharmaceutical composition of the present invention for use in preventing, treating or alleviating the following diseases: neurodegenerative diseases, nonalcoholic fatty liver diseases, liver fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer.

In some embodiments, the nonalcoholic fatty liver disease described in the present invention is nonalcoholic simple fatty liver, nonalcoholic steatohepatitis, cryptogenic cirrhosis associated with nonalcoholic fatty liver disease or primary liver cancer.

In some embodiments, the neurodegenerative disease described in the present invention is demyelinating disease, chronic demyelinating disease, leukodystrophy, dementia, ischemic stroke, lacunar stroke, multiple sclerosis, MCT8 deficiency, X-linked adrenal dystrophy (ALD), amyotrophic lateral sclerosis (ALS) or Alzheimer's disease.

The foregoing merely summarizes certain aspects disclosed herein, but is not limited to these aspects. These and other aspects are described more fully below.

### EXAMPLES

The invention provides a class of compounds with good agonistic activity on thyroid hormone beta receptors, its preparation method, its pharmaceutical composition and its application. Skilled in the art can learn from this article to properly improve the process parameters to implement the preparation method. Of particular note is that all similar substitutions and modifications to the skilled person is obvious, and they are deemed to be included in the present invention.

### DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the following definitions shall apply unless otherwise indicated. For purposes of this invention, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are hereby incorporated by reference.

The grammatical articles "a", "an" and "the", as used herein, are intended to include "at least one" or "one or more" unless otherwise indicated herein or clearly contradicted by the context. Thus, the articles are used herein to refer to one or more than one (*i.e.* at least one) of the grammatical objects of the article. By way of example, "a component" means one or more components, and thus, possibly, more than one component is contemplated and may be employed or used in an implementation of the described embodiments.

Unless otherwise stated, terms used in the present invention in the specification and claims have the following definitions.

The term "comprise" is an open expression, it means comprising the contents disclosed herein, but don't exclude other contents.

As described herein, compounds disclosed herein may optionally be substituted with one or more substituents, such as are illustrated generally above, or as exemplified by particular classes, subclasses, and species of the invention. It will be appreciated that the phrase "optionally substituted" is used interchangeably with the phrase "substituted or unsubstituted". The term "optionally" means that the subsequently described event or circumstance can but need not occur, and the description includes instances where the event or circumstance occurs and instances where the event or circumstance does not occur. In general, unless otherwise indicated, an optionally substituted group may have a substituent at each substitutable position of the group. When more than one position in a given structure can be substituted with more than one substituent selected from a specified group, the substituent may be either the same or different at each position. The substituents described therein can be, but are not limited to, H, deuterium, F, Cl, Br, I, -CN, -NO₂, -COOH, -OH, -NH₂, -SH, alkyl, alkoxy, alkylthio, alkylamino, haloalkyl, haloalkoxy, hydroxyalkyl, aminoalkyl, cyanoalkyl, carboxyalkyl, alkenyl, alkynyl, cycloalkyl, cycloalkyl-alkylene, heterocyclyl-alkylene, carbocyclyl, heterocyclyl, aryl, aryl-alkylene, heteroaryl, heteroaryl-alkylene, and the like.

Furthermore, what need to be explained is that the phrase "each... is independently" and "each of... and... is independently", unless otherwise stated, should be broadly understood. The specific options expressed by the same symbol are independent of each other in different groups; or the specific options expressed by the same symbol are independent of each other in same groups.

At various places in the present specification, substituents of compounds disclosed herein are disclosed in groups or in ranges. It is specifically intended that the invention include each and every individual subcombination of the members of such groups and ranges. For example, the term "C₁₋₆ alkyl" specifically refers to independently disclosed C₁ alkyl (methyl), C₂ alkyl (ethyl), C₃ alkyl, C₄ alkyl, C₅ alkyl and C₆ alkyl; "C₃₋₈ cycloalkyl" especially refers to independently disclosed C₃ cycloalkyl, C₄ cycloalkyl, C₅ cycloalkyl, C₆ cycloalkyl, C₇ cycloalkyl and C₈ cycloalkyl; "heterocyclyl consisting of 3-6 atoms" refers to heterocyclyl consisting of 3 atoms, heterocyclyl consisting of 4 atoms, heterocyclyl consisting of 5 atoms and heterocyclyl consisting of 6 atoms.

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl", it is understood that the "alkyl" or "aryl" represents a linking alkylene group or arylene group, respectively.

The term "alkylene" refers to a saturated divalent hydrocarbon group derived from a straight or branched chain saturated hydrocarbon by the removal of two hydrogen atoms. Unless otherwise specified, the alkylene group contains 1-12 carbon atoms. In some embodiments, the alkylene group contains 1-6 carbon atoms, *i.e*., C₁₋₆ alkylene; in some embodiments, the alkylene group contains 1-4 carbon atoms, *i.e*., C₁₋₄ alkylene; such examples include, but are not limited to, methylene (-CH₂-), ethylene (including -CH₂CH₂- or -CH(CH₃)-), isopropylidene (including -CH(CH₃)CH₂- or -C(CH₃)₂-), *n*-propylene (including -CH₂CH₂CH₂-, -CH(CH₂CH₃)- or -CH₂CH(CH₃)-), and the like. Wherein, the alkylene group may be optionally substituted with one or more substituents disclosed herein.

The term "alkyl" or "alkyl group" refers to a saturated linear or branched-chain monovalent hydrocarbon group of 1-20 carbon atoms, wherein the alkyl group is optionally substituted with one or more substituents described herein. In some embodiments, the alkyl group contains 1-10 carbon atoms; in other embodiments, the alkyl group contains 1-8 carbon atoms, *i.e.*, C₁₋₈ alkyl; in still other embodiments, the alkyl group contains 1-6 carbon atoms, *i.e.*, C₁₋₆ alkyl; in yet other embodiments, the alkyl group contains 1-4 carbon atoms, *i.e.*, C₁₋₄ alkyl.

Examples of alkyl group include, but are not limited to, methyl (Me, -CH₃), ethyl (Et, -CH₂CH₃), *n*-propyl (*n*-Pr, -CH₂CH₂CH₃), isopropyl (*i*-Pr, -CH(CH₃)₂), *n*-butyl (*n*-Bu, -CH₂CH₂CH₂CH₃), isobutyl (*i*-Bu, -CH₂CH(CH₃)₂), *sec*-butyl (*s*-Bu, -CH(CH₃)CH₂CH₃), *tert*-butyl (*t*-Bu, -C(CH₃)₃), *n*-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH (CH₃)₂), 3-methyl-1-butyl (-CH₂CH₂CH(CH₃)₂), 2-methyl-1-butyl (-CH₂CH(CH₃)CH₂CH₃), *n*-heptyl, *n*-octyl, *etc.*

The term "alkenyl" refers to linear or branched-chain monovalent hydrocarbon radical of 2 to 12 carbon atoms, wherein at least one unsaturated site is a carbon-carbon sp² double bond, wherein the alkenyl radical may be optionally substituted with one or more substituents described herein, including radicals having "*cis*" and "*trans*" orientations, or alternatively, "*E*" and "*Z*" orientations. In some embodiments, the alkenyl contains 2-8 carbon atoms; in other embodiments, the alkenyl contains 2-6 carbon atoms, *i.e.*, C₂₋₆ alkenyl; in still other embodiments, the alkenyl contains 2-4 carbon atoms, *i.e.*, C₂₋₄ alkenyl.

Examples of alkenyl group include, but are not limited to, vinyl (-CH=CH₂), allyl (-CH₂CH=CH₂), propenyl (-CH=CHCH₃), butenyl (-CH=CHCH₂CH₃, -CH₂CH=CHCH₃, -CH₂CH₂CH=CH₂, -CH=C(CH₃)₂, -CH=C(CH₃)₂, -CH₂C(CH₃)=CH₂), pentenyl (-CH₂CH₂CH₂CH=CH₂, -CH₂CH₂CH=CHCH₃, -CH₂CH₂CH=CHCH₃, -CH₂CH=CHCH₂CH₃, -CH=CHCH₂CH₂CH₃, -CH₂CH₂C(CH₃)=CH₂, -CH₂CH=C(CH₃)₂, -CH=CHCH(CH₃)₂, -C(CH₂CH₃)=CHCH₃, -CH(CH₂CH₃)CH=CH₂), and so on.

The term "alkynyl" refers to a linear or branched monovalent hydrocarbon radical of 2 to 12 carbon atoms, wherein at least one unsaturated site is a carbon-carbon sp triple bond, wherein the alkynyl radical may be optionally substituted with one or more substituents described herein. In some embodiments, the alkynyl contains 2-8 carbon atoms; in other embodiments, the alkynyl contains 2-6 carbon atoms, *i.e.*, C₂₋₆ alkynyl; in still other embodiments, the alkynyl contains 2-4 carbon atoms, *i.e.*, C₂₋₄ alkynyl. Some non-limiting examples of the alkynyl group include ethynyl (-C≡CH), 1-propynyl (-C≡CH-CH₃), propargyl (-CH₂C≡CH), 1-butynyl, 2-butynyl, 1-pentynyl, 2-pentynyl, 3-methyl-1-butynyl, 1-hexynyl, 1-heptynyl and 1-octynyl, *etc.*

The term "alkoxy" refers to an alkyl group, attached to the parent molecular moiety via an oxygen atom, *i.e.*, -O-alkyl, wherein the alkyl group has the meaning as described in the present invention, wherein the alkoxy group can be optionally substituted by one or more substituents described in the present invention. In some embodiments, the alkoxy group contains 1-20 carbon atoms; in some embodiments, the alkoxy group contains 1-10 carbon atoms; in some embodiments, the alkoxy group contains 1-8 carbon atoms; in some embodiments, the alkoxy group contains 1-6 carbon atoms, *i.e.*, C₁₋₆ alkoxy; in some embodiments, the alkoxy group contains 1-4 carbon atoms, *i.e.*, C₁₋₄ alkoxy.

Examples of alkoxy group include, but are not limited to, methoxy (MeO, -OCH₃), ethoxy (EtO, -OCH₂CH₃), *n*-propyloxy (*n*-PrO, *n*-propoxy, -OCH₂CH₂CH₃), isopropyloxy (*i*-PrO, *i*-propoxy, -OCH(CH₃)₂), 1-butoxy (*n*-BuO, *n*-butoxy, -OCH₂CH₂CH₂CH₃), 2-methyl-1-propoxy (*i*-BuO, *i*-butoxy, -OCH₂CH(CH₃)₂), 2-butoxy (*s*-BuO, *s*-butoxy, -OCH(CH₃)CH₂CH₃), 2-methyl-isopropyloxy (*t*-BuO, *t*-butoxy, -OC(CH₃)₃), *etc.*

The term "alkylamino" includes "*N*-alkylamino" and "*N,N*-dialkylamino", which means that the amino group is independently substituted with one or two alkyl radicals and the alkyl group is as defined herein. Wherein, the alkylamino group may be optionally substituted with one or more substituents disclosed herein. In some embidiments, the alkylamino group is an alkylamino radical having one or two C₁₋₆ alkyl groups attached to a nitrogen atom. In other embodiments, the alkylamino group is an alkylamino radical having one or two C₁₋₄ alkyl groups attached to a nitrogen atom, *i.e.*, C₁₋₄ alkylamino. Some non-limiting examples of the alkylamino group include methylamino (*N*-methylamino), ethylamino (*N*-ethylamino), dimethylamino (*N,N-*dimethylamino), diethylamino (*N,N*-diethylamino), *n*-propylamino (*N*-*n*-propylamino), isopropylamino (*N*-isopropylamino), *etc.*

The term "alkylthio" refers to an alkyl group, attached to the parent molecular moiety via a sulfur atom, *i.e.*, -S-alkyl, wherein the alkyl group has the meaning as described in the present invention, wherein the alkylthio group can be optionally substituted by one or more substituents described in the present invention. In some embodiments, the alkylthio group contains 1-10 carbon atoms; in some embodiments, the alkylthio group contains 1-8 carbon atoms; in some embodiments, the alkylthio group contains 1-6 carbon atoms, *i.e.*, C₁₋₆ alkylthio; in some embodiments, the alkylthio group contains 1-4 carbon atoms, *i.e.*, C₁₋₄ alkylthio; in some embodiments, the alkylthio group contains 1-3 carbon atoms, *i.e.*, C₁₋₃ alkylthio. Examples of alkylthio group include, but are not limited to, methylthio, ethylthio, and the like.

The term "haloalkyl" refers to an alkyl group having one or more halogen substituents, wherein the haloalkyl group may be optionally substituted with one or more substituents described herein. In some embodiments, the haloalkyl group contains 1-10 carbon atoms; in some embodiments, the haloalkyl group contains 1-8 carbon atoms; in some embodiments, the haloalkyl group contains 1-6 carbon atoms, *i.e.*, C₁₋₆ haloalkyl; in some embodiments, the haloalkyl group contains 1-4 carbon atoms, *i.e.*, C₁₋₄ haloalkyl; in some embodiments, the haloalkyl group contains 1-3 carbon atoms, *i.e.*, C₁₋₃ haloalkyl. Examples of haloalkyl include, but are not limited to, fluoromethyl (-CH₂F), difluoromethyl (-CHF₂), trifluoromethyl (-CF₃), fluoroethyl (-CHFCH₃, -CH₂CH₂F), difluoromethyl (-CF₂CH₃, -CFHCFH₂, -CH₂CHF₂), perfluoroethyl, fluoropropyl (-CHFCH₂CH₃, -CH₂CHFCH₃, -CH₂CH₂CH₂F), *etc.*

The term "haloalkoxy" refers to an alkoxy group substituted with one or more halogen substituents, wherein the haloalkoxy group may optionally be substituted with one or more substituents described herein. In some embodiments, the haloalkoxy group contains 1-10 carbon atoms; in some embodiments, the haloalkoxy group contains 1-8 carbon atoms; in some embodiments, the haloalkoxy group contains 1-6 carbon atoms, *i.e.*, C₁₋₆ haloalkoxy; in some embodiments, the haloalkoxy group contains 1-4 carbon atoms, *i.e.*, C₁₋₄ haloalkoxy; in some embodiments, the haloalkoxy group contains 1-3 carbon atoms, *i.e.*, C₁₋₃ haloalkoxy. Examples of haloalkoxy include, but are not limited to, -OCF₃, -OCHF₂, -OCH₂F, and the like.

The term "hydroxyalkyl" refers to an alkyl group substituted with one or more hydroxy groups (-OH), and the alkyl group has the meaning described in the present invention, wherein the hydroxyalkyl group may be optionally substituted with one or more substituents described herein. In some embodiments, the hydroxyalkyl group described in the present invention refers to a C₁₋₆ alkyl group substituted with one or more hydroxy groups (-OH), *i.e.*, hydroxy C₁₋₆ alkyl; in some embodiments, the hydroxyalkyl group refers to a C₁₋₄ alkyl group substituted with one or more hydroxy groups (-OH), *i.e.*, hydroxy C₁₋₄ alkyl. Examples of hydroxyalkyl group include, but are not limited to, hydroxymethyl (*e.g*., -CH₂OH), hydroxyethyl (*e.g*., 2-hydroxyethyl), and the like.

The term "aminoalkyl" refers to an alkyl group substituted with one or more amino groups (-NH₂), and the alkyl group has the meaning described in the present invention, wherein the aminoalkyl group may be optionally substituted with one or more substituents described herein. In some embodiments, the aminoalkyl group described in the present invention refers to a C₁₋₆ alkyl group substituted with one or more amino groups (-NH₂), *i.e.*, amino C₁₋₆ alkyl; in some embodiments, the aminoalkyl group refers to a C₁₋₄ alkyl group substituted with one or more amino groups (-NH₂), *i.e.*, amino C₁₋₄ alkyl. Examples of aminoalkyl group include, but are not limited to, aminomethyl (-CH₂NH₂), diaminomethyl (-CH(NH₂)₂), aminoethyl (*e.g*., 2-aminoethyl), and the like.

The term "cyanoalkyl" refers to an alkyl group substituted with one or more cyano groups (-CN), and the alkyl group has the meaning described in the present invention, wherein the cyanoalkyl group may be optionally substituted with one or more substituents described herein. In some embodiments, the cyanoalkyl group described in the present invention refers to a C₁₋₆ alkyl group substituted with one or more cyano groups (-CN), *i.e.*, cyano C₁₋₆ alkyl; in some embodiments, the cyanoalkyl group refers to a C₁₋₄ alkyl group substituted with one or more cyano groups (-CN), *i.e.*, cyano C₁₋₄ alkyl. Examples of cyanoalkyl group include, but are not limited to, cyanomethyl (*e.g.*, -CH₂CN), cyanoethyl (*e.g*., 2-cyanoethyl), and the like.

The term "carboxyalkyl" refers to an alkyl group substituted with one or more carboxyl groups (-COOH), and the alkyl group has the meaning described in the present invention, wherein the carboxyalkyl group may be optionally substituted with one or more substituents described herein. In some embodiments, the carboxyalkyl group described in the present invention refers to a C₁₋₆ alkyl group substituted with one or more carboxyl groups (-COOH), *i.e.*, carboxy C₁₋₆ alkyl; in some embodiments, the carboxyalkyl group refers to a C₁₋₄ alkyl group substituted with one or more carboxyl groups (-COOH), *i.e.*, carboxy C₁₋₄ alkyl. Examples of carboxyalkyl group include, but are not limited to, carboxymethyl, carboxyethyl (*e.g*., 2-carboxyethyl), and the like.

The term "cycloalkyl" or "carbocyclyl" refers to a monocyclic, bicyclic or tricyclic ring systems having from 3 to 14 ring carbon atoms, saturated or partially unsaturated, having one or more points of attachment to the rest of the molecule. Wherein the cycloalkyl group is optionally substituted with substituents described herein. In some embodiments, the cycloalkyl is a ring system containing 3-10 ring carbon atoms, *i.e.*, C₃₋₁₀ cycloalkyl; in still other embodiments, the cycloalkyl is a ring system containing 3-8 ring carbon atoms, *i.e.*, C₃₋₈ cycloalkyl; in yet other embodiments, the cycloalkyl is a ring system containing 3-6 ring carbon atoms, *i.e.*, C₃₋₆ cycloalkyl. Examples of cycloalkyl group include, but are not limited to, cyclopropyl, cyclobutyl, cyclopentyl, cyclohexyl, cyclopentadienyl and the like.

The term "heterocyclyl" refers to a saturated or partially unsaturated, non-aromatic, monocyclic, bicyclic or tricyclic ring system containing 3-12 ring atoms of which at least one ring member is selected from nitrogen, sulfur, oxygen and phosphorus. Wherein, the heterocyclyl is non-aromatic and does not contain any aromatic ring, and the ring system has one or more connection points connected to the rest of the molecule. Wherein, the heterocyclyl may be optionally substituted with one or more substituents disclosed herein. The term "heterocyclyl" includes monocyclic, bicyclic or polycyclic fused, spiro or bridged heterocyclic ring systems. Bicyclic heterocyclyl includes bridged bicyclic heterocyclyl, fused bicyclic heterocyclyl and spirobicyclic heterocyclyl. The terms "heterocyclyl" and "heterocycle" are used interchangeably herein. Unless otherwise specified, the heterocyclyl group may be carbon or nitrogen linked, and a -CH₂- group can be optionally substituted with -C(=O)-. In which, the sulfur can be optionally oxygenized to *S*-oxide, the nitrogen can be optionally oxygenized to *N*-oxide, and the phosphorus can be optionally oxygenized to *P*-oxide. In some embodiments, the heterocyclyl is a ring system consisting of 3-10 atoms; in some embodiments, the heterocyclyl is a ring system consisting of 5-10 atoms; in some embodiments, the heterocyclyl is a ring system consisting of 5-8 atoms; in some embodiments, the heterocyclyl is a ring system consisting of 6-8 atoms; in some embodiments, the heterocyclyl is a ring system consisting of 5-6 atoms, *i.e.*, a heterocyclyl consisting of 5-6 atoms; in some embodiments, the heterocyclyl is a ring system consisting of 3-6 atoms, *i.e.*, a heterocyclyl consisting of 3-6 atoms; in some embodiments, the heterocyclyl is a ring system consisting of 3 ring atoms; in some embodiments, the heterocyclyl is a ring system consisting of 4 atoms; in other embodiments, the heterocyclyl is a ring system consisting of 5 atoms; in other embodiments, the heterocyclyl is a ring system consisting of 6 atoms.

Some non-limiting examples of the heterocyclyl group include oxiranyl, azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothienyl, dihydrothienyl, 1,3-dioxolanyl, dithiolanyl, tetrahydropyranyl, dihydropyranyl, 2*H*-pyranyl, 4*H*-pyranyl, piperidinyl, morpholinyl, thiomorpholinyl, piperazinyl, dioxanyl, thioxanyl, homopiperazinyl, homopiperidinyl, oxepanyl, thiepanyl, tetrahydropyrrolyl, dihydropyrrolyl, tetrahydropyridyl, tetrahydropyrimidinyl, tetrahydropyrazinyl, tetrahydropyridazinyl, indolinyl, 1,2,3,4-tetrahydroisoquinolinyl, and the like. Some non-limiting examples of heterocyclyl wherein -CH₂- group is replaced by -C(=O)- include 2-oxopyrrolidinyl, oxo-1,3-thiazolidinyl, 2-piperidinonyl, 3,5-dioxopiperidinyl, pyrimidinedione-yl, 3,4-dihydroisoquinolin-1(2*H*)-one. Some non-limited examples of heterocyclyl wherein the ring sulfur atom is oxidized is sulfolanyl and 1,1-dioxo-thiomorpholinyl. Bridged heterocyclyl groups include, but are not limited to, 2-oxabicyclo[2.2.2]octyl, 1-azabicyclo[2.2.2]octyl, 3-azabicyclo[3.2.1]octyl, and the like.

The term "consisting of m atoms" where m is an integer typically describes the number of ring-forming atoms in a moiety where the number of ring-forming atoms is m. For example, piperidinyl is a heterocyclyl group consisting of 6 atoms and furyl is a heteroaryl group consisting of 5 atoms. As another example, "heterocyclyl consisting of 3-6 atoms" refers to a heterocyclyl group consisting of 3, 4, 5 or 6 atoms.

The term "aryl" refers to monocyclic, bicyclic and tricyclic aromatic carbocyclic ring systems containing 6-14 ring atoms, or 6-10 ring atoms, wherein each ring contains 3-7 ring atoms and has a single point or multipoint of attachment to the rest of the molecule. Wherein the aryl may be optionally substituted with one or more substituents disclosed herein. The term "aryl" may be used interchangeably with the term "aromatic ring". Examples of aryl groups include, but are not limited to, phenyl, indenyl, naphthyl, and anthracenyl, *etc.*

The term "heteroaryl" refers to monocyclic, bicyclic and tricyclic aromatic systems containing 5-14 ring atoms, wherein at least one ring contains one or more heteroatoms, the entire ring system is aromatic, and the heteroaryl has a single point or multipoint of attachment to the rest of the molecule. Wherein the heteroaryl may be optionally substituted with one or more substituents disclosed herein. Unless otherwise stated, the heteroaryl group can be attached to the rest of the molecule (such as the main structure in the general formula) through any reasonable point (which can be C in CH, or N in NH). When a -CH2- group is present on a heteroaryl group, the -CH₂- group may optionally be replaced by a -C(=O)-. The term "hetreroaryl" and "heteroaromatic ring" or "heteroaromatic compound" can be used interchangeably herein. In some embodiments, heteroaryl is a heteroaryl consisting of 5-8 atoms comprising 1, 2, 3 or 4 heteroatoms independently selected from O, S, and N; in some embodiments, heteroaryl is a heteroaryl consisting of 5-7 atoms comprising 1, 2, 3 or 4 heteroatoms independently selected from O, S, and N; in some embodiments, heteroaryl is a heteroaryl consisting of 5-6 atoms comprising 1, 2, 3 or 4 heteroatoms independently selected from O, S, and N; in some embodiments, heteroaryl is a heteroaryl consisting of 5 atoms comprising 1, 2, 3 or 4 heteroatoms independently selected from O, S, and N; in some embodiments, heteroaryl is a heteroaryl consisting of 6 atoms comprising 1, 2, 3 or 4 heteroatoms independently selected from O, S, and N

Examples of heteroaryl groups include, but are not limited to, the following monocyclic groups: furyl (2-furyl, 3-furyl), imidazolyl (*N*-imidazolyl, 2-imidazolyl, 4-imidazolyl, 5-imidazolyl), isoxazolyl (3-isoxazolyl, 4-isoxazolyl, 5-isoxazolyl), oxazolyl (2-oxazolyl, 4-oxazolyl, 5-oxazolyl), pyrrolyl (*N*-pyrrolyl, 2-pyrrolyl, 3-pyrrolyl), pyridyl (2-pyridyl, 3-pyridyl, 4-pyridyl), pyrimidinyl (2-pyrimidinyl, 4-pyrimidinyl, 5-pyrimidinyl), pyridazinyl (such as 3-pyridazinyl), thiazolyl (2-thiazolyl, 4-thiazolyl, 5-thiazolyl), thienyl (2-thienyl, 3-thienyl), pyrazolyl (such as 2-pyrazolyl and 3-pyrazolyl), isothiazolyl, 1,2,3-oxadiazolyl, 1,2,5-oxadiazolyl, 1,2,4-oxadiazolyl, 1,3,4-oxadiazolyl, 1,2,3-thiodiazolyl, 1,3,4-thiodiazolyl, 1,2,5-thiodiazolyl, pyrazinyl, 1,3,5-triazinyl; and also include, but are by no means limited to, the following bicyclic or tricyclic groups: benzimidazolyl, benzofuryl, benzothienyl, indolyl (such as 2-indolyl), purinyl, quinolyl (such as 2-quinolyl, 3-quinolyl, 4-quinolyl), isoquinolyl (such as 1-isoquinolyl, 3-isoquinolyl or 4-isoquinolyl), dibenzoimidazolyl, dibenzofuryl, dibenzothienyl.

The term "cycloalkyl-alkylene" refers to a cycloalkyl group attached to the rest of the molecule through an alkylene group, wherein the cycloalkyl and alkylene are as defined herein. The "cycloalkyl-alkylene" group may be optionally substituted with one or more substituents disclosed herein. The "C₃₋₆ cycloalkyl-C₁₋₄ alkylene" mentioned in the present invention means that the C₃₋₆ cycloalkyl is linked to the rest of the molecule through a C₁₋₄ alkylene group. The "C₃₋₆ cycloalkyl-C₁₋₂ alkylene" mentioned in the present invention means that the C₃₋₆ cycloalkyl is linked to the rest of the molecule through a C₁₋₂ alkylene group. Such examples include, but are not limited to, cyclopropyl-CH₂-, cyclopropyl-CH₂CH₂-, cyclobutyl-CH₂-, cyclobutyl-CH₂CH₂-, cyclopentyl-CH₂-, cyclopentyl-CH₂CH₂ -, cyclohexyl-CH₂-, cyclohexyl-CH₂CH₂-, *etc.*

The term "heterocyclyl-alkylene" refers to a heterocyclyl group attached to the rest of the molecule through an alkylene group, wherein the heterocyclyl and alkylene are as defined herein. The "heterocyclyl-alkylene" group may be optionally substituted with one or more substituents disclosed herein. The "(heterocyclyl consisting of 5-6 atoms)-C₁₋₄ alkylene" mentioned in the present invention means that the heterocyclyl consisting of 5-6 atoms is linked to the rest of the molecule through a C₁₋₄ alkylene group. The "(heterocyclyl consisting of 5-6 atoms)-C₁₋₂ alkylene" mentioned in the present invention means that the heterocyclyl consisting of 5-6 atoms is linked to the rest of the molecule through a C₁₋₂ alkylene group. Such examples include, but are not limited to, tetrahydropyranyl-CH₂-, tetrahydropyranyl-CH₂CH₂-, tetrahydrofuranyl-CH₂-, tetrahydrofuranyl-CH₂CH₂-, pyrrolidinyl-CH₂-, piperidinyl -CH₂-, piperidinyl-CH₂CH₂-, morpholinyl-CH₂-, morpholinyl-CH₂CH₂-, and the like.

The term "aryl-alkylene" refers to an aryl group attached to the rest of the molecule through an alkylene group, wherein the aryl and alkylene are as defined herein. The "aryl-alkylene" group may be optionally substituted with one or more substituents disclosed herein. For example, the "C₆₋₁₀ aryl-C₁₋₄ alkylene" mentioned in the present invention means that the C₆₋₁₀ aryl is linked to the rest of the molecule through a C₁₋₄ alkylene group. The "C₆₋₁₀ aryl-C₁₋₂ alkylene" mentioned in the present invention means that the C₆₋₁₀ aryl is linked to the rest of the molecule through a C₁₋₂ alkylene group. Such examples include, but are not limited to, phenyl-CH₂-, phenyl-CH₂CH₂-, naphthyl-CH₂-, and the like.

The term "heteroaryl-alkylene" refers to a heteroaryl group attached to the rest of the molecule through an alkylene group, wherein the heteroaryl and alkylene are as defined herein. The heteroaryl-alkylene group may be optionally substituted with one or more substituents disclosed herein. The "(heteroaryl consisting of 5-6 atoms)-C₁₋₄ alkylene" mentioned in the present invention means that the heteroaryl consisting of 5-6 atoms is linked to the rest of the molecule through a C₁₋₄ alkylene group. The "(heteroaryl consisting of 5-6 atoms)-C₁₋₂ alkylene" mentioned in the present invention means that the heteroaryl consisting of 5-6 atoms is linked to the rest of the molecule through a C₁₋₂ alkylene group. Such examples include, but are not limited to, pyridyl-CH₂-, pyrrolyl-CH₂CH₂-, quinolinyl-CH₂-, thienyl-CH₂-, furyl-CH₂-, pyrimidyl-CH₂-, pyridyl-CH₂-, *etc.*

The term "heteroatom" refers to one or more of oxygen, sulfur, nitrogen, phosphorus and silicon, including any oxidized form of nitrogen, sulfur, or phosphorus; the quaternized form of any basic nitrogen; or a substitutable nitrogen of a heterocyclic ring, for example, N (as in 3,4-dihydro-2*H*-pyrrolyl), NH (as in pyrrolidinyl) or NR^{T} (as NR^{T} in N-substituted pyrrolidinyl, R^{T} is a substituent on N).

The term "carbonyl", whether used alone or with other terms, such as "aminocarbonyl" or "acyloxy", represents -(C=O)-.

The term "deuterium" means deuterated, *i.e.*, ²H.

The term "pharmaceutically acceptable" means that the substance or composition must be chemically and/or toxicologically compatible with the other ingredients comprising the formulation and/or the mammal being treated therewith. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" includes any solvent, dispersion medium, coating material, surfactant, antioxidant, preservative (such as antibacterial, antifungal), isotonic agent, salt, drug stabilizer, binder, excipient, dispersant, lubricant, sweetener, flavoring agent, colorant, or combination thereof, these carriers are known to those skilled in the art (such described in Remington's Pharmaceutical Sciences, 18th Ed. Mack Printing Company, 1990, pp. 1289-1329). Except where any conventional carrier is incompatible with the active ingredient, its use in therapeutic or pharmaceutical compositions is contemplated.

The term "pharmaceutical composition" refers to a mixture of one or more of the compounds described herein, or physiologically/pharmaceutically acceptable salts or prodrugs thereof, and other chemical components, such as physiologically/pharmaceutically acceptable carriers, excipients, diluents, binders, fillers and other auxiliary materials, and other additional therapeutic agents, such as anti-diabetic agents, antihyperglycemic agents, antiadipositas agents, antihypertensive agents, antiplatelet agents, antiatherosclerotic agents, lipid-lowering agents, *etc.* The purpose of the pharmaceutical composition is to facilitate administration of a compound to an organism.

The term "prodrug" refers to a compound that is transformed *in vivo* into a compound of Formula (I). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic (C₁₋₂₄) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in Higuchi et al., Pro-drugs as Novel Delivery Systems, Vol. 14, A.C.S. Symposium Series; Roche, et al. ed., Bioreversible Carriers in Drug Design, American Pharmaceutical Association and Pergamon Press, 1987; Rautio et al., Prodrugs: Design and Clinical Applications, Nature Reviews Drug Discovery, 2008, 7, 255-270, and Hecker et al., Prodrugs of Phosphates and Phosphonates, J. Med. Chem., 2008, 51, 2328-2345.

The term "metabolite" refers to a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

The term "pharmaceutically acceptable salt" refers to organic or inorganic salts of a compound disclosed herein. Pharmaceutically acceptable salts are well known in the art. For example, the pharmaceutically acceptable salts are described in detail in Berge et al., J. Pharmacol Sci, 1977, 66: 1-19, which is incorporated herein by reference in its entirety.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid and ethanolamine. The term "hydrate" refers to the complex where the solvent molecule is water.

The term "*N*-oxide" refers to one or more than one nitrogen atoms oxidised to form an *N*-oxide, where a compound contains several amine functions. Particular examples of *N*-oxides are the *N-*oxides of tertiary amines or the *N-*oxides of nitrogen atoms of nitrogen-containing heterocycle. *N-*oxides can be formed by treatment of the corresponding amine with an oxidizing agent such as hydrogen peroxide or a per-acid (*e.g*., a peroxycarboxylic acid) (See, Advanced Organic Chemistiy, by Jerry March, 4th Edition, Wiley Interscience, pages). More particularly, *N-*oxides can be made by the procedure of L. W. Deady (Syn. Comm. 1977, 7, 509-514) in which the amine compound is reacted with m-chloroperoxybenzoic acid (MCPBA), for example, in an inert solvent such as dichloromethane.

Any asymmetric atom (*e.g*., carbon or the like) of the compound(s) disclosed herein can be present in racemic or enantiomerically enriched, for example the (*R*)-, (*S*)- or (*R*, *S*)-configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)- configuration. Where possible, substituents on atoms with unsaturated double bonds may be present in cis-(*Z*)- or trans-(*E*)-form.

Therefore, as described herein, the compounds of the present invention may exist in the form of one or a mixture of possible isomers, rotamers, atropisomers, tautomers, for example in the form of substantially pure geometric (cis or trans) isomers, diastereomers, optical isomers (enantiomers), racemates or mixtures thereof.

Any resulting mixtures of stereoisomers can be separated on the basis of the physicochemical differences of the constituents, into the pure or substantially pure geometric isomers, enantiomers, diastereomers, for example, by chromatography and/or fractional crystallization. Cis and trans isomers are diastereomer.

Any resulting racemates of final products or intermediates can be resolved into the optical antipodes by methods known to those skilled in the art, *e.g.*, by separation of the diastereomeric salts thereof. Racemic products can also be resolved by chiral chromatography, *e.g*., high performance liquid chromatography (HPLC) using a chiral adsorbent. Preferred enantiomers can also be prepared by asymmetric syntheses. (*e.g*., Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); and Wilen, S.H. Tables of Resolving Agents and Optical Resolutions p. 268 (E.L. Eliel, Ed., Univ. of Notre Dame Press, Notre Dame, IN 1972)))

The invention also includes isotopically labeled compounds of the invention which are identical to those described herein except for the fact that one or more atoms are replaced by atoms having an atomic mass or mass number different from that found in nature. Examples of isotopes that can be incorporated into compounds of the invention include isotopes of hydrogen, carbon, nitrogen, oxygen, phosphorous, sulfur, fluorine, and chlorine, such as ²H, ³H, ¹³C, ¹⁴C, ¹⁵N, ¹⁶O, ¹⁷O, ³¹P, ³²P, ³⁶S, ¹⁸F and ³⁷Cl, respectively.

Compounds of the present invention comprising the aforementioned isotopes and/or other isotopes of other atoms, as well as pharmaceutically acceptable salts of the compounds, are included within the scope of the present invention. Isotopically-labeled compounds of the present invention, for example those into which radioactive isotopes such as ³H and ¹⁴C are incorporated, are useful in drug and/or substrate tissue distribution assays. Tritiated, *i.e.*, ³H, and carbon-14, *i.e.*, ¹⁴C, isotopes are particularly preferred due to ease of preparation and detection. Furthermore, substitution with a higher mass isotope, such as deuterium, *i.e.*, ²H, may afford some therapeutic advantage of greater metabolic stability, such as increasing *in vivo* half-life or reducing dosage requirements. Therefore, it may be preferable in some situations.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds disclosed herein may contain asymmetric or chiral centers, and therefore exist in different stereoisomeric forms. It is intended that all stereoisomeric forms of the compounds disclosed herein, including, but not limited to, diastereomers, enantiomers and atropisomers, as well as mixtures thereof such as racemic mixtures, form part of the present invention. Many organic compounds exist in optically active forms, *i.e.*, they have the ability to rotate the plane of plane-polarized light. In describing an optically active compound, the prefixes *D* and *L,* or *R* and *S*, are used to denote the absolute configuration of the molecule with respect to the chiral center (or centers) in the molecule. The prefixes *d* and *l* or (+) and (-) are employed to designate the sign of rotation of plane-polarized light by the compound, with (-) or *l* meaning that the compound is levorotatory. A compound prefixed with (+) or *d* is dextrorotatory. For a given chemical structure, these stereoisomers are identical except that they are mirror images of each other. A specific stereoisomer is referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process.

Depending on the choice of the starting materials and procedures, the compounds can be present in the form of one of the possible stereoisomers or as mixtures thereof, such as pure optical isomers, or as mixtures of isomers, *e.g*., as racemates and diastereoisomer mixtures, depending on the number of asymmetric carbon atoms. Optically active (*R*)- and (*S*)- isomers may be prepared using chiral synthons or chiral reagents, or resolved using conventional techniques. If the compound contains a double bond, the substituent may be *E* or *Z* configuration. If the compound contains a disubstituted cycloalkyl, the cycloalkyl substituent may have a cis- or trans-configuration.

Unless otherwise indicated, structures depicted herein are also meant to include all isomeric (*e.g.*, enantiomeric, diastereomeric, atropisomer, and geometric (or conformational)) forms of the structure; for example, *R* and *S* configurations, (*Z*) and (*E*) double bond isomers, and (*Z*) and (*E*) conformational isomers for each asymmetric center. Therefore, single stereochemical isomers as well as enantiomeric, diastereomeric, or geometric mixtures of the present compounds are within the scope disclosed herein.

The term "tautomer" or "tautomeric form" refers to structural isomers of different energies which are interconvertible via a low energy barrier. Where tautomerization is possible (*e.g.*, in solution), a chemical equilibrium of tautomers can be reached. For example, proton tautomers (also known as prototropic tautomers) include interconversions via migration of a proton, such as keto-enol and imine-enamine isomerizations. Valence tautomers include interconversions by reorganization of some of the bonding electrons. A specific example of keto-enol tautomerization is the interconversion of pentane-2,4-dione and 4-hydroxypent-3-en-2-one tautomers. Another example of tautomerization is phenol-keto tautomerization. The specific example of phenol-keto tautomerisms is pyridin-4-ol and pyridin-4(1*H*)-one tautomerism. Unless otherwise stated, all tautomeric forms of the compounds disclosed herein are within the scope of the invention.

The term "geometric isomer" is also called "cis-trans isomer", which are isomers caused by double bonds (including the double bond of olefin, C=N double bond and N=N double bond) or single bonds of ring carbon atoms that cannot rotate freely.

As used herein, the term "subject" refers to an animal. Typically the animal is a mammal. A subject also refers to primates (*e.g*., humans), cows, sheep, goats, horses, dogs, cats, rabbits, rats, mice, fish, birds, and the like. In certain embodiments, the subject is a primate. In yet other embodiments, the subject is a human.

As used herein, the terms "subject" and "patient" are used interchangeably. The terms "subject" and "patient" refer to animals (*e.g*., birds such as chickens, quails, or turkeys, or mammals), particularly "mammals" including non-primates (*e.g*., cows, pigs, horses, sheep, rabbits, guinea pigs, rats, cats, dogs and mice) and primates (*e.g*., monkeys, chimpanzees and humans), more particularly humans. In one embodiment, the subject is a non-human animal, such as a livestock (*e.g*., horse, cow, pig, or sheep) or pet (*e.g.*, dog, cat, guinea pig, or rabbit). In other embodiments, "patient" refers to a human.

Additionally, unless otherwise stated, structures depicted herein are also meant to include compounds that differ only in the presence of one or more isotopically enriched atoms.

As used herein, the term "treat", "treating" or "treatment" of any disease or disorder refers in one embodiment, to ameliorating the disease or disorder (*i.e.*, slowing or arresting or reducing the development of the disease or at least one of the clinical symptoms thereof). In another embodiment, "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g*., stabilization of a discernible symptom), physiologically, (*e.g*., stabilization of a physical parameter), or both. In yet another embodiment, "treat", "treating" or "treatment" refers to preventing or delaying the onset or development or progression of the disease or disorder.

### DESCRIPTION OF COMPOUNDS OF THE INVENTION

The invention provides a class of compounds with good agonistic activity on thyroid hormone β receptors, which are used in the manufacture of a medicament for treating neurodegenerative diseases, nonalcoholic fatty liver diseases, liver Fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer. The present invention also provides methods for preparing these compounds, pharmaceutical compositions containing these compounds, and methods for using these compounds and pharmaceutical compositions in the manufacture of a medicament for treating mammals, especially human beings, for the above-mentioned diseases. Compared with the existing similar compounds, the compounds of the present invention not only have good pharmacological activity and selectivity, but also have excellent *in vivo* metabolic kinetic properties and *in vivo* pharmacodynamic properties. The preparation methods of the compounds described in the invention are simple and easy, the process methods are stable, and are suitable for industrialized production. Therefore, the compounds provided by the present invention have better druggability than the existing similar compounds.

### Specifically:

In one aspect, the present invention provides a compound having Formula (I) or a stereoisomer, a geometric isomer, a tautomer, an *N*-oxide, a solvate, a metabolite, a pharmaceutically acceptable salt or a prodrug thereof, 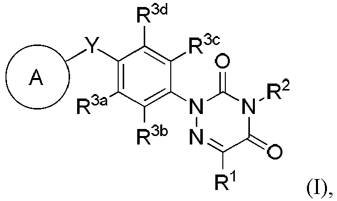 wherein, ring A, Y, R', R², R^{3a}, R^{3b}, R^{3c} and R^{3d} have the definitions as described in the present invention.

In some embodiments, Y is -O-, -S-, -NR⁰-, -C(=O)-, C₁₋₆ alkylene, C₂₋₆ alkenylene, C₂₋₆ alkynylene, -NR°C(=O)- or -C(=O)NR⁰-; wherein the Y is optionally substituted with 1, 2 or 3 R^{x}; wherein the R⁰ and R^{x} have the definitions as described in the present invention.

In some embodiments, R⁰ is H, deuterium, C₁₋₆ alkyl, C₁₋₆ haloalkyl, hydroxy C₁₋₆ alkyl, amino C₁₋₆ alkyl or cyano C₁₋₆ alkyl.

In some embodiments, R⁰ is H, deuterium, methyl, ethyl, n-propyl, isopropyl, C₁₋₄ haloalkyl, hydroxymethyl, hydroxyethyl, aminomethyl or cyanomethyl.

In some embodiments, each of R^{3a}, R^{3b}, R^{3c} and R^{3d} is independently H, deuterium, F, Cl, Br, I, -CN, -NO₂, -COOH, -OH, -NH₂, -SH, C₁₋₆ alkyl, C₁₋₆ alkoxy, C₁₋₆ alkylthio, C₁₋₆ alkylamino, C₁₋₆ haloalkyl, C₁₋₆ haloalkoxy, hydroxy C₁₋₆ alkyl, amino C₁₋₆ alkyl or cyano C₁₋₆ alkyl.

In some embodiments, R¹ is H, deuterium, F, Cl, Br, I, -CN, -NO₂, -COOH, -OH, -NH₂, -SH, C₁₋₆ alkyl, C₂₋₆ alkenyl, C₂₋₆ alkynyl, -C(=O)-C₁₋₆ alkoxy, -C(=O)-C₁₋₆ alkyl, -C(=O)-C₁₋₆ alkylamino, -C(=O)NH₂, -S (=O)₂-C₁₋₆ alkyl, -S(=O)₂-C₁₋₆ alkylamino, -S(=O)₂NH₂, C₁₋₆ alkylamino, C₁₋₆ alkoxy, C₁₋₆ haloalkyl, C₁₋₆ haloalkoxy, hydroxy C₁₋₆ alkyl, amino C₁₋₆ alkyl, carboxy C₁₋₆ alkyl or cyano C₁₋₆ alkyl.

In some embodiments, R² is H, deuterium, C₁₋₆ alkyl, C₂₋₆ alkenyl, C₂₋₆ alkynyl, C₃₋₆ cycloalkyl, heterocyclyl consisting of 5-6 atoms, C₆₋₁₀ aryl or heteroaryl consisting of 5-6 atoms.

In some embodiments, R² is H, deuterium, methyl, ethyl, *n*-propyl, isopropyl, *tert*-butyl, C₂₋₄ alkenyl, C₂₋₄ alkynyl, C₃₋₆ cycloalkyl, heterocyclyl consisting of 5-6 atoms, phenyl or heteroaryl consisting of 5-6 atoms.

In some embodiments, ring A is 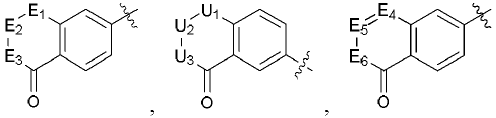 or 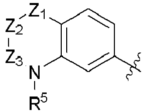 ; wherein, ring A may be optionally substituted by 1, 2 or 3 R^{y}; the E₁, E₂, E₃, E₄, E₅, E₆, U₁, U₂, U₃, Z₁, Z₂, Z₃, R⁵ and R^{y} have the definitions described in the present invention.

In some embodiments, each of E₁, U₁ and Z₁ is independently -(CR^{4a}R^{4b})_{q}-, -C(=O)-, -O-, -S-, -S(=O)-, -S(=O)₂- or -NR^{a}-; the R^{4a}, R^{4b}, R^{a} and q have the definitions described in the present invention.

In some embodiments, each of E₂, U₂ and Z₂ is independently -CR^{4c}R^{4d}-, -C(=O)-, -O-, -S-, -S(=O)-, -S(=O)₂- or -NR^{b}-; the R^{4c}, R^{4a} and R^{b} have the definitions described in the present invention.

In some embodiments, each of E₃, E₆, U₃ and Z₃ is independently -CR^{4e}R^{4f}-, -C(=O)-, -O-, -S-, -S(=O)-, -S(=O)₂- or -NR^{c}-; the R^{4e}, R^{4f} and R^{c} have the definitions described in the present invention.

In some embodiments, E₄ is -CR^{4g}= or -N=; wherein, the R^{4g} has the definition described herein.

In some embodiments, E₅ is -CR^{4h}= or -N=; wherein, the R^{4h} has the definition described herein.

In some embodiments, q is 0, 1, 2 or 3.

In some embodiments, each R^{a}, R^{b}, R^{c} and R⁵ is independently H, deuterium, C₁₋₆ alkyl, C₂₋₆ alkenyl, C₂₋₆ alkynyl, C₁₋₆ haloalkyl, C₃₋₆ cycloalkyl, heterocyclyl consisting of 5-6 atoms, C₆₋₁₀ aryl or heteroaryl consisting of 5-6 atoms, wherein each R^{a}, R^{b}, R^{c} and R⁵ is independently and optionally substituted with 1, 2 or 3 R^{y1}, R^{y1} has the meaning described in the present invention.

In some embodiments, each R^{4a}, R^{4b}, R^{4c}, R^{4a}, R^{4e}, R^{4f}, R^{4g} and R^{4h} is independently H, deuterium, F, Cl, Br, I, -CN, -NO₂, -COOH, -OH, -NH₂, -SH, C₁₋₆ alkyl, C₂₋₆ alkenyl, C₂₋₆ alkynyl, C₁₋₆ alkoxy, C₁₋₆ alkylamino, C₁₋₆ haloalkyl, C₁₋₆ haloalkoxy, C₃₋₆ cycloalkyl, C₃₋₆ cycloalkyl-C₁₋₄ alkylene, heterocyclyl consisting of 5-6 atoms, (heterocyclyl consisting of 5-6 atoms)-C₁₋₄ alkylene, C₆₋₁₀ aryl, C₆₋₁₀ aryl-C₁₋₄ alkylene, heteroaryl consisting of 5-6 atoms or (heteroaryl consisting of 5-6 atoms)-C₁₋₄ alkylene, wherein each R^{4a}, R^{4b}, R^{4c}, R^{4a}, R^{4e}, R^{4f}, R^{4g} and R^{4h} is optionally substituted with 1, 2 or 3 R^{y2}, R^{y2} has the definition as described in the present invention.

In some embodiments, R^{4a} and R^{4b}, together with the carbon atoms to which they are attached, form a C₃₋₈ carbon ring or a heterocyclyl consisting of 5-6 atoms, wherein each C₃₋₈ carbon ring and heterocyclyl consisting of 5-6 atoms is independently unsubstituted or substituted by 1, 2 or 3 R^{y3}, R^{y3} has the definition described in the present invention.

In some embodiments, R^{4c} and R^{4d}, together with the carbon atoms to which they are attached, form a C₃₋₈ carbon ring or a heterocyclyl consisting of 5-6 atoms, wherein each C₃₋₈ carbon ring and heterocyclyl consisting of 5-6 atoms is independently unsubstituted or substituted by 1, 2 or 3 R^{y3}, R^{y3} has the definition described in the present invention.

In some embodiments, R^{4e} and R^{4f}, together with the carbon atoms to which they are attached, form a C₃₋₈ carbon ring or a heterocyclyl consisting of 5-6 atoms, wherein each C₃₋₈ carbon ring and heterocyclyl consisting of 5-6 atoms is independently unsubstituted or substituted by 1, 2 or 3 R^{y3}, R^{y3} has the definition described in the present invention.

In some embodiments, two R^{y} linked on adjacent atoms, together with the atoms to which they are attached, form a C₃₋₈ carbon ring or a heterocyclyl consisting of 5-6 atoms, wherein each C₃₋₈ carbon ring and heterocyclyl consisting of 5-6 atoms is independently unsubstituted or substituted with 1, 2 or 3 R^{y4}, R^{y4} has the definition described in the present invention.

In some embodiments, each R^{x} is independently deuterium, F, Cl, Br, I, -CN, -OH, -NH₂, C₁₋₆ alkyl, C₁₋₆ haloalkyl, C₁₋₆ haloalkoxy, C₁₋₆ alkoxy or C₁₋₆ alkylamino.

In some embodiments, each R^{x} is independently deuterium, F, Cl, Br, I, -CN, -OH, -NH₂, methyl, ethyl, *n*-propyl, isopropyl, *tert*-butyl, *n*-butyl, trifluoromethyl, difluoromethyl, trifluoromethoxy, difluoromethoxy, methoxy, ethoxy, isopropoxy, methylamino or dimethylamino.

In some embodiments, each R^{y} is independently deuterium, F, Cl, Br, I, -CN, -OH, -NH₂, C₁₋₆ alkyl, C₁₋₆ haloalkyl, C₁₋₆ haloalkoxy, C₁₋₆ alkoxy or C₁₋₆ alkylamino.

In some embodiments, each R^{y} is independently deuterium, F, Cl, Br, I, -CN, -OH, -NH₂, methyl, ethyl, *n*-propyl, isopropyl, *tert*-butyl, *n*-butyl, trifluoromethyl, difluoromethyl, trifluoromethoxy, difluoromethoxy, methoxy, ethoxy, isopropoxy, methylamino or dimethylamino.

In some embodiments, each R^{y1} is independently deuterium, F, Cl, Br, I, -CN, -OH, -NH₂, -SH, oxo, -OC(=O)-C₁₋₆ alkyl, -C(=O)-C₁₋₆ alkoxy, -C(=O)-C₁₋₆ alkyl, -C(=O)-C₁₋₆ alkylamino, -C(=O)NH₂, -S(=O)₂-C₁₋₆ alkyl, -S(=O)₂-C₁₋₆ alkylamino, -S(=O)₂NH₂, C₁₋₆ alkyl, C₁₋₆ haloalkyl, C₁₋₆ haloalkoxy, C₁₋₆ alkoxy, C₁₋₆ alkylthio, C₁₋₆ alkylamino, C₃₋₆ cycloalkyl, heterocyclyl consisting of 5-6 atoms, C₆₋₁₀ aryl or heteroaryl consisting of 5-6 atoms, wherein each R^{y1} is independently and optionally substituted with 1, 2 or 3 R^{z}, R^{z} has the definition described in the present invention.

In some embodiments, each R^{z}, R^{y2}, R^{y3} and R^{y4} is independently deuterium, F, Cl, Br, I, -CN, -OH, -NH₂, -COOH, C₁₋₆ alkyl, C₁₋₆ haloalkyl, C₁₋₆ haloalkoxy, C₁₋₆ alkoxy, C₁₋₆ alkylthio or C₁₋₆ alkylamino.

In some embodiments, each of R^{3a}, R^{3b}, R^{3c} and R^{3d} is independently H, deuterium, F, Cl, Br, I, -CN, -NO₂, -COOH, -OH, -NH₂, -SH, methyl, ethyl, *n*-propyl, isopropyl, methoxy, ethoxy, methylthio, methylamino, -CF₃, -CHF₂, -CH₂F, -CH₂CF₃, -CH₂CHF₂, -OCF₃, -OCHF₂, -OCH₂F, hydroxymethyl, aminomethyl or cyanomethyl.

In some embodiments, R¹ is H, deuterium, F, Cl, Br, I, -CN, -NO₂, -COOH, -OH, -NH₂, -SH, methyl, ethyl, *n*-propyl, isopropyl, -CH=CH₂, -CH₂CH=CH₂, -CH=CHCH₃, -C≡CH, -C(=O)-OCH₃, -C(=O)-OCH₂CH₃, -C(=O)-OCH(CH₃)₂, -C(=O)-OCH₂CH₂CH₃, -C(=O)-O(CH₂)₃CH₃, -C(=O)-OCH₂CH(CH₃)₂, -C(=O)-CH₃, -C(=O)-CH₂CH₃, -C(=O)-NHCH₃, -C(=O)-N(CH₃)₂, -C(=O)NH₂, -S(=O)₂-CH₃, -S(=O)₂-CH₂CH₃, -S(=O)₂-NHCH₃, -S(=O)₂NH₂, methylamino, ethylamino, methoxy, ethoxy, -CF₃, -CHF₂, -CH₂F, -CH₂CF₃, -CH₂CHF₂, -OCF₃, -OCHF₂, -OCH₂F, hydroxymethyl, aminomethyl, carboxymethyl or cyanomethyl.

In some embodiments, each R^{a}, R^{b}, R^{c} and R⁵ is independently H, deuterium, C₁₋₄ alkyl, C₂₋₄ alkenyl, C₂₋₄ alkynyl, C₁₋₄ haloalkyl, C₃₋₆ cycloalkyl, heterocyclyl consisting of 5-6 atoms, C₆₋₁₀ aryl or heteroaryl consisting of 5-6 atoms, wherein each R^{a}, R^{b}, R^{c} and R⁵ is independently and optionally substituted with 1, 2 or 3 R^{y1}, R^{y1} has the meaning described in the present invention.

In some embodiments, each R^{a}, R^{b}, R^{c} and R⁵ is independently H, deuterium, methyl, ethyl, *n*-propyl, isopropyl, *n*-butyl, *tert*-butyl, -CH=CH₂, -CH₂CH=CH₂, -CH=CHCH₃, -C≡CH, -CF₃, -CHF₂, -CH₂F, -CH₂CF₃, -CH₂CHF₂, cyclopropyl, cyclobutyl, cyclopentyl, cyclohexyl, pyrrolidinyl, pyrazolidinyl, tetrahydrofuranyl, tetrahydrothiophenyl, tetrahydropyranyl, piperidinyl, morpholinyl, thiomorpholinyl, piperazinyl, phenyl, furanyl, thienyl, imidazolyl, pyrimidinyl, pyridyl, pyrrolyl, pyrazinyl, thiazolyl or oxazolyl, wherein each R^{a}, R^{b}, R^{c} and R⁵ is independently and optionally substituted with 1, 2 or 3 R^{y1}, R^{y1} has the meaning described in the present invention.

In some embodiments, each R^{y1} is independently deuterium, F, Cl, Br, I, -CN, -OH, -NH₂, -SH, oxo, -OC(=O)-C₁₋₄ alkyl, -C(=O)-C₁₋₄ alkoxy, -C(=O)-C₁₋₄ alkyl, -C(=O)-C₁₋₄ alkylamino, -C(=O)NH₂, -S(=O)₂-C₁₋₄ alkyl, -S(=O)₂-C₁₋₄ alkylamino, -S(=O)₂NH₂, C₁₋₄ alkyl, C₁₋₄ haloalkyl, C₁₋₄ haloalkoxy, C₁₋₄ alkoxy, C₁₋₄ alkylthio, C₁₋₄ alkylamino, C₃₋₆ cycloalkyl, heterocyclyl consisting of 5-6 atoms, C₆₋₁₀ aryl or heteroaryl consisting of 5-6 atoms, wherein each R^{y1} is optionally substituted with 1, 2 or 3 R^{z}, R^{z} has the definition described in the present invention.

In some embodiments, each R^{y1} is independently deuterium, F, Cl, Br, I, -CN, -OH, -NH₂, -SH, oxo, -OC(=O)-methyl, -OC(=O)-ethyl, -OC(=O)-*n*-propyl, -OC(=O)-isopropyl, -OC(=O)-*n*-butyl, -OC(=O)-*tert*-butyl, -OC(=O)-isobutyl, -C(=O)O-methyl, -C(=O)O-ethyl, -C(= O)O-*n*-propyl, -C(=O)O-isopropyl, -C(=O)O-*n*-butyl, -C(=O)O-*tert*-butyl, -C(=O)O-isobutyl, -C(=O)-methyl, -C(=O)-ethyl, -C(=O)-*n*-propyl, -C(=O)-isopropyl, -C (=O)-*n*-butyl, -C(=O)-*tert*-butyl, -C(=O)-isobutyl, -C(=O)-methylamino, -C(=O)-ethylamino, -C(=O)NH₂, -S(=O)₂-C₁₋₃ alkyl, -S(=O)₂-C₁₋₃ alkylamino, -S(=O)₂NH₂, methyl, ethyl, *n*-propyl, isopropyl, *tert*-butyl, -CF₃, -CHF₂, -CH₂F, -CH₂CF₃, -CH₂CHF₂, -OCF₃, -OCHF₂, -OCH₂F, -OCH₂CF₃, -OCH₂CHF₂, -OCHFCH₃, methoxy, ethoxy, *n*-propoxy, isopropoxy, methylthio, ethylthio, methylamino, ethylamino, cyclopropyl, cyclobutyl, cyclopentyl, cyclohexyl, pyrrolidinyl, pyrazolidinyl, tetrahydrofuryl, tetrahydrothiophenyl, tetrahydropyranyl, piperidinyl, morpholinyl, thiomorpholinyl, piperazinyl, phenyl, furyl, thienyl, imidazolyl, pyrimidinyl, pyridinyl, pyrrolyl, pyridazinyl, pyrazinyl, thiazolyl or oxazolyl, wherein each R^{y1} is optionally substituted with 1, 2 or 3 R^{z}; wherein R^{z} has the meaning described in the present invention.

In some embodiments, each R^{4a}, R^{4b}, R^{4c}, R^{4a}, R^{4e}, R^{4f}, R^{4g} and R^{4h} is independently H, deuterium, F, Cl, Br, I, -CN, -NO₂, -COOH, -OH, -NH₂, -SH, methyl, ethyl, *n*-propyl, isopropyl, *n*-butyl, *tert*-butyl, -CH=CH₂, -CH₂CH=CH₂, -CH=CHCH₃, -C≡CH, methoxy, ethoxy, methylamino, ethylamino, -CF₃, -CHF₂, -CH₂F, -CH₂CF₃, -CH₂CHF₂, -OCF₃, -OCHF₂, -OCH₂F, cyclopropyl, cyclobutyl, cyclopentyl, cyclohexyl, cyclopropyl-CH₂-, cyclobutyl-CH₂-, cyclopentyl-CH₂-, cyclohexyl-CH₂-, pyrrolidinyl, pyrazolidinyl, tetrahydrofuranyl, tetrahydrothienyl, piperidinyl, morpholinyl, thiomorpholinyl, piperazinyl, pyrrolidinyl-CH₂-, pyrazolidinyl-CH₂-, tetrahydrofuranyl-CH₂-, tetrahydrothiophenyl-CH₂-, piperidinyl-CH₂-, morpholinyl-CH₂-, thiomorpholinyl-CH₂-, piperazinyl-CH₂-, phenyl, phenyl-CH₂-, phenyl-CH₂CH₂-, furanyl, thienyl, imidazolyl, pyrimidinyl, pyridyl, pyrrolyl, pyridazinyl, pyrazinyl, thiazolyl, oxazolyl, furyl-CH₂-, thienyl-CH₂-, imidazolyl-CH₂-, pyrimidinyl-CH₂-, pyridyl-CH₂- or pyrrolyl-CH₂-, wherein each R^{4a}, R^{4b}, R^{4c}, R^{4a}, R^{4e}, R^{4f}, R^{4g} and R^{4h} is independently and optionally substituted with 1, 2 or 3 R^{y2}, R^{y2} has the definition described in the present invention.

In some embodiments, R^{4a} and R^{4b}, together with the carbon atoms to which they are attached, form a C₃₋₆ carbon ring or a heterocyclyl consisting of 5-6 atoms, wherein each C₃₋₆ carbon ring and heterocyclyl consisting of 5-6 atoms is independently unsubstituted or substituted by 1, 2 or 3 R^{y3}, R^{y3} has the definition described in the present invention.

In some embodiments, R^{4c} and R^{4d}, together with the carbon atoms to which they are attached, form a C₃₋₆ carbon ring or a heterocyclyl consisting of 5-6 atoms, wherein each C₃₋₆ carbon ring and heterocyclyl consisting of 5-6 atoms is independently unsubstituted or substituted by 1, 2 or 3 R^{y3}, R^{y3} has the definition described in the present invention.

In some embodiments, R^{4e} and R^{4f}, together with the carbon atoms to which they are attached, form a C₃₋₆ carbon ring or a heterocyclyl consisting of 5-6 atoms, wherein each C₃₋₆ carbon ring and heterocyclyl consisting of 5-6 atoms is independently unsubstituted or substituted by 1, 2 or 3 R^{y3}, R^{y3} has the definition described in the present invention.

In some embodiments, each R^{z}, R^{y2}, R^{y3} and R^{y4} is independently deuterium, F, Cl, Br, I, -CN, -OH, -NH₂, -COOH, methyl, ethyl, *n*-propyl, isopropyl, -CF₃, -CHF₂, -CH₂F, -OCF₃, -OCHF₂, -OCH₂F, methoxy, ethoxy or methylamino.

In another aspect, the present invention provides one of the following structures, or a stereoisomer, a geometric isomer, a tautomer, an *N*-oxide, a solvate, a metabolite, a pharmaceutically acceptable salt or a prodrug thereof,

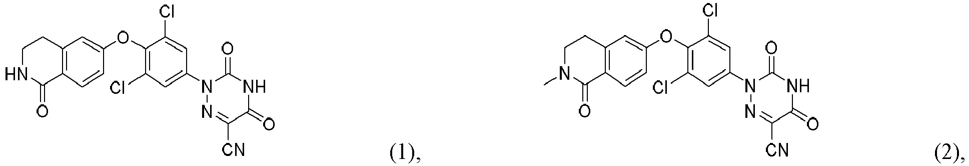

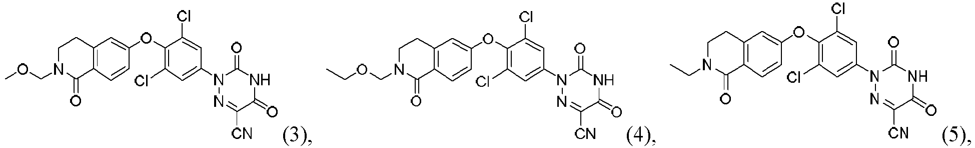

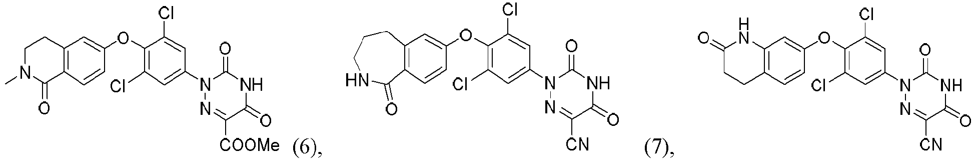

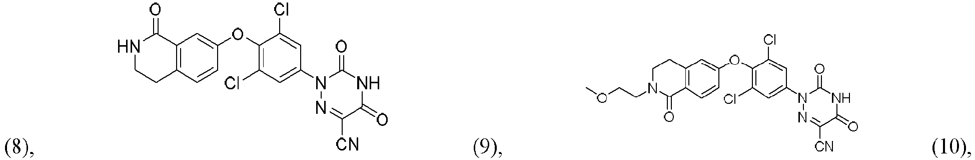

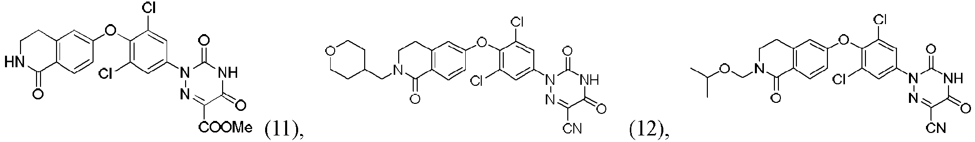

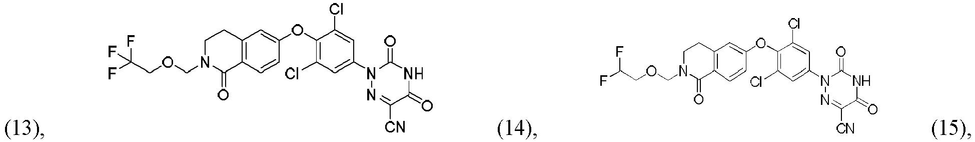

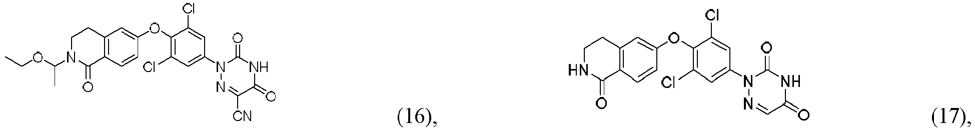

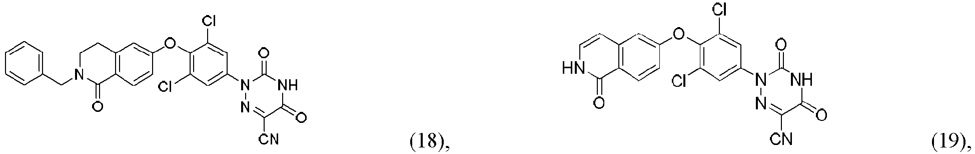

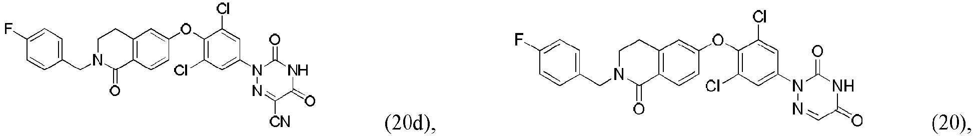

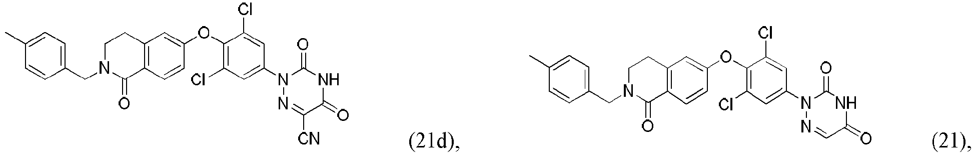

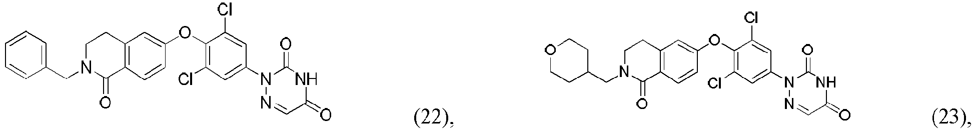

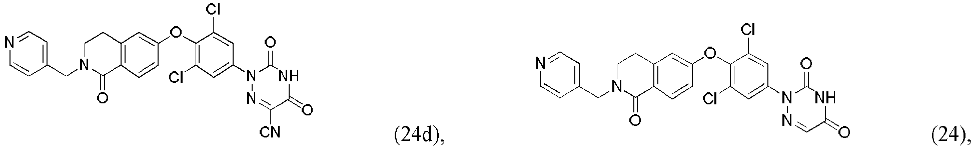

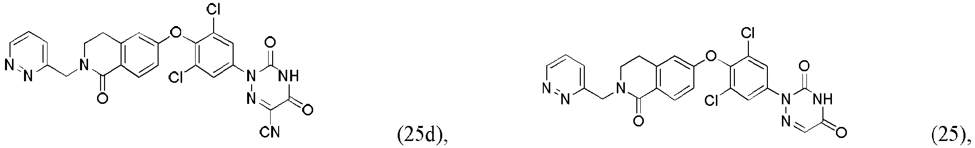

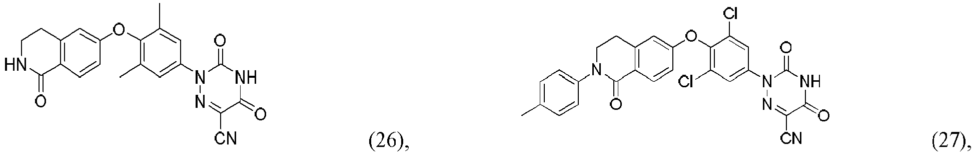

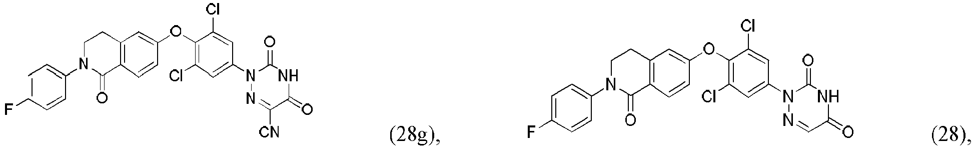

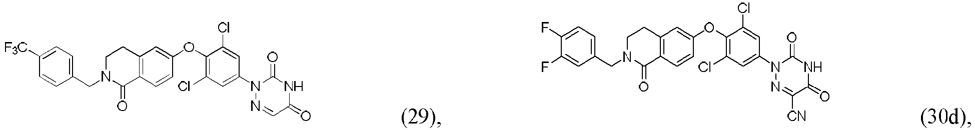

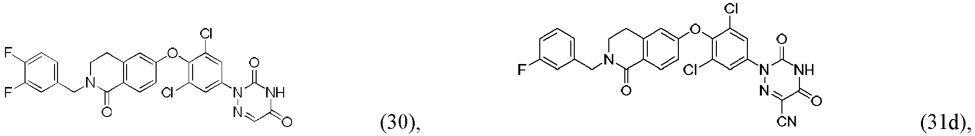

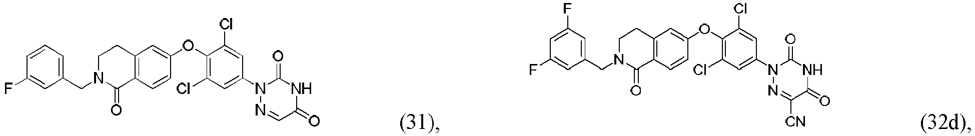

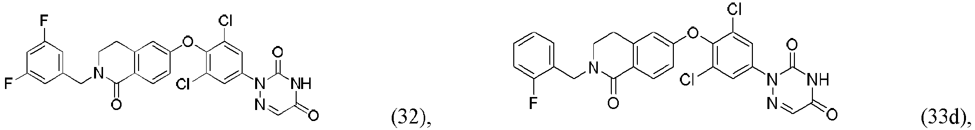

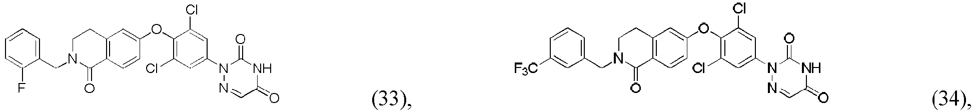

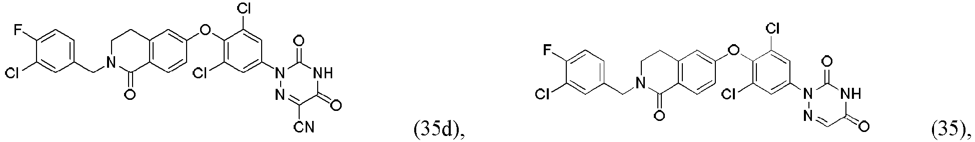

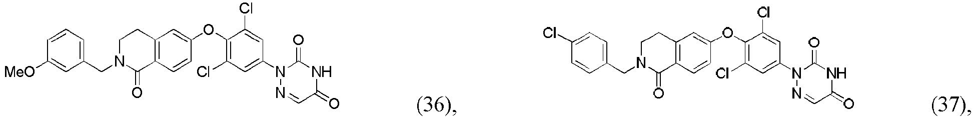

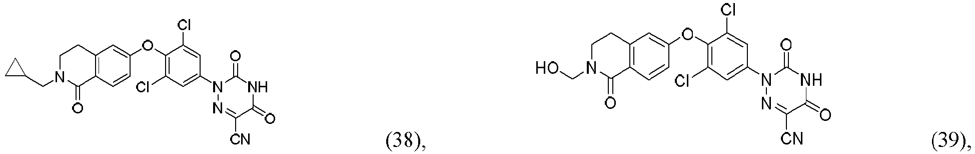

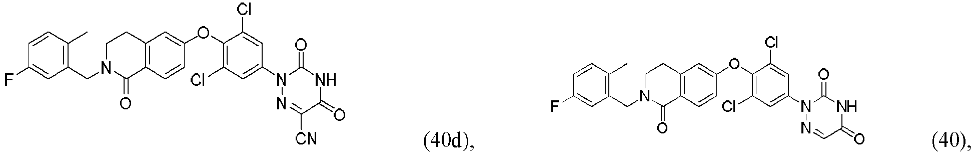

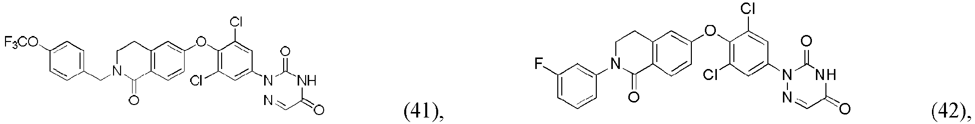

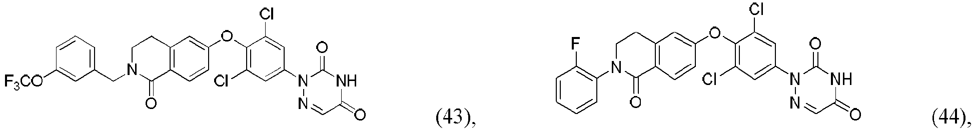

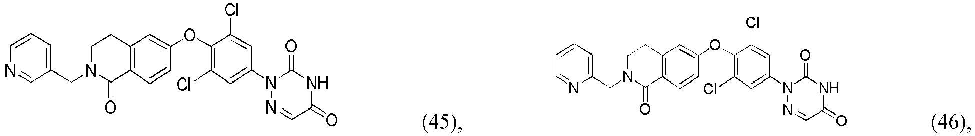

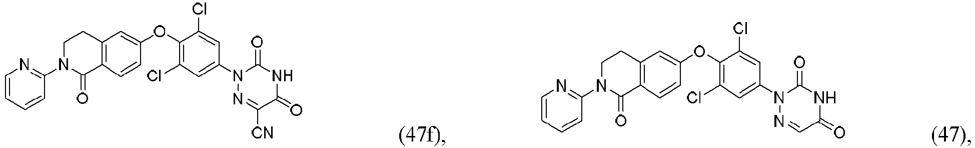

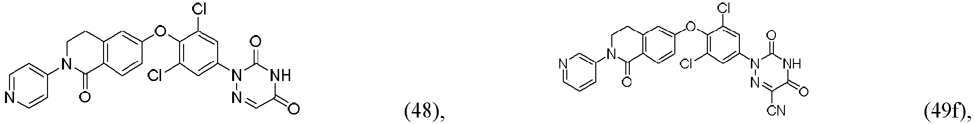

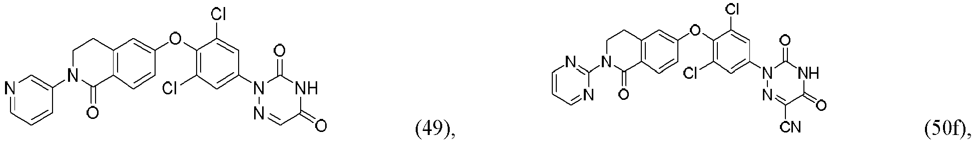

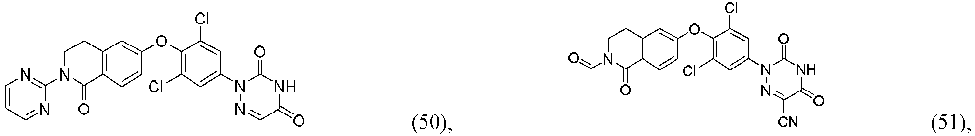

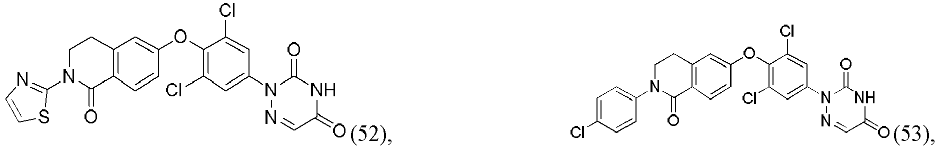

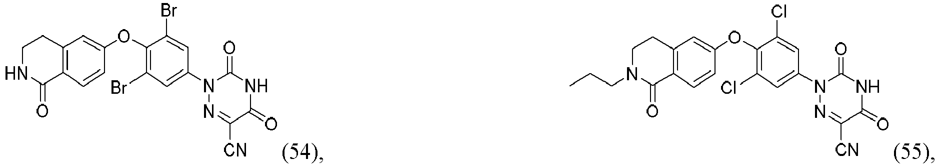

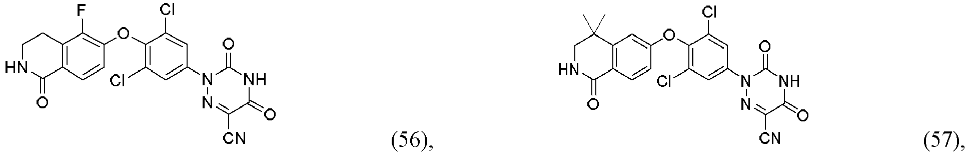

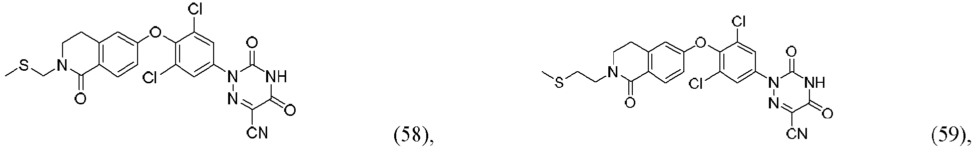

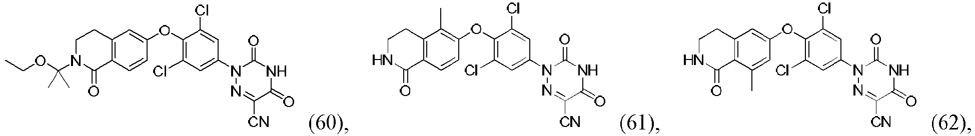

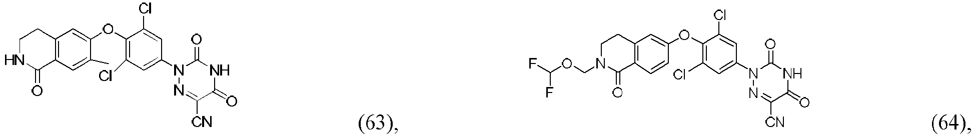

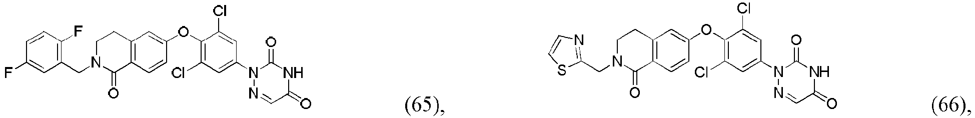

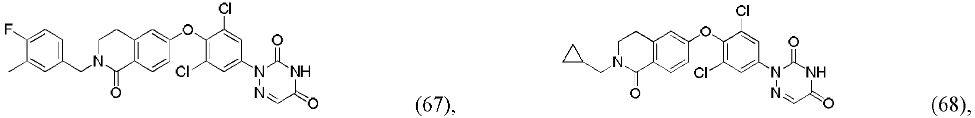

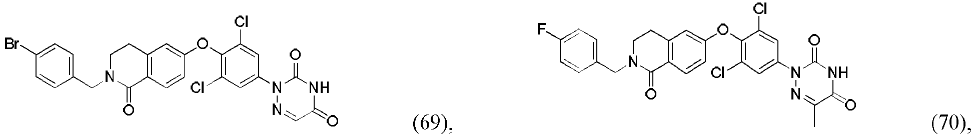

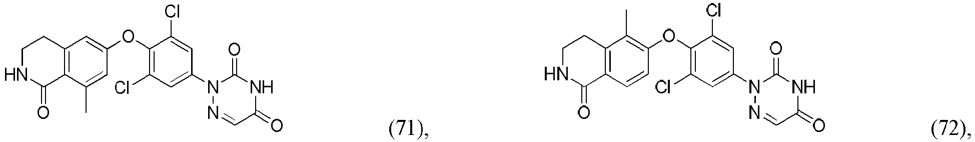

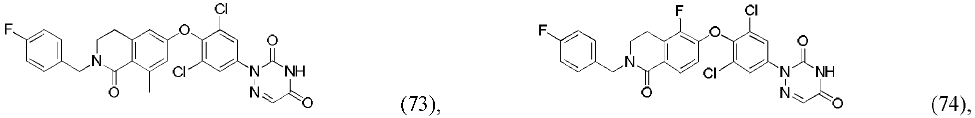

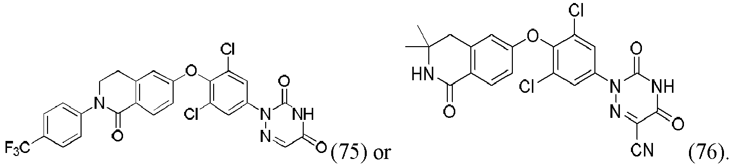

In other aspect, provided herein is a pharmaceutical composition comprising the compound disclosed herein.

In some embodiments, the pharmaceutical composition disclosed herein optionally further comprises any one of a pharmaceutically acceptable carrier, excipient, adjuvant, vehicle or any combination thereof.

In another aspect, the present invention relates to use of the compound of the present invention or the pharmaceutical composition of the present invention in the manufacture of a medicament for stimulating thyroid hormone receptors; or for preventing, treating or alleviating diseases regulated by thyroid hormone receptors.

In another aspect, the present invention relates to a method of stimulating thyroid hormone receptors with the compound or the pharmaceutical composition of the present invention, or a method of preventing, treating or alleviating diseases regulated by thyroid hormone receptors in a subject comprising administering to the subject a therapeutically effective amount of the compound or the pharmaceutical composition of the present invention. Moreover, the above-mentioned compounds provided by the present invention or their pharmaceutical compositions can be co-administered with other therapies or therapeutic agents. The mode of administration can be carried out simultaneously, sequentially or at certain time intervals.

In another aspect, the present invention relates to the compound of the present invention or the pharmaceutical composition of the present invention for use in stimulating thyroid hormone receptors, or in preventing, treating or alleviating diseases regulated by thyroid hormone receptors.

In some embodiments, the thyroid hormone receptor of the present invention is a thyroid hormone β receptor.

In some embodiments, the diseases regulated by thyroid hormone receptors in the present invention are neurodegenerative diseases, nonalcoholic fatty liver diseases, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer.

In one aspect, the present invention relates to use of the compound of the present invention or the pharmaceutical composition of the present invention in the manufacture of a medicament for preventing, treating or alleviating the following diseases: neurodegenerative diseases, nonalcoholic fatty liver diseases, liver fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer.

In one aspect, the present invention relates to the compound of the present invention or the pharmaceutical composition of the present invention for use in preventing, treating or alleviating the following diseases: neurodegenerative diseases, nonalcoholic fatty liver diseases, liver fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer.

In one aspect, the present invention relates to a method of using the compound of the present invention or the pharmaceutical composition of the present invention to prevent, treat or alleviate the following diseases: neurodegenerative diseases, nonalcoholic fatty liver diseases, liver fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer. The method is administering to an individual in need thereof a therapeutically effective amount of the compound or the pharmaceutical composition.

In some embodiments, the nonalcoholic fatty liver disease described in the present invention is nonalcoholic simple fatty liver, nonalcoholic steatohepatitis, cryptogenic cirrhosis associated with nonalcoholic fatty liver disease or primary liver cancer.

In some embodiments, the neurodegenerative disease described in the present invention is demyelinating disease, chronic demyelinating disease, leukodystrophy, dementia, ischemic stroke, lacunar stroke, multiple sclerosis, MCT8 deficiency, X-linked adrenal dystrophy (ALD), amyotrophic lateral sclerosis (ALS) or Alzheimer's disease.

The dosage of the compound or pharmaceutical composition required to implement the effects of treatment, prevention or delay usually depends on the specific compound to be administered, the patient, the specific disease or condition and its severity, the route and frequency of administration, *etc.*, and needs to be determined by the attending physician according to the actual situation. For example, when the compounds or pharmaceutical compositions provided by the present invention are administered by intravenous route, the administration can be performed once a week or even at longer time intervals.

In some embodiments, the salt refers to a pharmaceutically acceptable salt. The term "pharmaceutically acceptable" means that the substance or composition must be chemically and/or toxicologically compatible with the other ingredients comprising the formulation and/or the mammal being treated therewith.

The compounds of the present invention also include other salts of such compounds, which are not necessarily pharmaceutically acceptable salts, and can be used as intermediates for the preparation and/or purification of the compounds of the present invention and/or for the separation of enantiomers of the compounds of the present invention.

Furthermore, the compounds of the present invention, including their salts, may also be obtained in the form of their hydrates, or include other solvents used for their crystallization. The compounds of the present invention may inherently or by design form solvates with pharmaceutically acceptable solvents (including water); therefore, it is intended that the invention embrace both solvated and unsolvated forms.

### PHARMACEUTICAL COMPOSITION OF THE COMPOUND OF THE INVENTION AND PREPARATIONS AND ADMINISTRATION

The present invention relates to a pharmaceutical composition, which includes the compound of the present invention or the compound of the structure shown in the examples, or a stereoisomer, a geometric isomer, a tautomer, an *N*-oxide, a solvate, a metabolite, a pharmaceutically acceptable salt or a prodrug thereof. The pharmaceutical composition further comprises at least one pharmaceutically acceptable carrier, excipient, adjuvant, vehicle or a combination thereof, and optionally, other therapeutic and/or prophylactic ingredients. In some embodiments, the pharmaceutical composition disclosed herein comprises an effective amount of a compound described herein and at least one pharmaceutically acceptable carrier, excipient, adjuvant or vehicle. The amount of the compound in the pharmaceutical composition of the invention is effective to detectably agonize the thyroid hormone beta receptor in a biological specimen or patient.

Pharmaceutically acceptable carriers may contain inert ingredients that do not unduly inhibit the biological activity of the compound. A pharmaceutically acceptable carrier should be biocompatible, *e.g*., non-toxic, non-inflammatory, non-immunogenic or otherwise free of adverse effects or side effects once administered to a patient. Standard pharmaceutical techniques may be employed.

As described above, the pharmaceutical composition or pharmaceutically acceptable composition of the present invention further comprises a pharmaceutically acceptable carrier, an excipient, an adjuvant or a vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington: The Science and Practice of Pharmacy, 21st ed., 2005, Lippincott Williams & Wilkins, Philadelphia, and Swarbrick et al., Encyclopedia of Pharmaceutical Technology, eds. 1988-1999, Marcel Dekker, New York, discloses various carriers used in formulating pharmaceutically acceptable compositions and known techniques for the preparation thereof. Except insofar as any conventional carrier medium incompatible with the compounds disclosed herein, such as by producing any undesirable biological effect or otherwise interacting in a deleterious manner with any other components of the pharmaceutically acceptable composition, any other conventional carrier medium and its use is contemplated to be within the scope of this invention.

Some examples of substances that can be used as pharmaceutically acceptable carriers include, but are not limited to, ion exchangers, alumina, aluminum stearate, lecithin, serum proteins (such as human serum albumin), buffer substances (such as Tween 80, phosphate, glycine, sorbic acid, or potassium sorbate), partial glyceride mixtures of saturated vegetable fatty acids, water, salts, or electrolytes (such as protamine sulfate, disodium hydrogen phosphate, potassium hydrogen phosphate, sodium chloride, or zinc salts), silica gel, magnesium trisilicate, polyvinylpyrrolidone, polyacrylates, waxes, polyethylene-polyoxypropylene-block copolymers, methylcellulose, hydroxypropylmethylcellulose, lanolin, sugars (such as lactose, glucose, and sucrose), starches (such as corn starch and potato starch), cellulose and its derivatives (such as sodium carboxymethylcellulose, ethyl cellulose, and cellulose acetate), powdered tragacanth, malt, gel, talc, excipients (such as cocoa butter and suppository waxes), oils (such as peanut oil, cottonseed oil, safflower oil, sesame oil, ofive oil, corn oil, and soybean oil), glycols (such as propylene glycol or polyethylene glycol), esters (such as ethyl oleate and ethyl dodecanoate), agar, buffers (such as magnesium hydroxide and aluminum hydroxide), alginic acid, pyrogen-free water, isotonic saline, Ringer's solution, ethanol and phosphate buffered saline and other nontoxic compatible lubricants (such as sodium lauryl sulfate and magnesium stearate) and, at the discretion of the formulator, colorants, detackifying agents, coating agents, sweetening and flavoring agents, preservatives and antioxidants can also be present in the compositions.

The pharmaceutical composition of the present invention can be administered directly or in the form of a pharmaceutical composition or drug together with a suitable carrier or excipient, which is well known in the art. The methods of treatment of the present invention may comprise administering to a subject in need thereof an effective compound of the present invention. In some embodiments, the individual is a mammalian individual, in other embodiments, the individual is a human individual.

The effective amount of the compound, pharmaceutical composition or drug of the present invention can be easily determined by conventional methods and tests, and the most effective and convenient route of administration and the most suitable preparation can also be determined by conventional tests.

A compound or composition of the invention may be administered in any suitable means, and the above-mentioned compound and pharmaceutically acceptable composition can be administered to humans or other animals by oral, rectal, parenteral, intracisternal, intravaginal, intraperitoneal, topical (as by powder, ointment or drops) or nasal spray according to the severity of the disease.

Liquid dosage forms for oral administration include, but are not limited to, pharmaceutically acceptable emulsions, microemulsions, solutions, suspensions, syrups and elixirs. In addition to the active ingredient, the liquid dosage forms may contain inert diluent commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, dimethylformamide, oils (in particular cottonseed, groundnut, corn, germ, olive, castor and sesame oils), glycerol, tetrahydrofurfuryl alcohol, polyethylene glycols and fatty acid esters of sorbitan, and mixtures thereof. In addition to the inert diluent, the oral compositions may also contain adjuvants such as wetting agents, emulsifying or suspending agents, sweetening agents, flavoring agents and fragrances.

Injectable preparations can be formulated according to known techniques using suitable dispersing or wetting agents and suspending agents, such as sterile injectable aqueous or oily suspensions. The sterile injectable preparation may also be a sterile injectable solution, suspension or emulsion in a non-toxic parenterally acceptable diluent or solvent, for example a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic monoglycerides or diglycerides. In addition, fatty acids such as octadecenoic acid are used in the preparation of injectables.

The injectable formulations can be sterilized, for example, by filtration through a bacteria-retaining filter or by the addition of a sterile solid composition which can be dissolved or dispersed in sterile water or other sterile injectable medium prior to use.

In order to prolong the effect of the compounds or compositions described herein, it is often desirable to slow the absorption of the compound from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally administered compound is accomplished by dissolving or suspending the compound in an oil vehicle. Injectable depot forms are made by forming microencapsule matrices of the compound in biodegradable polymers such as polylactide-polyglycolic acid. Depending on the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration, in particular suppositories, may be prepared by mixing a compound according to the invention with a suitable non-irritating excipient or carrier, such as cocoa butter, polyethylene glycol or a suppository wax. The excipient or carrier is solid at ambient temperature but liquid at body temperature and therefore melts in the rectum or vaginal cavity and releases the active compound.

Solid dosage forms for oral administration include capsules, tablets, pills, powders and granules. In these dosage forms, the active compound of the present invention are mixed with at least one pharmaceutically acceptable inert excipient or carrier such as sodium citrate or dicalcium phosphate and/or a) fillers or bulking agents such as starch, lactose, sucrose, glucose, mannitol and silicic acid; b) binders such as carboxymethylcellulose, alginates, gelatin, polyvinylpyrrolidone, sucrose, and acacia; c) humectants such as glycerin; d) disintegrants such as agar-agar, calcium carbonate, potato or tapioca starch, alginic acid, certain silicates and sodium carbonate; e) solution retarders, such as paraffin; f) absorption accelerators, such as quaternary ammonium compounds; g) humectants, such as cetyl alcohol and glyceryl monostearate; h) absorbents, such as kaolin and bentonite; and i) lubricants such as talc, calcium stearate, magnesium stearate, solid polyethylene glycol, sodium lauryl sulfate, and mixtures thereof. In the case of capsules, tablets and pills, the dosage form may also comprise buffering agents.

Solid compositions of a similar type can also be used as fillers in soft and hard gelatin capsules using excipients such as lactose or milk sugar and high molecular weight polyethylene glycols. The solid dosage forms of tablets, dragees, capsules, pills, and granules can be prepared with coatings and shells such as enteric coatings and others well known in the pharmaceutical art. They may optionally contain opacifying agents and may also be of a composition so that they release the active ingredient(s) only, or preferably, in a certain part of the intestinal tract, optionally, in a delayed manner. Examples of embedding compositions that can be used include polymeric substances and waxes.

The active compounds can also be in microencapsulated form with one or more of the above-mentioned excipients. In these solid dosage forms, the active compound may be mixed with at least one inert diluent, such as sucrose, lactose or starch. In general, such dosage forms may also contain additional substances besides inert diluents, such as tableting lubricants and other tableting aids, such as magnesium stearate and microcrystalline cellulose. They may optionally contain opacifying agents and may also be of a composition so that they release the active ingredient(s) only, or preferably, in a certain part of the intestinal tract, optionally, in a delayed manner. Examples of embedding compositions that can be used include polymeric substances and waxes.

Dosage forms for topical or transdermal administration of a compound of this invention include unguents, ointments, creams, lotions, gels, powders, solutions, sprays, inhalants or patches. Under sterile conditions, the active compound is combined with a pharmaceutically acceptable carrier and any required preservatives or buffers that may be required. Ophthalmic formulations, ear drops, and eye drops are also contemplated within the scope of this invention. Additionally, the present invention contemplates the use of skin patches with the added advantage of providing controlled delivery of compounds to the body. Such dosage forms can be made by dissolving or dispersing the compound in the proper medium. Absorption enhancers can also be used to increase the flux of the compound across the skin. The rate can be controlled by providing a rate controlling membrane or by dispersing the compound in a polymer matrix or gel.

The compositions described herein may also be administered orally, parenterally, or topically, rectally, nasally, buccally, vaginally by inhalation spray, or via an implanted kit. The term "parenteral" as used herein includes, but is not limited to, subcutaneous, intravenous, intramuscular, intra-articular, intrasynovial, intrasternal, intrathecal, intrahepatic, intralesional and intracranial injection or infusion techniques. In particular, the compositions are administered orally, intraperitoneally or intravenously.

Sterile injectable forms of the compositions of this invention may be aqueous or oily suspensions. These suspensions may be formulated following techniques known in the art using suitable dispersing or wetting agents and suspending agents. The sterile injectable preparation may also be a sterile injectable solution or suspension in a non-toxic parenterally acceptable diluent or solvent, for example a solution in 1,3-butanediol. Among the acceptable vehicles and solvents that may be employed are water, Ringer's solution. and isotonic sodium chloride solution. In addition, sterile, fixed oils are conventionally employed as a solvent or suspending medium. For this purpose any bland fixed oil may be employed including synthetic monoglycerides or diglycerides. In addition, fatty acids such as octadecenoic acid and its glyceride derivatives are useful in the preparation of injectables, as are natural pharmaceutically-acceptable oils, such as ofive oil or castor oil, especially in their polyoxyethylated forms. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical composition of the present invention can be orally administered in any orally acceptable dosage form, including but not limited to capsules, tablets, aqueous suspensions or solutions. In the case of tablets for oral administration, carriers commonly used include, but are not limited to, lactose and starch. Lubricating agents, such as magnesium stearate, are also usually added. For oral administration in a capsule form, useful diluents include lactose and dried cornstarch. When aqueous suspensions are required for oral administration, the active ingredient is combined with emulsifying and suspending agents. Certain sweetening, flavoring or coloring agents can also be added, if desired.

Alternatively, the pharmaceutical compositions described herein may be administered in the form of suppositories for rectal use. These pharmaceutical compositions can be prepared by mixing reagents with non-irritating excipients, such substances include but are not limited to cocoa butter, beeswax and polyethylene glycols.

The pharmaceutical compositions of the present invention may also be administered topically, especially when the target of treatment includes topical instillation of easily accessible areas or organs, including diseases of the eye, skin or lower intestinal tract. Suitable topical formulations are readily prepared for each of these areas or organs.

Topical instillation to the lower intestinal tract can be achieved in rectal suppository formulations (see above) or in suitable enema formulations. Topical skin patches may also be used.

For topical administration, the pharmaceutical composition can be formulated as a suitable ointment containing the active components suspended or dissolved in one or more carriers. Carrier compounds for topical administration of the present invention include, but are not limited to, mineral oil, petrolatum, white petrolatum, propylene glycol, polyoxyethylene, polyoxypropylene compounds, emulsified waxes and water. Alternatively, the pharmaceutical composition can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical composition is formulated as a micronized suspension in, or especially as a solution in, isotonic pH-adjusted sterile saline, with or without a preservative such as benzalkonium chloride. Alternatively, for ophthalmic use, the pharmaceutical composition can be formulated as an ointment, such as petrolatum.

The pharmaceutical compositions can also be administered by nasal aerosol spray or inhalation. Such compositions are prepared according to techniques well known in the pharmaceutical art and solutions in saline are prepared using benzyl alcohol and other suitable preservatives, absorption enhancers to enhance bioavailability, fluorocarbons and/or other conventional solubilizing or dispersing agents.

### USE OF THE COMPOUNDS AND PHARMACEUTICAL COMPOSITIONS

The compound or pharmaceutical composition provided by the present invention can be used in the manufacture of a medicament for stimulating thyroid hormone receptors, or in the manufacture of a medicament for preventing, treating or alleviating diseases regulated by thyroid hormone receptors.

The compound or pharmaceutical composition provided by the present invention can be used to stimulate thyroid hormone receptors, or to prevent, treat or alleviate diseases regulated by thyroid hormone receptors.

The present invention relates to a method of stimulating thyroid hormone receptors or preventing, treating or alleviating diseases regulated by thyroid hormone receptors in a subject comprising administering a therapeutically effective amount of the above compound or its pharmaceutical composition to the subject in need. Moreover, the above-mentioned compounds provided by the present invention or their pharmaceutical compositions can be co-administered with other therapies or therapeutic agents. The mode of administration can be carried out simultaneously, sequentially or at certain time intervals.

The thyroid hormone receptor of the present invention is a thyroid hormone β receptor.

The diseases described in the present invention are nonalcoholic fatty liver diseases, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer, wherein the nonalcoholic fatty liver disease is nonalcoholic simple fatty liver, nonalcoholic steatohepatitis, nonalcoholic fatty liver disease-associated cryptogenic cirrhosis or primary liver cancer.

The compound or pharmaceutical composition of the present invention can be used to treat fibrotic diseases, including but not limited to liver fibrosis, idiopathic pulmonary fibrosis and the like.

Besides being useful for human treatment, these compounds are also useful for veterinary treatment of animals such as companion animals, exotic animals and farm animals, including mammals, rodents, and the like. In other embodiments, the animals disclosed herein include horses, dogs, and cats. As used herein, the compounds disclosed herein include the pharmaceutically acceptable derivatives thereof.

An "effective amount" or "effective dose" of the compound or pharmaceutically acceptable composition is an amount that is effective in treating or lessening the severity of one or more of the aforementioned disorders. The compounds and pharmaceutically acceptable compositions are effective administered in a fairly wide dose range. For example, the daily dose is from about 0.1 mg to 1000 mg per person, the compounds or pharmaceutically acceptable compositions can be administered in a single dose or in several divided doses a day. The compounds and compositions, according to the method disclosed herein, may be administered using any amount and any route of administration which is effective for treating or lessening the severity of the disorder or disease. The exact amount required will vary from subject to subject, depending on the species, age, and general condition of the subject, the severity of the infection, the particular agent, its mode of administration, and the like. A compound or composition can also be administered with one or more other therapeutic agents as discussed above.

### GENERAL SYNTHESIS AND DETECTION METHODS

In order to describe the present invention, examples are listed below. However, it should be understood that the present invention is not limited to these examples, but only provides a method of practicing the present invention.

In the present invention, if the chemical name of the compound doesn't match the corresponding structure, the compound is characterized by the corresponding structure.

Generally, the compounds disclosed herein may be prepared by methods described herein, wherein the substituents are as defined for Formula (I), except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

The structures of the compounds were identified by nuclear magnetic resonance (¹H-NMR, ¹³C-NMR and/or ¹⁹F-NMR). ¹H-NMR, ¹³C-NMR and/or ¹⁹F-NMR chemical shifts (δ) were recorded as ppm (10⁻⁶). ¹H-NMR, ¹³C-NMR and/or ¹⁹F-NMR were measured with Bruker Ultrashield-400 nuclear magnetic resonance spectrometer and Bruker Avance III HD 600 nuclear magnetic resonance spectrometer, and the measuring solvent was deuterated chloroform (CDCl₃), deuterated methanol (CD₃OD or MeOH-*d₄*) or deuterated dimethylsulfoxide (DMSO-*d₆*). TMS (0 ppm) or chloroform (7.25 ppm) was used as reference standards. When peak multiplicities are reported, the following abbreviations are used: s (singlet), d (doublet), t (triplet), q (quartet), m (multiplet), br (broadened), dd (doublet of doublets), ddd (doublet of doublet of doublets), dt (doublet of triplets), td (triplet of doublets), brs (broadened singlet). Coupling constant *J*, when given, was reported in Hertz (Hz).

Generally, Novasep pump 250 high-performance liquid chromatography was used for preparative purification or preparative resolution.

LC-MS spectra were determined on Agilen-6120 Quadrupole LC/MS mass spectrometer.

The silica gel used in column chromatography generally was Qingdao Ocean Chemical Factory 300 to 400 mesh silica gel.

The staring materials of the present invention were known or purchased from Shanghai Accela Company, Energy Company, J&K, Alfa Company and the like, or they could be prepared by the conventional synthesis methods in the prior art.

Unless otherwise stated, the reactions disclosed herein were carried out in a nitrogen atmosphere.

The term "nitrogen atmosphere" refers to such an atmosphere that a reaction flask was equipped with a balloon or a stainless steel autoclave filled with about 1 L nitrogen.

The term "hydrogen atmosphere" refers to such an atmosphere that a reaction flask was equipped with a balloon or a stainless steel autoclave filled with about 1 L hydrogen.

Unless otherwise stated, the solution used in the examples disclosed herein was an aqueous solution.

Unless otherwise stated, the reaction temperature was room temperature.

Unless otherwise stated, the room temperature was from 20°C to 40°C.

The reaction process in the examples was monitored by thin layer chromatography (TLC). The solvent system for development of a TLC plate comprised dichloromethane and methanol, dichloromethane and ethyl acetate, petroleum ether and ethyl acetate. The volume ratio of the solvents in the solvent system was adjusted according to the polarity of the compounds.

The elution system of column chromatography comprised: A: petroleum ether and ethyl acetate, B: dichloromethane and ethyl acetate, C: dichloromethane and methanol. The volume ratio of the solvents in the elution system was adjusted according to the polarity of the compounds, and sometimes it was also adjusted by adding a small amount of aqueous ammonia and acetic acid.

HPLC refers to High Performance Liquid Chromatography.
HPLC was determined on Agilent 1260 high pressure liquid chromatography spectrometer (chromatographic column: Agilent ZORBAX Eclipse Plus C18 4.6mm×150mm, 3.5µm);
The test condition of HPLC: the run time was 25 minutes (min); the column temperature was 35 °C; the detection was carried out at the wavelength of 210 nm and 245 nm;
the mobile phases were 0.05% phosphoric acid solution (A) and acetonitrile (B); and the flow rate was 1.0 mL/min.

The mobile phase gradient is shown in Table A:

**Table A**

| Time | Gradient of mobile phase A | Gradient of mobile phase B |
|---|---|---|
| 0 min | 90% | 10% |
| 15 min | 10% | 90% |
| 20 min | 10% | 90% |
| 25 min | 90% | 10% |

The LC/MS/MS system used for the analysis in the biological test experiment included Agilent 1200 series vacuum degassing oven, binary syringe pump, orifice plate autosampler, column oven, Agilent G6430 triple quadrupole mass spectrometer with electrospray ionization (ESI) source. Quantitative analysis was carried out in MRM mode, and the parameters of MRM conversion are shown in Table B:

**Table B**

| | |
|---|---|
| Full scan | 50~1400 |
| Fragmentation voltage | 230 V |
| Capillary voltage | 55 V |
| Dryer temperature | 350°C |
| Atomizer | 0.28MPa |
| Dryer flow rate | 10 L/min |

An Agilent XDB-C18, 2.1 × 30 mm, 3.5 µm column was used for analysis, and 5 µL of the sample was injected. Analytical conditions: Mobile phases were 0.1% formic acid in water (A) and 0.1% formic acid in methanol (B). The flow rate was 0.4 mL/min. The mobile phase gradient is shown in Table C:

**Table C**

| Time | Gradient of mobile phase B |
|---|---|
| 0.5 min | 5% |
| 1.0 min | 95 % |
| 2.2 min | 95 % |
| 2.3 min | 5 % |
| 5.0 min | Termination |

Low-resolution mass spectral (MS) data were determined on an Agilent 6120 Quadrupole HPLC-MS spectrometer equipped with an Agilent Zorbax SB-C18 (2.1 × 30 mm, 3.5 µm). The flow rate was 0.6 mL/min; the mobile phases consisted of a combination of A (0.1% formic acid in CH₃CN) and B (0.1% formic acid in H₂O) in gradient mode (5% to 95%), and an ESI source was used, the peak of HPLC was recorded with UV-Vis detection at 210 nm/254 nm.

The following abbreviations are used throughout the specification:

| DMSO-*d₆*: | Deuterated dimethylsulfoxide; | DMSO: | Dimethylsulfoxide; |
|---|---|---|---|
| TFA: | Trifluoroacetic acid; | mL, ml: | Milliliter; |
| µL, µl: | Microliter; | mol/L, mol/1: | Mole/liter; |
| mol: | Moore; | mmol/L, mmol/l, mM: | Millimol/L; |
| µmol/L, µmol/l, µM: | Micromol/L; | nmol/L, nmol/l, nM: | Nanomol/L; |
| g: | Gram; | h: | Hour; |
| mg: | Milligram; | µg: | Microgram; |
| ng: | Nanogram; | %wt, mass%: | % by weight; |
| µm: | Micron; | MPa: | Megapascal; |
| min: | Minute; | ACN: | Acetonitrile; |
| Me: | Methyl; | Et | Ethyl; |

### GENERAL SYNTHETIC PROCEDURES

Typical synthetic procedures for the preparation of the compounds disclosed herein are shown in the following synthetic schemes. Unless otherwise stated, each ring A, R', R^{3a}, R^{3b}, R^{3c} and R^{3d} have the definitions as described in the present invention, and X is halogen.

### Synthesis scheme 1:

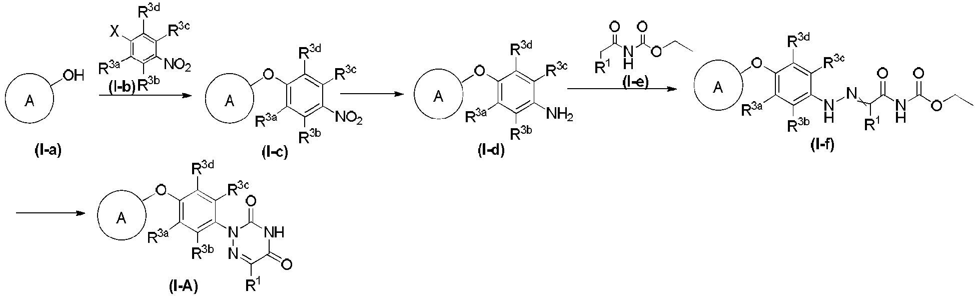

The compound with the structure shown in general formula (I-A) can be prepared by the general synthesis method described in synthesis scheme 1, and the specific steps can be referred to the examples. First, compound (I-a) can be reacted with compound (I-b) under the action of a base (such as potassium carbonate) to obtain compound (I-c); compound (I-c) can be reduced by nitro to obtain compound (I-d); compound (I-d) can be diazotized with the amino group and reacted with compound (I-e) to obtain compound (I-f); compound (I-f) can be ring-closed under the action of a base (such as sodium acetate) to obtain the target compound represented by general formula (I-A).

### Synthesis scheme 2:

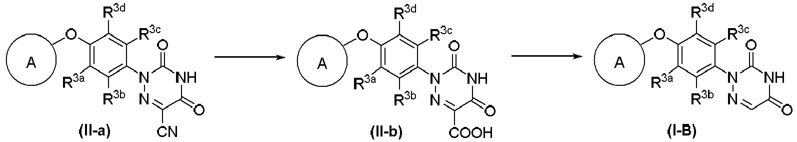

The compound with the structure shown in general formula (I-B) can be prepared by the general synthesis method described in synthesis scheme 2, and the specific steps can be referred to the examples. First, compound (II-a) can be hydrolyzed under acidic conditions (such as concentrated hydrochloric acid) to obtain compound (II-b); compound (II-b) can be decarboxylated to obtain the target compound represented by general formula (I-B).

### Example

### Example 1

### 2-(3,5-dichloro-4-((1-oxo-1,2,3,4-tetrahydroisoquinolin-6-yl)oxy)phenyl)-3,5-dioxo-2,3,4,5-tetrahydro-1,2,4-t riazine-6-carbonitrile 1

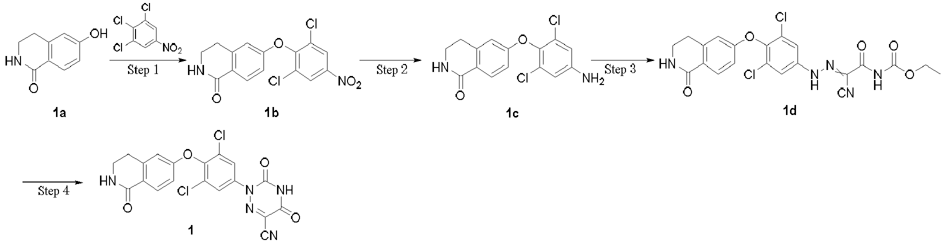

### Step 1: Synthesis of 6-(2,6-dichloro-4-nitrophenoxy)-3,4-dihydroisoquinolin-1(2H)-one 1b

To a solution of 6-hydroxy-3,4-dihydroisoquinolin-1(2*H*)-one **1a** (1.00 g, 6.13 mmol) and 1,2,3-trichloro-5-nitrobenzene (1.39 g, 6.14 mmol) in *N*,*N*-dimethylformamide (10 mL) was added potassium carbonate (2.14 g, 15.3 mmol), and the mixture was reacted at 120°C for 16 hours. The reaction solution was cooled to room temperature, then water (20 mL) was added. The reaction mixture was stirred for 15 minutes and filtered to collect filter cake. The filter cake was recrystallized with ethanol/ethyl acetate/petroleum ether (1/2/4, 35 mL), and filtered to collect filter cake. After drying, a yellow solid **1b** (1.41 g, yield 65%) was obtained.

MS (ESI, pos. ion) *m*/*z*: 353.1 [M+H]⁺.

### Step 2: Synthesis of 6-(4-amino-2,6-dichlorophenoxy)-3,4-dihydroisoquinolin-1(2H)-one 1c

To a solution of 6-(2,6-dichloro-4-nitrophenoxy)-3,4-dihydroisoquinolin-1(2*H*)-one **1b** (1.4 g, 4.0 mmol) in acetic acid (8 mL) was added iron powder (0.22 g, 3.9 mmol), and the mixture was reacted at 70°C for 2 hours. The reaction solution was cooled to room temperature, iron powder was removed, then water (30 mL) was added. The reaction mixture was stirred for 10 minutes, filtered, and rinsed with water (10 mL). The filter cake was collected and dried to obtain a yellow solid **1c** (1.0 g, yield 78%).

MS (ESI, pos. ion) *m*/*z*: 323.2 [M+H]⁺.

### Step 3: Synthesis of ethyl

### (2-cyano-2-(2-(3,5-dichloro-4-((1-oxo-1,2,3,4-tetrahydroisoquinolin-6-yl)oxy)phenyl)hydrazono)acetyl)carbamate 1d

To a solution of 6-(4-amino-2,6-dichlorophenoxy)-3,4-dihydroisoquinolin-1(2*H*)-one **1c** (0.20 g, 0.62 mmol) and *N*-cyanoacetylurethane (0.11 g, 0.69 mmol) in acetic acid (4 mL) was added a solution of sodium nitrite (65 mg, 0.93 mmol) in water (2 mL) at 0°C, and the mixture was reacted for 4.5 hours. At 0°C, water (10 mL) was added to the reaction solution. The mixture was stirred for 10 minutes, filtered, washed with water (2 mL), and the filter cake was collected and dried to obtain an orange solid **1d** (0.30 g, yield 99%).

### Step 4: Synthesis of

### 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile 1

To a solution of ethyl (2-cyano-2-(2-(3,5-dichloro-4-((1-oxo-1,2,3,4-tetrahydroisoquinolin-6-yl)oxy)phenyl)hydrazono)acetyl)carbamate **1d** (0.30 g, 0.61 mmol) in *N*,*N*-dimethylformamide (3 mL) was added sodium acetate (55 mg, 0.67 mmol), and the mixture was reacted at 120°C for 5 hours. The reaction solution was cooled to room temperature, water (20 mL) was added, solid was precipitated out. The mixture was stirred for 10 minutes, filtered, and rinsed with water (5 mL). The filter cake was collected and dried, and the obtained reddish-brown solid was recrystallized (acetonitrile/ethanol/ *N*,*N*-dimethylformamide/water=10/5/2/20, 37 mL), filtered, and rinsed with water (2 mL). The filter cake was collected and dried to obtain an orange solid **1** (0.13 g, yield 48%, purity 91.86%).

MS (ESI, neg. ion) *m*/*z*: 442.0 [M-H]⁻.

¹H NMR (400 MHz, DMSO-*d₆*) δ (ppm) 13.27 (s, 1H), 7.85 (d, *J* = 6.4 Hz, 4H), 6.87 (s, 1H), 6.81 (dd, *J* = 8.6, 2.3 Hz, 1H), 3.32 (s, 2H), 2.90 (t, *J* = 6.2 Hz, 2H).

### Example 2 2-(3,5-dichloro-4-((2-methyl-1-oxo-1,2,3,4-tetrahydroisoquinolin-6-yl)oxy)phenyl)-3,5-dioxo-2,3,4,5-tetrahy dro-1,2,4-triazine-6-carbonitrile 2

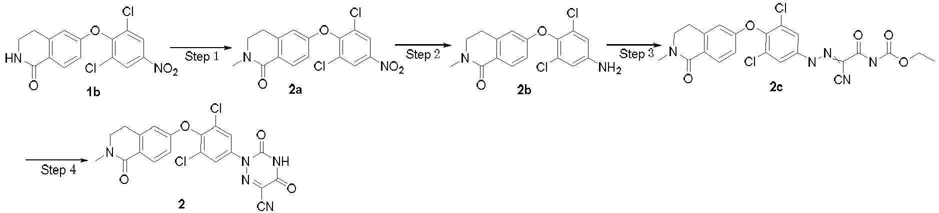

### Step 1: Synthesis of 6-(2,6-dichloro-4-nitrophenoxy)-2-methyl-3,4-dihydroisoquinolin-1(2H)-one 2a

To a solution of 6-(2,6-dichloro-4-nitrophenoxy)-3,4-dihydroisoquinolin-1(2*H*)-one **1b** (1.0 g, 2.8 mmol) in THF (15 mL) were added sodium hydride (0.23 g, 5.8 mmol, 60% in oil) and methyl iodide (0.26 mL, 4.2 mmol), and the mixture was reacted at room temperature for 2.5 hours. The reaction was quenched with water (10 mL). The mixture was extracted with ethyl acetate (50 mL × 2). The combined organic layers were washed with saturated sodium chloride (20 mL), dried over anhydrous sodium sulfate and concentrated by suction filtration. The residue was purified by silica gel column chromatography (petroleum ether/ethyl acetate = 3/1) to give a yellow solid **2a** (1.0 g, yield 99%).

### Step 2: Synthesis of 6-(4-amino-2,6-dichlorophenoxy)-2-methyl-3,4-dihydroisoquinolin-1(2H)-one 2b

To a solution of 6-(2,6-dichloro-4-nitrophenoxy)-2-methyl-3,4-dihydroisoquinolin-1(2*H*)-one **2a** (1.0 g, 2.7 mmol) in acetic acid (8 mL) was added iron powder (0.30 g, 5.4 mmol), and the mixture was reacted at 70°C. The reaction solution was cooled to room temperature, iron powder was removed, then water (30 mL) was added. The reaction mixture was stirred for 10 minutes, filtered, and rinsed with water (10 mL). The filter cake was collected and dried, and the obtained solid was purified by silica gel column chromatography (petroleum ether/ethyl acetate = 1/1) to obtain a yellow solid **2b** (0.40 g, yield 44%).

### Step 3: Synthesis of ethyl

### (2-cyano-2-(2-(3,5-dichloro-4-((2-methyl-1-oxo-1,2,3,4-tetrahydroisoquinolin-6-yl)oxy)phenyl)hydrazono)acetyl) carbamate 2c

To a solution of 6-(4-amino-2,6-dichlorophenoxy)-2-methyl-3,4-dihydroisoquinolin-1(2*H*)-one **2b** (0.40 g, 1.2 mmol) and *N*-cyanoacetylurethane (0.21 g, 1.3 mmol) in acetic acid (8 mL) was added a solution of sodium nitrite (0.17g, 2.4 mmol) in water (4 mL) at 0°C, and the mixture was reacted for 4 hours. At 0°C, water (10 mL) was added to the reaction solution. The mixture was stirred for 10 minutes, filtered, washed with water (2 mL), and the filter cake was collected and dried to obtain a red solid **2c** (0.62 g, yield 99%).

### Step 4: Synthesis of

### 2-(3,5-dichloro-4-((2-methyl-1-oxo-1,2,3,4-tetrahydroisoquinolin-6-yl)oxy)phenyl)-3,5-dioxo-2,3,4,5-tetrahydro-1,2,4-triazine-6-carbonitrile 2

To a solution of ethyl (2-cyano-2-(2-(3,5-dichloro-4-((2-methyl-1-oxo-1,2,3,4-tetrahydroisoquinolin-6-yl)oxy)phenyl)hydrazono)acetyl) carbamate **2c** (0.62 g, 1.2 mmol) in *N*,*N*-dimethylformamide (6 mL) was added sodium acetate (0.11 g, 1.3 mmol), and the mixture was reacted at 120°C for 6 hours. The reaction solution was cooled to room temperature, and water (20 mL) was added. The mixture was extracted with ethyl acetate (80 mL × 2). The combined organic layers were washed with saturated sodium chloride solution (40 mL), dried over anhydrous sodium sulfate and concentrated by suction filtration. The resulting residue was purified by silica gel column chromatography (petroleum ether/ethyl acetate = 1/3), and the obtained solid was recrystallized (acetonitrile/ethyl acetate/ethanol/petroleum ether=3/10/15/30, 58 mL) to obtain a light red solid **2** (0.21 g, yield 38%, purity: 97.61%).
MS (ESI, neg. ion) *m*/*z*: 456.0 [M-H]⁻;
¹H NMR (400 MHz, DMSO-*d₆*) δ (ppm) 13.29 (s, 1H), 8.11-7.63 (m, 3H), 6.91-6.76 (m, 2H), 3.53 (t, *J* = 6.6 Hz, 2H), 3.00 (s, 3H), 2.97 (t, *J* = 6.6 Hz, 2H).

### Example 3 2-(3,5-dichloro-4-((2-(methoxymethyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 3

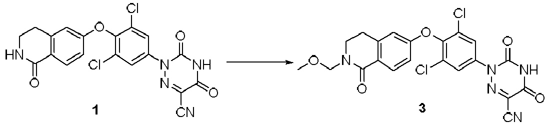

To a solution of 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.10 g, 0.23 mmol) in methanol (4 mL) was added 37% formaldehyde (0.37 mL, 5.0 mmol). The mixture was sealed and reacted at 100°C for 16 hours. The reaction solution was cooled to room temperature, and concentrated. The resulting residue was purified by silica gel column chromatography (petroleum ether/ethyl acetate = 1/1), and the obtained solid was recrystallized (methanol/ethyl acetate/petroleum ether = 1/6/12, 9.5 mL) to obtain a white solid **3** (50 mg, yield 45%, HPLC purity: 97.51%).
MS (ESI, neg. ion) *m*/*z*: 486.0 [M-H]⁻;
¹H NMR (400 MHz, DMSO-*d₆*) δ (ppm) 13.30 (s, 1H), 7.91 (d, *J* = 8.6 Hz, 1H), 7.84 (s, 2H), 6.95-6.81 (m, 2H), 4.86 (s, 2H), 3.56 (t, *J* = 6.4 Hz, 2H), 3.22 (s, 3H), 2.98 (t, *J* = 6.2 Hz, 2H).

### Example 4

### 2-(3,5-dichloro-4-((2-(ethoxymethyl)-1-oxo-1,2,3,4-tetrahydroisoquinolin-6-yl)oxy)phenyl)-3,5-dioxo-2,3,4,5-tetrahydro-1,2,4-triazine-6-carbonitrile 4

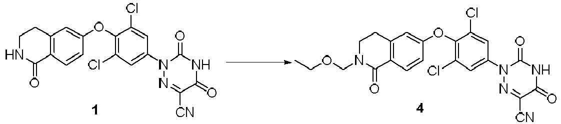

To a solution of 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.40 g, 0.90 mmol) in ethanol (10 mL) was added 37% formaldehyde (2.0 mL, 27 mmol). The mixture was sealed and reacted at 100°C for 48 hours. The reaction solution was cooled to room temperature, and concentrated. The resulting residue was purified by silica gel column chromatography (petroleum ether/ethyl acetate = 1/1), and the obtained solid was recrystallized (ethanol/ethyl acetate/petroleum ether = 1/5/3, 18 mL) to obtain a white solid **4** (0.28 g, yield 62%, HPLC purity: 96.19%).
MS (ESI, neg. ion) *m*/*z*: 500.0 [M-H]⁻;
¹H NMR (400 MHz, DMSO-*d₆*) δ (ppm) 13.31 (s, 1H), 8.06-7.74 (m, 3H), 6.99-6.74 (m, 2H), 4.90 (s, 2H), 3.56 (t, *J* = 6.1 Hz, 2H), 3.49-3.43 (m, 2H), 2.98 (t, *J* = 5.8 Hz, 2H), 1.11 (t, *J* = 6.9 Hz, 3H).

### Example 5

### 2-(3,5-dichloro-4-((2-ethyl-1-oxo-1,2,3,4-tetrahydroisoquinolin-6-yl)oxy)phenyl)-3,5-dioxo-2,3,4,5-tetrahydr o-1,2,4-triazine-6-carbonitrile 5

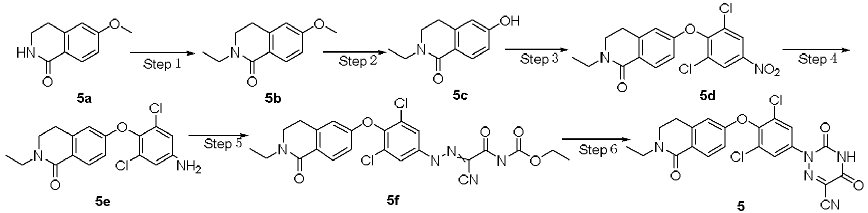

### Step 1: Synthesis of 2-ethyl-6-methoxy-3,4-dihydroisoquinolin-1(2H)-one 5b

6-Methoxy-3,4-dihydroisoquinolin-1(2*H*)-one **5a** (2.05 g, 11.6 mmol) was dissolved in a mixture solution of THF (40 mL) and *N*,*N*-dimethylacetamide (15 mL). Sodium hydride (0.58 g, 14.5 mmol, 60% in oil) was added in portions at 0°C, then ethyl iodide (1.13 mL, 13.8 mmol) was added dropwise, and the mixture was reacted at 50°C for 24 hours. The reaction solution was cooled to room temperature, quenched by the addition of ice water (40 mL), and then concentrated *in vacuo* to remove tetrahydrofuran. The remaining solution was extracted by ethyl acetate (50 mL × 3). The combined organic layers were washed with saturated sodium chloride (45 mL × 3), dried over anhydrous sodium sulfate and concentrated *in vacuo.* The resulting residue was purified by silica gel column chromatography (petroleum ether/ethyl acetate = 3/1) to give light yellow oil **5b** (2.30 g, yield 97%).

MS (ESI, pos. ion) *m*/*z*: 206.2 [M+H]⁺.

### Step 2: Synthesis of 2-ethyl-6-hydroxy-3,4-dihydroisoquinolin-1(2H)-one 5c

2-Ethyl-6-methoxy-3,4-dihydroisoquinolin-1(2*H*)-one **5b** (2.40 g, 11.7 mmol) was dissolved in dichloromethane (50 mL). Boron tribromide (2.28 mL, 23.4 mmol) was slowly added dropwise at 0°C and the mixture was continued stirring for 3.5 hours. The reaction was quenched by slowly adding methanol (10 mL) dropwise at 0°C. The mixture was concentrated *in vacuo*, extracted with ethyl acetate (40 mL × 3). The combined organic layers werewashed with saturated sodium chloride solution (40 mL × 2), dried over anhydrous sodium sulfate, and concentrated by suction filtration to give a brown-yellow solid **5c** (2.01 g, yield 90%).

MS (ESI, neg. ion) *m*/*z*: 190.1 [M-H]⁻.

### Step 3: Synthesis of 6-(2,6-dichloro-4-nitrophenoxy)-2-ethyl-3,4-dihydroisoquinolin-1(2H)-one 5d

To a solution of 2-ethyl-6-hydroxy-3,4-dihydroisoquinolin-1(2*H*)-one **5c** (2.00 g, 10.5 mmol) in *N*,*N*-dimethylacetamide (25 mL) were added 1,2,3-trichloro-5-nitrobenzene (2.37 g, 10.5 mmol) and potassium carbonate (5.61 g, 40.2 mmol) in sequence, and the mixture was reacted at 80°C for 7 hours. The reaction solution was cooled to room temperature, poured into water (50 mL), stirred for 20 minutes, then hydrochloric acid (3 N, 15 mL) was added dropwise. The reaction mixture was filtered, the solid was collected and dried, and the obtained solid was purified by silica gel column chromatography (petroleum ether/ethyl acetate = 2/1) to obtain a white solid **5d** (3.42 g, yield 86%).

MS (ESI, pos. ion) *m*/*z*: 381.0 [M+H]⁺.

### Step 4: Synthesis of 6-(4-amino-2,6-dichlorophenoxy)-2-ethyl-3,4-dihydroisoquinolin-1(2H)-one 5e

To a solution of 6-(2,6-dichloro-4-nitrophenoxy)-2-ethyl-3,4-dihydroisoquinolin-1(2*H*)-one **5d** (2.05 g, 5.38 mmol) in acetic acid (25 mL) was added iron powder (1.23 g, 21.6 mmol), and the mixture was reacted at 60°C for 4 hours. The reaction solution was cooled to room temperature, iron powder was removed, then water (50 mL) was added. The reaction mixture was stirred for 10 minutes, filtered, and rinsed with water (10 mL). The filter cake was collected and dried to obtain a yellow solid **5e** (1.80 g, yield 95%).

MS (ESI, pos. ion) *m*/*z*: 351.1 [M+H]⁺.

### Step 5: Synthesis of ethyl

### (2-cyano-2-(2-(3,5-dichloro-4-((2-ethyl-1-oxo-1,2,3,4-tetrahydroisoquinolin-6-yl)oxy)phenyl)hydrazono)acetyl)ca rbamate 5f

To a solution of 6-(4-amino-2,6-dichlorophenoxy)-2-ethyl-3,4-dihydroisoquinolin-1(2*H*)-one **5e** (0.80 g, 2.28 mmol) and *N*-cyanoacetylurethane (0.40 g, 2.50 mmol) in acetic acid (15 mL) was added a solution of sodium nitrite (0.24 g, 3.42 mmol) in water (2 mL) at 0°C, and the mixture was reacted for 3 hours. At 0°C, water (35 mL) was added to the reaction solution. The mixture was stirred for 10 minutes, filtered, washed with water (10 mL), and the filter cake was collected and dried to obtain a yellow solid **5f** (1.10 g, yield 93%).

MS (ESI, neg. ion) *m*/*z*: 515.9 [M-H]⁻.

### Step 6: Synthesis of

### 2-(3,5-dichloro-4-((2-ethyl-1-oxo-1,2,3,4-tetrahydroisoquinolin-6-yl)oxy)phenyl)-3,5-dioxo-2,3,4,5-tetrahydro-1, 2,4-triazine-6-carbonitrile 5

To a solution of ethyl (2-cyano-2-(2-(3,5-dichloro-4-((2-ethyl-1-oxo-1,2,3,4-tetrahydroisoquinolin-6-yl)oxy)phenyl)hydrazono)acetyl)ca rbamate **5f** (1.10 g, 2.12 mmol) in *N*,*N*-dimethylformamide (25 mL) was added sodium acetate (0.88 g, 12.9 mmol), and the mixture was reacted at 120°C for 6 hours. The reaction solution was cooled to room temperature. The reaction solution was poured into water (100 mL), stirred for 30 minutes, filtered, the filter cake was collected and dried, and the obtained pale yellow solid was separated and purified by pre-HPLC [50%ACN/50%H₂O (0.1% TFA), Kromasil Specifications: C18 10µm×50mm×250mm, flow rate: 100 mL/min] to obtain a white solid **5** (0.56 g, yield 54%, HPLC purity: 99.80%).
MS (ESI, neg. ion) *m*/*z*: 470.0 [M-H]⁻;
¹H NMR (400 MHz, DMSO-*d₆*) δ (ppm) 13.29 (s, 1H), 7.92-7.81 (m, 3H), 6.82 (dd, *J* = 13.1, 4.5 Hz, 2H), 3.55-3.50 (m, 2H), 3.50-3.45 (m, 2H), 2.95 (t, *J* = 6.4 Hz, 2H), 1.10 (t, *J* = 7.1 Hz, 3H).

### Example 6 Methyl

### 2-(3,5-dichloro-4-((2-methyl-1-oxo-1,2,3,4-tetrahydroisoquinolin-6-yl)oxy)phenyl)-3,5-dioxo-2,3,4,5-tetrahy dro-1,2,4-triazine-6-carboxylate 6

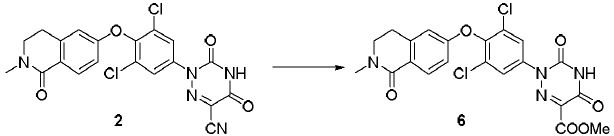

2-(3,5-Dichloro-4-((2-methyl-1-oxo-1,2,3,4-tetrahydroisoquinolin-6-yl)oxy)phenyl)-3,5-dioxo-2,3, 4,5-tetrahydro-1,2,4-triazine-6-carbonitrile **2** (0.30 g, 0.66 mmol) was dissolved in a methanol solution of hydrogen chloride (10 mL, 40 mmol, 4.0 mol/L). The mixture was reacted at 70°C for 48 hours. The reaction solution was concentrated. The resulting residue was purified by silica gel column chromatography (petroleum ether/ethyl acetate = 1/4) to obtain a light yellow solid **6** (35 mg, yield 11%, HPLC purity: 98.29%).
MS (ESI, pos. ion) *m*/*z*: 492.00 [M+H]⁺;
¹H NMR (400 MHz, DMSO-*d₆*) δ (ppm) 7.87 (d, *J* = 8.7 Hz, 3H), 6.87-6.76 (m, 2H), 4.13 (d, *J* = 5.0 Hz, 1H), 3.85 (s, 3H), 3.52 (t, *J* = 6.6 Hz, 2H), 3.17 (d, *J* = 4.1 Hz, 3H), 2.97 (t, *J* = 6.7 Hz, 2H).

### Example 7 2-(3,5-Dichloro-4-((1-oxo-2,3,4,5-tetrahydro-2H benzo[c]azepin-7-yl)oxy)phenyl)-3,5-dioxo-2,3,4,5-tetrahyd ro-1,2,4-triazine-6-carbonitrile 7

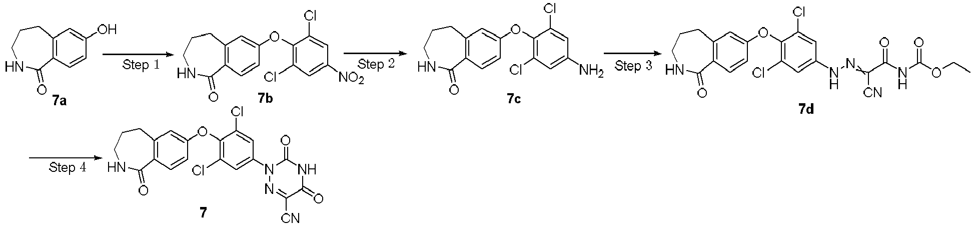

### Step 1: Synthesis of 7-(2,6-dichloro-4-nitrophenoxy)-2,3,4,5-tetrahydro-1H-benzo[c]azepin-1-one 7b

To a solution of 7-hydroxy-2,3,4,5-tetrahydro-1*H*-benzo[c]azepin-1-one **7a** (0.60 g, 3.4 mmol) and 1,2,3-trichloro-5-nitrobenzene (0.77 g, 3.4 mmol) in *N*,*N*-dimethylformamide (8 mL) was added potassium carbonate (1.2 g, 8.6 mmol), and the mixture was reacted at 120°C for 16 hours. The reaction solution was cooled to room temperature, then water (10 mL) was added. The reaction mixture was stirred for 15 minutes and filtered. The collected filter cake was slurried with ethanol/ethyl acetate/petroleum ether (1/2/4, 28 mL), and filtered to collect filter cake. After drying, a brown solid **7b** (0.90 g, yield 72%) was obtained.

### Step 2: Synthesis of 7-(4-amino-2,6-dichlorophenoxy)-2,3,4,5-tetrahydro-1H-benzo[c]azepin-1-one 7c

To a solution of 7-(2,6-dichloro-4-nitrophenoxy)-2,3,4,5-tetrahydro-1*H*-benzo[*c*]azepin-1-one **7b** (0.50 g, 1.4 mmol) in acetic acid (5 mL) was added iron powder (91 mg, 1.63 mmol), and the mixture was reacted at 70°C for 2 hours. The reaction solution was cooled to room temperature, iron powder was removed, then water (48 mL) was added. The reaction mixture was stirred for 10 minutes, filtered, and rinsed with water (10 mL). The solid was collected and dried to obtain a yellow solid **7c** (0.36 g, yield 78%).

### Step 3: Synthesis of ethyl

### (2-cyano-2-(2-(3,5-dichloro-4-((1-oxo-2,3,4,5-tetrahydro-1H-benzo[c]azepin-7-yl)oxy)phenyl)hydrazono)acetyl)c arbamate 7d

To a solution of 7-(4-amino-2,6-dichlorophenoxy)-2,3,4,5-tetrahydro-1*H*-benzo[c]azepin-1-one **7c** (0.26 g, 0.77 mmol) and *N*-cyanoacetylurethane (0.14 g, 0.88 mmol) in acetic acid (8 mL) was added a solution of sodium nitrite (0.11 g, 1.6 mmol) in water (4 mL) at 0°C, and the mixture was reacted for 2 hours. At 0°C, water (10 mL) was added to the reaction solution. The mixture was stirred for 10 minutes, filtered, washed with water (2 mL), and the filter cake was collected and dried to obtain a yellow solid **7d** (0.39 g, yield 100%).

### Step 4: Synthesis of

### 2-(3,5-dichloro-4-((1-oxo-2,3,4,5-tetrahydro-2H-benzo[c]azepin-7-yl)oxy)phenyl)-3,5-dioxo-2,3,4,5-tetrahydro-1, 2,4-triazine-6-carbonitrile 7

To a solution of ethyl (2-cyano-2-(2-(3,5-dichloro-4-((1-oxo-2,3,4,5-tetrahydro-1*H*-benzo[*c*]azepin-7-yl)oxy)phenyl)hydrazono)acetyl)c arbamate **7d** (0.39 g, 0.77 mmol) in *N*,*N*-dimethylformamide (4 mL) was added sodium acetate (70 mg, 0.85 mmol), and the mixture was reacted at 120°C for 5 hours. The reaction solution was cooled to room temperature, and water (20 mL) was added. The mixture was extracted with ethyl acetate (20 mL × 5). The combined organic layers were washed with saturated sodium chloride solution (10 mL), dried over anhydrous sodium sulfate, filtered and concentrated. The resulting residue was purified by silica gel column chromatography (100% ethyl acetate), and the obtained solid was recrystallized (ethanol/ethyl acetate/petroleum ether=1/5/6, 12 mL) to obtain a reddish-brown solid 7 (26 mg, yield 7.3%, HPLC purity: 82.24%).
MS (ESI, neg. ion) *m*/*z*: 456.5 [M-H]⁻;
¹H NMR (400 MHz, DMSO-*d₆*) δ (ppm) 7.99 (t, *J* = 5.7 Hz, 1H), 7.84 (s, 2H), 7.51 (d, *J* = 8.5 Hz, 1H), 6.87 (d, *J* = 2.4 Hz, 1H), 6.76 (dd, *J* = 8.5, 2.5 Hz, 1H), 2.97-2.89 (m, 2H), 2.74 (t, *J* = 6.9 Hz, 2H), 1.93-1.81 (m, 2H).

### Example 8

### 2-(3,5-dichloro-4-((2-oxo-1,2,3,4-tetrahydroquinolin-7-yl)oxy)phenyl)-3,5-dioxo-2,3,4,5-tetrahydro-1,2,4-tria zine-6-carbonitrile 8

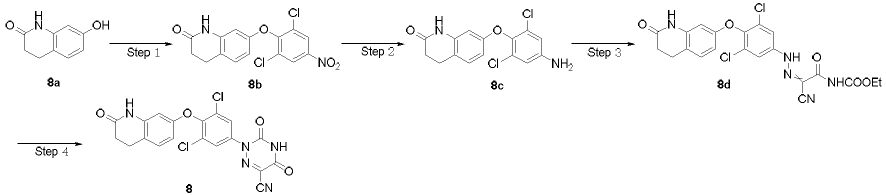

### Step 1: Synthesis of 7-(2,6-dichloro-4-nitrophenoxy)-3,4-dihydroquinolin-2(1H)-one 8b

To a solution of 7-hydroxy-3,4-dihydroquinolin-2(1*H*)-one **8a** (1.20 g, 7.35 mmol) in *N*,*N*-dimethylformamide (15 mL) were added 1,2,3-trichloro-5-nitrobenzene (1.67 g, 7.38 mmol) and potassium carbonate (2.46 g, 17.6 mmol), and the mixture was reacted at 80°C for 4 hours. The reaction solution was cooled to room temperature, then poured into water (50 mL). The reaction mixture was stirred for 30 minutes, filtered, and the filter cake was collected and dried to obtain a light yellow solid **8b** (2.46 g, yield 95%).

MS (ESI, neg. ion) *m*/*z*: 351.0 [M-H]⁻.

### Step 2: Synthesis of 7-(4-amino-2,6-dichlorophenoxy)-3,4-dihydroquinolin-2(1H)-one 8c

To a solution of 7-(2,6-dichloro-4-nitrophenoxy)-3,4-dihydroquinolin-2(1*H*)-one **8b** (2.00 g, 5.66 mmol) in acetic acid (40 mL) was added iron powder (1.29 g, 22.6 mmol), and the mixture was reacted at 60°C for 4.5 hours. The reaction solution was cooled to room temperature, iron powder was removed, then water (100 mL) was added dropwise. The reaction mixture was stirred for 30 minutes, and filtered. The filter cake was collected and dried to obtain a light gray solid **8c** (1.71 g, yield 93%).

MS (ESI, pos. ion) *m*/*z*: 323.0 [M+H]⁺.

### Step 3: Synthesis of ethyl

### (2-cyano-2-(2-(3,5-dichloro-4-((2-oxo-1,2,3,4-tetrahydroquinolin-7-yl)oxy)phenyl)hydrazono)acetyl)carbamate 8d

To a solution of 7-(4-amino-2,6-dichlorophenoxy)-3,4-dihydroquinolin-2(1*H*)-one **8c** (0.70 g, 2.17 mmol) and *N*-cyanoacetylurethane (0.38 g, 2.39 mmol) in acetic acid (10 mL) was added a solution of sodium nitrite (0.23 g, 3.25 mmol) in water (3 mL) at 0°C, and the mixture was reacted for 3 hours. At 0°C, water (20 mL) was added to the reaction solution. The mixture was stirred for 10 minutes, filtered, washed with water (4 mL), and the filter cake was collected and dried to obtain a yellow solid **8d** (0.88 g, yield 83%).

MS (ESI, neg. ion) *m*/*z*: 488.1 [M-H]⁻.

### Step 4: Synthesis of

### 2-(3,5-dichloro-4-((2-oxo-1,2,3,4-tetrahydroquinolin-7-yl)oxy)phenyl)-3,5-dioxo-2,3,4,5-tetrahydro-1,2,4-triazine -6-carbonitrile 8

To a solution of ethyl (2-cyano-2-(2-(3,5-dichloro-4-((2-oxo-1,2,3,4-tetrahydroquinolin-7-yl)oxy)phenyl)hydrazono)acetyl)carbamate **8d** (0.85 g, 1.73 mmol) in *N*,*N*-dimethylformamide (10 mL) was added sodium acetate (0.72 g, 8.65 mmol), and the mixture was reacted at 120°C for 6 hours. The reaction solution was cooled to room temperature, then water (40 mL) was added. The reaction mixture was stirred for 10 minutes, then hydrochloric acid (2 N, 3 mL) was added. The mixture was stirred for 10 minutes, filtered, and the filter cake was collected and dried to obtain a gray solid **8** (0.56 g, yield 73 %, HPLC purity: 89.58%).
MS (ESI, neg. ion) *m*/*z*: 442.1 [M-H]⁻;
¹H NMR (600 MHz, DMSO-*d₆*) δ (ppm) 13.29 (s, 1H), 9.98 (s, 1H), 7.82 (s, 2H), 7.14 (d, *J* = 8.3 Hz, 1H), 6.44 (dd, *J* = 8.2, 2.6 Hz, 1H), 6.40 (d, *J* = 2.5 Hz, 1H), 2.83 (t, *J* = 7.5 Hz, 2H), 2.45 (t, *J* = 7.5 Hz, 2H).

### Example 9 2-(3,5-Dichloro-4-((1-oxo-1,2,3,4-tetrahydroisoquinolin-7-yl)oxy)phenyl)-3,5-dioxo-2,3,4,5-tetrahydro-1,2,4-triazine-6-carbonitrile 9

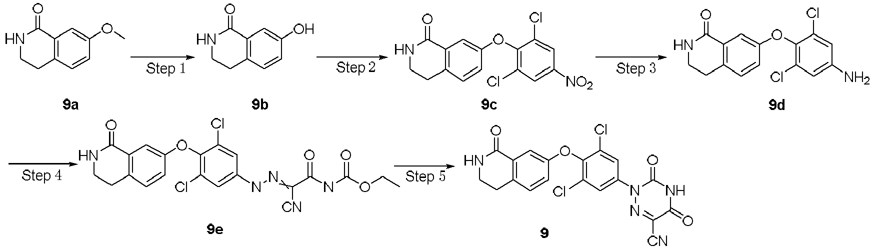

### Step 1: Synthesis of 7-hydroxy-3,4-dihydroisoquinolin-1(2H)-one 9b

At 0°C, to a solution of 7-methoxy-3,4-dihydroisoquinolin-1(2H)-one **9a** (5.0 g, 28 mmol) in dichloromethane (30 mL) was added boron tribromide (5.5 mL, 57 mmol) dropwise. The reaction was continued for 3.5 hours. The reaction solution was poured into ice water (40 mL) to quench the reaction. The mixture was stirred for 30 minutes, and filtered. The filter cake was washed with water (5 mL × 2) and dried. The obtained solid was recrystallized (ethanol/ethyl acetate/petroleum ether= 1/3/3, 42 mL) to give a brown solid **9b** (5.0 g, yield 100%).

### Step 2: Synthesis of 7-(2,6-dichloro-4-nitrophenoxy)-3,4-dihydroisoquinolin-1(2H)-one 9c

To a solution of 7-hydroxy-3,4-dihydroisoquinolin-1(2*H*)-one **9b** (1.5 g, 9.2 mmol) and 1,2,3-trichloro-5-nitro-benzene (2.3 g, 10 mmol) in *N*,*N*-dimethylformamide (40 mL) was added potassium carbonate (2.6 g, 19 mmol), and the mixture was reacted at 80°C for 4 hours. The reaction solution was cooled to room temperature, then water (80 mL) was added. The reaction mixture was stirred for 15 minutes and filtered. The filter cake was collected to obtain a yellow solid. The collected filter cake was slurried with ethanol/ethyl acetate/petroleum ether (1/3/6, 50 mL), and filtered to collect filter cake. After drying, a gray solid **9c** (2.4 g, yield 74%) was obtained.

### Step 3: Synthesis of 7-(4-amino-2,6-dichlorophenoxy)-3,4-dihydroisoquinolin-1(2H)-one 9d

To a solution of 7-(2,6-dichloro-4-nitrophenoxy)-3,4-dihydroisoquinolin-1(2*H*)-one **9c** (2.4 g, 6.8 mmol) in acetic acid (15 mL) was added iron powder (1.5 g, 3.9 mmol), and the mixture was reacted at 70°C for 3 hours. The reaction solution was cooled to room temperature, iron powder was removed, then water (30 mL) was added. The reaction mixture was stirred for 10 minutes, filtered, and rinsed with water (10 mL). The solid was collected and dried to obtain a white solid **9d** (1.00 g, yield 46%).

### Step 4: Synthesis of ethyl

### (2-cyano-2-(2-(3,5-dichloro-4-((1-oxo-1,2,3,4-tetrahydroisoquinolin-7-yl)oxy)phenyl)hvdrazono)acetyl)carbamate 9e

To a solution of 7-(4-amino-2,6-dichlorophenoxy)-3,4-dihydroisoquinolin-1(2*H*)-one **9d** (0.20 g, 0.62 mmol) and *N*-cyanoacetylurethane (0.11 g, 0.69 mmol) in acetic acid (4 mL) was added a solution of sodium nitrite (65 mg, 0.93 mmol) in water (2 mL) at 0°C, and the mixture was reacted for 3.5 hours. At 0°C, water (10 mL) was added to the reaction solution. The mixture was stirred for 10 minutes, filtered, washed with water (2 mL), and the filter cake was collected and dried to obtain a yellow solid **9e** (0.30 g, yield 99%).

### Step 5: Synthesis of

### 2-(3,5-dichloro-4-((1-oxo-1,2,3,4-tetrahydroisoquinolin-7-yl)oxy)phenyl)-3,5-dioxo-2,3,4,5-tetrahydro-1,2,4-triazi ne-6-carbonitrile 9

To a solution of ethyl (2-cyano-2-(2-(3,5-dichloro-4-((1-oxo-1,2,3,4-tetrahydroisoquinolin-7-yl)oxy)phenyl)hydrazono)acetyl)carbamate **9e** (0.30 g, 0.61 mmol) in *N*,*N*-dimethylformamide (4 mL) was added sodium acetate (55 mg, 0.67 mmol), and the mixture was reacted at 120°C for 5 hours. The reaction solution was cooled to room temperature, and water (30 mL) was added. The mixture was extracted with ethyl acetate (100 mL × 3). The combined organic layers were washed with saturated sodium chloride solution (20 mL × 2), dried over anhydrous sodium sulfate, filtered and concentrated. The resulting residue was recrystallized (ethanol/ethyl acetate/petroleum ether=1/7/11, 19 mL) to obtain an off-white solid 9 (0.18 g, yield 66%, HPLC purity: 98.20%).
MS (ESI, neg. ion) *m*/*z*: 442.0 [M-H]⁻;
¹H NMR (600 MHz, DMSO-*d₆*) δ (ppm) 13.30 (s, 1H), 8.06 (s, 1H), 7.85 (s, 2H), 7.35 (d, *J* = 8.4 Hz, 1H), 7.16 (dd, *J* = 8.3, 2.8 Hz, 1H), 7.09 (d, *J* = 2.8 Hz, 1H), 3.36 (d, *J* = 2.3 Hz, 2H), 2.87 (t, *J* = 6.5 Hz, 2H).

### Example 10

### 2-(3,5-Dichloro-4-((2-(2-methoxyethyl)-1-oxo-1,2,3,4-tetrahydroisoquinolin-6-yl)oxy)phenyl)-3,5-dioxo-2,3,4 ,5-tetrahydro-1,2,4-triazine-6-carbonitrile 10

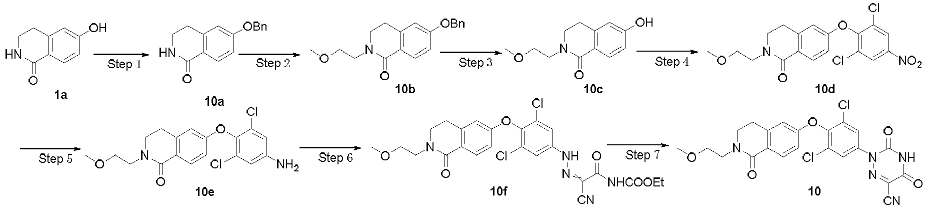

### Step 1: Synthesis of 6-(benzyloxy)-3,4-dihydroisoquinolin-1(2H)-one 10a

To a solution of 6-hydroxy-3,4-dihydroisoquinolin-1(2*H*)-one **1a** (5.0 g, 31 mmol) in *N*,*N*-dimethylformamide (50 mL) was added potassium carbonate (6.4 g, 46 mmol). The mixture was reacted at room temperature for 15 minutes, benzyl bromide (4.4 mL, 37 mmol) was added dropwise, and the mixture was reacted at room temperature for 48 hours. Water (100 mL) was added to quench the reaction. The reaction mixture was stirred for 20 minutes, and filtered. The solid was collected and dried to obtain a white solid **10a** (6.2 g, yield 80%).

### Step 2: Synthesis of 6-(benzyloxy)-2-(2-methoxyethyl)-3,4-dihydroisoquinolin-1(2H)-one 10b

6-(Benzyloxy)-3,4-dihydroisoquinolin-1(2*H*)-one **10a** (3.0 g, 12 mmol) was dissolved in a mixture solution of THF (24 mL) and *N*,*N*-dimethylformamide (6 mL). Sodium hydride (1.4 g, 35 mmol, 60% in oil) was added. The mixture was reacted at room temperature for 15 minutes. Then 1-bromo-2-methoxy-ethane (1.7 mL, 18 mmol) was added dropwise, and the mixture was reacted at 60°C for 4 hours. The reaction was quenched by adding water (30 mL). The mixture was extracted with ethyl acetate (50 mL × 2). The combined organic layers were washed with saturated sodium chloride (20 mL × 3), dried over anhydrous sodium sulfate, and concentrated by suction filtration to give yellow oil **10b** (3.7 g, 100% yield).

### Step 3: Synthesis of 6-hydroxy-2-(2-methoxyethyl)-3,4-dihydroisoquinolin-1(2H)-one 10c

6-(Benzyloxy)-2-(2-methoxyethyl)-3,4-dihydroisoquinolin-1(2*H*)-one 10b (3.7 g, 12 mmol) was dissolved in ethanol (30 mL), and 10% palladium carbon (0.37 g) was added. The reaction mixture was degassed and refilled with hydrogen (3 MPa), and hydrogenation was carried out for 16 hours. The reaction solution was filtered, and the filtrate was collected and concentrated to obtain a yellow solid **10c** (2.6 g, yield 99%).

### Step 4: Synthesis of 6-(2,6-dichloro-4-nitrophenoxy)-2-(2-methoxyethyl)-3,4-dihydroisoquinolin-1(2H)-one 10d

To a solution of 6-hydroxy-2-(2-methoxyethyl)-3,4-dihydroisoquinolin-1(2*H*)-one **10c** (0.50 g, 2.3 mmol) and 1,2,3-trichloro-5-nitro-benzene (0.56 g, 2.5 mmol) in *N*,*N*-dimethylformamide (5 mL) was added potassium carbonate (0.63 g, 4.5 mmol), and the mixture was reacted at 70°C for 3 hours. The reaction solution was cooled to room temperature, then water (10 mL) was added. The reaction mixture was stirred for 15 minutes and filtered to collect filter cake. The filter cake was recrystallized (ethyl acetate/petroleum ether = 3/5, 12 mL) to obtain a yellow solid **10d** (0.49 g, yield 53%).

### Step 5: Synthesis of

### 6-(4-amino-2,6-dichlorophenoxy)-2-(2-methoxyethyl)-3,4-dihydroisoquinolin-1(2H)-one 10e

To a solution of 6-(2,6-dichloro-4-nitrophenoxy)-2-(2-methoxyethyl)-3,4-dihydroisoquinolin-1(2*H*)-one **10d** (0.40 g, 0.97 mmol) in acetic acid (4 mL) was added iron powder (0.14 g, 2.48 mmol), and the mixture was reacted at 60°C for 5 hours. The reaction solution was cooled to room temperature, then water (12 mL) was added to quench the reaction. The reaction mixture was stirred for 10 minutes, filtered, and rinsed with water (20 mL). The solid was collected and dried to obtain an off-white solid **10e** (0.33 g, yield 89%).

### Step 6: Synthesis of ethyl

### (2-cyano-2-(2-(3,5-dichloro-4-((2-(2-methoxyethyl)-1-oxo-1,2,3,4-tetrahydroisoquinolin-6-yl)oxy)phenyl)hydrazo no)acetyl)carbamate 10f

To a solution of 6-(4-amino-2,6-dichlorophenoxy)-2-(2-methoxyethyl)-3,4-dihydroisoquinolin-1(2*H*)-one **10e** (0.33 g, 0.87 mmol) in acetic acid (6.6 mL) was added a solution of sodium nitrite (90 mg, 1.29 mmol) in water (3.3 mL) at 0°C. The mixture was reacted for 10 minutes, then N-cyanoacetylurethane (0.17 g, 1.1 mmol) was added, and the reaction was continued at 0°C for 1 hour. At 0°C, water (12 mL) was added to the reaction solution. The mixture was stirred for 10 minutes, filtered, washed with water (10 mL), and the filter cake was collected and dried to obtain a yellow solid **10f** (0.47 g, yield 99%).

### Step 7: Synthesis of

### 2-(3,5-dichloro-4-((2-(2-methoxyethyl)-1-oxo-1,2,3,4-tetrahydroisoquinolin-6-yl)oxy)phenyl)-3,5-dioxo-2,3,4,5-t etrahydro-1,2,4-triazine-6-carbonitrile 10

To a solution of ethyl (2-cyano-2-(2-(3,5-dichloro-4-((2-(2-methoxyethyl)-1-oxo-1,2,3,4-tetrahydroisoquinolin-6-yl)oxy)phenyl)hydrazo no)acetyl)carbamate **10f** (0.47 g, 0.86 mmol) in *N*,*N*-dimethylformamide (3 mL) was added sodium acetate (0.77 g, 0.94 mmol), and the mixture was reacted at 120°C. The reaction solution was cooled to room temperature, then water (10 mL) was added. The reaction mixture was stirred for 10 minutes and filtered. The collected solid was recrystallized (ethanol/ethyl acetate/petroleum ether = 7.5/20/10, 37.5 mL), and filtered to collect filter cake. After drying, an off-white solid **10** (0.23 g, yield 53%, HPLC purity:96.55%) was obtained.
MS (ESI, neg. ion) *m*/*z*: 500.1 [M-H]⁻;
¹H NMR (400 MHz, DMSO-*d₆*) δ (ppm) 13.28 (s, 1H), 7.86 (d, *J* = 12.5 Hz, 3H), 6.82 (d, *J* = 10.8 Hz, 2H), 3.61 (d, *J* = 5.3 Hz, 2H), 3.59-3.54 (m, 2H), 3.52-3.49 (m, 2H), 3.26 (s, 3H), 2.93 (t, *J* = 5.7 Hz, 2H).

### Example 11 Methyl

### 2-(3,5-dichloro-4-((1-oxo-1,2,3,4-tetrahydroisoquinolin-6-yl)oxy)phenyl)-3,5-dioxo-2,3,4,5-tetrahydro-1,2,4-t riazine-6-carboxylate 11

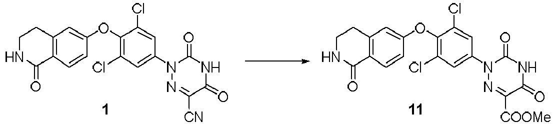

To 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.10 g, 0.23 mmol) was added a solution of hydrogen chloride in methanol (5 mL, 4.5 mol/L). The mixture was reacted at 70°C for 24 hours. The reaction solution was cooled to room temperature and concentrated. The resulting residue was purified by silica gel column chromatography (100% ethyl acetate) to obtain a white solid **11** (45 mg, yield 42%, HPLC purity: 95.69%).
MS (ESI, pos. ion) *m*/*z*: 477.0 [M+H]⁺;
¹H NMR (400 MHz, DMSO-*d₆*) δ (ppm) 12.79 (s, 1H), 7.86 (s, 3H), 7.84 (s, 1H), 6.87 (d, *J* = 2.2 Hz, 1H), 6.81 (dd, *J* = 8.6, 2.5 Hz, 1H), 3.85 (s, 3H), 3.45 (d, *J* = 9.4 Hz, 2H), 2.90 (t, *J* = 6.4 Hz, 2H).

### Example 12

### 2-(3,5-Dichloro-4-((1-oxo-2-((tetrahydro-2H-pyran-4-yl)methyl)-1,2,3,4-tetrahydroisoquinolin-6-yl)oxy)phe nyl)-3,5-dioxo-2,3,4,5-tetrahydro-1,2,4-triazine-6-carbonitrile 12

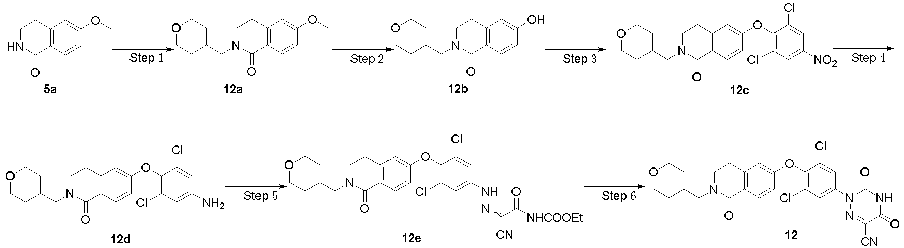

### Step 1: Synthesis of 6-methoxy-2-((tetrahydro-2H-pyran-4-yl)methyl)-3,4-dihydroisoquinolin-1(2H)-one 12a

6-Methoxy-3,4-dihydroisoquinolin-1(2*H*)-one **5a** (3.0 g, 16.9 mmol) was dissolved in a mixture solution of THF (30 mL) and *N*,*N*-dimethylacetamide (15 mL). Sodium hydride (0.85 g, 21.2 mmol, 60% in oil) was added in portions at 0°C, then bromomethylpyran (1.13 mL, 13.8 mmol) was added dropwise. After the addition was complete, the reaction was continued at room temperature for 24 hours. The reaction was quenched with water (30 mL). The mixture was extracted with ethyl acetate (40 mL × 3). The combined organic layers were washed with saturated sodium chloride (40 mL × 3), dried over anhydrous sodium sulfate and concentrated by suction filtration. The residue was purified by silica gel column chromatography (petroleum ether/ethyl acetate = 5/1) to give light yellow oil **12a** (4.35 g, yield 93%).

MS (ESI, pos. ion) *m*/*z*: 276.2 [M+H]⁺.

### Step 2: Synthesis of 6-hydroxy-2-((tetrahydro-2H-pyran-4-yl)methyl)-3,4-dihydroisoquinolin-1(2H)-one 12b

6-Methoxy-2-((tetrahydro-2*H*-pyran-4-yl)methyl)-3,4-dihydroisoquinolin-1(2*H*)-one **12a** (0.81 g, 2.94 mmol) was dissolved in dichloromethane (20 mL). Boron tribromide (0.57 mL, 5.89 mmol) was slowly added dropwise at 0°C and the mixture was reacted at 0°C for 2.5 hours. The reaction was quenched by adding methanol (5 mL) at 0°C. The mixture was concentrated, and water (50 mL) was added. The mixture was extracted with ethyl acetate (40 mL × 3). The combined organic layers were washed with saturated sodium chloride (40 mL × 2), dried over anhydrous sodium sulfate and concentrated by suction filtration. The obtained residue was purified by silica gel column chromatography (petroleum ether/ethyl acetate = 2/1) to give a light yellow solid **12b** (0.42 g, yield 55%).

### Step 3: Synthesis of

### 6-(2,6-dichloro-4-nitrophenoxy)-2-((tetrahydro-2H-pyran-4-yl)methyl)-3,4-dihydroisoguinolin-1(2H)-one 12c

To a solution of 6-hydroxy-2-((tetrahydro-2*H*-pyran-4-yl)methyl)-3,4-dihydroisoquinolin-1(2*H*)-one **12b** (0.33 g, 1.3 mmol) in *N*,*N*-dimethylformamide (10 mL) were added 1,2,3-trichloro-5-nitrobenzene (0.39 g, 1.7 mmol) and potassium carbonate (0.55 g, 3.9 mmol), and the mixture was reacted at 80°C for 5 hours. The reaction solution was cooled to room temperature, and water (15 mL) was added. The mixture was extracted with ethyl acetate (15 mL × 3). The combined organic layers were washed with saturated sodium chloride (15 mL × 3), dried over anhydrous sodium sulfate and concentrated by suction filtration. The obtained residue was purified by silica gel column chromatography (petroleum ether/ethyl acetate = 2/1) to give a yellow solid **12c** (0.31 g, yield 54%).

### Step 4: Synthesis of

### 6-(4-amino-2,6-dichlorophenoxy)-2-((tetrahydro-2H-pyran-4-yl)methyl)-3,4-dihydroisoquinolin-1(2H)-one 12d

To a solution of 6-(2,6-dichloro-4-nitrophenoxy)-2-((tetrahydro-2*H*-pyran-4-yl)methyl)-3,4-dihydroisoquinolin-1(2*H*)-one **12c** (0.31 g, 0.69 mmol) in acetic acid (10 mL) was added iron powder (0.19 g, 3.3 mmol), and the mixture was reacted at 60°C for 3.5 hours. The reaction solution was cooled to room temperature, iron powder was removed, and water (10 mL) was added. The mixture was extracted with ethyl acetate (15 mL × 3). The combined organic layers were washed with saturated sodium chloride (15 mL × 3), dried over anhydrous sodium sulfate and concentrated by suction filtration. The residue was purified by silica gel column chromatography (petroleum ether/ethyl acetate = 1/1) to give a light yellow solid **12d** (0.18 g, yield 62%).

MS (ESI, pos. ion) *m*/*z*: 421.1 [M+H]⁺.

### Step 5: Synthesis of ethyl

### (2-cyano-2-(2-(3,5-dichloro-4-((1-oxo-2-((tetrahydro-2H-pyran-4-yl)methyl)-1,2,3,4-tetrahydroisoguinolin-6-yl)o xy)phenyl)hydrazono)acetyl)carbamate 12e

To a solution of 6-(4-amino-2,6-dichlorophenoxy)-2-((tetrahydro-2*H*-pyran-4-yl)methyl)-3,4-dihydroisoquinolin-1(2*H*)-one **12d** (0.18 g, 0.43 mmol) in acetic acid (8 mL) was slowly added a solution of sodium nitrite (46 mg, 0.65 mmol) in water (0.5 mL) dropwise at 0°C. The mixture was reacted for 20 minutes, then N-cyano-acetylurethane (75 mg, 0.47 mmol) was added, and the reaction was continued for 4.5 hours. Water (10 mL) was added to the reaction solution. The mixture was extracted with ethyl acetate (15 mL × 3). The combined organic layers were washed with saturated sodium chloride (15 mL × 3), dried over anhydrous sodium sulfate, and concentrated by suction filtration to give a yellow solid **12e** (0.25 g, yield 99%).

MS (ESI, neg. ion) *m*/*z*: 586.1 [M-H]⁻.

### Step 6: Synthesis of

### 2-(3,5-dichloro-4-((1-oxo-2-((tetrahydro-2H-pyran-4-yl)methyl)-1,2,3,4-tetrahydroisoquinolin-6-yl)oxy)phenyl)-3 ,5-dioxo-2,3,4,5-tetrahydro-1,2,4-triazine-6-carbonitrile 12

To a solution of ethyl (2-cyano-2-(2-(3,5-dichloro-4-((1-oxo-2-((tetrahydro-2*H*-pyran-4-yl)methyl)-1,2,3,4-tetrahydroisoquinolin-6-yl)o xy)phenyl)hydrazono)acetyl)carbamate **12e** (0.25 g, 0.43 mmol) in *N*,*N*-dimethylformamide (8 mL) was added sodium acetate (0.18 g, 2.2 mmol), and the mixture was reacted at 120°C for 4 hours. The reaction solution was cooled to room temperature, and water (15 mL) was added. The mixture was extracted with ethyl acetate (15 mL × 3). The combined organic layers were washed with saturated sodium chloride solution (15 mL × 3), dried over anhydrous sodium sulfate and concentrated by suction filtration. The resulting residue was purified by silica gel column chromatography (petroleum ether/ethyl acetate = 2/1), and the obtained solid was recrystallized (petroleum ether/ethyl acetate = 3/1, 5 mL) to obtain a white solid **12** (0.21 g, yield 89%, HPLC purity: 97.04%).

MS (ESI, neg. ion) *m*/*z*: 540.1 [M-H]⁻.

¹H NMR (400 MHz, DMSO-*d₆*) δ (ppm) 13.29 (s, 1H), 7.88-7.80 (m, 3H), 6.85-6.78 (m, 2H), 3.86-3.80 (m, 2H), 3.53 (t, *J* = 6.4 Hz, 2H), 3.35 (s, 2H), 3.25 (t, *J* = 11.2 Hz, 2H), 2.95 (t, *J* = 6.3 Hz, 2H), 1.95-1.85 (m, 1H), 1.53 (d, *J* = 11.6 Hz, 2H), 1.21 (ddd, *J* = 16.5, 12.4, 5.4 Hz, 2H).

### Example 13

### 2-(3,5-Dichloro-4-((2-(isopropoxymethyl)-1-oxo-1,2,3,4-tetrahydroisoquinolin-6-yl)oxy)phenyl)-3,5-dioxo-2, 3,4,5-tetrahydro-1,2,4-triazine-6-carbonitrile 13

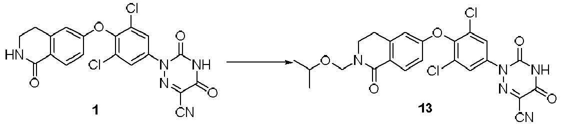

To a solution of 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.30 g, 0.68 mmol) in isopropanol (10 mL) was added 37% formaldehyde (1.5 mL, 20 mmol). The mixture was sealed and reacted at 100°C for 24 hours. The reaction solution was cooled to room temperature, and concentrated. The resulting residue was purified by silica gel column chromatography (petroleum ether/ethyl acetate = 1/1), and the obtained solid was recrystallized (ethyl acetate/petroleum ether = 1/2, 6 mL) to obtain a white solid **13** (85 mg, yield 24%, HPLC purity: 99.85%).
MS (ESI, neg. ion) *m*/*z*: 514.1 [M-H]⁻;
¹H NMR (400 MHz, DMSO-*d₆*) δ (ppm) 13.30 (s, 1H), 7.97-7.79 (m, 3H), 6.87 (dd, *J* = 17.0, 4.9 Hz, 2H), 4.92 (s, 2H), 3.67 (dt, *J* = 12.2, 6.1 Hz, 1H), 3.56 (t, *J* = 6.5 Hz, 2H), 2.97 (t, *J* = 6.3 Hz, 2H), 1.10 (d, *J* = 6.1 Hz, 6H).

### Example 14

### 2-(3,5-Dichloro-4-((1-oxo-2-((2,2,2-trifluoroethoxy)methyl)-1,2,3,4-tetrahydroisoquinolin-6-yl)oxy)phenyl)-3 ,5-dioxo-2,3,4,5-tetrahydro-1,2,4-triazine-6-carbonitrile 14

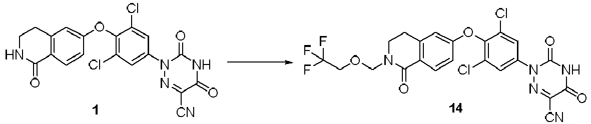

To a solution of 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.48 g, 1.08 mmol) in trifluoroethanol (8 mL) was added 37% formaldehyde (1.60 mL, 22 mmol). The mixture was sealed and reacted at 100°C for 24 hours. The reaction solution was cooled to room temperature, and concentrated. The resulting residue was purified by silica gel column chromatography (petroleum ether/ethyl acetate = 1/2), and the obtained solid was recrystallized (ethyl acetate/petroleum ether = 1/2, 6 mL) to obtain a white solid **14** (0.23 g, yield 38%, HPLC purity: 96.05%).
MS (ESI, neg. ion) *m*/*z*: 554.0 [M-H]⁻;
¹H NMR (400 MHz, DMSO-*d₆*) δ (ppm) 13.30 (s, 1H), 7.93 (d, *J* = 8.6 Hz, 1H), 7.90-7.79 (m, 2H), 6.93-6.83 (m, 2H), 5.07 (s, 2H), 4.12 (q, *J* = 9.4 Hz, 2H), 3.63 (t, *J* = 6.4 Hz, 2H), 3.01 (t, *J* = 6.4 Hz, 2H);
¹⁹F NMR (376 MHz, DMSO-*d₆*) δ (ppm) -73.04.

### Example 15

### 2-(3,5-dichloro-4-((1-oxo-2-((2,2-difluoroethoxy)methyl)-1,2,3,4-tetrahydroisoquinolin-6-yl)oxy)phenyl)-3,5-dioxo-2,3,4,5-tetrahydro-1,2,4-triazine-6-carbonitrile 15

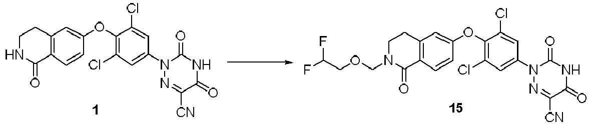

To a solution of 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.30 g, 0.68 mmol) in 2,2-difluoroethanol (6 mL) was added 37% formaldehyde (1.0 mL, 14 mmol). The mixture was sealed and reacted at 100°C for 24 hours. The reaction solution was cooled to room temperature, and concentrated. The resulting residue was purified by silica gel column chromatography (petroleum ether/ethyl acetate = 1/2), and the obtained solid was recrystallized (ethyl acetate/petroleum ether = 1/2, 6 mL) to obtain a white solid **15** (85 mg, yield 23%, HPLC purity: 97.53%).
MS (ESI, neg. ion) *m*/*z*: 536.1 [M-H]⁻;
¹H NMR (400 MHz, DMSO-*d₆*) δ (ppm) 13.31 (s, 1H), 7.89 (dd, *J* = 27.8, 8.3 Hz, 3H), 6.95-6.78 (m, 2H), 6.14 (t, *J* = 55.1 Hz, 1H), 5.01 (s, 2H), 3.74 (t, *J* = 14.8 Hz, 2H), 3.61 (s, 2H), 3.00 (s, 2H);
¹⁹F NMR (376 MHz, DMSO-*d₆*) δ (ppm) -125.56.

### Example 16

### 2-(3,5-dichloro-4-((2-(1-ethoxyethyl)-1-oxo-1,2,3,4-tetrahydroisoquinolin-6-yl)oxy)phenyl)-3,5-dioxo-2,3,4,5-tetrahydro-1,2,4-triazine-6-carbonitrile 16

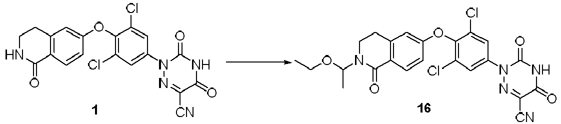

To a solution of 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.20 g, 0.45 mmol) in ethanol (4 mL) was added acetaldehyde (0.8 mL, 14 mmol). The mixture was sealed and reacted at 90°C for 48 hours. The reaction solution was cooled to room temperature, and concentrated. The resulting residue was purified by silica gel column chromatography (petroleum ether/ethyl acetate = 1/2), and the obtained solid was recrystallized (ethanol/ethyl acetate/petroleum ether = 1/7/10, 18 mL) to obtain a yellow solid **16** (60 mg, yield 26%, HPLC purity: 87.08%).
MS (ESI, neg. ion) *m*/*z:* 514.1 [M-H]⁻;
¹H NMR (400 MHz, DMSO-*d₆*) δ (ppm) 13.30 (s, 1H), 7.90 (d, *J* = 8.6 Hz, 1H), 7.85 (s, 2H), 6.93-6.78 (m, 2H), 5.89 (q, *J* = 6.0 Hz, 1H), 3.41 (s, 2H), 3.39 (d, *J* = 7.0 Hz, 2H), 2.95 (t, *J* = 6.5 Hz, 2H), 1.26 (d, *J* = 6.1 Hz, 3H), 1.11 (t, *J* = 7.0 Hz, 3H).

### Example 17

### 2-(3,5-Dichloro-4-((1-oxo-1,2,3,4-tetrahydroisoquinolin-6-yl)oxy)phenyl)-1,2,4-triazine-3,5(2H,4H)-dione 17

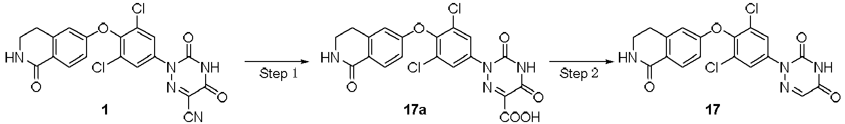

### Step 1: Synthesis of

### 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carboxylic acid 17a

To a solution of 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.20 g, 0.45 mmol) in acetic acid (4 mL) was added concentrated hydrochloric acid (1.5 mL). The mixture was reacted at 120°C for 16 hours. The reaction solution was cooled to room temperature, then water (6 mL) was added. The reaction mixture was stirred for 5 minutes, then filtered, and the filter cake was collected and dried to obtain a yellow solid **17a** (90 mg, yield 87%).

MS (ESI, neg. ion) *m*/*z*: 463.0 [M-H]⁻.

### Step 2: Synthesis of

### 2-(3,5-dichloro-4-((1-oxo-1,2,3,4-tetrahydroisoquinolin-6-yl)oxy)phenyl)-1,2,4-triazine-3,5(2H,4H)-dione 17

2-(3,5-Dichloro-4-((1-oxo-1,2,3,4-tetrahydroisoquinolin-6-yl)oxy)phenyl)-3,5-dioxo-2,3,4,5-tetrah ydro-1,2,4-triazine-6-carboxylic acid **17a** (175 mg, 0.38 mmol) was dissolved in thioglycolic acid (2.5 mL). The mixture was reacted at 140°C for 17 hours. The reaction solution was cooled to room temperature, and water (20 mL) was added to quench the reaction. The mixture was extracted with ethyl acetate (20 mL × 2). The combined organic layers were washed with saturated sodium chloride (20 mL), dried over anhydrous sodium sulfate and concentrated by suction filtration. The obtained residue was purified by silica gel column chromatography (100% ethyl acetate) to give a light red solid **17** (63 mg, yield 40%, HPLC purity: 95.58%).
MS (ESI, neg. ion) *m*/*z*: 418.0[M-H]⁻;
¹H NMR (400 MHz, DMSO-*d₆*) δ (ppm) 12.52 (s, 1H), 7.85 (d, *J* = 10.5 Hz, 4H), 7.73 (s, 1H), 6.85 (d, *J* = 2.6 Hz, 1H), 6.78 (dd, *J* = 8.6, 2.6 Hz, 1H), 3.57- 3.38 (m, 2H), 2.89 (t, *J* = 6.6 Hz, 2H).

### Example 18

### 2-(4-((2-Benzyl-1-oxo-1,2,3,4-tetrahydroisoquinolin-6-yl)oxy)-3,5-dichlorophenyl)-3,5-dioxo-2,3,4,5-tetrahy dro-1,2,4-triazine-6-carbonitrile 18

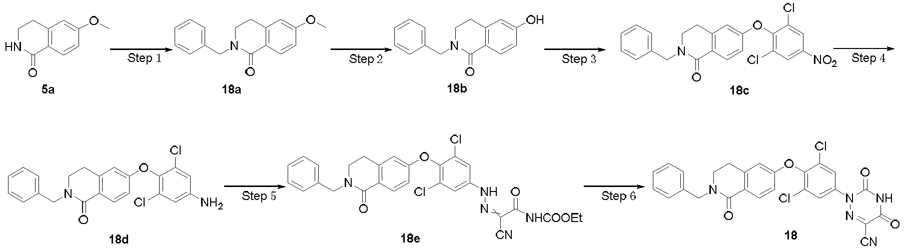

### Step 1: Synthesis of 2-benzyl-6-methoxy-3,4-dihydroisoquinolin-1(2H)-one 18a

At 0°C, to a mixed solution of sodium hydride (0.81 g, 20 mmol, 60% in oil) in *N*,*N*-dimethylformamide (24 mL) and tetrahydrofuran (18 mL) was added 6-methoxy-3,4-dihydroisoquinolin-1(2*H*)-one **5a** (3.0 g, 17 mmol). Benzyl bromide (2.2 mL, 19 mmol) was added dropwise. The mixture was reacted at room temperature for 6 hours. The reaction was quenched by adding water (120 mL). The mixture was extracted with ethyl acetate (200 mL × 2). The combined organic layers were washed with saturated sodium chloride (50 mL × 2), dried over anhydrous sodium sulfate, and concentrated by suction filtration to give light yellow oil **18a** (4.51 g, 100% yield).

### Step 2: Synthesis of 2-benzyl-6-hydroxy-3,4-dihydroisoquinolin-1(2H)-one 18b

At 0°C, to a solution of 2-benzyl-6-methoxy-3,4-dihydroisoquinolin-1(2*H*)-one **18a** (4.5 g, 17 mmol) in dichloromethane (36 mL) was added boron tribromide (3.2 mL, 34 mmol) dropwise. Then the mixture was reacted at room temperature for 5 hours. The reaction solution was quenched by pouring into ice water (100 mL). The mixture was stirred for 10 minutes, then filtered, and the filter cake was washed with water (20 mL × 2), and the filter cake was collected and dried to obtain a white solid **18b** (3.8 g, yield 89%).

### Step 3: Synthesis of 2-benzyl-6-(2,6-dichloro-4-nitrophenoxy)-3,4-dihydroisoquinolin-1(2H)-one 18c

To a solution of 2-benzyl-6-hydroxy-3,4-dihydroisoquinolin-1(2*H*)-one **18b** (3.80 g, 15.0 mmol) and 1,2,3-trichloro-5-nitrobenzene (3.74 g, 16.5 mmol) in *N*,*N*-dimethylformamide (23 mL) was added potassium carbonate (3.14 g, 22.5 mmol), and the mixture was reacted at 70°C for 7 hours. The reaction solution was cooled to room temperature, then water (50 mL) was added. The reaction mixture was stirred for 10 minutes and filtered. The filter cake was washed with water (20 mL × 2). The collected filter cake was recrystallized (ethyl acetate/petroleum ether = 1/2, 30 mL) to obtain an off-white solid **18c** (6.10 g, yield 92%).

### Step 4: Synthesis of 6-(4-amino-2,6-dichlorophenoxy)-2-benzyl-3,4-dihydroisoquinolin-1(2H)-one 18d

To a solution of 2-benzyl-6-(2,6-dichloro-4-nitrophenoxy)-3,4-dihydroisoquinolin-1(2*H*)-one **18c** (3.0 g, 6.8 mmol) in acetic acid (18 mL) was added iron powder (1.1 g, 20 mmol), and the mixture was reacted at 60°C for 3 hours. The reaction solution was cooled to room temperature, then water (40 mL) was added. The reaction mixture was stirred for 10 minutes and filtered. The filter cake was washed with water (20 mL × 2). The collected filter cake was recrystallized (ethyl acetate/petroleum ether = 2/1, 15 mL) to obtain a brown solid **18d** (1.1 g, yield 39%).

### Step 5: Synthesis of ethyl

### (2-(2-(4-((2-benzyl-1-oxo-1,2,3,4-tetrahydroisoquinolin-6-yl)oxy)-3,5-dichlorophenyl)hydrazono)-2-cyanoacetyl) carbamate 18e

To a solution of 6-(4-amino-2,6-dichlorophenoxy)-2-benzyl-3,4-dihydroisoquinolin-1(2*H*)-one **18d** (1.0 g, 2.4 mmol) and *N*-(2-cyanoacetyl) carbamate (0.45 g, 2.9 mmol) in acetic acid (20 mL) was added a solution of sodium nitrite (0.33 g, 4.8 mmol) in water (10 mL), and the mixture was continued to react at 0°C for 2 hours. At 0°C, water (30 mL) was added to the reaction solution. The mixture was stirred for 10 minutes, then filtered, and the filter cake was washed with water (10 mL). Then the filter cake was collected and dried to obtain a yellow solid **18e** (0.60 g, yield 43%).

### Step 6: Synthesis of

### 2-(4-((2-benzyl-1-oxo-1,2,3,4-tetrahydroisoquinolin-6-yl)oxy)-3,5-dichlorophenyl)-3,5-dioxo-2,3,4,5-tetrahydro-1 ,2,4-triazine-6-carbonitrile 18

To a solution of ethyl (2-(2-(4-((2-benzyl-1-oxo-1,2,3,4-tetrahydroisoquinolin-6-yl)oxy)-3,5-dichlorophenyl)hydrazono)-2-cyanoacetyl) carbamate **18e** (0.70 g, 1.2 mmol) in *N*,*N*-dimethylformamide (7 mL) was added sodium acetate (0.11 g, 1.3 mmol), and the mixture was reacted at 120°C for 4 hours. The reaction solution was cooled to room temperature, and water (20 mL) was added. The mixture was extracted with ethyl acetate/petroleum ether (2/1, 30 mL × 3). The combined organic layers were washed with saturated sodium chloride solution (10 mL × 3), dried over anhydrous sodium sulfate, filtered and concentrated. The resulting residue was recrystallized (ethanol/ethyl acetate/petroleum ether=1/3/6, 50 mL) to obtain a red solid **18** (0.26 g, yield 40%, HPLC purity: 98.24%).
MS (ESI, neg. ion) *m*/*z*: 533.4[M-H]⁻;
¹H NMR (400 MHz, DMSO-*d₆*) δ (ppm) 7.94 (d, *J* = 8.6 Hz, 1H), 7.84 (s, 2H), 7.32(dq, *J* = 19.3, 11.0, 9.3 Hz, 5H), 6.85 (d, *J* = 6.9 Hz, 2H), 4.70 (s, 2H), 3.47(s, 3H), 2.95 (s, 2H).

### Example 19

### 2-(3,5-Dichloro-4-((1-oxo-1,2-dihydroisoquinolin-6-yl)oxy)phenyl)-3,5-dioxo-2,3,4,5-tetrahydro-1,2,4-triazin e-6-carbonitrile 19

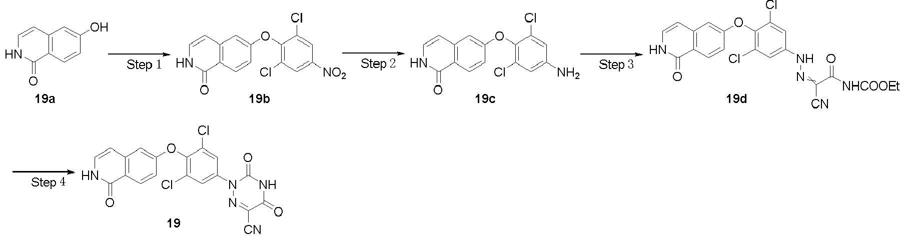

### Step 1: Synthesis of 6-(2,6-dichloro-4-nitrophenoxy)isoquinolin-1(2H)-one 19b

To a solution of 6-hydroxylsoquinolin-1(2*H*)-one **19a** (0.92 g, 5.7 mmol) in *N*,*N*-dimethylformamide (20 mL) were added 1,2,3-trichloro-5-nitrobenzene (1.40 g, 6.18 mmol) and potassium carbonate (1.90 g, 13.6 mmol), and the mixture was reacted at 80°C for 3 hours. The reaction solution was cooled to room temperature, and water (50 mL) was added. The mixture was extracted with ethyl acetate (35 mL × 3). The combined organic layers were washed with saturated sodium chloride (35 mL × 3), dried over anhydrous sodium sulfate and concentrated by suction filtration. The residue was purified by silica gel column chromatography (petroleum ether/ethyl acetate = 2/1) to give a yellow solid **19b** (1.83 g, yield 91%).

MS (ESI, pos. ion) *m*/*z*: 351.1 [M+H]⁺.

### Step 2: Synthesis of 6-(4-amino-2,6-dichlorophenoxy)isoquinolin-1(2H)-one 19c

To a solution of 6-(2,6-dichloro-4-nitrophenoxy)isoquinolin-1(2*H*)-one **19b** (0.45 g, 1.30 mmol) in acetic acid (15 mL) was added iron powder (0.29 g, 5.1 mmol), and the mixture was reacted at 60°C for 2.5 hours. The reaction solution was cooled to room temperature, iron powder was removed, then water (30 mL) was added. The reaction mixture was stirred for 10 minutes, and filtered. The filter cake was collected and dried to obtain a light yellow solid **19c** (0.38 g, yield 92%).

### Step 3: Synthesis of ethyl

### (2-cyano-2-(2-(3,5-dichloro-4-((1-oxo-1,2-dihydroisoquinolin-6-yl)oxy)phenyl)hydrazono)acetyl)carbamate 19d

To a solution of 6-(4-amino-2,6-dichlorophenoxy)isoquinolin-1(2*H*)-one **19c** (0.40 g, 1.20 mmol) in acetic acid (12 mL) was added a solution of sodium nitrite (0.13 g, 1.8 mmol) in water (1.5 mL) dropwise at 0°C. The mixture was reacted for 20 minutes, then *N*-cyanoacetylurethane (0.22 g, 1.40 mmol) was added, and the reaction was continued for 4 hours. Water (30 mL) was added to the reaction solution. The mixture was extracted with ethyl acetate (15 mL × 3). The combined organic layers were washed with saturated sodium chloride (35 mL × 3), dried over anhydrous sodium sulfate, and concentrated by suction filtration to give a yellow solid **19d** (0.60 g, yield 99%).

### Step 4: Synthesis of

### 2-(3,5-dichloro-4-((1-oxo-12-dihydroisoquinolin-6-yl)oxy)phenyl)-3,5-dioxo-2,3,4,5-tetrahydro-1,2,4-triazine-6-carbonitrile 19

To a solution of ethyl (2-cyano-2-(2-(3,5-dichloro-4-((1-oxo-1,2-dihydroisoquinolin-6-yl)oxy)phenyl)hydrazono)acetyl)carbamate **19d** (0.45 g, 0.92 mmol) in *N*,*N*-dimethylformamide (15 mL) was added sodium acetate (0.38 g, 4.61 mmol), and the mixture was reacted at 120°C for 3 hours. The reaction solution was cooled to room temperature, and water (50 mL) was added. The mixture was extracted with ethyl acetate (30 mL × 3). The combined organic layers were washed with saturated sodium chloride (30 mL × 3), dried over anhydrous sodium sulfate and concentrated by suction filtration. The residue was purified by silica gel column chromatography (petroleum ether/ethyl acetate = 1/2) to give a white solid **19** (0.23 g, yield 55%, HPLC purity: 96.04%).
MS (ESI, neg. ion) *m*/*z*: 440 [M-H]⁻;
¹H NMR (400 MHz, DMSO-*d₆*) δ (ppm) 13.32 (s, 1H), 11.20 (d, *J* = 3.8 Hz, 1H), 8.21 (d, *J* = 8.8 Hz, 1H), 7.86 (s, 2H), 7.24-7.10 (m, 2H), 7.02 (s, 1H), 6.52 (d, *J* = 7.1 Hz, 1H).

### Example 20 2-(3,5-dichloro-4-((2-(4-fluorobenzyl)-1-oxo-1,2,3,4-tetrahydroisoquinolin-6-yl)oxy)phenyl)-1,2,4-triazine-3, 5(2H,4H)-dione 20

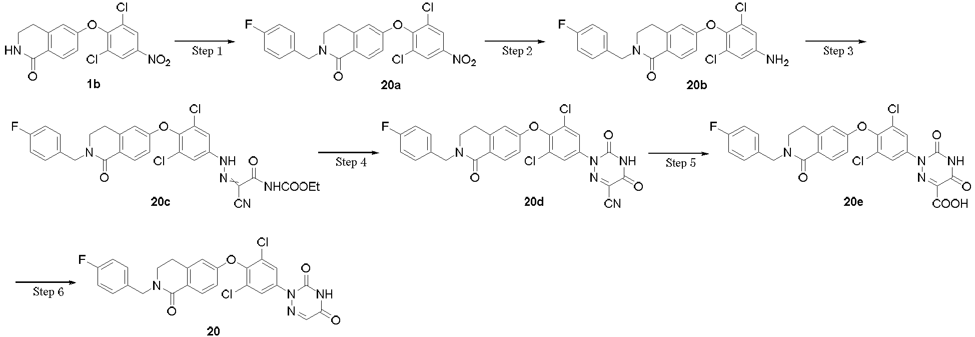

### Step 1: Synthesis of 6-(2,6-dichloro-4-nitrophenoxy)-2-(4-fluorobenzyl)-3,4-dihydroisoquinolin-1(2H)-one 20a

To a solution of 6-(2,6-dichloro-4-nitrophenoxy)-3,4-dihydroisoquinolin-1(2*H*)-one **1b** (0.50 g, 1.4 mmol) in THF (20 mL) was added sodium hydride (85 mg, 2.1 mmol, 60 mass% in oil) at 0°C. After 20 minutes of reaction, 1-bromomethyl-4-fluorobenzene (0.55 g, 2.8 mmol) and *N*,*N*-dimethylformamide (5 mL) were added dropwise, and the mixture was reacted at room temperature for 15 hours. The reaction was quenched with ice water (30 mL). The mixture was extracted with ethyl acetate (30 mL × 2). The combined organic layers were washed with saturated sodium chloride (30 mL), dried over anhydrous sodium sulfate and concentrated by suction filtration. The residue was purified by silica gel column chromatography (petroleum ether/ethyl acetate = 3/1) to give light yellow **oil 20a** (0.65 g, yield 99%).
MS (ESI, pos. ion) *m*/*z*: 461.2[M+H]⁺;
¹H NMR (400 MHz, CDCl₃) δ (ppm) 8.35 (s, 2H), 8.14 (d, *J* = 8.6 Hz, 1H), 7.37-7.30 (m, 2H), 7.07-7.00 (m, 2H), 6.79 (dd, *J* = 8.7, 2.6 Hz, 1H), 6.64 (d, *J* = 2.5 Hz, 1H), 4.76 (s, 2H), 3.51 (t, *J* = 6.6 Hz, 2H), 2.93 (t, *J* = 6.6 Hz, 2H).

### Step 2: Synthesis of 6-(4-amino-2,6-dichlorophenoxy)-2-(4-fluorobenzyl)-3,4-dihydroisoquinolin-1(2H)-one 20b

To a solution of 6-(2,6-dichloro-4-nitrophenoxy)-2-(4-fluorobenzyl)-3,4-dihydrolsoquinolin-1(2*H*)-one **20a** (0.65 g, 1.4 mmol) in acetic acid (10 mL) was added iron powder (0.16 g, 2.8 mmol), and the mixture was reacted at 50°C for 2.5 hours. The reaction solution was cooled to room temperature. The reaction was quenched with water (30 mL). The mixture was extracted with ethyl acetate (50 mL × 2). The combined organic layers were washed with saturated sodium chloride (50 mL), dried over anhydrous sodium sulfate and concentrated by suction filtration. The resulting residue was purified by silica gel column chromatography (petroleum ether/ethyl acetate = 1/1) to give light yellow oil 20b (0.37 g, yield 61%).

MS (ESI, pos. ion) *m*/*z*: 431.0 [M+H]⁺.

### Step 3: Synthesis of ethyl

### (2-cyano-2-(2-(3,5-dichloro-4-((2-(4-fluorobenzyl)-1-oxo-1,2,3,4-tetrahydroisoquinolin-6-yl)oxy)phenyl)hydrazo no)acetyl)carbamate 20c

To a solution of 6-(4-amino-2,6-dichlorophenoxy)-2-(4-fluorobenzyl)-3,4-dihydroisoquinolin-1(2*H*)-one **20b** (0.37 g, 0.86 mmol) in acetic acid (12 mL) was added a solution of sodium nitrite (0.12 g, 1.7 mmol) in water (6 mL) dropwise at 0°C, then *N*-cyanoacetylurethane (0.20 g, 1.3 mmol) was added. The mixture was reacted for 3 hours. Water (30 mL) was added to quench the reaction. The mixture was stirred for 20 minutes, then filtered, and the filter cake was washed with water (5 mL). Then the filter cake was collected and dried to obtain a light yellow solid **20c** (0.50 g, yield 97%).

MS (ESI, pos. ion) *m*/*z*: 596.0 [M+H]⁺.

### Step 4: Synthesis of

### 2-(3,5-dichloro-4-((2-(4-fluorobenzyl)-1-oxo-1,2,3,4-tetrahydroisoguinolin-6-yl)oxy)phenyl)-3,5-dioxo-2,3,4,5-tet rahydro-1,2,4-triazine-6-carbonitrile 20d

To a solution of ethyl (2-cyano-2-(2-(3,5-dichloro-4-((2-(4-fluorobenzyl)-1-oxo-1,2,3,4-tetrahydroisoquinolin-6-yl)oxy)phenyl)hydrazo no)acetyl)carbamate **20c** (0.50 g, 0.84 mmol) in *N*,*N*-dimethylformamide (10 mL) was added sodium acetate (0.15 g, 1.1 mmol), and the mixture was reacted at 120°C for 6 hours. The reaction solution was cooled to room temperature, then water (20 mL) was added to quench the reaction. The reaction mixture was stirred for 15 minutes, and filtered. The filter cake was collected and dried, recrystallized (ethyl acetate/petroleum ether = 2/1, 15 mL) at 60°C to obtain a yellow solid **20d** (0.28 g, yield 61%, HPLC purity: 100%).
MS (ESI, neg. ion) *m*/*z*: 550.0 [M-H]⁻;
¹H NMR (400 MHz, DMSO-*d₆*) δ (ppm) 13.30 (s, 1H), 7.94 (dd, *J* = 9.0, 6.2 Hz, 1H), 7.84 (s, 2H), 7.36 (dd, *J* = 8.4, 5.5 Hz, 2H), 7.16 (t, *J* = 8.7 Hz, 2H), 6.85 (d, *J* = 6.7 Hz, 2H), 4.67 (s, 2H), 3.47 (t, *J* = 6.6 Hz, 2H), 2.95 (t, *J* = 6.6 Hz, 2H);
¹⁹F NMR (376 MHz, DMSO-*d₆*) δ (ppm) -115.68.

### Step 5: Synthesis of

### 2-(3,5-dichloro-4-((2-(4-fluorobenzyl)-1-oxo-1,2,3,4-tetrahydroisoguinolin-6-yl)oxy)phenyl)-3,5-dioxo-2,3,4,5-tet rahydro-1,2,4-triazine-6-carboxylic acid 20e

2-(3,5-Dichloro-4-((2-(4-fluorobenzyl)-1-oxo-1,2,3,4-tetrahydroisoquinolin-6-yl)oxy)phenyl)-3,5-d ioxo-2,3,4,5-tetrahydro-1,2,4-triazine-6-carbonitrile **20d** (0.17 g, 0.31 mmol) was dissolved in acetic acid (4 mL), then concentrated hydrochloric acid (2 mL) was added. The mixture was reacted at 100°C for 18 hours. The reaction solution was cooled to room temperature, then water (18 mL) was added. The reaction mixture was stirred for 10 minutes, filtered, and rinsed with water (10 mL × 2). The filter cake was collected and dried to obtain a yellow solid **20e** (0.15 g, yield 85%).

### Step 6: Synthesis of

### 2-(3,5-dichloro-4-((2-(4-fluorobenzyl)-1-oxo-1,2,3,4-tetrahydroisoquinolin-6-yl)oxy)phenyl)-1,2,4-triazine-3,5(2 H,4H)-dione 20

2-(3,5-Dichloro-4-((2-(4-fluorobenzyl)-1-oxo-1,2,3,4-tetrahydroisoquinolin-6-yl)oxy)phenyl)-3,5-d ioxo-2,3,4,5-tetrahydro-1,2,4-triazine-6-carboxylic acid **20e** (0.15 g, 0.26 mmol) was dissolved in thioglycolic acid (3 mL). The mixture was reacted at 150°C for 24 hours. The reaction solution was cooled to room temperature, and ethyl acetate (30 mL) was added. The mixture was washed successively with water (10 mL), saturated sodium bicarbonate solution (10 mL) and saturated sodium chloride solution (10 mL), dried over anhydrous sodium sulfate and concentrated by suction filtration. The resulting residue was purified by silica gel column chromatography (petroleum ether/ethyl acetate = 1/1) to give a white solid **20** (90 mg, yield 65%, HPLC purity: 98.12%).
MS (ESI, neg. ion) *m*/*z*: 525.0 [M-H]⁻;
¹H NMR (400 MHz, DMSO-*d₆*) δ (ppm) 12.52 (s, 1H), 7.93 (d, *J* = 8.6 Hz, 1H), 7.87 (s, 2H), 7.73 (s, 1H), 7.36 (dd, *J* = 8.5, 5.6 Hz, 2H), 7.16 (dd, *J* = 10.1, 7.6 Hz, 2H), 6.83 (s, 1H), 6.81 (d, *J* = 2.6 Hz, 1H), 4.67 (s, 2H), 3.47 (t, *J* = 6.6 Hz, 2H), 2.95 (t, *J* = 6.6 Hz, 2H).

### Example 21

### 2-(3,5-dichloro-4-((2-(4-methylbenzyl)-1-oxo-1,2,3,4-tetrahydroisoquinolin-6-yl)oxy)phenyl)-1,2,4-triazine-3 ,5(2H,4H)-dione 21

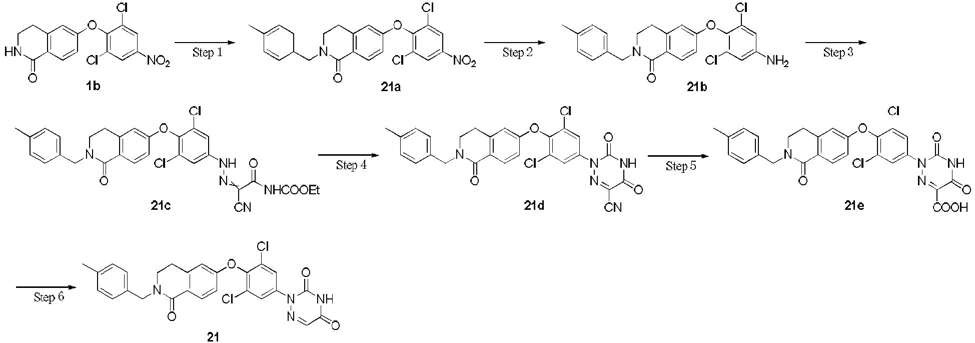

### Step 1: Synthesis of 6-(2,6-dichloro-4-nitrophenoxy)-2-(4-methylbenzyl)-3,4-dihydroisoquinolin-1(2H)-one 21a

To a solution of 6-(2,6-dichloro-4-nitrophenoxy)-3,4-dihydroisoquinolin-1(2*H*)-one **1b** (0.50 g, 1.4 mmol) in THF (20 mL) was added sodium hydride (85 mg, 2.1 mmol, 60 mass% in oil). After 20 minutes of reaction, 1-bromomethyl-4-toluene (0.54 g, 2.8 mmol) and N,N-dimethylformamide (5 mL) were added dropwise, and the mixture was reacted at room temperature for 14 hours. The reaction was quenched with ice water (30 mL). The mixture was extracted with ethyl acetate (30 mL × 2). The combined organic layers were washed with saturated sodium chloride (30 mL), dried over anhydrous sodium sulfate and concentrated by suction filtration. The residue was purified by silica gel column chromatography (petroleum ether/ethyl acetate = 3/1) to give light yellow **oil 21a** (0.63 g, yield 98%).

### Step 2: Synthesis of 6-(4-amino-2,6-dichlorophenoxy)-2-(4-methylbenzyl)-3,4-dihydroisoquinolin-1(2H)-one 21b

To a solution of 6-(2,6-dichloro-4-nitrophenoxy)-2-(4-methylbenzyl)-3,4-dihydroisoquinolin-1(2*H*)-one **21a** (0.63 g, 1.4 mmol) in acetic acid (10 mL) was added iron powder (0.16 g, 2.8 mmol), and the mixture was reacted at 50°C for 2.5 hours. The reaction solution was cooled to room temperature. The reaction was quenched with water (30 mL). The mixture was extracted with ethyl acetate (50 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate = 1/1) to give light yellow **oil 21b** (0.46 g, yield 78%).

MS (ESI, pos. ion) *m*/*z*: 428.1 [M+H]⁺.

### Step 3: Synthesis of ethyl

### (2-cyano-2-(2-(3,5-dichloro-4-((2-(4-methylbenzyl)-1-oxo-1,2,3,4-tetrahydroisoquinolin-6-yl)oxy)phenyl)hydrazo no)acetyl)carbamate 21c

To a solution of 6-(4-amino-2,6-dichlorophenoxy)-2-(4-methylbenzyl)-3,4-dihydroisoquinolin-1(2*H*)-one **21b** (0.46 g, 1.1 mmol) in acetic acid (16 mL) was added a solution of sodium nitrite (0.15 g, 2.1 mmol) in water (8 mL) at 0°C, then *N*-cyanoacetylurethane (0.25 g, 1.6 mmol) was added. The mixture was reacted for 2.5 hours. Water (30 mL) was added to quench the reaction. The mixture was stirred for 20 minutes, filtered, and the filter cake was washed with water (5 mL). Then the filter cake was collected and dried to obtain a light yellow solid **21c** (0.48 g, yield 75%).

MS (ESI, pos. ion) *m*/*z*: 595.0 [M+H]⁺.

### Step 4: Synthesis of

### 2-(3,5-dichloro-4-((2-(4-methylbenzyl)-1-oxo-1,2,3,4-tetrahydroisoquinolin-6-yl)oxy)phenyl)-3,5-dioxo-2,3,4,5-te trahydro-1,2,4-triazine-6-carbonitrile 21d

To a solution of ethyl (2-cyano-2-(2-(3,5-dichloro-4-((2-(4-methylbenzyl)-1-oxo-1,2,3,4-tetrahydroisoquinolin-6-yl)oxy)phenyl)hydrazo no)acetyl)carbamate **21c** (0.48 g, 0.81 mmol) in *N*,*N*-dimethylformamide (10 mL) was added sodium acetate (0.14 g, 1.0 mmol), and the mixture was reacted at 120°C for 8 hours. The reaction solution was cooled to room temperature, then water (20 mL) was added to quench the reaction. The reaction mixture was stirred for 15 minutes, and filtered. The filter cake was collected and dried, recrystallized (ethyl acetate/petroleum ether = 2/1, 15 mL) at 60°C to obtain a yellow solid **21d** (0.41 g, yield 93%, HPLC purity: 98.71%).
MS (ESI, neg. ion) *m*/*z*: 547.0 [M-H]⁻;
¹H NMR (400 MHz, DMSO-*d₆*) δ (ppm) 13.33 (s, 1H), 7.97-7.89 (m, 1H), 7.84 (s, 2H), 7.20 (d, *J* = 7.7 Hz, 2H), 7.14 (d, *J* = 7.8 Hz, 2H), 6.85 (dd, *J* = 5.8, 2.9 Hz, 2H), 4.64 (s, 2H), 3.44 (t, *J* = 6.5 Hz, 2H), 2.93 (t, *J* = 6.6 Hz, 2H), 2.28 (s, 3H).

### Step 5: Synthesis of

### 2-(3,5-dichloro-4-((2-(4-methylbenzyl)-1-oxo-1,2,3,4-tetrahydroisoquinolin-6-yl)oxy)phenyl)-3,5-dioxo-2,3,4,5-te trahydro-1,2,4-triazine-6-carboxylic acid 21e

2-(3,5-Dichloro-4-((2-(4-methylbenzyl)-1-oxo-1,2,3,4-tetrahydroisoquinolin-6-yl)oxy)phenyl)-3,5-dioxo-2,3,4,5-tetrahydro-1,2,4-triazine-6-carbonitrile **21d** (0.21 g, 0.37 mmol) was dissolved in acetic acid (4 mL), then concentrated hydrochloric acid (2 mL) was added. The mixture was reacted at 100°C for 18 hours. The reaction solution was cooled to room temperature, then water (18 mL) was added. The reaction mixture was stirred for 10 minutes, filtered, and rinsed with water (10 mL × 2). The filter cake was collected and dried to obtain a yellow solid **21e** (0.20 g, yield 93%).

### Step 6: Synthesis of

### 2-(3,5-dichloro-4-((2-(4-methylbenzyl)-1-oxo-1,2,3,4-tetrahydroisoquinolin-6-yl)oxy)phenyl)-1,2,4-triazine-3,5(2 H,4H)-dione 21

2-(3,5-Dichloro-4-((2-(4-methylbenzyl)-1-oxo-1,2,3,4-tetrahydroisoquinolin-6-yl)oxy)phenyl)-3,5-dioxo-2,3,4,5-tetrahydro-1,2,4-triazine-6-carboxylic acid **21e** (0.20 g, 0.35 mmol) was dissolved in thioglycolic acid (3 mL). The mixture was reacted at 150°C for 24 hours. The reaction solution was cooled to room temperature, and ethyl acetate (30 mL) was added. The mixture was washed successively with water (10 mL), saturated sodium bicarbonate solution (10 mL) and saturated sodium chloride solution (10 mL), dried over anhydrous sodium sulfate and concentrated by suction filtration. The resulting residue was purified by silica gel column chromatography (petroleum ether/ethyl acetate = 1/1) to give a yellow solid **21** (96 mg, yield 52%, HPLC purity: 96.18%).
MS (ESI, neg. ion) *m*/*z*: 521.0 [M-H]⁻;
¹H NMR (400 MHz, DMSO-*d₆*) δ (ppm) 12.53 (s, 1H), 7.93 (d, *J* = 9.2 Hz, 1H), 7.86 (s, 2H), 7.72 (s, 1H), 7.23-7.11 (m, 4H), 6.82 (s, 1H), 6.80 (d, *J* = 2.6 Hz, 1H), 4.64 (s, 2H), 3.44 (d, *J* = 6.5 Hz, 2H), 2.92 (t, *J* = 6.6 Hz, 2H), 2.27 (s, 3H).

### Example 22

### 2-(4-((2-benzyl-1-oxo-1,2,3,4-tetrahydroisoquinolin-6-yl)oxy)-3,5-dichlorophenyl)-1,2,4-triazine-3,5(2H,4H)-dione 22

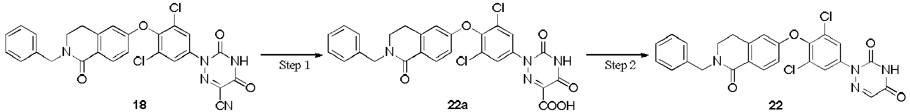

### Step 1: Synthesis of

### 2-(4-((2-benzyl-1-oxo-1,2,3,4-tetrahydroisoquinolin-6-yl)oxy)-3,5-dichlorophenyl)-3,5-dioxo-2,3,4,5-tetrahydro-1 ,2,4-triazine-6-carboxylic acid 22a

2-(4-((2-Benzyl-1-oxo-1,2,3,4-tetrahydroisoquinolin-6-yl)oxy)-3,5-dichlorophenyl)-3,5-dioxo-2,3, 4,5-tetrahydro-1,2,4-triazine-6-carbonitrile **18** (0.21 g, 0.37 mmol) was dissolved in acetic acid (4 mL), then concentrated hydrochloric acid (1.5 mL) was added. The mixture was reacted at 120°C for 24 hours. The reaction solution was cooled to room temperature, then water (20 mL) was added. The reaction mixture was stirred for 10 minutes, filtered, and rinsed with water (10 mL × 2). The filter cake was collected and dried to obtain a yellow solid **22a** (0.20 g, yield 100%).

### Step 2: Synthesis of

### 2-(4-((2-benzyl-1-oxo-1,2,3,4-tetrahydroisoquinolin-6-yl)oxy)-3,5-dichlorophenyl)-1,2,4-triazine-3,5(2H,4H)-dio ne 22

2-(4-((2-Benzyl-1-oxo-1,2,3,4-tetrahydroisoquinolin-6-yl)oxy)-3,5-dichlorophenyl)-3,5-dioxo-2,3, 4,5-tetrahydro-1,2,4-triazine-6-carboxylic acid **22a** (0.20 g, 0.36 mmol) was dissolved in thioglycolic acid (4 mL). The mixture was reacted at 160°C for 24 hours. The reaction solution was cooled to room temperature, and water (10 mL) was added. The mixture was extracted with ethyl acetate (20 mL × 3). The combined organic layers were washed with saturated sodium chloride (20 mL), dried over anhydrous sodium sulfate and concentrated by suction filtration. The residue was purified by silica gel column chromatography (petroleum ether/ethyl acetate = 1/1) to give a light yellow solid **22** (0.13 g, yield 68%, HPLC purity: 98.02%).
MS (ESI, neg. ion) *m*/*z*: 507.5 [M-H]⁻;
¹H NMR (400 MHz, DMSO-*d₆*) δ (ppm) 12.51 (s, 1H), 7.92 (d, *J =* 8.7 Hz, 1H), 7.85 (s, 2H), 7.72 (s, 1H), 7.35- 7.22 (m, 5H), 6.81 (d, *J* = 7.6 Hz, 2H), 4.68 (s, 2H), 3.45 (t, *J* = 6.4 Hz, 2H), 2.93 (t, *J* = 6.2 Hz, 2H).

### Example 23 2-(3,5-dichloro-4-((1-oxo-2-((tetrahydro-2H pyran-4-yl)methyl)-1,2,3,4-tetrahydroisoquinolin-6-yl)oxy)phen yl)-1,2,4-triazine-3,5(2H,4H)-dione 23

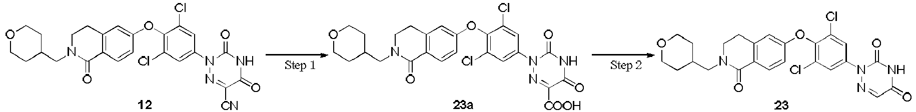

### Step 1: Synthesis of

### 2-(3,5-dichloro-4-((1-oxo-2-((tetrahydro-2H-pyran-4-yl)methyl)-1,2,3,4-tetrahydroisoquinolin-6-yl)oxy)phenyl)-3 ,5-dioxo-2,3,4,5-tetrahydro-1,2,4-triazine-6-carboxylic acid 23a

2-(3,5-Dichloro-4-((1-oxo-2-((tetrahydro-2*H-*pyran-4-yl)methyl)-1,2,3,4-tetrahydroisoquinolin-6-y 1)oxy)phenyl)-3,5-dioxo-2,3,4,5-tetrahydro-1,2,4-triazine-6-carbonitrile **12** (1.3 g, 2.4 mmol) was dissolved in acetic acid (20 mL), then concentrated hydrochloric acid (6 mL) was added. The mixture was reacted at 120°C for 4.5 hours. The reaction solution was cooled to room temperature, and concentrated. Then water (25 mL), saturated sodium bicarbonate solution (30 mL) and ethyl acetate (30 mL) were added. The organic layer was separated, and extracted with water (25 mL × 2). The aqueous layer was collected, and adjusted the pH value to 2-3 with dilute hydrochloric acid (4 N), then extracted with ethyl acetate (25 mL × 3). The extracted organic layers after acidification were combined, washed with saturated sodium chloride (25 mL × 3), dried over anhydrous sodium sulfate, and concentrated by suction filtration to give a yellow solid **23a** (0.87 g, yield 65%).

### Step 2: Synthesis of

### 2-(3,5-dichloro-4-((1-oxo-2-((tetrahydro-2H-pyran-4-yl)methyl)-1,2,3,4-tetrahydroisoquinolin-6-yl)oxy)phenyl)-1 ,2,4-triazine-3,5(2H,4H)-dione 23

2-(3,5-Dichloro-4-((1-oxo-2-((tetrahydro-2*H-*pyran-4-yl)methyl)-1,2,3,4-tetrahydroisoquinolin-6-y 1)oxy)phenyl)-3,5-dioxo-2,3,4,5-tetrahydro-1,2,4-triazine-6-carboxylic acid **23a** (0.85 g, 1.5 mmol) was dissolved in thioglycolic acid (4 mL). The mixture was reacted at 160°C for 24 hours. The reaction solution was cooled to room temperature, and saturated sodium bicarbonate solution (15 mL) was slowly added. The mixture was extracted with ethyl acetate (15 mL × 3). The combined organic layers were washed with saturated sodium chloride (15 mL × 3), dried over anhydrous sodium sulfate and concentrated by suction filtration. The residue was purified by silica gel column chromatography (petroleum ether/ethyl acetate = 1/2) to give a light yellow solid **23** (0.42 g, yield 54%, HPLC purity: 96.10%).
MS (ESI, neg. ion) *m*/*z*: 515.2 [M-H]⁻;
¹H NMR (400 MHz, DMSO-*d₆*) δ (ppm) 12.51 (s, 1H), 7.94-7.82 (m, 3H), 7.72 (s, 1H), 6.87-6.75 (m, 2H), 3.83 (d, *J* = 8.9 Hz, 2H), 3.53 (t, *J* = 6.4 Hz, 2H), 3.35 (s, 2H), 3.25 (t, *J* = 11.1 Hz, 2H), 2.94 (t, *J* = 6.2 Hz, 2H), 1.96-1.85 (m, 1H), 1.53 (d, *J* = 11.9 Hz, 2H), 1.24 (dd, *J* = 11.7, 4.0 Hz, 2H).

### Example 24

### 2-(3,5-dichloro-4-((1-oxo-2-(pyridin-4-ylmethyl)-1,2,3,4-tetrahydroisoquinolin-6-yl)oxy)phenyl)-1,2,4-triazi ne-3,5(2H,4H)-dione 24

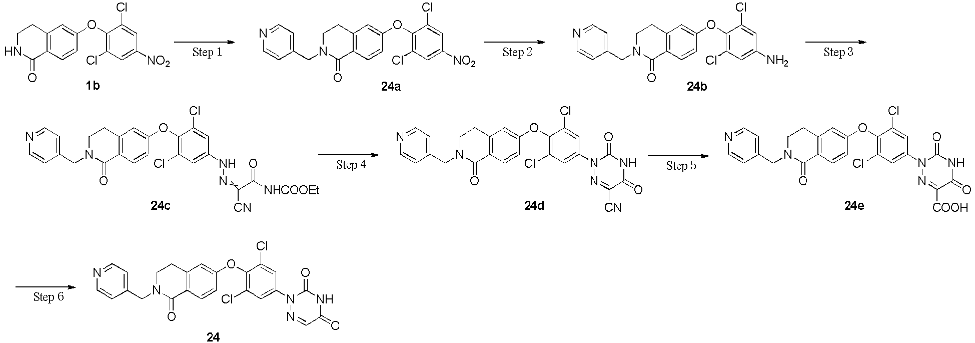

### Step 1: Synthesis of

### 6-(2,6-dichloro-4-nitrophenoxy)-2-(pyridin-4-ylmethyl)-3,4-dihydroisoquinolin-1(2H)-one 24a

To a solution of 6-(2,6-dichloro-4-nitrophenoxy)-3,4-dihydroisoquinolin-1(2*H*)-one **1b** (2.0 g, 5.7 mmol) in THF (20 mL) was added sodium hydride (0.80 g, 20 mmol, 60 mass% in oil) at 0°C. After 20 minutes of reaction, 4-(bromomethyl)pyridine hydrobromide (1.5 g, 8.7 mmol) and N,N-dimethylformamide (3 mL) were added, and the mixture was reacted at 5°C for 2 hours. Water (70 mL) was added to quench the reaction. The mixture was stirred for 10 minutes, filtered, and the filter cake was washed with water (20 mL × 3). Then the filter cake was collected and dried to obtain a yellow solid **24a** (2.3 g, yield 91%).

### Step 2: Synthesis of

### 6-(4-amino-2,6-dichlorophenoxy)-2-(pyridin-4-ylmethyl)-3,4-dihydroisoquinolin-1(2H)-one 24b

To a solution of 6-(2,6-dichloro-4-nitrophenoxy)-2-(pyridin-4-ylmethyl)-3,4-dihydroisoquinolin-1(2*H*)-one **24a** (1.9 g, 4.3 mmol) in acetic acid (25 mL) was added iron powder (0.60 g, 11 mmol), and the mixture was reacted at 55°C for 8 hours. The reaction solution was cooled to room temperature, then water (120 mL) was added to quench the reaction. The mixture was extracted with ethyl acetate (150 mL × 2). The combined organic layers were washed with saturated sodium chloride (30 mL × 2), dried over anhydrous sodium sulfate, and concentrated by suction filtration to give a gray solid **24b** (1.8 g, yield 100%).

### Step 3: Synthesis of ethyl

### (2-cyano-2-(2-(3,5-dichloro-4-((2-(pyridin-4-ylmethyl)-1-oxo-1,2,3,4-tetrahydroisoquinolin-6-yl)oxy)phenyl)hydr azono)acetyl)carbamate 24c

To a solution of 6-(4-amino-2,6-dichlorophenoxy)-2-(pyridin-4-ylmethyl)-3,4-dihydroisoquinolin-1(2*H*)-one **24b** (1.8 g, 4.3 mmol) in acetic acid (27 mL) was added a solution of sodium nitrite (0.60 g, 8.7 mmol) in water (14 mL) at 0°C, then *N*-cyanoacetylurethane (0.81 g, 5.3 mmol) was added. The mixture was reacted for 2 hours. Water (80 mL) was added to quench the reaction. The mixture was extracted with ethyl acetate (150 mL × 2). The combined organic layers were washed with saturated sodium chloride (50 mL × 3), dried over anhydrous sodium sulfate, and concentrated by suction filtration to give a red foamy solid **24c** (2.5 g, yield 99%).

### Step 4: Synthesis of

### 2-(3,5-dichloro-4-((2-(pyridin-4-ylmethyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 24d

To a solution of ethyl (2-cyano-2-(2-(3,5-dichloro-4-((2-(pyridin-4-ylmethyl)-1-oxo-1,2,3,4-tetrahydroisoquinolin-6-yl)oxy)phenyl)hydr azono)acetyl)carbamate **24c** (1.8 g, 3.1 mmol) in *N*,*N*-dimethylformamide (20 mL) was added sodium acetate (0.28 g, 3.4 mmol), and the mixture was reacted at 120°C for 6 hours. The reaction solution was cooled to room temperature, then water (100 mL) was added to quench the reaction. The reaction mixture was stirred for 15 minutes, and filtered. The filter cake was collected and dried, recrystallized (*N*,*N*-dimethylformamide/ethanol/ethyl acetate/petroleum ether = 4/25/15/20, 128 mL) at 85°C to obtain a yellow solid **24d** (0.90 g, yield 53%, HPLC purity: 82.28%).
MS (ESI, pos. ion) *m*/*z*: 536.3 [M+H]⁺;
¹H NMR (400 MHz, DMSO-*d₆*) δ (ppm) 8.56 (s, 2H), 7.92 (d, *J* = 8.4 Hz, 1H), 7.84 (s, 2H), 7.33 (s, 2H), 6.86 (d, *J* = 13.0 Hz, 2H), 4.71 (s, 2H), 3.54 (s, 2H), 3.01 (s, 2H).

### Step 5: Synthesis of

### 2-(3,5-dichloro-4-((2-(pyridin-4-ylmethyl)-1-oxo-1,2,3,4-tetrahydroisoquinolin-6-yl)oxy)phenyl)-3,5-dioxo-2,3,4, 5-tetrahydro-1,2,4-triazine-6-carboxylic acid 24e

2-(3,5-Dichloro-4-((2-(pyridin-4-ylmethyl)-1-oxo-1,2,3,4-tetrahydroisoquinolin-6-yl)oxy)phenyl)-3,5-dioxo-2,3,4,5-tetrahydro-1,2,4-triazine-6-carbonitrile **24d** (0.45 g, 0.84 mmol) was dissolved in acetic acid (8 mL), then concentrated hydrochloric acid (3 mL) was added. The mixture was reacted at 120°C for 24 hours. The reaction solution was cooled to room temperature, then water (30 mL) was added. The reaction mixture was stirred for 10 minutes, filtered, and rinsed with water (10 mL × 2). The filter cake was collected and dried to obtain a light yellow solid **24e** (0.45 g, yield 97%).

### Step 6: Synthesis of

### 2-(3,5-dichloro-4-((2-(pyridin-4-ylmethyl)-1-oxo-1,2,3,4-tetrahydroisoquinolin-6-yl)oxy)phenyl)-1,2,4-triazine-3, 5(2H,4H)-dione 24

2-(3,5-Dichloro-4-((2-(pyridin-4-ylmethyl)-1-oxo-1,2,3,4-tetrahydroisoquinolin-6-yl)oxy)phenyl)-3,5-dioxo-2,3,4,5-tetrahydro-1,2,4-triazine-6-carboxylic acid **24e** (0.37 g, 0.67 mmol) was dissolved in thioglycolic acid (5 mL). The mixture was reacted at 120°C for 36 hours. The reaction solution was cooled to room temperature, and ethyl acetate (80 mL) was added. The mixture was washed successively with water (40 mL), saturated sodium bicarbonate solution (20 mL) and saturated sodium chloride solution (20 mL), then dried over anhydrous sodium sulfate and concentrated by suction filtration. The residue was purified by silica gel column chromatography (petroleum ether/ethyl acetate = 1/2) to give a light yellow solid **24** (0.20 g, yield 59%, HPLC purity: 92.08%).
MS (ESI, neg. ion) *m*/*z*: 511.3 [M+H]⁺;
¹H NMR (400 MHz, DMSO-*d₆*) δ (ppm) 12.53 (s, 1H), 8.77 (s, 2H), 7.92 (d, *J* = 8.5 Hz, 1H), 7.87 (s, 2H), 7.73 (s, 1H), 7.65 (s, 2H), 6.87 (s, 1H), 6.83 (d, *J* = 7.6 Hz, 1H), 4.82 (s, 2H), 3.59 (d, *J* = 6.2 Hz, 2H), 3.03 (d, *J* = 6.5 Hz, 2H).

### Example 25

### 2-(3,5-dichloro-4-((1-oxo-2-(pyridazin-3-ylmethyl)-1,2,3,4-tetrahydroisoquinolin-6-yl)oxy)phenyl)-1,2,4-tria zine-3,5(2H,4H)-dione 25

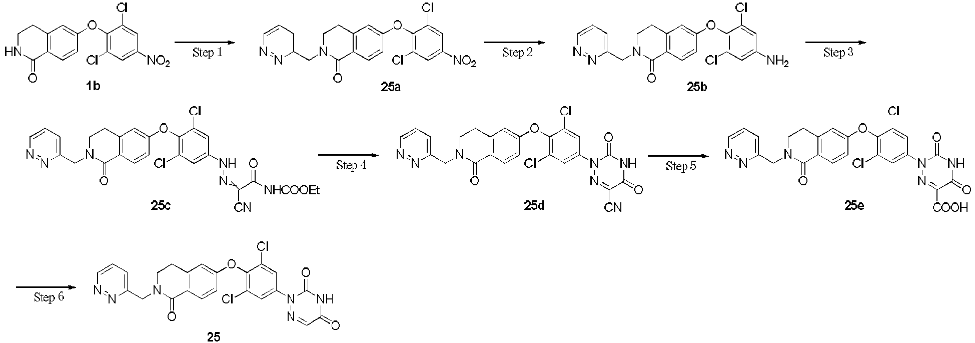

### Step 1: Synthesis of

### 6-(2,6-dichloro-4-nitrophenoxy)-2-(pyridazin-3-ylmethyl)-3,4-dihydroisoquinolin-1(2H)-one 25a

To a solution of 6-(2,6-dichloro-4-nitrophenoxy)-3,4-dihydroisoquinolin-1(2*H*)-one **1b** (2.0 g, 5.7 mmol) in THF (25 mL) was added sodium hydride (0.80 g, 20 mmol, 60 mass% in oil). After 20 minutes of reaction, 3-(bromomethyl)pyridazine hydrobromide (1.5 g, 8.7 mmol) and *N*,*N*-dimethylformamide (4 mL) were added, and the mixture was reacted at 8°C for 2 hours. Water (150 mL) was added to quench the reaction. The mixture was stirred for 10 minutes, and filtered. The filter cake was rinsed with water (20 mL × 3), and the collected filter cake was dried and slurried with ethyl acetate/petroleum ether (1/4, 50 mL). The mixture was filtered, and the filter cake was collected to obtain a yellow solid **25a** (1.8 g, yield 71%).

### Step 2: Synthesis of

### 6-(4-amino-2,6-dichlorophenoxy)-2-(pyridazin-3-ylmethyl)-3,4-dihydroisoquinolin-1(2H)-one 25b

To a solution of 6-(2,6-dichloro-4-nitrophenoxy)-2-(pyridazin-3-ylmethyl)-3,4-dihydroisoquinolin-1(2*H*)-one **25a** (1.0 g, 2.2 mmol) in acetic acid (15 mL) was added iron powder (0.35 g, 6.3 mmol), and the mixture was reacted at 60°C for 8 hours. The reaction solution was cooled to room temperature, then water (80 mL) was added to quench the reaction. The reaction mixture was stirred for 10 minutes, filtered, and rinsed with water (20 mL × 2). The filter cake was collected and dried to obtain a gray solid **25b** (0.74 g, yield 79%).

### Step 3: Synthesis of ethyl

### (2-cyano-2-(2-(3,5-dichloro-4-((2-(pyridazin-3-ylmethyl)-1-oxo-1,2,3,4-tetrahydroisoquinolin-6-yl)oxy)phenyl)hy drazono)acetyl)carbamate 25c

To a solution of 6-(4-amino-2,6-dichlorophenoxy)-2-(pyridazin-3-ylmethyl)-3,4-dihydroisoquinolin-1(2*H*)-one **25b** (0.74 g, 1.8 mmol) in acetic acid (15 mL) was added a solution of sodium nitrite (0.25 g, 3.6 mmol) in water (6 mL) at 0°C, then *N*-cyanoacetylurethane (0.35 g, 2.2 mmol) was added. The mixture was reacted for 2 hours. Water (50 mL) was added to quench the reaction. The mixture was stirred for 10 minutes, filtered, and the filter cake was washed with water (30 mL × 2). Then the filter cake was collected and dried to obtain a red solid **25c** (0.96 g, yield 93%).

### Step 4: Synthesis of

### 2-(3,5-dichloro-4-((2-(pyridazin-3-ylmethyl)-1-oxo-1,2,3,4-tetrahydroisoquinolin-6-yl)oxy)phenyl)-3,5-dioxo-2,3, 4,5-tetrahydro-1,2,4-triazine-6-carbonitrile 25d

To a solution of ethyl (2-cyano-2-(2-(3,5-dichloro-4-((2-(pyridazin-3-ylmethyl)-1-oxo-1,2,3,4-tetrahydroisoquinolin-6-yl)oxy)phenyl)hy drazono)acetyl)carbamate **25c** (0.96 g, 1.6 mmol) in *N*,*N*-dimethylformamide (12 mL) was added sodium acetate (0.15 g, 1.8 mmol), and the mixture was reacted at 120°C for 6 hours. The reaction solution was cooled to room temperature, then water (100 mL) was added to quench the reaction. The reaction mixture was stirred for 15 minutes, and filtered. The filter cake was collected and dried, recrystallized (*N*,*N*-dimethylformamide/ethanol/ethyl acetate/petroleum ether = 2/5/20/40, 128 mL) at 85°C to obtain a yellow solid **24d** (0.51 g, yield 58%, HPLC purity: 93.11%).
MS (ESI, pos. ion) *m*/*z*: 537.3 [M+H]⁺;
¹H NMR (400 MHz, DMSO-*d₆*) δ (ppm) 13.23 (s, 1H), 9.15 (d, *J* = 4.6 Hz, 1H), 7.88 (d, *J* = 28.0 Hz, 3H), 7.66 (dd, *J* = 10.8, 6.2 Hz, 2H), 7.06-6.64 (m, 2H), 4.97 (s, 2H), 3.66 (t, *J* = 6.6 Hz, 2H), 3.02 (t, *J* = 6.5 Hz, 2H).

### Step 5: Synthesis of

### 2-(3,5-dichloro-4-((2-(pyridazin-3-ylmethyl)-1-oxo-1,2,3,4-tetrahydroisoquinolin-6-yl)oxy)phenyl)-3,5-dioxo-2,3, 4,5-tetrahydro-1,2,4-triazine-6-carboxylic acid 25e

2-(3,5-Dichloro-4-((2-(pyridazin-3-ylmethyl)-1-oxo-1,2,3,4-tetrahydroisoquinolin-6-yl)oxy)phenyl )-3,5-dioxo-2,3,4,5-tetrahydro-1,2,4-triazine-6-carbonitrile **25d** (0.23 g, 0.43 mmol) was dissolved in acetic acid (2.5 mL), then concentrated hydrochloric acid (1.4 mL) was added. The mixture was reacted at 120°C for 24 hours. The reaction solution was cooled to room temperature, then water (30 mL) was added. The reaction mixture was stirred for 10 minutes, filtered, and rinsed with water (15 mL × 2). The filter cake was collected and dried to obtain a yellow solid **25e** (0.21 g, yield 88%).

### Step 6: Synthesis of

### 2-(3,5-dichloro-4-((2-(pyridazin-3-ylmethyl)-1-oxo-1,2,3,4-tetrahydroisoquinolin-6-yl)oxy)phenyl)-1,2,4-triazine-3,5(2H,4H)-dione 25

2-(3,5-Dichloro-4-((2-(pyridazin-3-ylmethyl)-1-oxo-1,2,3,4-tetrahydroisoquinolin-6-yl)oxy)phenyl )-3,5-dioxo-2,3,4,5-tetrahydro-1,2,4-triazine-6-carboxylic acid **25e** (0.20 g, 0.40 mmol) was dissolved in thioglycolic acid (2.5 mL). The mixture was reacted at 120°C for 36 hours. The reaction solution was cooled to room temperature, and ethyl acetate (100 mL) was added. The mixture was washed successively with water (20 mL), saturated sodium bicarbonate solution (20 mL) and saturated sodium chloride solution (20 mL), then dried over anhydrous sodium sulfate and concentrated by suction filtration. The resulting residue was separated and purified by pre-HPLC [35%ACN/65%H₂O (1%TFA), Kromasil specifications: C18 10µm×50mm×250mm, flow rate: 100 mL/min] to obtain a white solid **25** (20 mg, yield 20%, HPLC purity: 82.95%).
MS (ESI, neg. ion) m/z: 513.3 [M+H]⁺;
¹H NMR (400 MHz, DMSO-*d₆*) δ (ppm) 12.52 (s, 1H), 9.17 (s, 1H), 7.99-7.83 (m, 3H), 7.79-7.51 (m, 3H), 6.96-6.74 (m, 2H), 4.95 (d, *J* = 17.0 Hz, 2H), 3.67 (s, 2H), 3.02 (t, *J* = 6.5 Hz, 2H).

### Example 26

### 2-(3,5-dimethyl-4-((1-oxo-1,2,3,4-tetrahydroisoquinolin-6-yl)oxy)phenyl)-3,5-dioxo-2,3,4,5-tetrahydro-1,2,4-triazine-6-carbonitrile 26

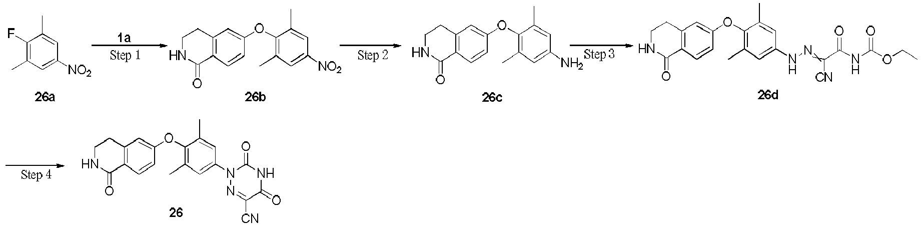

### Step 1: Synthesis of 6-(2,6-dimethyl-4-nitrophenoxy)-3,4-dihydroisoquinolin-1(2H)-one 1b

To a solution of 6-hydroxy-3,4-dihydroisoquinolin-1(2*H*)-one 1a (3.0 g, 18 mmol) and 2-fluoro-1,3-dimethyl-5-nitrobenzene 26a (3.5 g, 21 mmol) in *N*,*N*-dimethylformamide (30 mL) was added potassium carbonate (2.8 g, 28 mmol), and the mixture was reacted at 80°C for 12 hours. The reaction solution was cooled to room temperature, then water (150 mL) was added. The mixture was stirred for 10 minutes, and filtered. The filter cake was rinsed with water (30 mL × 2), and the collected filter cake was slurried with ethyl acetate/petroleum ether (1/6, 70 mL). The mixture was filtered, and the filter cake was collected and dried to obtain a white solid **26b** (4.2 g, yield 73%).

### Step 2: Synthesis of 6-(4-amino-2,6-dimethylphenoxy)-3,4-dihydroisoquinolin-1(2H)-one 26c

To a solution of 6-(2,6-dimethyl-4-nitrophenoxy)-3,4-dihydroisoquinolin-1(2*H*)-one **26b** (3.0 g, 9.6 mmol) in acetic acid (30 mL) was added iron powder (1.6 g, 29 mmol), and the mixture was reacted at 55°C for 5 hours. The reaction solution was cooled to room temperature, iron powder was removed, and ethyl acetate (200 mL) was added. The mixture was washed with water (150 mL) and saturated sodium chloride solution (40 mL × 2) successively, then dried over ahydrous sodium sulfate and concentrated by suction filtration. The resulting residue was purified by silica gel column chromatography (petroleum ether/ethyl acetate = 1/2) to give yellow oil **26c** (2.7 g, yield 100%).

### Step 3: Synthesis of ethyl

### (2-cyano-2-(2-(3,5-dimethyl-4-((1-oxo-1,2,3,4-tetrahydroisoquinolin-6-yl)oxy)phenyl)hydrazono)acetyl)carbamat e 26d

To a solution of 6-(4-amino-2,6-methylphenoxy)-3,4-dihydroisoquinolin-1(2*H*)-one **26c** (2.7 g, 9.6 mmol) in acetic acid (10 mL) was added a solution of sodium nitrite (1.0 g, 14 mmol) in water (5 mL) at 0°C. After reacting for 5 minutes, *N*-cyanoacetylurethane (1.9 g, 12 mmol) was added, and the mixture was reacted for 1.5 hours. At 0°C, water (10 mL) was added to the reaction solution. The mixture was stirred for 10 minutes, filtered, washed with water (5 mL), and the filter cake was collected and dried to obtain a yellow solid **26d** (4.1 g, yield 95%).

### Step 4: Synthesis of

### 2-(3,5-dimethyl-4-((1-oxo-1,2,3,4-tetrahydroisoquinolin-6-yl)oxy)phenyl)-3,5-dioxo-2,3,4,5-tetrahydro-1,2,4-triaz ine-6-carbonitrile 26

To a solution of ethyl (2-cyano-2-(2-(3,5-dimethyl-4-((1-oxo-1,2,3,4-tetrahydroisoquinolin-6-yl)oxy)phenyl)hydrazono)acetyl)carbamat e **26d** (4.1 g, 9.1 mmol) in *N*,*N*-dimethylformamide (20 mL) was added sodium acetate (0.90 g, 10 mmol), and the mixture was reacted at 120°C for 7.5 hours. The reaction solution was cooled to room temperature, and water (50 mL) was added. The mixture was extracted with ethyl acetate (20 mL × 3). The combined organic layers were washed with saturated sodium chloride solution (20 mL), dried over anhydrous sodium sulfate and concentrated by suction filtration. The resulting residue was separated and purified by pre-HPLC [42%ACN/58%H₂O (0.1%TFA), Kromasil specifications: C18 10µm×50mm×250mm, flow rate: 100 mL/min] to obtain a white solid **26** (1.0 g, yield 27%, purity 95.48%).
MS (ESI, pos. ion) *m*/*z*: 404.2 [M+H]⁺.
¹H NMR (400 MHz, DMSO-*d₆*) δ (ppm) 13.05 (s, 1H), 7.89 -7.72 (m, 2H), 7.32 (s, 2H), 6.76-6.61 (m, 3H), 3.27-3.33 (m, 2H), 2.86 (t, *J* = 6.4 Hz, 2H), 2.11 (s, 6H).

### Example 27

### 2-(3,5-dichloro-4-((1-oxo-2-(p-tolyl)-1,2,3,4-tetrahydroisoquinolin-6-yl)oxy)phenyl)-3,5-dioxo-2,3,4,5-tetrahy dro-1,2,4-triazine-6-carbonitrile 27

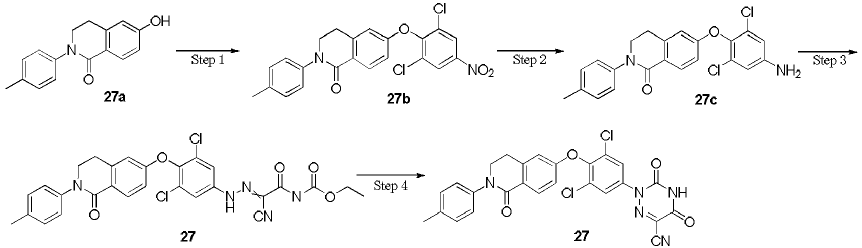

### Step 1: Synthesis of 6-(2,6-dichloro-4-nitrophenoxy)-2-(p-tolyl)-3,4-dihydroisoquinolin-1(2H)-one 27b

To a solution of 6-hydroxy-2-(p-tolyl)-3,4-dihydroisoquinolin-1(2*H*)-one **27a** (refer to steps 1 and 2 of Example 23 of the patent application CN 109988109 A for the preparation method) (0.32 g, 1.3 mmol) and 1,2,3-trichloro-5-nitrobenzene (0.34 g, 1.5 mmol) in *N*,*N*-dimethylformamide (6 mL) was added potassium carbonate (0.35 g, 2.5 mmol), and the mixture was reacted at 60°C for 3 hours. The reaction solution was cooled to room temperature, then water (8 mL) was added. The reaction mixture was stirred for 10 minutes, filtered, and rinsed with water (3 mL × 2). The filter cake was collected and dried to obtain a gray solid **27b** (0.56 g, yield 99%).

### Step 2: Synthesis of 6-(4-amino-2,6-dichlorophenonoxy)-2-(p-tolyl)-3,4-dihydroisoquinolin-1(2H)-one 27c

To a solution of 6-(2,6-dichloro-4-nitrophenoxy)-2-(p-tolyl)-3,4-dihydroisoquinolin-1(2*H*)-one **27b** (0.56 g, 1.25 mmol) in acetic acid (5 mL) was added iron powder (0.21 g, 3.75 mmol), and the mixture was reacted at 60°C for 5 hours. The reaction solution was cooled to room temperature, iron powder was removed, and water (20 mL) was added. The mixture was extracted with ethyl acetate (15 mL × 2). The combined organic layers were washed with saturated sodium chloride (10 mL × 3), dried over anhydrous sodium sulfate, and concentrated by suction filtration to give a white solid **27c** (0.37 g, yield 72%).

### Step 3: Synthesis of ethyl

### (2-cyano-2-(2-(3,5-dichloro-4-((1-oxo-2-(p-tolyl)-1,2,3,4-tetrahydroisoquinolin-6-yl)oxy)phenyl)hydrazono)acety l)carbamate 27d

To a solution of 6-(4-amino-2,6-dichlorophenoxy)-2-(*p*-tolyl)-3,4-dihydroisoquinolin-1(2*H*)-one **27c** (0.37 g, 0.90 mmol) in acetic acid (4 mL) was added *N*-cyanoacetylurethane (0.15 g, 1.08 mmol) at 0°C. The mixture was stirred for 5 minutes, then a solution of sodium nitrite (93 mg, 1.35 mmol) in water (1 mL) was added, and the mixture was reacted for 1 hours. At 0°C, water (5 mL) was added to the reaction solution. The mixture was stirred for 10 minutes, filtered, washed with water (3 mL), and the filter cake was collected and dried to obtain a yellow solid **27d** (0.50 g, yield 96%).

### Step 4: Synthesis of

### 2-(3,5-dichloro-4-((1-oxo-2-(p-tolyl)-1,2,3,4-tetrahydroisoquinolin-6-yl)oxy)phenyl)-3,5-dioxo-2,3,4,5-tetrahydro -1,2,4-triazine-6-carbonitrile 27

To a solution of ethyl (2-cyano-2-(2-(3,5-dichloro-4-((1-oxo-2-(*p*-tolyl)-1,2,3,4-tetrahydroisoquinolin-6-yl)oxy)phenyl)hydrazono)acety l)carbamate **27d** (0.50 g, 0.86 mmol) in *N*,*N*-dimethylformamide (6 mL) was added sodium acetate (0.14 g, 1.66 mmol), and the mixture was reacted at 120°C for 3 hours. The reaction solution was cooled to room temperature, and water (50 mL) was added to quench the reaction. The mixture was stirred for 10 minutes, filtered, the filter cake was collected and dried, and the obtained solid was separated and purified by pre-HPLC [60%ACN/40%H₂O (0.1% TFA), Kromasil Specifications: C18 10µm×50mm×250mm, flow rate: 100 mL/min] to obtain a white solid **27** (0.23 g, yield 50%, HPLC purity: 98.72%).
MS (ESI, neg. ion) *m*/*z*: 534.0 [M-H]⁻;
¹H NMR (400 MHz, DMSO-*d₆*) δ (ppm) 13.31 (s, 1H).7.94 (d, *J* = 6.4 Hz, 1H), 7.86 (s, 2H), 7.24 (dd, *J* =23.6 Hz, *J* = 8.4 Hz, 4H), 6.97- 6.82 (m, 2H), 3.91 (t, *J* = 6.4 Hz, 2H), 3.11 (t, *J* = 6.8 Hz, 2H), 2.32 (s, 3H).

### Example 28

### 2-(3,5-dichloro-4-((1-oxo-2-(4-fluorophenyl)-1,2,3,4-tetrahydroisoquinolin-6-yl)oxy)phenyl)-3,5-dioxo-2,3,4, 5-tetrahydro-1,2,4-triazine-6-carbonitrile 28

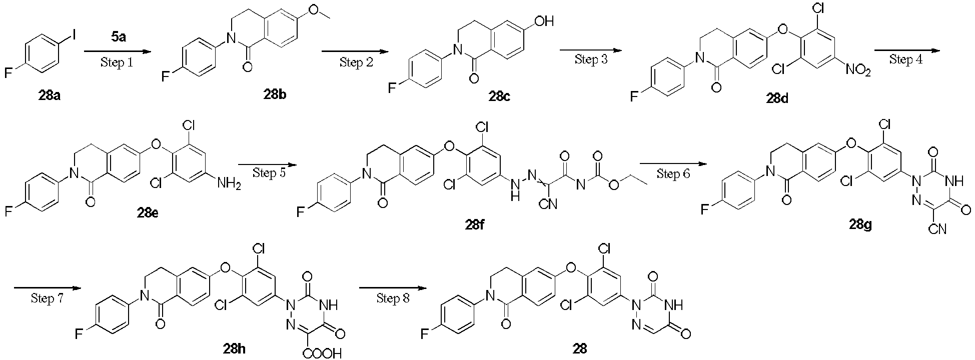

### Step 1: Synthesis of 2-(4-fluorophenyl)-6-methoxy-3,4-dihydroisoquinolin-1(2H)-one 28b

6-Methoxy-3,4-dihydroisoquinolin-1(2*H*)-one **5a** (1.50 g, 8.5 mmol), sodium iodide (0.32 g, 1.7 mmol), 1-fluoro-4-iodobenzene (3.76 g, 16.9 mmol) and potassium carbonate (1.17 g, 8.47 mmol) were dissolved in *N*,*N*-dimethylformamide (40 mL), and the mixture was reacted at 150°C for 16 hours. The reaction solution was cooled to room temperature, and water (100 mL) was added to quench the reaction. The mixture was extracted with ethyl acetate (200 mL). The combined organic layers were washed with saturated sodium chloride (100 mL), then dried over ahydrous sodium sulfate and concentrated by suction filtration. The resulting residue was purified by silica gel column chromatography (petroleum ether/ethyl acetate = 1/2) to give an off-white solid **28b** (1.05 g, yield 46%).

¹H NMR (400 MHz, CDCl₃) δ (ppm) 8.11 (d, *J* = 8.6 Hz, 1H), 7.36 (ddt, *J* = 6.8, 4.9, 2.8 Hz, 2H), 7.16-7.08 (m, 2H), 6.90 (dd, *J* = 8.7, 2.6 Hz, 1H), 6.74 (d, *J* = 2.5 Hz, 1H), 3.96 (t, *J* = 6.4 Hz, 2H), 3.89 (s, 3H), 3.13 (t, *J* = 6.4 Hz, 2H).

### Step 2: Synthesis of 2-(4-fluorophenyl)-6-hydroxy-3,4-dihydroisoquinolin-1(2H)-one 28c

At 0°C, to a solution of 2-(4-fluorophenyl)-6-methoxy-3,4-dihydroisoquinolin-1(2*H*)-one **28b** (1.00 g, 3.7 mmol) in dichloromethane (30 mL) was added boron tribromide (0.71 mL, 7.4 mmol) dropwise. Then the mixture was reacted at room temperature for 4 hours. The reaction solution was quenched by pouring into ice water (30 mL). The mixture was stirred for 10 minutes, filtered, and the filter cake was washed with water (10 mL), and the filter cake was collected and dried to obtain a white solid **28c** (0.87 g, yield 92%).

MS (ESI, pos. ion) *m*/*z*: 258.1 [M+H]⁺.

### Step 3: Synthesis of 6-(2,6-dichloro-4-nitrophenoxy)-2-(4-fluorophenyl)-3,4-dihydroisoquinolin-1(2H)-one 28d

To a solution of 2-(4-fluorophenyl)-6-hydroxy-3,4-dihydroisoquinolin-1(2*H*)-one **28c** (0.87 g, 3.4 mmol) and 1,2,3-trichloro-5-nitrobenzene (0.87 g, 3.8 mmol) in *N*,*N*-dimethylformamide (15 mL) was added potassium carbonate (0.93 g, 6.8 mmol), and the mixture was reacted at 70°C for 17 hours. The reaction solution was cooled to room temperature, then water (30 mL) was added. The reaction mixture was stirred for 10 minutes, filtered, and rinsed with water (10 mL). The filter cake was collected and dried to obtain an off-white solid **28d** (1.50 g, yield 99%).

MS (ESI, pos. ion) *m*/*z*: 448.0 [M+H]⁺.

### Step 4: Synthesis of 6-(4-amino-2,6-dichlorophenoxy)-2-(4-fluorophenyl)-3,4-dihydroisoquinolin-1(2H)-one 28e

To a solution of 6-(2,6-dichloro-4-nitrophenoxy)-2-(4-fluorophenyl)-3,4-dihydroisoquinolin-1(2*H*)-one **28d** (1.50 g, 3.30 mmol) in acetic acid (20 mL) was added iron powder (0.37 g, 6.70 mmol), and the mixture was reacted at 50°C for 3 hours. The reaction solution was cooled to room temperature, iron powder was removed, and water (80 mL) was added. The mixture was extracted with ethyl acetate (80 mL × 2). The combined organic layers were washed with saturated sodium chloride (100 mL), dried over anhydrous sodium sulfate and concentrated by suction filtration. The residue was purified by silica gel column chromatography (petroleum ether/ethyl acetate = 1/1) to give light yellow oil **28e** (1.05 g, yield 75%).

MS (ESI, pos. ion) *m*/*z*: 417.1 [M+H]⁺.

### Step 5: Synthesis of ethyl

### (2-cyano-2-(2-(3,5-dichloro-4-((2-(4-fluorophenyl)-1-oxo-1,2,3,4-tetrahydroisoquinolin-6-yl)oxy)phenyl)hydrazo no)acetyl)carbamate 28f

To a solution of 6-(4-amino-2,6-dichlorophenoxy)-2-(4-fluorophenyl)-3,4-dihydroisoquinolin-1(2*H*)-one **28e** (1.00 g, 2.40 mmol) in acetic acid (36 mL) was added a solution of sodium nitrite (0.33 g, 4.8 mmol) in water (18 mL) dropwise at 0°C. After reacting for 15 minutes, *N*-cyanoacetylurethane (0.56 g, 3.60 mmol) was added, and the mixture was reacted for 5 hours. Water (50 mL) was added to the reaction solution. The mixture was stirred for 10 minutes, filtered, washed with water (10 mL), and the filter cake was collected and dried to obtain a yellow solid **28f** (1.30 g, yield 93%).

MS (ESI, pos. ion) *m*/*z*: 585.1 [M+H]⁺.

### Step 6: Synthesis of

### 2-(3,5-dichloro-4-((2-(4-fluorophenyl)-1-oxo-1,2,3,4-tetrahydroisoquinolin-6-yl)oxy)phenyl)-3,5-dioxo-2,3,4,5-tet rahydro-1,2,4-triazine-6-carbonitrile 28g

To a solution of ethyl (2-cyano-2-(2-(3,5-dichloro-4-((2-(4-fluorophenyl)-1-oxo-1,2,3,4-tetrahydroisoquinolin-6-yl)oxy)phenyl)hydrazo no)acetyl)carbamate **28f** (1.30 g, 2.20 mmol) in *N*,*N*-dimethylformamide (40 mL) was added sodium acetate (0.40 g, 2.90 mmol), and the mixture was reacted at 120°C for 15 hours. The reaction solution was cooled to room temperature, and water (80 mL) was added to quench the reaction. The mixture was extracted with ethyl acetate (100 mL × 2). The combined organic layers were washed with saturated sodium chloride solution (100 mL), dried over anhydrous sodium sulfate and concentrated by suction filtration. The resulting residue was purified by silica gel column chromatography (100% ethyl acetate), and the obtained solid was recrystallized (petroleum ether/ethyl acetate = 1/2, 24 mL) at 80°C to obtain a yellow solid **28g** (0.71 g, yield 59%, HPLC purity: 97.86%).
MS (ESI, neg. ion) *m*/*z*: 537.1 [M-H]⁻;
¹H NMR (400 MHz, DMSO-*d₆*) δ (ppm) 13.31 (s, 1H), 7.95 (d, *J* = 8.6 Hz, 1H), 7.86 (s, 2H), 7.43 (dd, *J* = 8.8, 5.0 Hz, 2H), 7.25 (t, *J* = 8.8 Hz, 2H), 6.95 (d, *J* = 2.6 Hz, 1H), 6.88 (dd, *J* = 8.6, 2.7 Hz, 1H), 3.93 (t, *J* = 6.4 Hz, 2H), 3.13 (t, *J* = 6.5 Hz, 2H).

### Step 7: Synthesis of

### 2-(3,5-dichloro-4-((2-(4-fluorophenyl)-1-oxo-1,2,3,4-tetrahydroisoguinolin-6-yl)oxy)phenyl)-3,5-dioxo-2,3,4,5-tet rahydro-1,2,4-triazine-6-carboxylic acid 28h

2-(3,5-Dichloro-4-((2-(4-fluorophenyl)-1-oxo-1,2,3,4-tetrahydroisoquinolin-6-yl)oxy)phenyl)-3,5-dioxo-2,3,4,5-tetrahydro-1,2,4-triazine-6-carbonitrile **28g** (0.45 g, 0.84 mmol) was dissolved in acetic acid (8 mL), then concentrated hydrochloric acid (4 mL) was added. The mixture was reacted at 100°C for 17 hours. The reaction solution was cooled to room temperature, then water (20 mL) was added. The reaction mixture was stirred for 10 minutes, filtered, and rinsed with water (10 mL × 2). The filter cake was collected and dried to obtain a yellow solid **28h** (0.35 g, yield 75%).

### Step 8: Synthesis of

### 2-(3,5-dichloro-4-((2-(4-fluorophenyl)-1-oxo-1,2,3,4-tetrahydroisoquinolin-6-yl)oxy)phenyl)-1,2,4-triazine-3,5(2 H,4H)-dione 28

2-(3,5-Dichloro-4-((2-(4-fluorophenyl)-1-oxo-1,2,3,4-tetrahydroisoquinolin-6-yl)oxy)phenyl)-3,5-dioxo-2,3,4,5-tetrahydro-1,2,4-triazine-6-carboxylic acid **28h** (0.35 g, 0.63 mmol) was dissolved in thioglycolic acid (2.5 mL). The mixture was reacted at 140°C for 24 hours. The reaction solution was cooled to room temperature, and ethyl acetate (60 mL) was added. The mixture was washed successively with water (20 mL), saturated sodium bicarbonate solution (30 mL × 2) and saturated sodium chloride solution (30 mL), dried over anhydrous sodium sulfate and concentrated by suction filtration. The residue was purified by silica gel column chromatography (petroleum ether/ethyl acetate = 1/3) to give a yellow solid **28** (0.21 g, yield 61%, HPLC purity: 96.79%).
MS (ESI, neg. ion) *m*/*z*: 514.1 [M+H]⁺;
¹H NMR (400 MHz, DMSO-*d₆*) δ (ppm) 12.52 (s, 1H), 7.95 (d, *J* = 8.6 Hz, 1H), 7.88 (s, 2H), 7.73 (s, 1H), 7.43 (dd, *J* = 8.9, 5.2 Hz, 2H), 7.24 (t, *J* = 8.8 Hz, 2H), 6.93 (d, *J* = 2.6 Hz, 1H), 6.85 (dd, *J* = 8.6, 2.6 Hz, 1H), 3.92 (t, *J* = 6.4 Hz, 2H), 3.13 (t, *J* = 6.4 Hz, 2H).

### Example 29

### 2-(3,5-dichloro-4-((1-oxo-2-(4-(trifluoromethyl)benzyl)-1,2,3,4-tetrahydroisoquinolin-6-yl)oxy)phenyl)-1,2,4-triazine-3,5(2H,4H)-dione 29

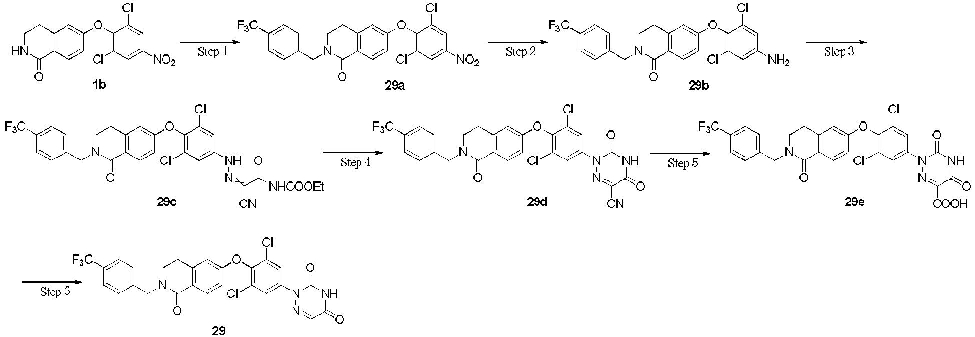

### Step 1: Synthesis of

### 6-(2,6-dichloro-4-nitrophenoxy)-2-(4-(trifluoromethyl)benzyl)-3,4-dihydroisoquinolin-1(2H)-one 29a

To a solution of 6-(2,6-dichloro-4-nitrophenoxy)-3,4-dihydroisoquinolin-1(2*H*)-one **1b** (1.50 g, 4.2 mmol) in THF (20 mL) was added sodium hydride (0.25 mg, 6.3 mmol, 60 mass% in oil) at 0°C. After 30 minutes of reaction, 1-bromomethyl-4-(trifluoromethyl)benzene (2.0 g, 8.37 mmol) and N,N-dimethylformamide (6 mL) were added dropwise, and the mixture was reacted at room temperature for 3.5 hours. The reaction was quenched with ice water (20 mL). The mixture was extracted with ethyl acetate (30 mL × 2). The combined organic layers were washed with saturated sodium chloride (10 mL), dried over anhydrous sodium sulfate and concentrated by suction filtration. The residue was purified by silica gel column chromatography (petroleum ether/ethyl acetate (v/v) = 5/1) to give light yellow oil **29a** (2.2 g, yield 100%).

MS (ESI, pos. ion) *m*/*z*: 511.0 [M+H]⁺.

### Step 2: Synthesis of

### 6-(4-amino-2,6-dichlorophenoxy)-2-(4-(trifluoromethyl)benzyl)-3,4-dihydroisoquinolin-1(2H)-one 29b

To a solution of 6-(2,6-dichloro-4-nitrophenoxy)-2-(4-(trifluoromethyl)benzyl)-3,4-dihydroisoquinolin-1(2*H*)-one **29a** (2.2 g, 4.2 mmol) in acetic acid (50 mL) was added iron powder (0.99 g, 17.8 mmol), and the mixture was reacted at 55°C for 10 hours. The reaction solution was cooled to room temperature. The reaction was quenched with water (100 mL). The mixture was extracted with ethyl acetate (50 mL × 3). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 3/1) to give light yellow oil **29b** (1.65 g, yield 77%).

### Step 3: Synthesis of ethyl

### (2-cyano-2-(2-(3,5-dichloro-4-((1-oxo-2-(4-(trifluoromethyl)benzyl)-1,2,3,4-tetrahydroisoqunolin-6-yl)oxy)phen yl)hydrazono)acetyl)carbamate 29c

To a solution of 6-(4-amino-2,6-dichlorophenoxy)-2-(4-(trifluoromethyl)benzyl)-3,4-dihydroisoquinolin-1(2*H*)-one **29b** (0.60 g, 1.25 mmol) in acetic acid (12 mL) was added a solution of sodium nitrite (0.19 g, 12.78 mmol) in water (6 mL) at 0°C, then *N*-cyanoacetylurethane (0.26 g, 1.67 mmol) was added. The mixture was reacted for 3 hours. Water (100 mL) was added to quench the reaction. The mixture was stirred for 20 minutes, filtered, and the filter cake was washed with water (5 mL × 2). Then the filter cake was collected and dried to obtain a light yellow solid **29c** (0.76 g, yield 94%).

### Step 4: Synthesis of

### 2-(3,5-dichloro-4-((1-oxo-2-(4-(trifluoromethyl)benzyl)-1,2,3,4-tetrahydroisoquinolin-6-yl)oxy)phenyl)-3,5-dioxo -2,3,4,5-tetrahydro-1,2,4-triazine-6-carbonitrile 29d

To a solution of ethyl (2-cyano-2-(2-(3,5-dichloro-4-((1-oxo-2-(4-(trifluoromethyl)benzyl)-1,2,3,4-tetrahydroisoquinolin-6-yl)oxy)phen yl)hydrazono)acetyl)carbamate **29c** (0.72 g, 1.12 mmol) in *N*,*N*-dimethylformamide (25 mL) was added sodium acetate (0.20 g, 2.45 mmol), and the mixture was reacted at 120°C for 6 hours. The reaction solution was cooled to room temperature, then water (20 mL) was added to quench the reaction. The reaction mixture was stirred for 15 minutes, and filtered. The filter cake was collected and dried, recrystallized (ethyl acetate/petroleum ether (v/v) = 2/1, 15 mL) at 80°C to obtain a yellow solid **29d** (0.65 g, yield 97%, HPLC purity: 96.68%).
MS (ESI, neg. ion) *m*/*z*: 600.0 [M-H]⁻;
¹H NMR (400 MHz, DMSO-*d₆*) δ (ppm) 13.30 (s, 1H), 7.94 (d, *J* = 8.4 Hz, 1H), 7.84 (s, 2H), 7.71 (d, *J* = 8.0 Hz, 2H), 7.54 (d, *J* = 8.0 Hz, 2H), 6.88 (d, *J* = 2.6 Hz, 1H), 6.85 (dd, *J* = 8.3, 2.7 Hz, 1H), 4.78 (s, 2H), 3.53 (t, *J* = 6.6 Hz, 2H), 2.99 (t, *J* = 6.6 Hz, 2H).

### Step 5: Synthesis of

### 2-(3,5-dichloro-4-((1-oxo-2-(4-(trifluoromethyl)benzyl)-1,2,3,4-tetrahydroisoquinolin-6-yl)oxy)phenyl)-3,5-dioxo -2,3,4,5-tetrahydro-1,2,4-triazine-6-carboxylic acid 29e

2-(3,5-Dichloro-4-((1-oxo-2-(4-(trifluoromethyl)benzyl)-1,2,3,4-tetrahydroisoquinolin-6-yl)oxy)ph enyl)-3,5-dioxo-2,3,4,5-tetrahydro-1,2,4-triazine-6-carbonitrile **29d** (0.65 g, 1.01 mmol) was dissolved in acetic acid (10 mL), then concentrated hydrochloric acid (5 mL) was added. The mixture was reacted at 120°C for 13 hours. The reaction solution was cooled to room temperature, then water (20 mL) was added. The reaction mixture was stirred for 10 minutes, filtered, and rinsed with water (10 mL × 2). The filter cake was collected and dried to obtain a yellow solid **29e** (0.55 g, yield 82%).

### Step 6: Synthesis of

### 2-(3,5-dichloro-4-((1-oxo-2-(4-(trifluoromethyl)benzyl)-1,2,3,4-tetrahydroisoquinolin-6-yl)oxy)phenyl)-1,2,4-tria zinc-3,5(2H-4H)-dione 29

2-(3,5-Dichloro-4-((1-oxo-2-(4-(trifluoromethyl)benzyl)-1,2,3,4-tetrahydroisoquinolin-6-yl)oxy)ph enyl)-3,5-dioxo-2,3,4,5-tetrahydro-1,2,4-triazine-6-carboxylic acid **29e** (0.55 g, 0.89 mmol) was dissolved in thioglycolic acid (5 mL). The mixture was reacted at 150°C for 24 hours. The reaction solution was cooled to room temperature, and ethyl acetate (30 mL) was added. The mixture was washed successively with water (10 mL), saturated sodium bicarbonate solution (10 mL) and saturated sodium chloride solution (10 mL), then dried over anhydrous sodium sulfate and concentrated by suction filtration. The resulting residue was purified by silica gel column chromatography (petroleum ether/ethyl acetate (v/v) = 2/1), and the obtained solid was recrystallized at 70°C (ethyl acetate/methanol/petroleum ether (v/v/v) = 5/1/10, 32 mL) to give a white solid **29** (0.32 g, yield 62%, HPLC purity: 96.97%).
MS (ESI, neg. ion) *m*/*z*: 575.0 [M-H]⁻;
¹H NMR (400 MHz, DMSO-*d₆*) δ (ppm) 12.52 (s, 1H), 7.94 (d, *J* = 8.4 Hz, 1H), 7.87 (s, 2H), 7.73 (s, 1H), 7.70 (d, *J* = 7.9 Hz, 2H), 7.54 (d, *J* = 7.9 Hz, 2H), 6.85 (s, 1H), 6.84-6.79 (m, 1H), 4.78 (s, 2H), 3.52 (t, *J* = 6.5 Hz, 2H), 2.98 (t, *J* = 6.6 Hz, 2H).

### Example 30

### 2-(3,5-dichloro-4-((2-(3,4-difluorobenzyl)-1-oxo-1,2,3,4-tetrahydroisoquinolin-6-yl)oxy)phenyl)-1,2,4-triazin e-3,5(2H,4H)-dione 30

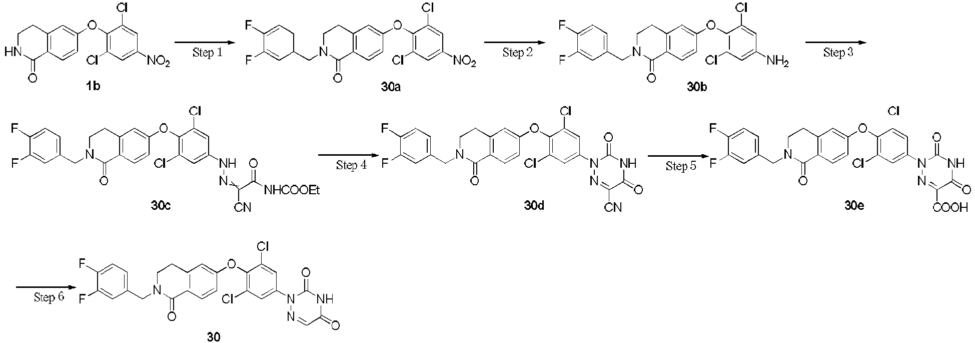

### Step 1: Synthesis of

### 6-(2,6-dichloro-4-nitrophenoxy)-2-(3,4-difluorobenzyl)-3,4-dihydroisoquinolin-1(2H)-one 30a

To a solution of 6-(2,6-dichloro-4-nitrophenoxy)-3,4-dihydroisoquinolin-1(2*H*)-one **1b** (1.00 g, 2.83 mmol) in THF (40 mL) was added sodium hydride (0.17 g, 4.25 mmol, 60 mass% in oil) at 0°C. After 30 minutes of reaction, 3,4-difluorobenzyl bromide (1.17 g, 5.66 mmol) and *N*,*N*-dimethylformamide (6 mL) were added dropwise, and the mixture was reacted at room temperature for 12 hours. The reaction was quenched with ice water (30 mL). The mixture was extracted with ethyl acetate (30 mL × 2). The combined organic layers were washed with saturated sodium chloride (20 mL), dried over anhydrous sodium sulfate and concentrated by suction filtration. The residue was purified by silica gel column chromatography (petroleum ether/ethyl acetate (v/v) = 4/1) to give light yellow oil **30a** (1.10 g, yield 81%).

MS (ESI, pos. ion) *m*/*z:* 479.1 [M+H]⁺.

### Step 2: Synthesis of

### 6-(4-amino-2,6-dichlorophenoxy)-2-(3,4-difluorobenzyl)-3,4-dihydroisoquinolin-1(2H)-one 30b

To a solution of 6-(2,6-dichloro-4-nitrophenoxy)-2-(3,4-difluorobenzyl)-3,4-dihydroisoquinolin-1(2*H*)-one **30a** (1.10 g, 2.29 mmol) in acetic acid (50 mL) was added iron powder (0.51 g, 9.15 mmol), and the mixture was reacted at 55°C for 6 hours. The reaction solution was cooled to room temperature. The reaction was quenched with water (80 mL). The mixture was extracted with ethyl acetate (50 mL × 3). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 3/1) to give light yellow oil **30b** (0.64 g, yield 62%).

MS (ESI, pos. ion) *m*/*z*: 431.0 [M+H]⁺.

### Step 3: Synthesis of ethyl

### (2-cyano-2-(2-(3,5-dichloro-4-((2-(3,4-difluorobenzyl)-1-oxo-1,2,3,4-tetrahydroisoquinolin-6-yl)oxy)phenyl)hydr azono)acetyl)carbamate 30c

To a solution of 6-(4-amino-2,6-dichlorophenoxy)-2-(3,4-difluorobenzyl)-3,4-dihydroisoquinolin-1(2*H*)-one **30b** (0.65 g, 1.42 mmol) in acetic acid (12 mL) was added a solution of sodium nitrite (0.20 g, 2.90 mmol) in water (6 mL) dropwise at 0°C, then *N*-cyanoacetylurethane (0.27 g, 1.71 mmol) was added. The mixture was reacted for 2 hours. Water (100 mL) was added to quench the reaction. The mixture was stirred for 20 minutes, filtered, and the filter cake was washed with water (5 mL × 2). Then the filter cake was collected and dried to obtain a light yellow solid **30c** (0.94 g, yield 100%).

### Step 4: Synthesis of

### 2-(3,5-dichloro-4-((2-(3,4-difluorobenzyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 30d

To a solution of ethyl (2-cyano-2-(2-(3,5-dichloro-4-((2-(3,4-difluorobenzyl)-1-oxo-1,2,3,4-tetrahydroisoquinolin-6-yl)oxy)phenyl)hydr azono)acetyl)carbamate **30c** (0.94 g, 1.53 mmol) in *N,N*-dimethylformamide (30 mL) was added sodium acetate (0.28 g, 3.36 mmol), and the mixture was reacted at 120°C for 6 hours. The reaction solution was cooled to room temperature, then water (20 mL) was added to quench the reaction. The reaction mixture was stirred for 15 minutes, and filtered. The filter cake was collected and dried, recrystallized (ethyl acetate/petroleum ether (v/v) = 2/1, 15 mL) at 80°C to obtain a yellow solid **30d** (0.45 g, yield 52%, HPLC purity: 94.04%).
MS (ESI, neg. ion) *m*/*z*: 550.0 [M-H]⁻;
¹H NMR (400 MHz, DMSO-*d₆*) δ (ppm) 13.29 (s, 1H), 7.93 (d, *J =* 8.3 Hz, 1H), 7.84 (s, 2H), 7.39 (td, *J* = 7.9, 6.1 Hz, 1H), 7.18-7.07 (m, 3H), 6.86 (s, 1H), 6.84 (d, *J* = 2.7 Hz, 1H), 4.70 (s, 2H), 3.51 (t, *J* = 6.6 Hz, 2H), 2.97 (t, *J* = 6.6 Hz, 2H).

### Step 5: Synthesis of

### 2-(3,5-dichloro-4-((2-((3,4-difluorobenzyl)-1-oxo-1,2,3,4-tetrahydroisoquinolin-6-yl)oxy)phenyl)-3,5-dioxo-2,3,4, 5-tetrahydro-1,2,4-triazine-6-carboxylic acid 30e

2-(3,5-Dichloro-4-((2-(3,4-difluorobenzyl)-1-oxo-1,2,3,4-tetrahydroisoquinolin-6-yl)oxy)phenyl)-3 ,5-dioxo-2,3,4,5-tetrahydro-1,2,4-triazine-6-carbonitrile **30d** (0.45 g, 0.79 mmol) was dissolved in acetic acid (6 mL), then concentrated hydrochloric acid (3 mL) was added. The mixture was reacted at 120°C for 12 hours. The reaction solution was cooled to room temperature, then water (20 mL) was added. The reaction mixture was stirred for 10 minutes, filtered, and rinsed with water (10 mL × 2). The filter cake was collected and dried to obtain a yellow solid **30e** (0.41 g, yield 89%).

### Step 6: Synthesis of

### 2-(3,5-dichloro-4-((2-(3,4-difluorobenzyl)-1-oxo-1,2,3,4-tetrahydroisoquinolin-6-yl)oxy)phenyl)-1,2,4-triazine-3, 5(2H,4H)-dione 30

2-(3,5-Dichloro-4-((2-(3,4-difluorobenzyl)-1-oxo-1,2,3,4-tetrahydroisoquinolin-6-yl)oxy)phenyl)-3 ,5-dioxo-2,3,4,5-tetrahydro-1,2,4-triazine-6-carboxylic acid **30e** (0.41 g, 0.68 mmol) was dissolved in thioglycolic acid (3 mL). The mixture was reacted at 140°C for 19 hours. The reaction solution was cooled to room temperature, and ethyl acetate (30 mL) was added. The mixture was washed successively with water (10 mL), saturated sodium bicarbonate solution (10 mL) and saturated sodium chloride solution (10 mL), dried over anhydrous sodium sulfate and concentrated by suction filtration. The residue was purified by silica gel column chromatography (petroleum ether/ethyl acetate (v/v) = 3/1), and the obtained solid was recrystallized at 70°C (ethyl acetate/methanol/petroleum ether (v/v/v) = 5/1/10, 32 mL) to give a white solid **30** (0.25 g, yield 67%, HPLC purity: 93.81%).
MS (ESI, neg. ion) *m*/*z*: 543.0 [M-H]⁻;
¹H NMR (400 MHz, DMSO-*d₆*) δ (ppm) 12.52 (s, 1H), 7.93 (d, *J =* 8.3 Hz, 1H), 7.87 (s, 2H), 7.72 (s, 1H), 7.45-7.32 (m, 2H), 7.18 (t, *J=* 6.4 Hz, 1H), 6.83 (s, 1H), 6.81 (d, *J* = 2.6 Hz, 1H), 4.66 (s, 2H), 3.50 (t, *J* = 6.5 Hz, 2H), 2.97 (t, *J* = 6.6 Hz, 2H).

### Example 31

### 2-(3,5-dichloro-4-((2-(3-fluorobenzyl)-1-oxo-1,2,3,4-tetrahydroisoquinolin-6-yl)oxy)phenyl)-1,2,4-triazine-3, 5(2H,4H)-dione 31

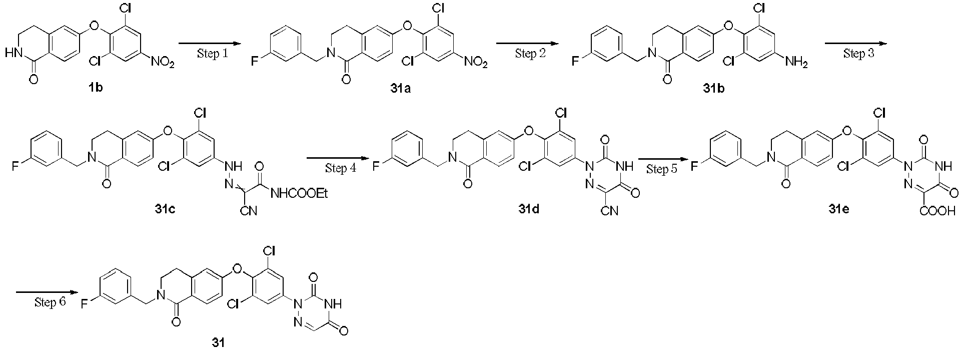

### Step 1: Synthesis of 6-(2,6-dichloro-4-nitrophenoxy)-2-(3-fluorobenzyl)-3,4-dihydroisoquinolin-1(2H)-one 31a

To a solution of 6-(2,6-dichloro-4-nitrophenoxy)-3,4-dihydroisoquinolin-1(2*H*)-one **1b** (1.00 g, 2.83 mmol) in THF (14 mL) was added sodium hydride (0.17 g, 7.08 mmol, 60 mass% in oil). After 30 minutes of reaction, 1-bromomethyl-3-fluorobenzene (1.07 g, 5.66 mmol) and *N,N*-dimethylformamide (6 mL) were added dropwise, and the mixture was reacted at room temperature for 3 hours. The reaction was quenched with ice water (10 mL). The mixture was extracted with ethyl acetate (30 mL × 2). The combined organic layers were washed with saturated sodium chloride (20 mL), dried over anhydrous sodium sulfate and concentrated by suction filtration. The residue was purified by silica gel column chromatography (petroleum ether/ethyl acetate (v/v) = 4/1) to give light yellow **oil 31a** (1.30 g, yield 99%).

MS (ESI, pos. ion) *m*/*z*: 461.1 [M+H]⁺.

### Step 2: Synthesis of 6-(4-amino-2,6-dichlorophenoxy)-2-(3-fluorobenzyl)-3,4-dihydroisoquinolin-1(2H)-one 31b

To a solution of 6-(2,6-dichloro-4-nitrophenoxy)-2-(3-fluorobenzyl)-3,4-dihydroisoquinolin-1(2*H*)-one **31a** (1.80 g, 3.90 mmol) in acetic acid (50 mL) was added iron powder (0.87 g, 15.2 mmol), and the mixture was reacted at 55°C for 7 hours. The reaction solution was cooled to room temperature. The reaction was quenched with water (80 mL). The mixture was extracted with ethyl acetate (50 mL × 3). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 3/1) to give a white solid **31b** (1.29 g, yield 77%).

### Step 3: Synthesis of ethyl

### (2-cyano-2-(2-(3,5-dichloro-4-((2-(3-fluorobenzyl)-1-oxo-1,2,3,4-tetrahydroisoquinolin-6-yl)oxy)phenyl)hydrazo no)acetyl)carbamate 31c

To a solution of 6-(4-amino-2,6-dichlorophenoxy)-2-(3-fluorobenzyl)-3,4-dihydroisoquinolin-1(2*H*)-one **31b** (0.60 g, 1.39 mmol) in acetic acid (12 mL) was added a solution of sodium nitrite (0.19 g, 2.78 mmol) in water (5 mL) dropwise at 0°C, then N-cyanoacetylurethane (0.26 g, 1.67 mmol) was added. The mixture was reacted for 2 hours. Water (100 mL) was added to quench the reaction. The mixture was stirred for 20 minutes, filtered, and the filter cake was washed with water (5 mL × 2). Then the filter cake was collected and dried to obtain a light yellow solid **31c** (0.92 g, yield 100%).

### Step 4: Synthesis of

### 2-(3,5-dichloro-4-((2-(3-fluorobenzyl)-1-oxo-1,2,3,4-tetrahydroisoquinolin-6-yl)oxy)phenyl)-3,5-dioxo-2,3,4,5-tet rahydro-1,2,4-triazine-6-carbonitrile 31d

To a solution of ethyl (2-cyano-2-(2-(3,5-dichloro-4-((2-(3-fluorobenzyl)-1-oxo-1,2,3,4-tetrahydroisoquinolin-6-yl)oxy)phenyl)hydrazo no)acetyl)carbamate **31c** (0.92 g, 1.54 mmol) in *N,N*-dimethylformamide (30 mL) was added sodium acetate (0.28 g, 3.39 mmol), and the mixture was reacted at 120°C for 6 hours. The reaction solution was cooled to room temperature, then water (20 mL) was added to quench the reaction. The reaction mixture was stirred for 15 minutes, and filtered. The filter cake was collected and dried, recrystallized (ethyl acetate/petroleum ether (v/v) = 2/1, 15 mL) at 80°C to obtain a yellow solid **31d** (0.75 g, yield 99%)

### Step 5: Synthesis of

### 2-(3,5-dichloro-4-((2-((3-fluorobenzyl)-1-oxo-1,2,3,4-tetrahydroisoquinolin-6-yl)oxy)phenyl)-3,5-dioxo-2,3,4,5-te trahydro-1,2,4-triazine-6-carboxylic acid 31e

2-(3,5-Dichloro-4-((2-(3-fluorobenzyl)-1-oxo-1,2,3,4-tetrahydroisoquinolin-6-yl)oxy)phenyl)-3,5-d ioxo-2,3,4,5-tetrahydro-1,2,4-triazine-6-carbonitrile **31d** (0.39 g, 0.71 mmol) was dissolved in acetic acid (10 mL), then concentrated hydrochloric acid (3 mL) was added. The mixture was reacted at 120°C for 12 hours. The reaction solution was cooled to room temperature, then water (20 mL) was added. The reaction mixture was stirred for 10 minutes, filtered, and rinsed with water (10 mL × 2). The filter cake was collected and dried to obtain a yellow solid **31e** (0.40 g, yield 98%).

### Step 6: Synthesis of

### 2-(3,5-dichloro-4-((2-(3-fluorobenzyl)-1-oxo-1,2,3,4-tetrahydroisoquinolin-6-yl)oxy)phenyl)-1,2,4-triazine-3,5(2 H,4H)-dione 31

2-(3,5-Dichloro-4-((2-(3-fluorobenzyl)-1-oxo-1,2,3,4-tetrahydroisoquinolin-6-yl)oxy)phenyl)-3,5-d ioxo-2,3,4,5-tetrahydro-1,2,4-triazine-6-carboxylic acid **31e** (0.40 g, 0.70 mmol) was dissolved in thioglycolic acid (3 mL). The mixture was reacted at 140°C for 19 hours. The reaction solution was cooled to room temperature, and ethyl acetate (30 mL) was added. The mixture was washed successively with water (10 mL), and saturated sodium bicarbonate solution (10 mL), dried over anhydrous sodium sulfate and concentrated by suction filtration. The residue was purified by silica gel column chromatography (petroleum ether/ethyl acetate (v/v) = 3/1), and the obtained solid was recrystallized at 70°C (ethyl acetate/methanol/petroleum ether (v/v/v) = 5/1/10, 32 mL) to give a white solid 31 (0.20 g, yield 54%, HPLC purity: 93.81%).
MS (ESI, neg. ion) *m*/*z*: 525.0[M-H]⁻;
¹H NMR (400 MHz, DMSO-*d₆*) δ(ppm) 12.52 (s, 1H), 7.93 (d, *J =* 8.3 Hz, 1H), 7.86 (s, 2H), 7.73 (s, 1H), 7.38 (td, *J=* 7.9, 6.0 Hz, 1H), 7.18- 7.06 (m, 3H), 6.86-6.77 (m, 2H), 4.69 (s, 2H), 3.50 (t, *J* = 6.5 Hz, 2H), 2.96 (t, *J* = 6.6 Hz, 2H).

### Example 32

### 2-(3,5-dichloro-4-((2-(3,5-difluorobenzyl)-1-oxo-1,2,3,4-tetrahydroisoquinolin-6-yl)oxy)phenyl)-1,2,4-triazin e-3,5(2H,4H)-dione 32

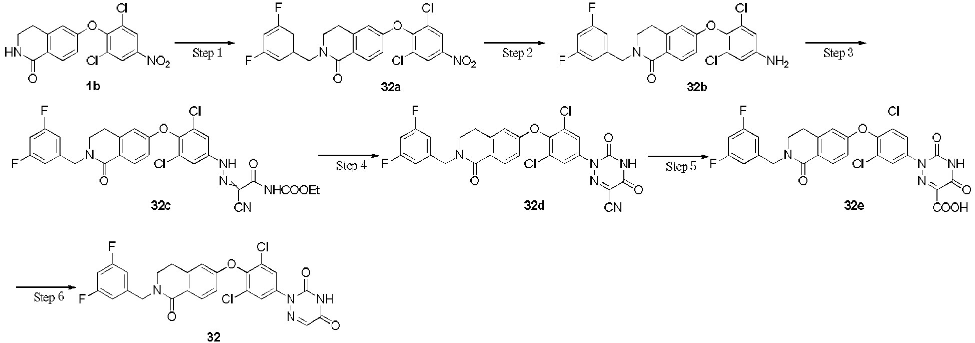

### Step 1: Synthesis of

### 2-(3,5-difluorobenzyl)-6-(2,6-dichloro-4-nitrophenoxy)-3,4-dihydroisoquinolin-1(2H)-one 32a

To a solution of 6-(2,6-dichloro-4-nitrophenoxy)-3,4-dihydroisoquinolin-1(2*H*)-one 1b (1.0 g, 2.83 mmol) in THF (14 mL) was added sodium hydride (0.17 g, 4.25 mmol, 60 mass% in oil) at 0°C. After 20 minutes of reaction, 3,5-difluorobenzyl bromide (1.17 g, 5.66 mmol) and *N*,*N*-dimethylformamide (10 mL) were added dropwise, and the mixture was reacted at room temperature for 6 hours. The reaction was quenched with ice water (30 mL). The mixture was extracted with ethyl acetate (30 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 3/1) to give light yellow **oil 32a** (1.26 g, yield 93%).

MS (ESI, pos. ion) *m*/*z*: 480.1 [M+H]⁺.

### Step 2: Synthesis of

### 6-(4-amino-2,6-dichlorophenoxy)-2-(3,5-difluorobenzyl)-3,4-dihydroisoquinolin-1(2H)-one 32b

To a solution of 2-(3,5-difluorobenzyl)-6-(2,6-dichloro-4-nitrophenoxy)-3,4-dihydroisoquinolin-1(2*H*)-one **32a** (0.99 g, 2.1 mmol) in acetic acid (20 mL) was added iron powder (0.24 g, 4.3 mmol), and the mixture was reacted at 50°C for 2.5 hours. The reaction solution was cooled to room temperature. The reaction was quenched with water (30 mL). The mixture was extracted with ethyl acetate (50 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 2/1) to give light yellow oil **32b** (0.68 g, yield 58%).

### Step 3: Synthesis of ethyl

### (2-cyano-2-(2-(3,5-dichloro-4-((2-(3,5-difluorobenzyl)-1-oxo-1,2,3,4-tetrahydroisoquinolin-6-yl)oxy)phenyl)hydr azono)acetyl)carbamate 32c

To a solution of 6-(4-amino-2,6-dichlorophenoxy)-2-(3,5-difluorobenzyl)-3,4-dihydroisoquinolin-1(2*H*)-one **32b** (0.68 g, 1.5 mmol) in acetic acid (20 mL) was added a solution of sodium nitrite (0.21 g, 3.0 mmol) in water (9 mL) dropwise at 0°C, then *N*-cyanoacetylurethane (0.35 g, 2.3 mmol) was added. The mixture was reacted for 3 hours. Water (60 mL) was added to quench the reaction. The mixture was stirred for 20 minutes, filtered, and the filter cake was washed with water (10 mL). Then the filter cake was collected and dried to obtain a light yellow solid **32c** (0.93 g, yield 100%).

MS (ESI, neg. ion) *m*/*z*: 615.2 [M-H]⁻.

### Step 4: Synthesis of

### 2-(3,5-dichloro-4-((2-(3,5-difluorobenzyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 32d

To a solution of ethyl (2-cyano-2-(2-(3,5-dichloro-4-((2-(3,5-difluorobenzyl)-1-oxo-1,2,3,4-tetrahydroisoquinolin-6-yl)oxy)phenyl)hydr azono)acetyl)carbamate **32c** (0.93 g, 1.5 mmol) in *N*,*N*-dimethylformamide (16 mL) was added sodium acetate trihydrate (0.27 g, 2.0 mmol), and the mixture was reacted at 120°C for 8 hours. The reaction solution was cooled to room temperature, then water (50 mL) was added to quench the reaction. The reaction mixture was stirred for 15 minutes, and filtered. The filter cake was collected and dried, recrystallized (ethyl acetate/petroleum ether (v/v) = 2/1, 30 mL) at 80°C to obtain a yellow solid **32d** (0.63 g, yield 73%, HPLC purity: 95.85%).
MS (ESI, neg. ion) *m*/*z*: 571.3 [M-H]⁻;
¹H NMR (400 MHz, DMSO-*d₆*) δ (ppm) 13.27 (s, 1H), 7.92 (d, *J* = 8.4 Hz, 1H), 7.84 (s, 2H), 7.12 (td, *J* = 9.4, 4.8 Hz, 1H), 7.03 (d, *J* = 7.3 Hz, 2H), 6.91-6.76 (m, 2H), 4.69 (s, 2H), 3.53 (t, *J* = 6.6 Hz, 2H), 2.99 (t, *J* = 6.6 Hz, 2H).

### Step 5: Synthesis of

### 2-(3,5-dichloro-4-((2-((3,5-difluorobenzyl)-1-oxo-1,2,3,4-tetrahydroisoquinolin-6-yl)oxy)phenyl)-3,5-dioxo-2,3,4, 5-tetrahydro-1,2,4-triazine-6-carboxylic acid 32e

2-(3,5-Dichloro-4-((2-(3,5-difluorobenzyl)-1-oxo-1,2,3,4-tetrahydroisoquinolin-6-yl)oxy)phenyl)-3 ,5-dioxo-2,3,4,5-tetrahydro-1,2,4-triazine-6-carbonitrile **32d** (0.55 g, 0.96 mmol) was dissolved in acetic acid (10 mL), then concentrated hydrochloric acid (5 mL) was added. The mixture was reacted at 100°C for 8 hours. The reaction solution was cooled to room temperature, then water (30 mL) was added. The reaction mixture was stirred for 10 minutes, filtered, and rinsed with water (10 mL × 2). The filter cake was collected and dried to obtain a yellow solid **32e** (0.48 g, yield 84%).

MS (ESI, pos. ion) *m*/*z:* 590.0 [M+H]⁺.

### Step 6: Synthesis of

### 2-(3,5-dichloro-4-((2-(3,4-difluorobenzyl)-1-oxo-1,2,3,4-tetrahydroisoquinolin-6-yl)oxy)phenyl)-1,2,4-triazine-3, 5(2H,4H)-dione 32

2-(3,5-Dichloro-4-((2-(3,5-difluorobenzyl)-1-oxo-1,2,3,4-tetrahydroisoquinolin-6-yl)oxy)phenyl)-3 ,5-dioxo-2,3,4,5-tetrahydro-1,2,4-triazine-6-carboxylic acid **32e** (0.48 g, 0.81 mmol) was dissolved in thioglycolic acid (5 mL). The mixture was reacted at 140°C for 16 hours. The reaction solution was cooled to room temperature, and ethyl acetate (60 mL) was added. The mixture was washed successively with 50% sodium bicarbonate solution (30 mL × 2) and saturated sodium chloride solution (30 mL), dried over anhydrous sodium sulfate and concentrated by suction filtration. The residue was purified by silica gel column chromatography (petroleum ether/ethyl acetate (v/v) = 3/2), and the obtained solid was recrystallized at 80°C (ethyl acetate/petroleum ether (v/v) = 1/2, 30 mL) to give a light yellow solid **32** (0.20 g, yield 45%, HPLC purity: 99.48%).
MS (ESI, neg. ion) *m*/*z:* 544.2 [M-H]⁻;
¹H NMR (400 MHz, DMSO-*d₆*) δ (ppm) 12.51 (s, 1H), 7.92 (d, *J =* 8.4 Hz, 1H), 7.86 (s, 2H), 7.72 (s, 1H), 7.12 (tt, *J* = 9.4, 2.5 Hz, 1H), 7.06-6.96 (m, 2H), 6.87-6.78 (m, 2H), 4.69 (s, 2H), 3.53 (t, *J =* 6.6 Hz, 2H), 2.98 (t, *J* = 6.6 Hz, 2H).

### Example 33

### 2-(3,5-dichloro-4-((2-(2-fluorobenzyl)-1-oxo-1,2,3,4-tetrahydroisoquinolin-6-yl)oxy)phenyl)-1,2,4-triazine-3, 5(2H,4H)-dione 33

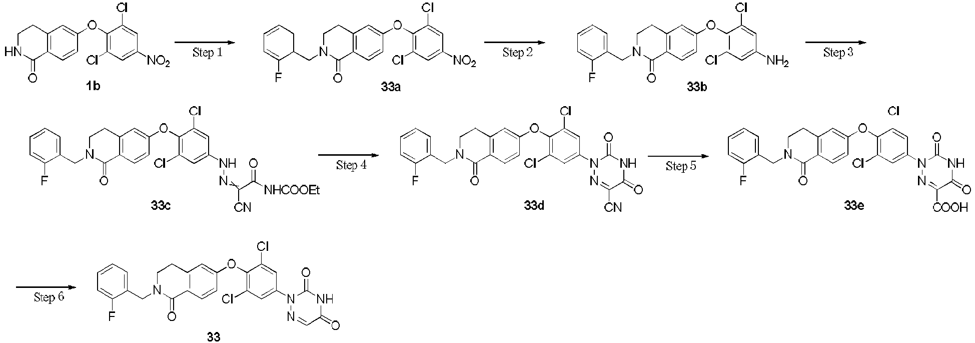

### Step 1: Synthesis of 2-(2-fluorobenzyl)-6-(2,6-dichloro-4-nitrophenoxy)-3,4-dihydroisoquinolin-1(2H)-one 33a

To a solution of 6-(2,6-dichloro-4-nitrophenoxy)-3,4-dihydroisoquinolin-1(2*H*)-one **1b** (1.0 g, 2.8 mmol) in THF (40 mL) was added sodium hydride (0.17 g, 4.2 mmol, 60 mass% in oil) at 0°C. After 20 minutes of reaction, 2-fluorobenzyl bromide (1.1 g, 5.7 mmol) and *N*,*N*-dimethylformamide (10 mL) were added dropwise, and the mixture was reacted at room temperature for 6 hours. The reaction was quenched with ice water (30 mL). The mixture was extracted with ethyl acetate (30 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 3/1) to give a light yellow solid **33a** (0.99 g, yield 76%).

MS (ESI, pos. ion) *m*/*z*: 461.1 [M+H]⁺.

### Step 2: Synthesis of 6-(4-amino-2,6-dichlorophenoxy)-2-(2-fluorobenzyl)-3,4-dihydroisoquinolin-1(2H)-one 33b

To a solution of 2-(2-fluorobenzyl)-6-(2,6-dichloro-4-nitrophenoxy)-3,4-dihydroisoquinolin-1(2*H*)-one **33a** (0.99 g, 2.1 mmol) in acetic acid (20 mL) was added iron powder (0.24 g, 4.3 mmol), and the mixture was reacted at 50°C for 2.5 hours. The reaction solution was cooled to room temperature. The reaction was quenched with water (30 mL). The mixture was extracted with ethyl acetate (50 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 3/1) to give light yellow **oil 33b** (0.62 g, yield 67%).

### Step 3: Synthesis of ethyl

### (2-cyano-2-(2-(3,5-dichloro-4-((2-(2-fluorobenzyl)-1-oxo-1,2,3,4-tetrahydroisoquinolin-6-yl)oxy)phenyl)hydrazo no)acetyl)carbamate 33c

To a solution of 6-(4-amino-2,6-dichlorophenoxy)-2-(2-fluorobenzyl)-3,4-dihydroisoquinolin-1(2*H*)-one **33b** (0.62 g, 1.4 mmol) in acetic acid (18 mL) was added a solution of sodium nitrite (0.20 g, 2.9 mmol) in water (9 mL) dropwise at 0°C, then *N*-cyanoacetylurethane (0.34 g, 2.2 mmol) was added. The mixture was reacted for 3 hours. Water (50 mL) was added to quench the reaction. The mixture was stirred for 20 minutes, filtered, and the filter cake was washed with water (10 mL). Then the filter cake was collected and dried to obtain a light yellow solid **33c** (0.86 g, yield 100%).

MS (ESI, neg. ion) *m*/*z*: 599.2 [M-H]⁻.

### Step 4: Synthesis of

### 2-(3,5-dichloro-4-((2-(2-fluorobenzyl)-1-oxo-1,2,3,4-tetrahydroisoquinolin-6-yl)oxy)phenyl)-3,5-dioxo-2,3,4,5-tet rahydro-1,2,4-triazine-6-carbonitrile 33d

To a solution of ethyl (2-cyano-2-(2-(3,5-dichloro-4-((2-(2-fluorobenzyl)-1-oxo-1,2,3,4-tetrahydroisoquinolin-6-yl)oxy)phenyl)hydrazo no)acetyl)carbamate **33c** (0.86 g, 1.4 mmol) in *N*,*N*-dimethylformamide (16 mL) was added sodium acetate trihydrate (0.26 g, 1.9 mmol), and the mixture was reacted at 120°C for 6 hours. The reaction solution was cooled to room temperature, then water (50 mL) was added to quench the reaction. The reaction mixture was stirred for 15 minutes, and filtered. The filter cake was collected and dried, recrystallized (ethyl acetate/petroleum ether (v/v) = 2/1, 30 mL) at 80°C to obtain a yellow solid **33d** (0.49 g, yield 63%, HPLC purity: 96.04%).
MS (ESI, pos. ion) *m*/*z*: 553.0 [M+H]⁺;
¹H NMR (400 MHz, DMSO-*d₆*) δ (ppm) 13.29 (s, 1H), 7.91 (d, *J =* 8.3 Hz, 1H), 7.83 (s, 2H), 7.34 (qd, *J* = 6.8, 5.8, 3.3 Hz, 2H), 7.26-7.10 (m, 2H), 6.92-6.80 (m, 2H), 4.73 (s, 2H), 3.52 (t, *J* = 6.6 Hz, 2H), 2.98 (t, *J =* 6.5 Hz, 2H).

### Step 5: Synthesis of

### 2-(3,5-dichloro-4-((2-((2-fluorobenzyl)-1-oxo-1,2,3,4-tetrahydroisoquinolin-6-yl)oxy)phenyl)-3,5-dioxo-2,3,4,5-te trahydro-1,2,4-triazine-6-carboxylic acid 33e

2-(3,5-Dichloro-4-((2-(2-fluorobenzyl)-1-oxo-1,2,3,4-tetrahydroisoquinolin-6-yl)oxy)phenyl)-3,5-d ioxo-2,3,4,5-tetrahydro-1,2,4-triazine-6-carbonitrile **33d** (0.43 g, 0.78 mmol) was dissolved in acetic acid (10 mL), then concentrated hydrochloric acid (5 mL) was added. The mixture was reacted at 100°C for 8 hours. The reaction solution was cooled to room temperature, then water (30 mL) was added. The reaction mixture was stirred for 10 minutes, filtered, and rinsed with water (10 mL × 2). The filter cake was collected and dried to obtain a yellow solid **33e** (0.38 g, yield 85%).

MS (ESI, pos. ion) *m*/*z*: 572.0 [M+H]⁺.

### Step 6: Synthesis of

### 2-(3,5-dichloro-4-((2-(2-fluorobenzyl)-1-oxo-1,2,3,4-tetrahydroisoquinolin-6-yl)oxy)phenyl)-1,2,4-triazine-3,5(2 H,4H)-dione 33

2-(3,5-Dichloro-4-((2-(2-fluorobenzyl)-1-oxo-1,2,3,4-tetrahydroisoquinolin-6-yl)oxy)phenyl)-3,5-d ioxo-2,3,4,5-tetrahydro-1,2,4-triazine-6-carboxylic acid **33e** (0.38 g, 0.66 mmol) was dissolved in thioglycolic acid (5 mL). The mixture was reacted at 140°C for 16 hours. The reaction solution was cooled to room temperature, and ethyl acetate (60 mL) was added. The mixture was washed successively with 50% sodium bicarbonate solution (30 mL × 2) and saturated sodium chloride solution (30 mL), dried over anhydrous sodium sulfate and concentrated by suction filtration. The residue was purified by silica gel column chromatography (petroleum ether/ethyl acetate (v/v) = 3/2), and the obtained solid was recrystallized at 80°C (ethyl acetate/petroleum ether (v/v) = 1/2, 30 mL) to give a light yellow solid **33** (0.29 g, yield 83%, HPLC purity: 99.47%).
MS (ESI, pos. ion) *m*/*z*: 527.0 [M+H]⁺;
¹H NMR (400 MHz, DMSO-*d₆*) δ (ppm) 12.51 (s, 1H), 7.91 (d, *J =* 8.4 Hz, 1H), 7.86 (s, 2H), 7.72 (s, 1H), 7.33 (q, *J* = 7.8 Hz, 2H), 7.24-7.12 (m, 2H), 6.87-6.78 (m, 2H), 4.73 (s, 2H), 3.52 (t, *J* = 6.6 Hz, 2H), 2.97 (t, *J* = 6.5 Hz, 2H).

### Example 34

### 2-(3,5-dichloro-4-((2-(3-(trifluoromethyl)benzyl)-1-oxo-1,2,3,4-tetrahydroisoquinolin-6-yl)oxy)phenyl)-1,2,4-triazine-3,5(2H,4H)-dione 34

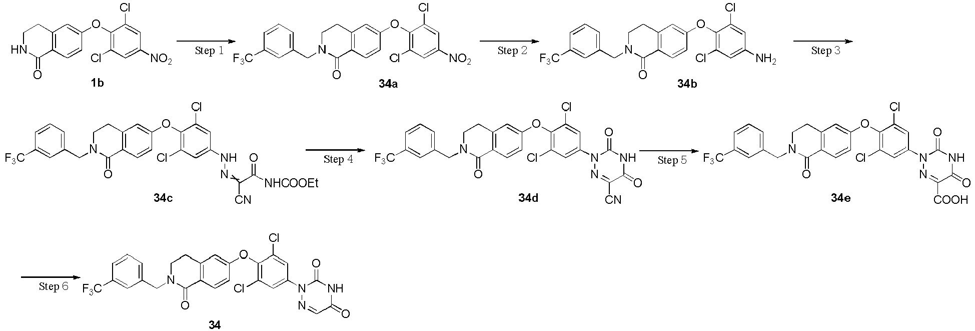

### Step 1: Synthesis of

### 2-(3-(trifluoromethyl)benzyl)-6-(2,6-dichloro-4-nitrophenoxy)-3,4-dihydroisoquinolin-1(2H)-one 34a

To a solution of 6-(2,6-dichloro-4-nitrophenoxy)-3,4-dihydroisoquinolin-1(2*H*)-one **1b** (1.0 g, 2.8 mmol) in THF (40 mL) was added sodium hydride (0.17 g, 4.2 mmol, 60 mass% in oil) at 0°C. After 20 minutes of reaction, 3-trifluoromethylbenzyl bromide (1.4 g, 5.7 mmol) and *N*,*N*-dimethylformamide (10 mL) were added dropwise, and the mixture was reacted at room temperature for 6 hours. The reaction was quenched with ice water (30 mL). The mixture was extracted with ethyl acetate (30 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 3/1) to give light yellow **oil 34a** (0.86 g, yield 59%).

MS (ESI, neg. ion) *m*/*z:* 647.1 [M-H]⁻.

### Step 2: Synthesis of

### 6-(4-amino-2,6-dichlorophenoxy)-2-(3-(trifluoromethyl)benzyl)-3,4-dihydroisoquinolin-1(2H)-one 34b

To a solution of 2-(3-(trifluoromethyl)benzyl)-6-(2,6-dichloro-4-nitrophenoxy)-3,4-dihydroisoquinolin-1(2*H*)-one **34a** (0.86 g, 1.7 mmol) in acetic acid (20 mL) was added iron powder (0.24 g, 4.3 mmol), and the mixture was reacted at 50°C for 2.5 hours. The reaction solution was cooled to room temperature. The reaction was quenched with water (30 mL). The mixture was extracted with ethyl acetate (50 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 1/2) to give light yellow oil **34b** (0.63 g, yield 78%).

### Step 3: Synthesis of ethyl

### (2-cyano-2-(2-(3,5-dichloro-4-((2-(3-(trifluoromethyl)benzyl)-1-oxo-1,2,3,4-tetrahydroisoquinolin-6-yl)oxy)phen yl)hydrazono)acetyl)carbamate 34c

To a solution of 6-(4-amino-2,6-dichlorophenoxy)-2-(3-(trifluoromethyl)benzyl)-3,4-dihydroisoquinolin-1(2*H*)-one **34b** (0.63 g, 1.3 mmol) in acetic acid (18 mL) was added a solution of sodium nitrite (0.18 g, 2.6 mmol) in water (9 mL) at 0°C, then *N*-cyanoacetylurethane (0.31 g, 2.0 mmol) was added. The mixture was reacted for 3 hours. Water (50 mL) was added to quench the reaction. The mixture was stirred for 20 minutes, filtered, and the filter cake was washed with water (10 mL). Then the filter cake was collected and dried to obtain a light yellow solid **34c** (0.85 g, yield 100%).

MS (ESI, neg. ion) *m*/*z*: 647.1 [M-H]⁻.

### Step 4: Synthesis of

### 2-(3,5-dichloro-4-((2-(3-(trifluoromethyl)benzyl)-1-oxo-1,2,3,4-tetrahydroisoquinolin-6-yl)oxy)phenyl)-3,5-dioxo -2,3,4,5-tetrahydro-1,2,4-triazine-6-carbonitrile 34d

To a solution of ethyl (2-cyano-2-(2-(3,5-dichloro-4-((2-(3-(trifluoromethyl)benzyl)-1-oxo-1,2,3,4-tetrahydroisoquinolin-6-yl)oxy)phen yl)hydrazono)acetyl)carbamate **34c** (0.85 g, 1.3 mmol) in *N*,*N*-dimethylformamide (16 mL) was added sodium acetate trihydrate (0.23 g, 1.7 mmol), and the mixture was reacted at 120°C for 6 hours. The reaction solution was cooled to room temperature, then water (50 mL) was added to quench the reaction. The reaction mixture was stirred for 15 minutes, and filtered. The filter cake was collected and dried, recrystallized (ethyl acetate/petroleum ether (v/v) = 1/2, 30 mL) at 80°C to obtain a yellow solid **34d** (0.55 g, yield 70%, HPLC purity: 97.82%).
MS (ESI, neg. ion) *m*/*z*: 601.3 [M-H]⁻;
¹H NMR (400 MHz, DMSO-d₆) δ (ppm) 13.29 (s, 1H), 7.93 (d, *J =* 8.3 Hz, 1H), 7.84 (s, 2H), 7.72-7.55 (m, 4H), 6.85 (d, *J* = 8.8 Hz, 2H), 4.77 (s, 2H), 3.52 (t, *J* = 6.6 Hz, 2H), 2.97 (t, *J* = 6.6 Hz, 2H).

### Step 5: Synthesis of

### 2-(3,5-dichloro-4-((2-(3-(trifluoromethyl)benzyl)-1-oxo-1,2,3,4-tetrahydroisoquinolin-6-yl)oxy)phenyl)-3,5-dioxo -2,3,4,5-tetrahydro-1,2,4-triazine-6-carboxylic acid 34e

2-(3,5-Dichloro-4-((2-(3-(trifluoromethyl)benzyl)-1-oxo-1,2,3,4-tetrahydroisoquinolin-6-yl)oxy)ph enyl)-3,5-dioxo-2,3,4,5-tetrahydro-1,2,4-triazine-6-carbonitrile **34d** (0.47 g, 0.80 mmol) was dissolved in acetic acid (10 mL), then concentrated hydrochloric acid (5 mL) was added. The mixture was reacted at 100°C for 8 hours. The reaction solution was cooled to room temperature, then water (30 mL) was added. The reaction mixture was stirred for 10 minutes, filtered, and rinsed with water (10 mL × 2). The filter cake was collected and dried to obtain a yellow solid **34e** (0.43 g, yield 89%).

MS (ESI, neg. ion) *m*/*z*: 622.0 [M-H]⁻.

### Step 6: Synthesis of

### 2-(3,5-dichloro-4-((2-(3-(trifluoromethyl)benzyl)-1-oxo-1,2,3,4-tetrahydroisoquinolin-6-yl)oxy)phenyl)-1,2,4-tria zinc-3,5(2H,4H)-dione 34

2-(3,5-Dichloro-4-((2-(3-(trifluoromethyl)benzyl)-1-oxo-1,2,3,4-tetrahydroisoquinolin-6-yl)oxy)ph enyl)-3,5-dioxo-2,3,4,5-tetrahydro-1,2,4-triazine-6-carboxylic acid **34e** (0.43 g, 0.71 mmol) was dissolved in thioglycolic acid (5 mL). The mixture was reacted at 140°C for 15 hours. The reaction solution was cooled to room temperature, and ethyl acetate (60 mL) was added. The mixture was washed successively with 50% sodium bicarbonate solution (30 mL × 2) and saturated sodium chloride solution (30 mL), dried over anhydrous sodium sulfate and concentrated by suction filtration. The residue was purified by silica gel column chromatography (petroleum ether/ethyl acetate (v/v) = 3/2), and the obtained solid was recrystallized at 80°C (ethyl acetate/petroleum ether (v/v) = 1/2, 30 mL) to give a light yellow solid **34** (0.24 g, yield 60%, HPLC purity: 99.41%).
MS (ESI, neg. ion) *m*/*z*: 576.0 [M-H]⁻;
¹H NMR (400 MHz, DMSO-*d₆*) δ (ppm) 12.51 (s, 1H), 7.93 (d, *J =* 8.4 Hz, 1H), 7.86 (s, 2H), 7.72 (s, 1H), 7.69-7.54 (m, 4H), 6.88-6.77 (m, 2H), 4.77 (s, 2H), 3.52 (t, *J* = 6.6 Hz, 2H), 2.96 (t, *J=* 6.5 Hz, 2H).

### Example 35 2-(3,5-dichloro-4-((2-(3-chloro-4-fluorobenzyl)-1-oxo-1,2,3,4-tetrahydroisoquinolin-6-yl)oxy)phenyl)-1,2,4-tr iazine-3,5(2H,4H)-dione 35

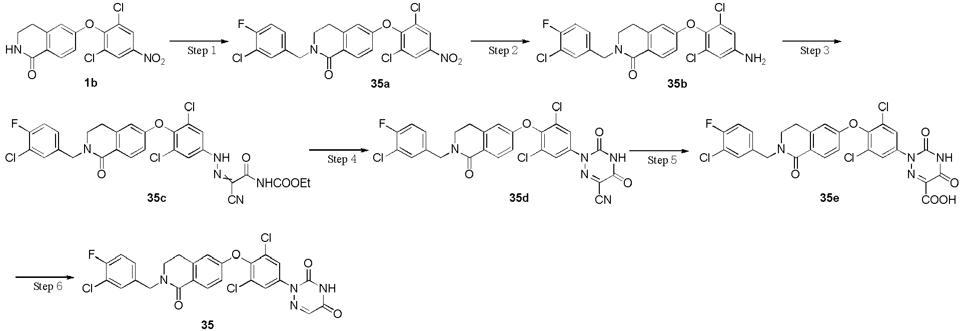

### Step 1: Synthesis of

### 2-(3-chloro-4-fluorobenzyl)-6-(2,6-dichloro-4-nitrophenoxy)-3,4-dihydroisoquinolin-1(2H)-one 35a

To a solution of 6-(2,6-dichloro-4-nitrophenoxy)-3,4-dihydroisoquinolin-1(2*H*)-one **1b** (1.0 g, 2.8 mmol) in THF (40 mL) was added sodium hydride (0.17 g, 4.2 mmol, 60 mass% in oil) at 0°C. After 20 minutes of reaction, 3-chloro-4-fluorobenzyl bromide (1.3 g, 5.7 mmol) and *N*,*N*-dimethylformamide (10 mL) were added dropwise, and the mixture was reacted at room temperature for 6 hours. The reaction was quenched with ice water (30 mL). The mixture was extracted with ethyl acetate (30 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 3/1) to give a light yellow solid **35a** (1.0 g, yield 71%).

### Step 2: Synthesis of

### 6-(4-amino-2,6-dichlorophenoxy)-2-(3-chloro-4-fluorobenzyl)-3,4-dihydroisoquinolin-1(2H)-one 35b

To a solution of 2-(3-chloro-4-fluorobenzyl)-6-(2,6-dichloro-4-nitrophenoxy)-3,4-dihydroisoquinolin-1(2*H*)-one **35a** (0.99 g, 2.0 mmol) in acetic acid (20 mL) was added iron powder (0.22 g, 4.0 mmol), and the mixture was reacted at 50°C for 2.5 hours. The reaction solution was cooled to room temperature. The reaction was quenched with water (30 mL). The mixture was extracted with ethyl acetate (50 mL × 2). The combined organic layers were washed with saturated sodium chloride (50 mL), dried over anhydrous sodium sulfate and concentrated by suction filtration. The residue was purified by silica gel column chromatography (petroleum ether/ethyl acetate (v/v) = 1/2) to give light yellow oil **35b** (0.61 g, yield 66%).

### Step 3: Synthesis of ethyl

### (2-cyano-2-(2-(3,5-dichloro-4-((2-(3-chloro-4-fluorobenzyl)-1-oxo-1,2,3,4-tetrahydroisoquinolin-6-yl)oxy)phenyl )hydrazono)acetyl)carbamate 35c

To a solution of 6-(4-amino-2,6-dichlorophenoxy)-2-(3-chloro-4-fluorobenzyl)-3,4-dihydroisoquinolin-1(2*H*)-one **35b** (0.61 g, 1.3 mmol) in acetic acid (18 mL) was added a solution of sodium nitrite (0.18 g, 2.6 mmol) in water (9 mL) dropwise at 0°C, then *N*-cyanoacetylurethane (0.31 g, 2.0 mmol) was added. The mixture was reacted for 3 hours. Water (50 mL) was added to quench the reaction. The mixture was stirred for 20 minutes, filtered, and the filter cake was washed with water (10 mL). Then the filter cake was collected and dried to obtain a light yellow solid **35c** (0.83 g, yield 100%).

MS (ESI, neg. ion) *m*/*z*: 631.1 [M-H]⁻.

### Step 4: Synthesis of

### 2-(3,5-dichloro-4-((2-(3-chloro-4-fluorobenzyl)-1-oxo-1,2,3,4-tetrahydroisoquinolin-6-yl)oxy)phenyl)-3,5-dioxo-2,3,4,5-tetrahydro-1,2,4-triazine-6-carbonitrile 35d

To a solution of ethyl (2-cyano-2-(2-(3,5-dichloro-4-((2-(3-chloro-4-fluorobenzyl)-1-oxo-1,2,3,4-tetrahydroisoquinolin-6-yl)oxy)phenyl )hydrazono)acetyl)carbamate **35c** (0.83 g, 1.3 mmol) in *N,N*-dimethylformamide (16 mL) was added sodium acetate trihydrate (0.23 g, 1.7 mmol), and the mixture was reacted at 120°C for 6 hours. The reaction solution was cooled to room temperature, then water (50 mL) was added to quench the reaction. The reaction mixture was stirred for 15 minutes, and filtered. The filter cake was collected and dried, recrystallized (ethyl acetate/petroleum ether (v/v) = 1/2, 30 mL) at 80°C to obtain a yellow solid **35d** (0.57 g, yield 74%, HPLC purity: 93.07%).
MS (ESI, neg. ion) *m*/*z*: 585.0 [M-H]⁻;
¹H NMR (400 MHz, DMSO-d₆) δ (ppm) 13.28 (s, 1H), 7.92 (d, *J =* 8.3 Hz, 1H), 7.83 (s, 2H), 7.53 (dd, *J* = 7.3, 2.1 Hz, 1H), 7.41-7.30 (m, 2H), 6.84 *(d, J=* 8.5 Hz, 2H), 4.66 (s, 2H), 3.51 (t, *J* = 6.6 Hz, 2H), 2.96 (t, *J* = 6.6 Hz, 2H).

### Step 5: Synthesis of

### 2-(3,5-dichloro-4-((2-((3-chloro-4-fluorobenzyl)-1-oxo-1,2,3,4-tetrahydroisoquinolin-6-yl)oxy)phenyl)-3,5-dioxo-2,3,4,5-tetrahydro-1,2,4-triazine-6-carboxylic acid 35e

2-(3,5-Dichloro-4-((2-(3-chloro-4-fluorobenzyl)-1-oxo-1,2,3,4-tetrahydroisoquinolin-6-yl)oxy)phe nyl)-3,5-dioxo-2,3,4,5-tetrahydro-1,2,4-triazine-6-carbonitrile **35d** (0.47 g, 0.80 mmol) was dissolved in acetic acid (10 mL), then concentrated hydrochloric acid (5 mL) was added. The mixture was reacted at 100°C for 8 hours. The reaction solution was cooled to room temperature, then water (30 mL) was added. The reaction mixture was stirred for 10 minutes, filtered, and rinsed with water (10 mL × 2). The filter cake was collected and dried to obtain a light yellow solid **35e** (0.43 g, yield 89%).

MS (ESI, pos. ion) *m*/*z:* 606.9 [M+H]⁺.

### Step 6: Synthesis of

### 2-(3,5-dichloro-4-((2-(3-chloro-4-fluorobenzyl)-1-oxo-1,2,3,4-tetrahydroisoquinolin-6-yl)oxy)phenyl)-1,2,4-triazi ne-3,5(2H,4H)-dione 35

2-(3,5-Dichloro-4-((2-(3-chloro-4-fluorobenzyl)-1-oxo-1,2,3,4-tetrahydroisoquinolin-6-yl)oxy)phe nyl)-3,5-dioxo-2,3,4,5-tetrahydro-1,2,4-triazine-6-carboxylic acid **35e** (0.43 g, 0.71 mmol) was dissolved in thioglycolic acid (5 mL). The mixture was reacted at 140°C for 15 hours. The reaction solution was cooled to room temperature, and ethyl acetate (60 mL) was added. The mixture was washed successively with 50% sodium bicarbonate solution (30 mL × 2) and saturated sodium chloride solution (30 mL), dried over anhydrous sodium sulfate and concentrated by suction filtration. The residue was purified by silica gel column chromatography (petroleum ether/ethyl acetate (v/v) = 3/2), and the obtained solid was recrystallized at 80°C (ethyl acetate/petroleum ether (v/v) = 1/2, 30 mL) to give a light yellow solid **35** (0.26 g, yield 65%, HPLC purity: 98.66%).
MS (ESI, neg. ion) *m*/*z:* 561.1 [M-H]⁻;
¹H NMR (400 MHz, DMSO-*d₆*) δ (ppm) 12.51 (s, 1H), 7.92 (d, *J* = 8.3 Hz, 1H), 7.86 (s, 2H), 7.72 (s, 1H), 7.52 (dd, *J* = 7.2, 2.0 Hz, 1H), 7.41-7.30 (m, 2H), 6.81 (d, *J* = 8.8 Hz, 2H), 4.66 (s, 2H), 3.50 (t, *J* = 6.6 Hz, 2H), 2.96 (t, *J* = 6.6 Hz, 2H).

### Example 36

### 2-(3,5-dichloro-4-((2-(3-methoxybenzyl)-1-oxo-1,2,3,4-tetrahydroisoquinolin-6-yl)oxy)phenyl)-1,2,4-triazine -3,5(2H,4H)-dione 36

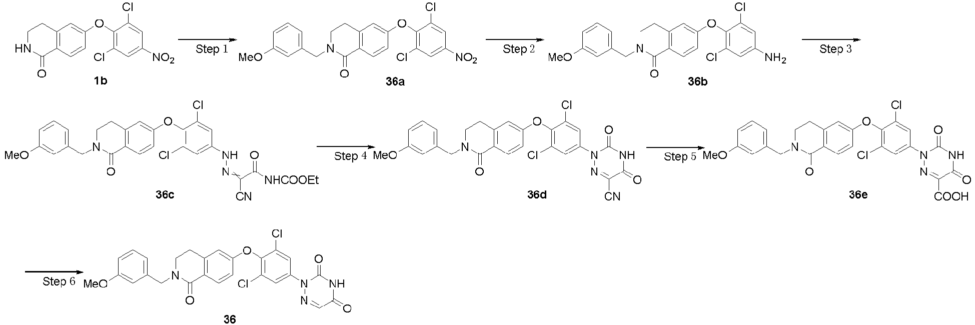

### Step 1: Synthesis of

### 6-(2,6-dichloro-4-nitrophenoxy)-2-(3-methoxybenzyl)-3,4-dihydroisoquinolin-1(2H)-one 36a

To a solution of 6-(2,6-dichloro-4-nitrophenoxy)-3,4-dihydroisoquinolin-1(2*H*)-one **1b** (1.8 g, 5.1 mmol) in THF (35 mL) was added sodium hydride (0.18 g, 7.6 mmol, 60 mass% in oil) at 0°C. After 5 minutes of reaction, 1-bromomethyl-3-methoxybenzene (1.0 g, 5.0 mmol) was added dropwise, and the mixture was reacted at room temperature for 8 hours. The reaction was quenched with ice water (20 mL). The mixture was extracted with ethyl acetate (30 mL × 2). The combined organic layers were washed with saturated sodium chloride (20 mL), dried over anhydrous sodium sulfate and concentrated by suction filtration. The residue was purified by silica gel column chromatography (petroleum ether/ethyl acetate = 3/1) to give a white solid **36a** (1.9 g, yield 79%).

### Step 2: Synthesis of

### 6-(4-amino-2,6-dichlorophenoxy)-2-(3-methoxybenzyl)-3,4-dihydroisoquinolin-1(2H)-one 36b

To a solution of 6-(2,6-dichloro-4-nitrophenoxy)-2-(3-methoxybenzyl)-3,4-dihydroisoquinolin-1(2*H*)-one **36a** (1.9 g, 4.0 mmol) in acetic acid (7 mL) was added iron powder (0.67 g, 12 mmol), and the mixture was reacted at 60°C for 4 hours. The reaction solution was cooled to room temperature, then water (5 mL) was added to quench the reaction. The excess iron powder was filtered off, and water (10 mL) was added to the filtrate. The mixture was stirred for 10 minutes, filtered, and rinsed with water (10 mL × 2). The filter cake was collected and dried to obtain a brown solid **36b** (1.7 g, yield 93%).

### Step 3: Synthesis of ethyl

### (2-cyano-2-(2-(3,5-dichloro-4-((2-(3-methoxybenzyl)-1-oxo-1,2,3,4-tetrahydroisoquinolin-6-yl)oxy)phenyl)hydra zono)acetyl)carbamate 36c

To a solution of 6-(4-amino-2,6-dichlorophenoxy)-2-(3-methoxybenzyl)-3,4-dihydroisoquinolin-1(2*H*)-one **36b** (1.65 g, 3.72 mmol) in acetic acid (6 mL) was added a solution of sodium nitrite (0.39 g, 5.58 mmol) in water (3 mL) at 0°C, then *N*-cyanoacetylurethane (0.86 g, 4.65 mmol) was added. The mixture was reacted for 1 hours. Water (20 mL) was added to quench the reaction. The mixture was stirred for 10 minutes, filtered, and the filter cake was washed with water (5 mL × 2). Then the filter cake was collected and dried to obtain a yellow solid **36c** (2.1 g, yield 92%).

### Step 4: Synthesis of

### 2-(3,5-dichloro-4-((2-(3-methoxybenzyl)-1-oxo-1,2,3,4-tetrahydroisoquinolin-6-yl)oxy)phenyl)-3,5-dioxo-2,3,4,5-tetrahydro-1,2,4-triazine-6-carbonitrile 36d

To a solution of ethyl (2-cyano-2-(2-(3,5-dichloro-4-((2-(3-methoxybenzyl)-1-oxo-1,2,3,4-tetrahydroisoquinolin-6-yl)oxy)phenyl)hydra zono)acetyl)carbamate **36c** (2.10 g, 3.40 mmol) in *N*,*N*-dimethylformamide (8 mL) was added sodium acetate (0.34 g, 3.39 mmol), and the mixture was reacted at 120°C for 12 hours. The reaction solution was cooled to room temperature, then water (20 mL) was added to quench the reaction. The reaction mixture was stirred for 15 minutes, and filtered. The filter cake was collected and dried, recrystallized (ethyl acetate/petroleum ether = 2/1, 15 mL) at 80°C to obtain a yellow solid **36d** (0.75 g, yield 99%)

### Step 5: Synthesis of

### 2-(3,5-dichloro-4-((2-((3-methoxybenzyl)-1-oxo-1,2,3,4-tetrahydroisoquinolin-6-yl)oxy)phenyl)-3,5-dioxo-2,3,4,5 -tetrahydro-1,2,4-triazine-6-carboxylic acid 36e

2-(3,5-Dichloro-4-((2-(3-methoxybenzyl)-1-oxo-1,2,3,4-tetrahydroisoquinolin-6-yl)oxy)phenyl)-3, 5-dioxo-2,3,4,5-tetrahydro-1,2,4-triazine-6-carbonitrile **36d** (0.45 g, 0.80 mmol) was dissolved in acetic acid (6 mL), then concentrated hydrochloric acid (3 mL) was added. The mixture was reacted at 120°C for 16 hours. The reaction solution was cooled to room temperature, then water (10 mL) was added. The reaction mixture was stirred for 10 minutes, filtered, and rinsed with water (5 mL × 2). The filter cake was collected and dried to obtain a yellow solid **36e** (0.40 g, yield 86%).

### Step 6: Synthesis of

### 2-(3,5-dichloro-4-((2-(3-methoxybenzyl)-1-oxo-1,2,3,4-tetrahydroisoquinolin-6-yl)oxy)phenyl)-1,2,4-triazine-3,5( 2H,4H)-dione 36

2-(3,5-Dichloro-4-((2-(3-methoxybenzyl)-1-oxo-1,2,3,4-tetrahydroisoquinolin-6-yl)oxy)phenyl)-3, 5-dioxo-2,3,4,5-tetrahydro-1,2,4-triazine-6-carboxylic acid **36e** (0.40 g, 0.69 mmol) was dissolved in thioglycolic acid (4 mL). The mixture was reacted at 160°C for 12 hours. The reaction solution was cooled to room temperature, and ethyl acetate (30 mL) was added. The mixture was washed successively with water (10 mL) and saturated sodium bicarbonate solution (10 mL), dried over anhydrous sodium sulfate and concentrated by suction filtration. The residue was purified by silica gel column chromatography (petroleum ether/ethyl acetate = 1/1) to give a white solid **36** (39 mg, yield 11%, HPLC purity: 94.35%).
MS (ESI, neg. ion) *m*/*z*: 537.1[M-H]⁻;
¹H NMR (400 MHz, DMSO-*d₆*) δ (ppm) 12.52 (s, 1H), 8.00-7.82 (m, 3H), 7.73 (s, 1H), 7.25 (t, *J =* 7.6 Hz, 1H), 6.97-6.77 (m, 5H), 4.66 (s, 2H), 3.73 (s, 3H), 3.46 (t, *J* = 6.4 Hz, 2H), 2.94 (t, *J* = 6.4 Hz, 2H).

### Example 37

### 2-(3,5-dichloro-4-((2-(4-chlorobenzyl)-1-oxo-1,2,3,4-tetrahydroisoquinolin-6-yl)oxy)phenyl)-1,2,4-triazine-3, 5(2H,4H)-dione 37

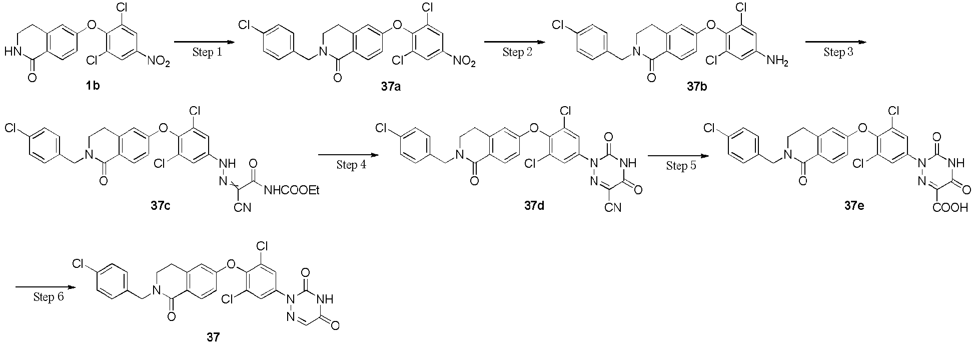

### Step 1: Synthesis of 6-(2,6-dichloro-4-nitrophenoxy)-2-(4-chlorobenzyl)-3,4-dihydroisoquinolin-1(2H)-one 37a

To a solution of 6-(2,6-dichloro-4-nitrophenoxy)-3,4-dihydroisoquinolin-1(2*H*)-one **1b** (3.0 g, 8.5 mmol) in THF (35 mL) was added sodium hydride (0.31 g, 13.0 mmol, 60 mass% in oil) at 0°C. After 5 minutes of reaction, 1-chloromethyl-4-chlorobenzene (2.7 g, 17.0 mmol) was added dropwise, and the mixture was reacted at room temperature for 8 hours. The reaction was quenched with ice water (20 mL). The mixture was extracted with ethyl acetate (20 mL × 2), dried over anhydrous sodium sulfate and concentrated by suction filtration. The residue was purified by silica gel column chromatography (petroleum ether/ethyl acetate = 3/1) to give a white solid **37a** (1.75 g, yield 43%).

### Step 2: Synthesis of 6-(4-amino-2,6-dichlorophenoxy)-2-(4-chlorobenzyl)-3,4-dihydroisoquinolin-1(2H)-one 37b

To a solution of 6-(2,6-dichloro-4-nitrophenoxy)-2-(4-chlorobenzyl)-3,4-dihydroisoquinolin-1(2*H*)-one **37a** (1.75 g, 3.66 mmol) in acetic acid (7 mL) was added iron powder (0.61 g, 11.0 mmol), and the mixture was reacted at 60°C for 4.5 hours. The reaction solution was cooled to room temperature, then water (5 mL) was added to quench the reaction. The excess iron powder was filtered off, and water (10 mL) was added to the filtrate. The mixture was stirred for 10 minutes, filtered, and rinsed with water (10 mL). The filter cake was collected and dried to obtain a brown solid **37b** (1.55 g, yield 95%).

### Step 3: Synthesis of ethyl

### (2-cyano-2-(2-(3,5-dichloro-4-((2-(4-chlorobenzyl)-1-oxo-1,2,3,4-tetrahydroisoquinolin-6-yl)oxy)phenyl)hydrazo no)acetyl)carbamate 37c

To a solution of 6-(4-amino-2,6-dichlorophenoxy)-2-(4-chlorobenzyl)-3,4-dihydroisoquinolin-1(2*H*)-one **37b** (1.55 g, 3.46 mmol) in acetic acid (6 mL) was added a solution of sodium nitrite (0.36 g, 5.25 mmol) in water (3 mL) at 0°C, then *N*-cyanoacetylurethane (0.81 g, 4.37 mmol) was added. The mixture was reacted for 1 hours. Water (20 mL) was added to quench the reaction. The mixture was stirred for 10 minutes, filtered, and the filter cake was washed with water (5 mL). Then the filter cake was collected and dried to obtain a yellow solid **37c** (1.90 g, yield 89%).

### Step 4: Synthesis of

### 2-(3,5-dichloro-4-((2-(4-chlorobenzyl)-1-oxo-1,2,3,4-tetrahydroisoquinolin-6-yl)oxy)phenyl)-3,5-dioxo-2,3,4,5-te trahydro-1,2,4-triazine-6-carbonitrile 37d

To a solution of ethyl (2-cyano-2-(2-(3,5-dichloro-4-((2-(4-chlorobenzyl)-1-oxo-1,2,3,4-tetrahydroisoquinolin-6-yl)oxy)phenyl)hydrazo no)acetyl)carbamate **37c** (1.9 g, 3.1 mmol) in *N*,*N*-dimethylformamide (8 mL) was added sodium acetate (0.30 g, 3.7 mmol), and the mixture was reacted at 120°C for 12 hours. The reaction solution was cooled to room temperature, then water (20 mL) was added to quench the reaction. The reaction mixture was stirred for 15 minutes, and filtered. The filter cake was collected and dried, recrystallized (ethyl acetate/petroleum ether = 2/1, 15 mL) at 80°C to obtain a yellow solid **37d** (0.50 g, yield 28%, HPLC purity: 95.83%).
MS (ESI, neg. ion) *m*/*z*: 566.0 [M-H]⁻;
¹H NMR (400 MHz, DMSO-*d₆*) δ (ppm) 13.32 (s, 1H), 8.03-7.76 (m, 3H), 7.49-7.25 (m, 4H), 6.94-6.76 (m, 2H), 4.68 (s, 2H), 3.48 (t, *J* = 6.4 Hz, 2H), 2.96 (t, *J* = 6.4 Hz, 2H).

### Step 5: Synthesis of

### 2-(3,5-dichloro-4-((2-(4-chlorobenzyl)-1-oxo-1,2,3,4-tetrahydroisoquinolin-6-yl)oxy)phenyl)-3,5-dioxo-2,3,4,5-te trahydro-1,2,4-triazine-6-carboxylic acid 37e

2-(3,5-Dichloro-4-((2-(4-chlorobenzyl)-1-oxo-1,2,3,4-tetrahydroisoquinolin-6-yl)oxy)phenyl)-3,5-dioxo-2,3,4,5-tetrahydro-1,2,4-triazine-6-carbonitrile **37d** (0.40 g, 0.70 mmol) was dissolved in acetic acid (6 mL), then concentrated hydrochloric acid (3 mL) was added. The mixture was reacted at 120°C for 17 hours. The reaction solution was cooled to room temperature, then water (10 mL) was added. The reaction mixture was stirred for 10 minutes, filtered, and rinsed with water (5 mL × 2). The filter cake was collected and dried to obtain a yellow solid **37e** (0.40 g, yield 97%).

### Step 6: Synthesis of

### 2-(3,5-dichloro-4-((2-(4-chlorobenzyl)-1-oxo-1,2,3,4-tetrahydroisoquinolin-6-yl)oxy)phenyl)-1,2,4-triazine-3,5(2 H,4H)-dione 37

2-(3,5-Dichloro-4-((2-(4-chlorobenzyl)-1-oxo-1,2,3,4-tetrahydroisoquinolin-6-yl)oxy)phenyl)-3,5-dioxo-2,3,4,5-tetrahydro-1,2,4-triazine-6-carboxylic acid **37e** (0.15 g, 0.26 mmol) was dissolved in thioglycolic acid (3 mL). The mixture was reacted at 160°C for 12 hours. The reaction solution was cooled to room temperature, and ethyl acetate (30 mL) was added. The mixture was washed successively with water (10 mL), saturated sodium bicarbonate solution (10 mL) and saturated sodium chloride solution (10 mL), dried over anhydrous sodium sulfate and concentrated by suction filtration. The residue was purified by silica gel column chromatography (petroleum ether/ethyl acetate = 1/1) to give a white solid **37** (0.14 g, yield 38%, HPLC purity: 97.64%).
MS (ESI, neg. ion) *m*/*z:* 541.0 [M-H]⁻;
¹H NMR (400 MHz, DMSO-*d₆*) δ (ppm) 12.52 (s, 1H), 8.01-7.80 (m, 3H), 7.73 (s, 1H), 7.46-7.26 (m, 4H), 6.90-6.74 (m, 2H), 4.68 (s, 2H), 3.48 (t, *J=* 6.8 Hz, 2H), 2.95 (t, *J=* 6.8 Hz, 2H).

### Example 38 2-(3,5-dichloro-4-((2-(cyclopropylmethyl)-1-oxo-1,2,3,4-tetrahydroisoquinolin-6-yl)oxy)phenyl)-3,5-dioxo-2, 3,4,5-tetrahydro-1,2,4-triazine-6-carbonitrile 38

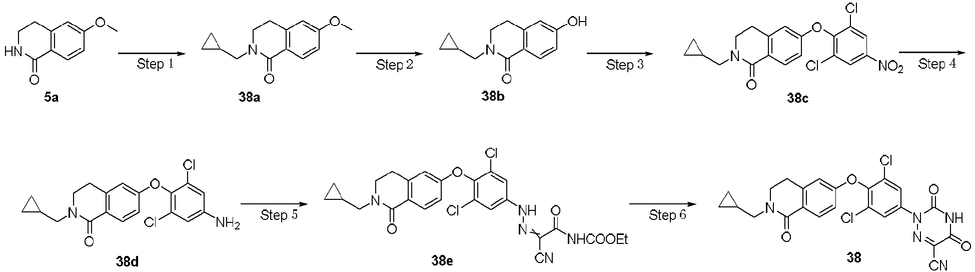

### Step 1: Synthesis of 6-methoxy-2-(cyclopropylmethyl)-3,4-dihydroisoquinolin-1(2H)-one 38a

6-Methoxy-3,4-dihydroisoquinolin-1(2*H*)-one **5a** (2.5 g, 14.1 mmol) was dissolved in a mixture solution of THF (30 mL) and *N,N*-dimethylacetamide (30 mL). Sodium hydride (1.41 g, 35.3 mmol, 60% in oil) was added in portions at 0°C, then chloromethylcyclopropane (2.60 mL, 28.0 mmol) was added dropwise and N,N-dimethylformamide (30 mL) was added in turn. After the addition was complete, the reaction was continued at room temperature for 24 hours. The reaction was quenched with water (40 mL). The mixture was extracted with ethyl acetate (45 mL × 3). The combined organic layers were washed with saturated sodium chloride (40 mL × 3), dried over anhydrous sodium sulfate and concentrated by suction filtration. The residue was purified by silica gel column chromatography (petroleum ether/ethyl acetate = 2/1) to give light yellow **oil 38a** (2.56 g, yield 78%).

MS (ESI, pos. ion) *m*/*z*: 232.2 [M+H]⁺.

### Step 2: Synthesis of 6-hydroxy-2-(cyclopropylmethyl)-3,4-dihydroisoquinolin-1(2H)-one 38a

6-Methoxy-2-(cyclopropylmethyl)-3,4-dihydroisoquinolin-1(2*H*)-one **38a** (2.40 g, 10.4 mmol) was dissolved in dichloromethane (35 mL). Boron tribromide (2.02 mL, 20.8 mmol) was slowly added dropwise at 0°C and the mixture was reacted at 0°C for 1.5 hours. The reaction was quenched by adding methanol (5 mL) at 0°C. The mixture was concentrated, and water (50 mL) was added. The mixture was extracted with ethyl acetate (30 mL × 3). The combined organic layers were washed with saturated sodium chloride (30 mL × 2), dried over ahydrous sodium sulfate and concentrated by suction filtration. The residue was purified by silica gel column chromatography (petroleum ether/ethyl acetate = 2/1) to give a light yellow solid **38b** (1.01 g, yield 45%).

### Step 3: Synthesis of

### 6-(2,6-dichloro-4-nitrophenoxy)-2-(cyclopropylmethyl)-3,4-dihydroisoquinolin-1(2H)-one 38c

To a solution of 6-hydroxy-2-(cyclopropylmethyl)-3,4-dihydroisoquinolin-1(2*H*)-one **38b** (1.01 g, 4.65 mmol) in *N,N*-dimethylformamide (10 mL) were added 1,2,3-trichloro-5-nitrobenzene (1.11 g, 4.90 mmol) and potassium carbonate (1.56 g, 11.2 mmol), and the mixture was reacted at 80°C for 6 hours. The reaction solution was cooled to room temperature, and water (15 mL) was added. The mixture was extracted with ethyl acetate (15 mL × 3). The combined organic layers were washed with saturated sodium chloride (15 mL × 3), dried over ahydrous sodium sulfate and concentrated by suction filtration. The residue was purified by silica gel column chromatography (petroleum ether/ethyl acetate = 2/1) to give a yellow solid **38c** (0.96 g, yield 51%).

### Step 4: Synthesis of

### 6-(4-amino-2,6-dichlorophenoxy)-2-(cyclopropylmethyl)-3,4-dihydroisoquinolin-1(2H)-one 38d

To a solution of 6-(2,6-dichloro-4-nitrophenoxy)-2-(cyclopropylmethyl)-3,4-dihydroisoquinolin-1(2*H*)-one **38c** (0.91 g, 2.23 mmol) in acetic acid (15 mL) was added iron powder (0.51 g, 8.93 mmol), and the mixture was reacted at 60°C for 6.5 hours. The reaction solution was cooled to room temperature, iron powder was removed, then water (25 mL) was added. The reaction mixture was stirred for 10 minutes, filtered, and rinsed with water (10 mL × 2). The filter cake was collected and dried to obtain a light yellow solid **38d** (0.72 g, yield 86%).

MS (ESI, pos. ion) *m*/*z*: 477.1 [M+H]⁺.

### Step 5: Synthesis of ethyl

### (2-cyano-2-(2-(3,5-dichloro-4-((2-(cyclopropylmethyl)-1-oxo-1,23,4-tetrahydroisoquinolin-6-yl)oxy)phenyl)hydr azono)acetyl)carbamate 38e

To a solution of 6-(4-amino-2,6-dichlorophenoxy)-2-(cyclopropylmethyl)-3,4-dihydroisoquinolin-1(2*H*)-one **38d** (0.72 g, 1.91 mmol) in acetic acid (10 mL) was slowly added a solution of sodium nitrite (0.20 g, 2.87 mmol) in water (0.5 mL) at 0°C. The mixture was reacted for 20 minutes, *N*-cyanoacetylurethane (0.34 g, 2.10 mmol) was added, and the reaction was continued at 0°C for 4.5 hours. Water (10 mL) was added to the reaction solution. The mixture was extracted with ethyl acetate (15 mL × 3). The combined organic layers were washed with saturated sodium chloride (15 mL × 3), dried over anhydrous sodium sulfate, and concentrated by suction filtration to give a yellow solid **38e** (0.97 g, yield 93%).

### Step 6: Synthesis of

### 2-(3,5-dichloro-4-((2-(cyclopropylmethyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 38

To a solution of ethyl (2-cyano-2-(2-(3,5-dichloro-4-((2-(cyclopropylmethyl)-1-oxo-1,2,3,4-tetrahydroisoquinolin-6-yl)oxy)phenyl)hydr azono)acetyl)carbamate **38e** (0.97 g, 1.78 mmol) in *N,N*-dimethylformamide (12 mL) was added sodium acetate (0.44 g, 5.33 mmol), and the mixture was reacted at 120°C for 12 hours. The reaction solution was cooled to room temperature, and water (20 mL) was added. The mixture was extracted with ethyl acetate (20 mL × 3). The combined organic layers were washed with saturated sodium chloride solution (20 mL × 3), dried over anhydrous sodium sulfate and concentrated by suction filtration. The resulting residue was purified by silica gel column chromatography (petroleum ether/ethyl acetate = 2/1), and the obtained solid was recrystallized (petroleum ether/ethyl acetate = 3/1, 20 mL) to obtain a white solid **38** (0.13 g, yield 14%, HPLC purity: 96.32%).
MS (ESI, neg. ion) *m*/*z*: 496.1 [M-H]⁻.
¹H NMR (400 MHz, DMSO-*d₆*) δ (ppm) 13.30 (s, 1H), 7.92-7.80 (m, 3H), 6.88-6.79 (m, 2H), 3.61 (t, *J* = 6.5 Hz, 2H), 3.45 (dt, *J* = 13.9, 7.1 Hz, 1H), 3.35 (d, *J* = 6.8 Hz, 2H), 2.97 (t, *J* = 6.4 Hz, 2H), 0.45 (q, *J* = 5.2 Hz, 2H), 0.26 (q, *J* = 4.7 Hz, 2H).

### Example 39

### 2-(3,5-dichloro-4-((2-(hydroxymethyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 39

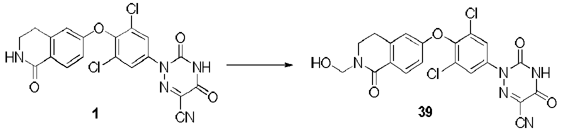

To a solution of 2-(3,5-dichloro-4-((1-oxo-1,2,3,4-tetrahydroisoquinolin-6-yl)oxy)phenyl)-3,5-dioxo-2,3,4,5-tetrahydro-1,2,4-triazi ne-6-carbonitrile **1** (0.20 g, 0.45 mmol) in *N*-methylpyrrolidone (2 mL) were added 37% formaldehyde (0.67 mL, 9.1 mmol) and ethyl phosphate. The mixture was reacted at 100°C for 24 hours. The reaction solution was cooled to room temperature, and water (10 mL) was added. The mixture was extracted with ethyl acetate (15 mL × 3). The combined organic layers were washed with saturated sodium chloride (15 mL × 3), dried over anhydrous sodium sulfate and concentrated by suction filtration. The residue was purified by silica gel column chromatography (petroleum ether/ethyl acetate = 1/2) to give a white solid **39** (0.12 g, yield 56%, HPLC purity: 98.48%).
MS (ESI, neg. ion) *m*/*z*: 472.1 [M-H]⁻;
¹H NMR (400 MHz, DMSO-*d₆*) δ (ppm) 13.30 (s, 1H), 7.90 (d, *J* = 8.5 Hz, 1H), 7.84 (s, 2H), 6.91-6.79 (m, 2H), 5.87 (s, 1H), 4.87 (s, 2H), 3.57 (t, *J* = 6.1 Hz, 2H), 2.96 (t, *J* = 5.9 Hz, 2H).

### Example 40

### 2-(3,5-dichloro-4-((2-(5-fluoro-2-methylbenzyl)-1-oxo-1,2,3,4-tetrahydroisoquinolin-6-yl)oxy)phenyl)-1,2,4-t riazine-3,5(2H,4H)-dione 40

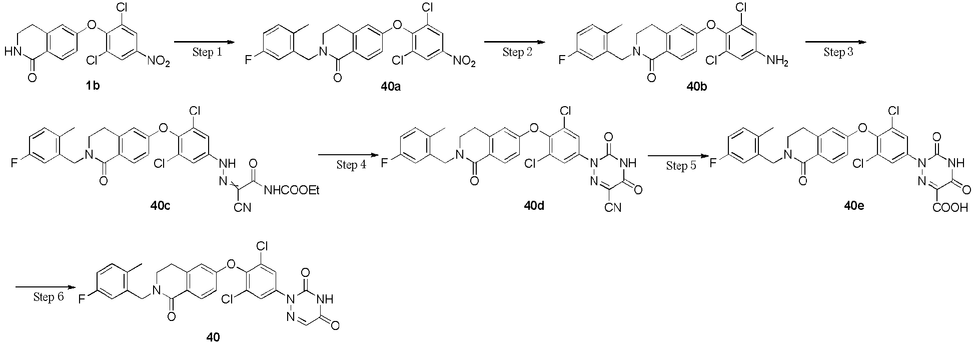

### Step 1: Synthesis of

### 6-(2,6-dichloro-4-nitrophenoxy)-2-(5-fluoro-2-methylbenzyl)-3,4-dihydroisoquinolin-1(2H)-one 40a

To a solution of 6-(2,6-dichloro-4-nitrophenoxy)-3,4-dihydroisoquinolin-1(2*H*)-one **1b** (2.0 g, 5.7 mmol) in THF (20 mL) was added sodium hydride (0.60 g, 15.0 mmol, 60 mass% in oil) at 0°C. After 10 minutes of reaction, 2-bromomethyl-4-fluoro-1-methylbenzene (1.0 mL, 7.2 mmol) and *N*,*N*-dimethylformamide (2 mL) were added dropwise, and the mixture was reacted at room temperature for 3 hours. The reaction was quenched with water (100 mL). The mixture was extracted with ethyl acetate (120 mL). The combined organic layers were washed with saturated sodium chloride (30 mL), dried over anhydrous sodium sulfate and concentrated by suction filtration. The residue was purified by silica gel column chromatography (petroleum ether/ethyl acetate = 5/1) to give a white solid **40a** (2.1 g, yield 77%).

### Step 2: Synthesis of

### 6-(4-amino-2,6-dichlorophenoxy)-2-(5-fluoro-2-methylbenzyl)-3,4-dihydroisoquinolin-1(2H)-one 40b

To a solution of 6-(2,6-dichloro-4-nitrophenoxy)-2-(5-fluoro-2-methylbenzyl)-3,4-dihydroisoquinolin-1(2*H*)-one **40a** (2.0 g, 4.2 mmol) in acetic acid (25 mL) was added iron powder (0.60 g, 10.0 mmol), and the mixture was reacted at 60°C for 6 hours. The reaction solution was cooled to room temperature, then water (100 mL) was added to quench the reaction. The reaction mixture was stirred for 10 minutes, filtered, and rinsed with water (50 mL). The filter cake was collected and dried, and the obtained solid was slurried with petroleum ether/ethyl acetate (6/1, 20 mL) to give a white solid **40b** (1.1 g, yield 59%).

### Step 3: Synthesis of ethyl

### (2-cyano-2-(2-(3,5-dichloro-4-((2-(5-fluoro-2-methylbenzyl)-1-oxo-1,2,3,4-tetrahydroisoquinolin-6-yl)oxy)phenyl )hydrazono)acetyl)carbamate 40c

To a solution of 6-(4-amino-2,6-dichlorophenoxy)-2-(5-fluoro-2-methylbenzyl)-3,4-dihydroisoquinolin-1(2*H*)-one **40b** (1.0 g, 2.2 mmol) in acetic acid (12 mL) was added a solution of sodium nitrite (0.31 g, 4.5 mmol) in water (5 mL) at 0°C, then N-cyanoacetylurethane (0.50 g, 3.0 mmol) was added. The mixture was reacted for 2 hours. Water (30 mL) was added to quench the reaction. The reaction mixture was stirred for 10 minutes, filtered, and rinsed with water (5 mL × 2). The filter cake was collected and dried, and the obtained solid was slurried with petroleum ether/ethyl acetate (5/2, 35 mL) to give a yellow solid **40c** (1.1 g, yield 80%).

### Step 4: Synthesis of

### 2-(3,5-dichloro-4-((2-(5-fluoro-2-methylbenzyl)-1-oxo-1,2,3,4-tetrahydroisoquinolin-6-yl)oxy)phenyl)-3,5-dioxo-2,3,4,5-tetrahydro-1,2,4-triazine-6-carbonitrile 40d

To a solution of ethyl (2-cyano-2-(2-(3,5-dichloro-4-((2-(5-fluoro-2-methylbenzyl)-1-oxo-1,2,3,4-tetrahydroisoquinolin-6-yl)oxy)phenyl )hydrazono)acetyl)carbamate **40c** (1.1 g, 1.8 mmol) in *N*,*N*-dimethylformamide (10 mL) was added sodium acetate (0.20 g, 2.4 mmol), and the mixture was reacted at 120°C for 6 hours. The reaction solution was cooled to room temperature, then water (25 mL) was added to quench the reaction. The reaction mixture was stirred for 10 minutes, and filtered. The filter cake was collected and dried, recrystallized (ethanol/ethyl acetate/petroleum ether = 5/14/20, 39 mL) at 80°C to obtain a white solid **40d** (0.66 g, yield 65%, HPLC purity: 97.55%).
MS (ESI, neg. ion) *m*/*z*: 564.0 [M-H]⁻;
¹H NMR (400 MHz, DMSO-*d₆*) δ (ppm) 13.34 (s, 1H), 7.93 (d, *J* = 8.2 Hz, 1H), 7.85 (s, 2H), 7.30-7.15 (m, 1H), 6.98 (dd, *J* = 17.3, 5.6 Hz, 2H), 6.86 (d, *J* = 9.5 Hz, 2H), 4.67 (s, 2H), 3.50-3.44 (m, 2H), 2.99 (s, 2H), 2.25 (s, 3H).

### Step 5: Synthesis of

### 2-(3,5-dichloro-4-((2-((5-fluoro-2-methylbenzyl)-1-oxo-1,2,3,4-tetrahydroisoquinolin-6-yl)oxy)phenyl)-3,5-dioxo -2,3,4,5-tetrahydro-1,2,4-triazine-6-carboxylic acid 40e

2-(3,5-Dichloro-4-((2-(5-fluoro-2-methylbenzyl)-1-oxo-1,2,3,4-tetrahydroisoquinolin-6-yl)oxy)phe nyl)-3,5-dioxo-2,3,4,5-tetrahydro-1,2,4-triazine-6-carbonitrile **40d** (0.60 g, 1.1 mmol) was dissolved in acetic acid (10 mL), then concentrated hydrochloric acid (3 mL) was added. The mixture was reacted at 120°C for 12 hours. The reaction solution was cooled to room temperature, then water (30 mL) was added. The reaction mixture was stirred for 10 minutes, filtered, and rinsed with water (10 mL × 2). The filter cake was collected and dried to obtain a yellow solid **40e** (0.40 g, yield 60%).

### Step 6: Synthesis of

### 2-(3,5-dichloro-4-((2-(5-fluoro-2-methylbenzyl)-1-oxo-1,2,3,4-tetrahydroisoquinolin-6-yl)oxy)phenyl)-1,2,4-triazi ne-3,5(2H,4H)-dione 40

2-(3,5-Dichloro-4-((2-(5-fluoro-2-methylbenzyl)-1-oxo-1,2,3,4-tetrahydroisoquinolin-6-yl)oxy)phe nyl)-3,5-dioxo-2,3,4,5-tetrahydro-1,2,4-triazine-6-carboxylic acid **40e** (0.40 g, 0.68 mmol) was dissolved in thioglycolic acid (3 mL). The mixture was reacted at 150°C for 12 hours. The reaction solution was cooled to room temperature, and ethyl acetate (50 mL) was added. The mixture was washed successively with water (10 mL) and saturated sodium chloride solution (10 mL), then dried over anhydrous sodium sulfate and concentrated by suction filtration. The residue was purified by silica gel column chromatography (petroleum ether/ethyl acetate = 1/1), and the obtained solid was recrystallized at 80°C (ethyl acetate/petroleum ether = 1/2, 24 mL) to give a white solid **40** (0.29 g, yield 78%, HPLC purity: 99.21%).
MS (ESI, neg. ion) *m*/*z:* 539.2 [M-H]⁻;
¹H NMR (400 MHz, DMSO-*d₆*) δ(ppm) 12.52 (s, 1H), 7.93 (d, *J* = 8.4 Hz, 1H), 7.87 (s, 2H), 7.73 (s, 1H), 7.23 (dd, *J* = 8.3, 6.0 Hz, 1H), 7.03-6.93 (m, 2H), 6.86-6.80 (m, 2H), 4.67 (s, 2H), 3.49 (t, *J* = 6.6 Hz, 2H), 2.99 (t, *J* = 6.6 Hz, 2H), 2.25 (s, 3H).

### Example 41

### 2-(3,5-dichloro-4-((1-oxo-2-(4-(trifluoromethoxy)benzyl)-1,2,3,4-tetrahydroisoquinolin-6-yl)oxy)phenyl)-1,2, 4-triazine-3,5(2H,4H)-dione 41

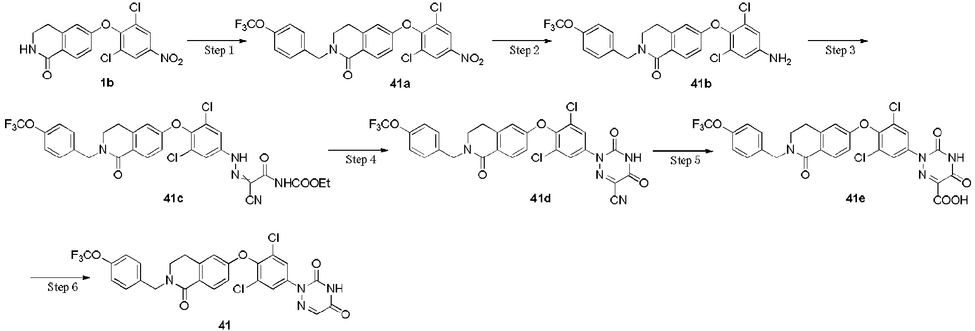

### Step 1: Synthesis of

### 6-(2,6-dichloro-4-nitrophenoxy)-2-(4-(trifluoromethoxy)benzyl)-3,4-dihydroisoquinolin-1(2H)-one 41a

To a solution of 6-(2,6-dichloro-4-nitrophenoxy)-3,4-dihydroisoquinolin-1(2*H*)-one **1b** (1.50 g, 4.20 mmol) in *N,N*-dimethylformamide (6 mL) and THF (20 mL) was added sodium hydride (0.25 g, 6.25 mmol, 60 mass% in oil) at 0°C. After 30 minutes of reaction, 4-trifluoromethoxybenzyl bromide (2.27 g, 8.90 mmol) was added dropwise, and the mixture was reacted at room temperature for 3 hours. The reaction was quenched with ice water (20 mL). The mixture was extracted with ethyl acetate (20 mL × 2), dried over anhydrous sodium sulfate and concentrated by suction filtration. The residue was purified by silica gel column chromatography (petroleum ether/ethyl acetate = 4/1) to give yellow **oil 41a** (2.07 g, yield 93%).

MS (ESI, pos. ion) *m*/*z*: 527.00 [M+H]⁺.

### Step 2: Synthesis of

### 6-(4-amino-2,6-dichlorophenoxy)-2-(4-(trifluoromethoxy)benzyl)-3,4-dihydroisoquinolin-1(2H)-one 41b

To a solution of 6-(2,6-dichloro-4-nitrophenoxy)-2-(4-(trifluoromethoxy)benzyl)-3,4-dihydroisoquinolin-1(2*H*)-one **41a** (2.07 g, 3.93 mmol) in acetic acid (50 mL) was added iron powder (0.88 g, 15.7 mmol), and the mixture was reacted at 55°C for 5 hours. The reaction solution was cooled to room temperature. The reaction was quenched with water (50 mL). The mixture was extracted with ethyl acetate (50 mL × 3). The combined organic layers were washed with saturated sodium chloride (30 mL), dried over anhydrous sodium sulfate and concentrated by suction filtration. The residue was purified by silica gel column chromatography (petroleum ether/ethyl acetate = 5/1) to give a white solid **41b** (1.59 g, yield 81%).

### Step 3: Synthesis of ethyl

### (2-cyano-2-(2-(3,5-dichloro-4-((1-oxo-2-(4-(trifluoromethoxy)benzyl)-1,2,3,4-tetrahydroisoquinolin-6-yl)oxy)phe nyl)hydrazono)acetyl)carbamate 41c

To a solution of 6-(4-amino-2,6-dichlorophenoxy)-2-(4-(trifluoromethoxy)benzyl)-3,4-dihydroisoquinolin-1(2*H*)-one **41b** (0.60 g, 1.21 mmol) in acetic acid (12 mL) was added a solution of sodium nitrite (0.17 g, 2.48 mmol) in water (6 mL) at 0°C, then *N*-cyanoacetylurethane (0.23 g, 1.49 mmol) was added. The mixture was reacted for 4 hours. Water (100 mL) was added to quench the reaction. The mixture was stirred for 30 minutes, filtered, and the filter cake was washed with water (10 mL × 2). Then the filter cake was collected and dried to obtain a yellow solid **41c** (0.87 g, yield 100%).

### Step 4: Synthesis of

### 2-(3,5-dichloro-4-((1-oxo-2-(4-(trifluoromethoxy)benzyl)-1,2,3,4-tetrahydroisoquinolin-6-yl)oxy)phenyl)-3,5-dio xo-2,3,4,5-tetrahydro-1,2,4-triazine-6-carbonitrile 41d

To a solution of ethyl (2-cyano-2-(2-(3,5-dichloro-4-((1-oxo-2-(4-(trifluoromethoxy)benzyl)-1,2,3,4-tetrahydroisoquinolin-6-yl)oxy)phe nyl)hydrazono)acetyl)carbamate **41c** (0.87 g, 1.21 mmol) in *N*,*N*-dimethylformamide (18 mL) was added sodium acetate (0.22 g, 2.65 mmol), and the mixture was reacted at 120°C for 6 hours. The reaction solution was cooled to room temperature, then water (20 mL) was added to quench the reaction. The reaction mixture was stirred for 15 minutes, and filtered. The filter cake was collected and dried, recrystallized (ethanol/ethyl acetate/petroleum ether = 1/1/4, 30 mL) at 80°C to obtain a light red solid **41d** (0.65 g, yield 88%, HPLC purity: 98.61%).
MS (ESI, neg. ion) *m*/*z*: 616.0 [M-H]⁻;
¹H NMR (400 MHz, DMSO-*d₆*) δ (ppm) 13.30 (s, 1H), 7.93 (d, *J* = 8.4 Hz, 1H), 7.84 (s, 2H), 7.44 (d, *J* = 8.2 Hz, 2H), 7.33 (d, *J =* 8.2 Hz, 2H), 6.85 (d, *J =* 10.7 Hz, 2H), 4.72 (s, 2H), 3.51 (t, *J =* 6.3 Hz, 2H), 2.98 (t, *J =* 6.1 Hz, 2H).

### Step 5: Synthesis of

### 2-(3,5-dichloro-4-((2-(1-oxo-4-(trifluoromethoxy)benzyl)-1,2,3,4-tetrahydroisoquinolin-6-yl)oxy)phenyl)-3,5-dio xo-2,3,4,5-tetrahydro-1,2,4-triazine-6-carboxylic acid 41e

2-(3,5-Dichloro-4-((1-oxo-2-(4-(trifluoromethoxy)benzyl)-1,2,3,4-tetrahydroisoquinolin-6-yl)oxy) phenyl)-3,5-dioxo-2,3,4,5-tetrahydro-1,2,4-triazine-6-carbonitrile **41d** (0.32 g, 0.52 mmol) was dissolved in acetic acid (10 mL), then concentrated hydrochloric acid (5 mL) was added. The mixture was reacted at 100°C for 11 hours. The reaction solution was cooled to room temperature, then water (20 mL) was added. The reaction mixture was stirred for 10 minutes, filtered, and rinsed with water (5 mL × 2). The filter cake was collected and dried to obtain a yellow solid **41e** (0.35 g, yield 100%).

### Step 6:

### 2-(3,5-dichloro-4-((1-oxo-2-(4-(trifluoromethoxy)benzyl)-1,2,3,4-tetrahydroisoquinolin-6-yl)oxy)phenyl)-1,2,4-tri azine-3,5(2H,4H)-dione 41

2-(3,5-Dichloro-4-((1-oxo-2-(4-(trifluoromethoxy)benzyl)-1,2,3,4-tetrahydroisoquinolin-6-yl)oxy) phenyl)-3,5-dioxo-2,3,4,5-tetrahydro-1,2,4-triazine-6-carboxylic acid **41e** (0.35 g, 0.53 mmol) was dissolved in thioglycolic acid (3 mL). The mixture was reacted at 140°C for 14 hours. The reaction solution was cooled to room temperature, and ethyl acetate (30 mL) was added. The mixture was washed successively with water (10 mL) and saturated sodium chloride solution (10 mL), then dried over anhydrous sodium sulfate and concentrated by suction filtration. The residue was purified by silica gel column chromatography (petroleum ether/ethyl acetate = 3/1), and the obtained solid was recrystallized at 85°C (ethyl acetate/petroleum ether = 1/1, 20 mL) to give a white solid **41** (0.22 g, yield 71%, HPLC purity: 99.21%).
MS (ESI, neg. ion) *m*/*z*: 591.0 [M-H]⁻;
¹H NMR (400 MHz, DMSO-*d₆*) δ (ppm) 12.52 (s, 1H), 7.93 (d, *J =* 8.4 Hz, 1H), 7.87 (s, 2H), 7.73 (s, 1H), 7.44 (d, *J* = 8.3 Hz, 2H), 7.33 (d, *J* = 8.2 Hz, 2H), 6.87-6.78 (m, 2H), 4.72 (s, 2H), 3.51 (t, *J* = 6.6 Hz, 2H), 2.97 (t, *J* = 6.6 Hz, 2H).

### Example 42

### 2-(3,5-dichloro-4-((2-(3-fluorophenyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 42

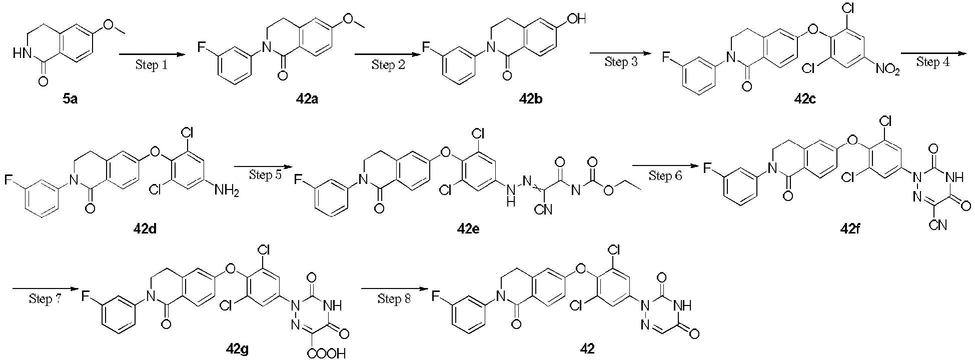

### Step 1: Synthesis of 2-(3-fluorophenyl)-6-methoxy-3,4-dihydroisoquinolin-1(2H)-one 42a

6-Methoxy-3,4-dihydroisoquinolin-1(2*H*)-one **5a** (1.50 g, 8.5 mmol), ketone iodide (0.32 g, 1.7 mmol), *m*-fluoroiodobenzene (3.76 g, 16.9 mmol) and potassium carbonate (1.17 g, 8.47 mmol) were dissolved in *N*,*N*-dimethylformamide (40 mL), and the mixture was reacted at 150°C for 19 hours. The reaction solution was cooled to room temperature, then water (40 mL) was added to quench the reaction. The mixture was extracted with ethyl acetate (60 mL × 2). The combined organic layers were washed with saturated sodium chloride (20 mL), dried over anhydrous sodium sulfate, and concentrated by suction filtration to give a yellow solid **42a** (2.22 g, yield 96%).

MS (ESI, pos. ion) *m*/*z:* 272.2 [M+H]⁺.

### Step 2: Synthesis of 2-(3-fluorophenyl)-6-hydroxy-3,4-dihydroisoquinolin-1(2H)-one 42b

At 0°C, to a solution of 2-(3-fluorophenyl)-6-methoxy-3,4-dihydroisoquinolin-1(2*H*)-one **42a** (2.22 g, 8.17 mmol) in dichloromethane (30 mL) was added boron tribromide (2.4 mL, 25.0 mmol) dropwise. Then the mixture was reacted at room temperature for 4 hours. The reaction solution was quenched by pouring into ice water (30 mL). The reaction mixture was stirred for 10 minutes, filtered, and rinsed with water (10 mL). The filter cake was collected and dried, and the obtained solid was purified by silica gel column chromatography (petroleum ether/ethyl acetate = 3/1) to obtain a white solid **42b** (0.13 g, yield 4.5%).

MS (ESI, pos. ion) *m*/*z:* 258.1 [M+H]⁺.

### Step 3: Synthesis of 6-(2,6-dichloro-4-nitrophenoxy)-2-(3-fluorophenyl)-3,4-dihydroisoquinolin-1(2H)-one 42c

To a solution of 2-(3-fluorophenyl)-6-hydroxy-3,4-dihydroisoquinolin-1(2*H*)-one **42b** (0.13 g, 0.51 mmol) and 1,2,3-trichloro-5-nitrobenzene (0.13 g, 0.57 mmol) in *N,N*-dimethylformamide (3 mL) was added potassium carbonate (0.14 g, 1.01 mmol), and the mixture was reacted at 70°C for 2 hours. The reaction solution was cooled to room temperature, then water (10 mL) was added. The reaction mixture was stirred for 10 minutes, filtered, and rinsed with water (5 mL). The filter cake was collected and dried to obtain an off-white solid **42c** (0.22 g, yield 97%).

### Step 4: Synthesis of 6-(4-amino-2,6-dichlorophenoxy)-2-(3-fluorophenyl)-3,4-dihydroisoquinolin-1(2H)-one 42d

To a solution of 6-(2,6-dichloro-4-nitrophenoxy)-2-(3-fluorophenyl)-3,4-dihydroisoquinolin-1(2*H*)-one **42c** (0.22 g, 0.49 mmol) in acetic acid (6 mL) was added iron powder (0.11 g, 1.95 mmol), and the mixture was reacted at 55°C for 6 hours. The reaction solution was cooled to room temperature, iron powder was removed, and water (50 mL) was added. The mixture was extracted with ethyl acetate (20 mL × 2). The combined organic layers were washed with saturated sodium chloride (20 mL × 3), dried over anhydrous sodium sulfate, and concentrated by suction filtration to give a white solid **42d** (0.18 g, yield 85%).

### Step 5: Synthesis of ethyl

### (2-cyano-2-(2-(3,5-dichloro-4-((2-(3-fluorophenyl)-1-oxo-1,2,3,4-tetrahydroisoquinolin-6-yl)oxy)phenyl)hydrazo no)acetyl)carbamate 42e

To a solution of 6-(4-amino-2,6-dichlorophenoxy)-2-(3-fluorophenyl)-3,4-dihydroisoquinolin-1(2*H*)-one **42d** (0.18 g, 0.42 mmol) in acetic acid (10 mL) was added a solution of sodium nitrite (58 mg, 0.84 mmol) in water (5 mL) dropwise at 0°C. After stirring for 15 minutes, *N*-cyanoacetylurethane (79 mg, 0.51 mmol) was added, and the mixture was reacted for 3.5 hours. Water (20 mL) was added to the reaction solution. The mixture was stirred for 10 minutes, filtered, washed with water (10 mL), and the filter cake was collected and dried to obtain a yellow solid **42e** (0.19 g, yield 73%).

### Step 6: Synthesis of

### 2-(3,5-dichloro-4-((2-(3-fluorophenyl)-1-oxo-1,2,3,4-tetrahydroisoquinolin-6-yl)oxy)phenyl)-3,5-dioxo-2,3,4,5-tet rahydro-1,2,4-triazine-6-carbonitrile 42f

To a solution of ethyl (2-cyano-2-(2-(3,5-dichloro-4-((2-(3-fluorophenyl)-1-oxo-1,2,3,4-tetrahydroisoquinolin-6-yl)oxy)phenyl)hydrazo no)acetyl)carbamate **42e** (0.35 g, 0.60 mmol) in *N,N*-dimethylformamide (40 mL) was added sodium acetate (0.12 g, 1.40 mmol), and the mixture was reacted at 120°C for 8 hours. The reaction solution was cooled to room temperature, and water (80 mL) was added to quench the reaction. The mixture was extracted with ethyl acetate (100 mL × 2). The combined organic layers were washed with saturated sodium chloride solution (50 mL), dried over anhydrous sodium sulfate and concentrated by suction filtration. The resulting residue was purified by silica gel column chromatography (100% ethyl acetate), and the obtained solid was recrystallized (petroleum ether/ethyl acetate = 1/2, 24 mL) at 80°C to obtain a yellow solid **42f** (0.25 g, yield 76%, HPLC purity: 99.44%).
MS (ESI, neg. ion) *m*/*z*: 536.1 [M-H]⁻;
¹H NMR (400 MHz, DMSO-*d₆*) δ (ppm) 13.31 (s, 1H), 7.96 (d, *J* = 8.7 Hz, 1H), 7.86 (s, 2H), 7.45 (q, J = 7.8 Hz, 1H), 7.34-7.23 (m, 2H), 7.09 (td, *J* = 8.6, 2.6 Hz, 1H), 6.95 (d, *J* = 2.6 Hz, 1H), 6.89 (dd, *J* = 8.7, 2.6 Hz, 1H), 3.97 (t, *J* = 6.4 Hz, 2H), 3.13 (t, *J* = 6.4 Hz, 2H).

### Step 7: Synthesis of

### 2-(3,5-dichloro-4-((2-(3-fluorophenyl)-1-oxo-1,2,3,4-tetrahvdroisoquinolin-6-yl)oxy)phenyl)-3,5-dioxo-2,3,4,5-tet rahydro-1,2,4-triazine-6-carboxylic acid 42g

2-(3,5-Dichloro-4-((2-(3-fluorophenyl)-1-oxo-1,2,3,4-tetrahydroisoquinolin-6-yl)oxy)phenyl)-3,5-dioxo-2,3,4,5-tetrahydro-1,2,4-triazine-6-carbonitrile **42f** (0.26 g, 0.49 mmol) was dissolved in acetic acid (5 mL), then concentrated hydrochloric acid (2.5 mL) was added. The mixture was reacted at 100°C for 7 hours. The reaction solution was cooled to room temperature, then water (20 mL) was added. The reaction mixture was stirred for 10 minutes, filtered, and rinsed with water (10 mL × 2). The filter cake was collected and dried to obtain a yellow solid **42g** (0.22 g, yield 78%).

### Step 8: Synthesis of

### 2-(3,5-dichloro-4-((2-(3-fluorophenyl)-1-oxo-1,2,3,4-tetrahydroisoquinolin-6-yl)oxy)phenyl)-1,2,4-triazine-3,5(2 H,4H)-dione 42

2-(3,5-Dichloro-4-((2-(3-fluorophenyl)-1-oxo-1,2,3,4-tetrahydroisoquinolin-6-yl)oxy)phenyl)-3,5-dioxo-2,3,4,5-tetrahydro-1,2,4-triazine-6-carboxylic acid **42g** (0.22 g, 0.40 mmol) was dissolved in thioglycolic acid (2 mL). The mixture was reacted at 140°C for 14 hours. The reaction solution was cooled to room temperature, and ethyl acetate (30 mL) was added. The mixture was washed successively with water (10 mL) and saturated sodium chloride solution (10 mL), then dried over anhydrous sodium sulfate and concentrated by suction filtration. The residue was purified by silica gel column chromatography (petroleum ether/ethyl acetate = 3/1), and the obtained solid was recrystallized at 85°C (petroleum ether/ethyl acetate = 1/1, 20 mL) to give a white solid **42** (88 mg, yield 43%, HPLC purity: 98.94%).
MS (ESI, neg. ion) *m*/*z:* 511.1 [M-H]⁻;
¹H NMR (400 MHz, DMSO-d₆) δ (ppm) 12.53 (s, 1H), 7.96 (s, 1H), 7.88 (s, 2H), 7.74 (s, 1H), 7.45 (s, 1H), 7.28 (dd, *J* = 21.9, 9.6 Hz, 2H), 7.10 (s, 1H), 6.93 (s, 1H), 6.86 (s, 1H), 3.97 (s, 2H), 3.13 (s, 2H).

### Example 43

### 2-(3,5-dichloro-4-((1-oxo-2-(3-(trifluoromethoxy)benzyl)-1,2,3,4-tetrahydroisoquinolin-6-yl)oxy)phenyl)-1,2, 4-triazine-3,5(2H,4H)-dione 43

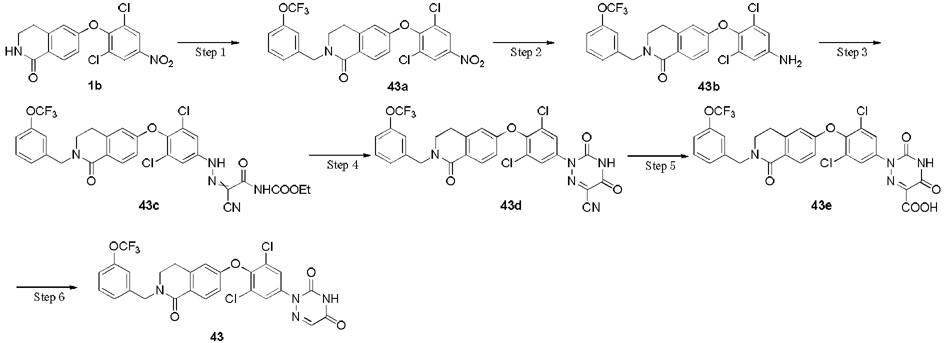

### Step 1: Synthesis of

### 6-(2,6-dichloro-4-nitrophenoxy)-2-(3-(trifluoromethoxy)benzyl)-3,4-dihydroisoquinolin-1(2H)-one 43a

To a solution of 6-(2,6-dichloro-4-nitrophenoxy)-3,4-dihydroisoquinolin-1(2*H*)-one **1b** (2.5 g, 7.1 mmol) in THF (25 mL) was added sodium hydride (0.60 g, 20 mmol, 60 mass% in oil) at 0°C. Then 3-trifluoromethoxybenzyl bromide (1.4 g, 8.6 mmol) and *N,N*-dimethylformamide (2 mL) were added dropwise and the mixture was reacted at 0°C for 5 hours. The reaction was quenched with water (100 mL). The mixture was extracted with ethyl acetate (200 mL). The combined organic layers were washed with saturated sodium chloride (30 mL × 3), dried over anhydrous sodium sulfate and concentrated by suction filtration. The residue was purified by silica gel column chromatography (petroleum ether/ethyl acetate = 4/1) to give yellow oil **43a** (2.6 g, yield 70%).

### Step 2: Synthesis of

### 6-(4-amino-2,6-dichlorophenoxy)-2-(3-(trifluoromethoxy)benzyl)-3,4-dihydroisoquinolin-1(2H)-one 43b

To a solution of 6-(2,6-dichloro-4-nitrophenoxy)-2-(3-(trifluoromethoxy)benzyl)-3,4-dihydroisoquinolin-1(2*H*)-one **43a** (2.5 g, 4.7 mmol) in acetic acid (30 mL) was added iron powder (0.55 g, 9.8 mmol), and the mixture was reacted at 60°C for 6 hours. The reaction solution was cooled to room temperature. The reaction was quenched with water (60 mL). The mixture was extracted with ethyl acetate (150 mL). The combined organic layers were washed with saturated sodium chloride (20 mL × 2), dried over anhydrous sodium sulfate and concentrated by suction filtration. The residue was purified by silica gel column chromatography (petroleum ether/ethyl acetate = 4/1) to give yellow oil **43b** (1.5 g, yield 64%).

### Step 3: Synthesis of ethyl

### (2-cyano-2-(2-(3,5-dichloro-4-((1-oxo-2-(3-(trifluoromethoxy)benzyl)-1,2,3,4-tetrahydroisoquinolin-6-yl)oxy)phe nyl)hydrazono)acetyl)carbamate 43c

To a solution of 6-(4-amino-2,6-dichlorophenoxy)-2-(3-(trifluoromethoxy)benzyl)-3,4-dihydroisoquinolin-1(2*H*)-one **43b** (1.5 g, 3.0 mmol) in acetic acid (20 mL) was added a solution of sodium nitrite (0.42 g, 6.1 mmol) in water (6 mL) at 0°C, then *N*-cyanoacetylurethane (0.61 g, 3.9 mmol) was added. The mixture was reacted for 2 hours. Water (30 mL) was added to quench the reaction. The mixture was stirred for 10 minutes, filtered, and the filter cake was washed with water (10 mL × 2). Then the filter cake was collected and dried to obtain a yellow solid **43c** (1.6 g, yield 80%).

### Step 4: Synthesis of

### 2-(3,5-dichloro-4-((1-oxo-2-(3-(trifluoromethoxy)benzyl)-1,2,3,4-tetrahydroisoquinolin-6-yl)oxy)phenyl)-3,5-dio xo-2,3,4,5-tetrahydro-1,2,4-triazine-6-carbonitrile 43d

To a solution of ethyl (2-cyano-2-(2-(3,5-dichloro-4-((1-oxo-2-(3-(trifluoromethoxy)benzyl)-1,2,3,4-tetrahydroisoquinolin-6-yl)oxy)phe nyl)hydrazono)acetyl)carbamate **43c** (1.5 g, 3.7 mmol) in *N,N*-dimethylformamide (15 mL) was added sodium acetate (0.30 g, 3.7 mmol), and the mixture was reacted at 120°C for 6 hours. The reaction solution was cooled to room temperature, then water (35 mL) was added to quench the reaction. The reaction mixture was stirred for 10 minutes, and filtered. The filter cake was collected and dried, recrystallized (ethanol/ethyl acetate/petroleum ether = 5/20/18, 43 mL) at 85°C to obtain a white solid **43d** (0.80 g, yield 57%, HPLC purity: 98.83%).
MS (ESI, neg. ion) *m*/*z*: 616.0 [M-H]⁻;
¹H NMR (400 MHz, DMSO-*d₆*) δ (ppm) 13.33 (s, 1H), 7.93 (d, *J* = 8.3 Hz, 1H), 7.84 (s, 2H), 7.48 (t, *J* = 7.8 Hz, 1H), 7.36 (d, *J* = 7.7 Hz, 1H), 7.33-7.13 (m, 2H), 6.85 (d, *J* = 9.3 Hz, 2H), 4.74 (s, 2H), 3.52 (t, *J* = 6.4 Hz, 2H), 2.97 (t, *J* = 6.2 Hz, 2H).

### Step 5: Synthesis of

### 2-(3,5-dichloro-4-((1-oxo-2-(3-(trifluoromethoxy)benzyl)-1,2,3,4-tetrahydroisoquinolin-6-yl)oxy)phenyl)-3,5-dio xo-2,3,4,5-tetrahydro-1,2,4-triazine-6-carboxylic acid 43e

2-(3,5-Dichloro-4-((1-oxo-2-(3-(trifluoromethoxy)benzyl)-1,2,3,4-tetrahydroisoquinolin-6-yl)oxy) phenyl)-3,5-dioxo-2,3,4,5-tetrahydro-1,2,4-triazine-6-carbonitrile **43d** (0.80 g, 1.3 mmol) was dissolved in acetic acid (12 mL), then concentrated hydrochloric acid (4 mL) was added. The mixture was reacted at 120°C for 12 hours. The reaction solution was cooled to room temperature, then water (30 mL) was added. The reaction mixture was stirred for 10 minutes, filtered, and rinsed with water (5 mL × 2). The filter cake was collected and dried to obtain a white solid **43e** (0.62 g, yield 75%).

### Step 6: Synthesis of

### 2-(3,5-dichloro-4-((1-oxo-2-(3-(trifluoromethoxy)benzyl)-1,2,3,4-tetrahydroisoquinolin-6-yl)oxy)phenyl)-1,2,4-tri azine-3,5(2H,4H)-dione 43

2-(3,5-Dichloro-4-((1-oxo-2-(3-(trifluoromethoxy)benzyl)-1,2,3,4-tetrahydroisoquinolin-6-yl)oxy) phenyl)-3,5-dioxo-2,3,4,5-tetrahydro-1,2,4-triazine-6-carboxylic acid **43e** (0.62 g, 0.97 mmol) was dissolved in thioglycolic acid (3.5 mL). The mixture was reacted at 150°C for 12 hours. The reaction solution was cooled to room temperature, and water (20 mL) was added to quench the reaction. The mixture was extracted with ethyl acetate (60 mL × 2). The combined organic layers were washed with saturated sodium chloride solution (15 mL × 3), dried over anhydrous sodium sulfate and concentrated by suction filtration. The resulting residue was purified by silica gel column chromatography (petroleum ether/ethyl acetate = 1/3), and the obtained solid was recrystallized at 85°C (ethyl acetate/petroleum ether = 2/5, 21 mL) to give a white solid **43** (0.38 g, yield 66%, purity: 98.38%).
MS (ESI, neg. ion) *m*/*z:* 591.1 [M-H]⁻;
¹H NMR (400 MHz, DMSO-*d₆*) δ (ppm) 12.52 (s, 1H), 7.93 (d, *J* = 8.3 Hz, 1H), 7.87 (d, *J* = 1.0 Hz, 2H), 7.73 (d, *J* = 1.4 Hz, 1H), 7.48 (t, *J* = 7.9 Hz, 1H), 7.35 (d, *J* = 7.7 Hz, 1H), 7.32-7.15 (m, 2H), 6.96-6.64 (m, 2H), 4.74 (s, 2H), 3.51 (t, *J* = 6.6 Hz, 2H), 2.97 (t, *J* = 6.6 Hz, 2H).

### Example 44

### 2-(3,5-dichloro-4-((2-(2-fluorophenyl)-1-oxo-1,2,3,4-tetrahydroisoquinolin-6-yl)oxy)phenyl)-1,2,4-triazine-3, 5(2H,4H)-dione 44

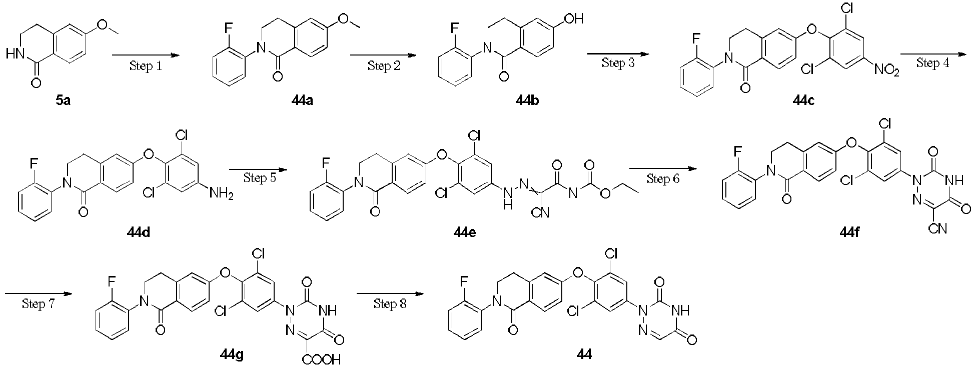

### Step 1: Synthesis of 2-(2-fluorophenyl)-6-methoxy-3,4-dihydroisoquinolin-1(2H)-one 44a

6-Methoxy-3,4-dihydroisoquinolin-1(2*H*)-one **5a** (1.50 g, 8.5 mmol), ketone iodide (0.32 g, 1.7 mmol), o-fluoroiodobenzene (3.76 g, 16.9 mmol) and potassium carbonate (1.17 g, 8.47 mmol) were dissolved in N,N-dimethylformamide (40 mL), and the mixture was reacted at 150°C for 19 hours. The reaction solution was cooled to room temperature, then water (40 mL) was added to quench the reaction. The mixture was extracted with ethyl acetate (60 mL × 2). The combined organic layers were washed with saturated sodium chloride (20 mL), dried over anhydrous sodium sulfate, and concentrated by suction filtration to give a yellow solid **44a** (1.76 g, yield 75%).

MS (ESI, pos. ion) *m*/*z:* 272.2 [M+H]⁺.

### Step 2: Synthesis of 2-(2-fluorophenyl)-6-hydroxy-3,4-dihydroisoquinolin-1(2H)-one 44b

At 0°C, to a solution of 2-(2-fluorophenyl)-6-methoxy-3,4-dihydroisoquinolin-1(2*H*)-one **44a** (1.72 g, 6.34 mmol) in dichloromethane (30 mL) was added boron tribromide (1.84 mL, 19.1 mmol) dropwise. Then the mixture was reacted at room temperature for 4 hours. The reaction solution was quenched by pouring into ice water (30 mL). The reaction mixture was stirred for 10 minutes, filtered, and rinsed with water (10 mL). The filter cake was collected and dried, and the obtained solid was purified by silica gel column chromatography (petroleum ether/ethyl acetate = 3/1) to obtain a white solid **44b** (1.09 g, yield 67%).

MS (ESI, pos. ion) *m*/*z:* 258.2 [M+H]⁺.

### Step 3: Synthesis of 6-(2,6-dichloro-4-nitrophenoxy)-2-(2-fluorophenyl)-3,4-dihydroisoquinolin-1(2H)-one 44c

To a solution of 2-(2-fluorophenyl)-6-hydroxy-3,4-dihydroisoquinolin-1(2*H*)-one **44b** (0.50 g, 1.94 mmol) and 1,2,3-trichloro-5-nitrobenzene (0.52 g, 2.28 mmol) in *N,N*-dimethylformamide (3 mL) was added potassium carbonate (0.54 g, 3.89 mmol), and the mixture was reacted at 40°C for 4.5 hours. The reaction solution was cooled to room temperature, then water (30 mL) was added. The reaction mixture was stirred for 10 minutes, filtered, and rinsed with water (5 mL). The filter cake was collected and dried to obtain an off-white solid **44c** (0.92 g, yield 100%).

### Step 4: Synthesis of 6-(4-amino-2,6-dichlorophenoxy)-2-(2-fluorophenyl)-3,4-dihydroisoquinolin-1(2H)-one 44d

To a solution of 6-(2,6-dichloro-4-nitrophenoxy)-2-(2-fluorophenyl)-3,4-dihydroisoquinolin-1(2*H*)-one **44c** (0.92 g, 2.06 mmol) in acetic acid (10 mL) was added iron powder (0.46 g, 8.24 mmol), and the mixture was reacted at 55°C for 8 hours. The reaction solution was cooled to room temperature, iron powder was removed, and water (50 mL) was added. The mixture was extracted with ethyl acetate (20 mL × 2). The combined organic layers were washed with saturated sodium chloride (20 mL × 3), dried over anhydrous sodium sulfate, and concentrated by suction filtration to give a white solid **44d** (0.84 g, yield 98%).

### Step 5: Synthesis of ethyl

### (2-cyano-2-(2-(3,5-dichloro-4-((2-(2-fluorophenyl)-1-oxo-1,2,3,4-tetrahydroisoquinolin-6-yl)oxy)phenyl)hydrazo no)acetyl)carbamate 44e

To a solution of 6-(4-amino-2,6-dichlorophenoxy)-2-(2-fluorophenyl)-3,4-dihydroisoquinolin-1(2*H*)-one **44d** (0.55 g, 1.32 mmol) in acetic acid (11 mL) was added a solution of sodium nitrite (0.18 g, 2.64 mmol) in water (6 mL) dropwise at 0°C. After reacting for 15 minutes, N-cyanoacetylurethane (0.25 g, 1.58 mmol) was added, and the mixture was reacted for 3.5 hours. Water (20 mL) was added to the reaction solution. The mixture was stirred for 10 minutes, filtered, washed with water (10 mL), and the filter cake was collected and dried to obtain a yellow solid **44e** (0.76 g, yield 99%).

### Step 6: Synthesis of

### 2-(3,5-dichloro-4-((2-(2-fluorophenyl)-1-oxo-1,2,3,4-tetrahydroisoquinolin-6-yl)oxy)phenyl)-3,5-dioxo-2,3,4,5-tet rahydro-1,2,4-triazine-6-carbonitrile 44f

To a solution of ethyl (2-cyano-2-(2-(3,5-dichloro-4-((2-(2-fluorophenyl)-1-oxo-1,2,3,4-tetrahydroisoquinolin-6-yl)oxy)phenyl)hydrazo no)acetyl)carbamate **44e** (0.42 g, 0.72 mmol) in *N,N*-dimethylformamide (10 mL) was added sodium acetate (0.13 g, 1.59 mmol), and the mixture was reacted at 120°C for 8 hours. The reaction solution was cooled to room temperature, then water (80 mL) was added to quench the reaction. The reaction mixture was stirred for 10 minutes, filtered, and rinsed with water (10 mL). The filter cake was collected and dried, and the obtained solid was recrystallized at 80°C (petroleum ether/ethyl acetate = 1/2, 30 mL) to give a yellow solid **44f** (0.25 g, yield 65%, HPLC purity: 96.68%).
MS (ESI, neg. ion) *m*/*z*: 536.0 [M-H]⁻;
¹H NMR (400 MHz, DMSO-*d₆*) δ (ppm) 13.31 (s, 1H), 7.95 (d, *J* = 8.5 Hz, 1H), 7.86 (s, 2H), 7.48 (t, *J* = 7.7 Hz, 1H), 7.42-7.35 (m, 1H), 7.33 (d, *J* = 10.7 Hz, 1H), 7.28 (d, *J* = 8.2 Hz, 1H), 6.97 (s, 1H), 6.89 (d, *J* = 8.7 Hz, 1H), 3.93-3.83 (m, 2H), 3.20-3.11 (m, 2H).

### Step 7: Synthesis of

### 2-(3,5-dichloro-4-((2-(2-fluorophenyl)-1-oxo-1,2,3,4-tetrahydroisoquinolin-6-yl)oxy)phenyl)-3,5-dioxo-2,3,4,5-tet rahydro-1,2,4-triazine-6-carboxylic acid 44g

2-(3,5-Dichloro-4-((2-(2-fluorophenyl)-1-oxo-1,2,3,4-tetrahydroisoquinolin-6-yl)oxy)phenyl)-3,5-dioxo-2,3,4,5-tetrahydro-1,2,4-triazine-6-carbonitrile **44f** (0.55 g, 1.02 mmol) was dissolved in acetic acid (10 mL), then concentrated hydrochloric acid (5 mL) was added. The mixture was reacted at 100°C for 7 hours. The reaction solution was cooled to room temperature, then water (20 mL) was added. The reaction mixture was stirred for 10 minutes, filtered, and rinsed with water (10 mL × 2). The filter cake was collected and dried to obtain a yellow solid **44g** (0.56 g, yield 98%).

### Step 8: Synthesis of

### 2-(3,5-dichloro-4-((2-(2-fluorophenyl)-1-oxo-1,2,3,4-tetrahydroisoquinolin-6-yl)oxy)phenyl)-1,2,4-triazine-3,5(2 H,4H)-dione 44

2-(3,5-Dichloro-4-((2-(2-fluorophenyl)-1-oxo-1,2,3,4-tetrahydroisoquinolin-6-yl)oxy)phenyl)-3,5-dioxo-2,3,4,5-tetrahydro-1,2,4-triazine-6-carboxylic acid **44g** (0.56 g, 1.01 mmol) was dissolved in thioglycolic acid (2 mL). The mixture was reacted at 130°C for 14 hours. The reaction solution was cooled to room temperature, and ethyl acetate (30 mL) was added. The mixture was washed successively with water (10 mL) and saturated sodium chloride solution (10 mL), dried over anhydrous sodium sulfate and concentrated by suction filtration. The residue was purified by silica gel column chromatography (petroleum ether/ethyl acetate = 3/1), and the obtained solid was recrystallized at 85°C (petroleum ether/ethyl acetate = 1/1, 20 mL) to give a white solid **44** (0.23 g, yield 44%, HPLC purity: 97.70%).
MS (ESI, neg. ion) *m*/*z*: 511.0 [M-H]⁻;
¹H NMR (400 MHz, DMSO-*d₆*) δ (ppm) 12.53 (s, 1H), 7.95 (d, *J* = 8.6 Hz, 1H), 7.89 (s, 2H), 7.74 (s, 1H), 7.48 (td, *J* = 7.8, 1.8 Hz, 1H), 7.41-7.25 (m, 3H), 6.95 (d, *J* = 2.6 Hz, 1H), 6.86 (dd, *J* = 8.6, 2.6 Hz, 1H), 3.88 (t, *J* = 6.4 Hz, 2H), 3.14 (t, *J* = 6.5 Hz, 2H).

### Example 45

### 2-(3,5-dichloro-4-((1-oxo-2-(pyridin-3-ylmethyl)-1,2,3,4-tetrahydroisoquinolin-6-yl)oxy)phenyl)-1,2,4-triazi ne-3,5(2H,4H)-dione 45

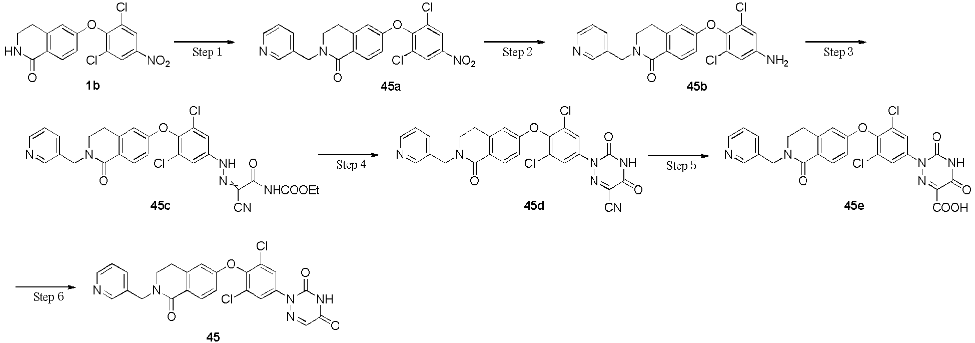

### Step 1: Synthesis of

### 6-(2,6-dichloro-4-nitrophenoxy)-2-(pyridin-3-ylmethyl)-3,4-dihydroisoquinolin-1(2H)-one 45a

To a solution of 6-(2,6-dichloro-4-nitrophenoxy)-3,4-dihydroisoquinolin-1(2*H*)-one **1b** (2.0 g, 5.7 mmol) in THF (20 mL) was added sodium hydride (0.70 g, 18 mmol, 60 mass% in oil) at 0°C. Then 3-(bromomethyl)pyridine hydrobromide (1.9 g, 7.5 mmol) and *N,N*-dimethylformamide (6 mL) were added, and the mixture was reacted at 20°C for 4 hours. Water (50 mL) was added to quench the reaction at 0°C. The mixture was stirred for 10 minutes, and filtered. The filter cake was rinsed with water (20 mL × 3), and the collected filter cake was dried and slurried with ethyl acetate/petroleum ether (1/2, 20 mL). The mixture was filtered, and the filter cake was collected and dried to obtain a yellow solid **45a** (2.2 g, yield 87%).

### Step 2: Synthesis of

### 6-(4-amino-2,6-dichlorophenoxy)-2-(pyridin-3-ylmethyl)-3,4-dihydroisoquinolin-1(2H)-one 45b

To a solution of 6-(2,6-dichloro-4-nitrophenoxy)-2-(pyridin-3-ylmethyl)-3,4-dihydroisoquinolin-1(2*H*)-one **45a** (2.2 g, 5.0 mmol) in acetic acid (25 mL) was added iron powder (0.72 g, 13 mmol), and the mixture was reacted at 60°C for 6 hours. The reaction solution was cooled to room temperature, then water (100 mL) was added to quench the reaction. The mixture was extracted with ethyl acetate (150 mL × 2). The combined organic layers were washed with saturated sodium chloride (20 mL × 2), dried over anhydrous sodium sulfate, and concentrated by suction filtration to give a yellow solid **45b** (1.6 g, yield 78%).

### Step 3: Synthesis of ethyl

### (2-cyano-2-(2-(3,5-dichloro-4-((2-(pyridin-3-ylmethyl)-1-oxo-1,2,3,4-tetrahydroisoquinolin-6-yl)oxy)phenyl)hydr azono)acetyl)carbamate 45c

To a solution of 6-(4-amino-2,6-dichlorophenoxy)-2-(pyridin-3-ylmethyl)-3,4-dihydroisoquinolin-1(2*H*)-one **45b** (1.6 g, 3.9 mmol) in acetic acid (20 mL) was added a solution of sodium nitrite (0.35 g, 5.1 mmol) in water (5 mL) at 0°C, then N-cyanoacetylurethane (0.72 g, 4.6 mmol) was added. The mixture was reacted for 2 hours. Water (100 mL) was added to quench the reaction. The mixture was stirred for 10 minutes, filtered, and the filter cake was collected and dried to obtain a yellow solid **45c** (2.2 g, yield 98%).

### Step 4: Synthesis of

### 2-(3,5-dichloro-4-((2-(pyridin-3-ylmethyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 45d

To a solution of ethyl (2-cyano-2-(2-(3,5-dichloro-4-((2-(pyridin-3-ylmethyl)-1-oxo-1,2,3,4-tetrahydroisoquinolin-6-yl)oxy)phenyl)hydr azono)acetyl)carbamate **45c** (2.2 g, 3.8 mmol) in *N*,*N*-dimethylformamide (18 mL) was added sodium acetate (0.40 g, 4.9 mmol), and the mixture was reacted at 120°C for 6 hours. The reaction solution was cooled to room temperature, then water (100 mL) was added to quench the reaction. The reaction mixture was stirred for 15 minutes, and filtered. The filter cake was collected and dried, then slurried with ethanol/ethyl acetate (1/3, 50 mL) at 85 °C to give a white solid **45d** (0.90 g, yield 40%, HPLC purity: 83.86%).
MS (ESI, pos. ion) *m*/*z*: 535.9 [M+H]⁺;
¹H NMR (400 MHz, DMSO-*d₆*) δ (ppm) 8.56 (d, *J* = 28.8 Hz, 2H), 8.05-7.68 (m, 4H), 7.49-7.35 (m, 1H), 6.83 (d, *J* = 9.2 Hz, 2H), 4.71 (s, 2H), 3.52 (d, *J* = 6.7 Hz, 2H), 2.96 (t, *J* = 6.6 Hz, 2H).

### Step 5: Synthesis of

### 2-(3,5-dichloro-4-((2-(pyridin-3-ylmethyl)-1-oxo-1,2,3,4-tetrahydroisoquinolin-6-yl)oxy)phenyl)-3,5-dioxo-2,3,4, 5-tetrahydro-1,2,4-triazine-6-carboxylic acid 45e

2-(3,5-Dichloro-4-((2-(pyridin-3-ylmethyl)-1-oxo-1,2,3,4-tetrahydroisoquinolin-6-yl)oxy)phenyl)-3,5-dioxo-2,3,4,5-tetrahydro-1,2,4-triazine-6-carbonitrile **45d** (0.80 g, 1.5 mmol) was dissolved in acetic acid (12 mL), then concentrated hydrochloric acid (6.0 mL) was added. The mixture was reacted at 120°C for 12 hours. The reaction solution was cooled to room temperature, and concentrated *in vacuo* to obtain a yellow solid **45e** (0.80 g, yield 97%).

### Step 6: Synthesis of

### 2-(3,5-dichloro-4-((2-(pyridin-3-ylmethyl)-1-oxo-1,2,3,4-tetrahydroisoquinolin-6-yl)oxy)phenyl)-1,2,4-triazine-3, 5(2H,4H)-dione 45

2-(3,5-Dichloro-4-((2-(pyridin-3-ylmethyl)-1-oxo-1,2,3,4-tetrahydroisoquinolin-6-yl)oxy)phenyl)-3,5-dioxo-2,3,4,5-tetrahydro-1,2,4-triazine-6-carboxylic acid **45e** (0.80 g, 1.4 mmol) was dissolved in thioglycolic acid (2 mL). The mixture was reacted at 140°C for 12 hours. The reaction solution was cooled to room temperature and the reaction solution was purified by silica gel column chromatography (petroleum ether/ethyl acetate=1/1) to obtain a white solid **45** (0.12 g, yield 20%, HPLC purity: 93.09%).
MS (ESI, neg. ion) *m*/*z:* 511.0 [M+H]⁺;
¹H NMR (400 MHz, DMSO-*d₆*) δ (ppm) 12.53 (s, 1H), 9.08-8.68 (m, 2H), 8.41 (d, *J* = 12.8 Hz, 1H), 8.14-7.80 (m, 4H), 7.74 (d, *J* = 1.8 Hz, 1H), 7.05-6.70 (m, 2H), 4.84 (d, *J* = 2.9 Hz, 2H), 3.62 *(d, J=* 6.8 Hz, 2H), 3.02 (t, *J* = 6.6 Hz, 2H).

### Example 46

### 2-(3,5-dichloro-4-((1-oxo-2-(pyridin-2-ylmethyl)-1,2,3,4-tetrahydroisoquinolin-6-yl)oxy)phenyl)-1,2,4-triazi ne-3,5(2H,4H)-dione 46

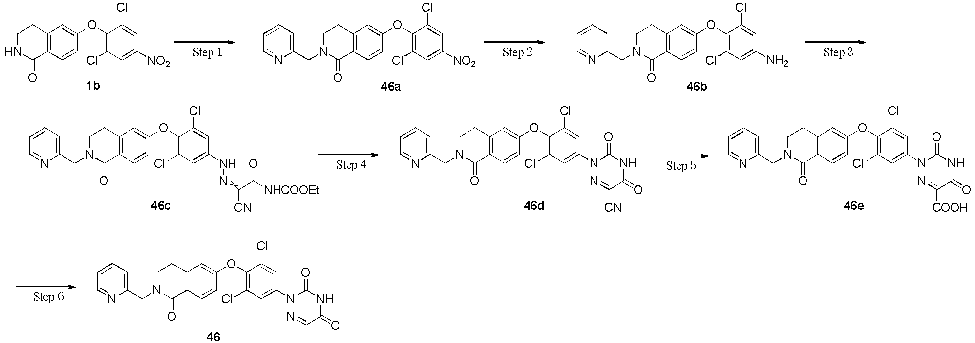

### Step 1: Synthesis of

### 6-(2,6-dichloro-4-nitrophenoxy)-2-(pyridin-2-ylmethyl)-3,4-dihydroisoquinolin-1(2H)-one 46a

To a solution of 6-(2,6-dichloro-4-nitrophenoxy)-3,4-dihydroisoquinolin-1(2*H*)-one **1b** (2.0 g, 5.7 mmol) in THF (20 mL) was added sodium hydride (0.60 g, 15 mmol, 60 mass% in oil) at 0°C. Then 3-(bromomethyl)pyridine hydrobromide (1.9 g, 7.5 mmol) and N,N-dimethylformamide (2 mL) were added, and the mixture was reacted at 15°C for 6 hours. The reaction was quenched by adding water (50 mL) at 0°C. The mixture was stirred for 10 minutes, extracted with ethyl acetate (120 mL). The combined organic layers were washed with saturated sodium chloride (20 mL × 3), dried over anhydrous sodium sulfate, and concentrated by suction filtration to give a yellow solid **46a** (2.3 g, 91% yield).

### Step 2: Synthesis of

### 6-(4-amino-2,6-dichlorophenoxy)-2-(pyridin-2-ylmethyl)-3,4-dihydroisoquinolin-1(2H)-one 46b

To a solution of 6-(2,6-dichloro-4-nitrophenoxy)-2-(pyridin-2-ylmethyl)-3,4-dihydroisoquinolin-1(2*H*)-one **46a** (2.3 g, 5.2 mmol) in acetic acid (25 mL) was added iron powder (0.72 g, 13 mmol), and the mixture was reacted at 60°C for 6 hours. The reaction solution was cooled to room temperature, then water (80 mL) was added to quench the reaction. The mixture was extracted with ethyl acetate (150 mL × 2). The combined organic layers were washed with saturated sodium chloride (20 mL × 3), dried over anhydrous sodium sulfate, and concentrated by suction filtration to give black oil **46b** (2.1 g, yield 98%).

### Step 3: Synthesis of ethyl

### (2-cyano-2-(2-(3,5-dichloro-4-((2-(pyridin-2-ylmethyl)-1-oxo-1,2,3,4-tetrahydroisoquinolin-6-yl)oxy)phenyl)hydr azono)acetyl)carbamate 46c

To a solution of 6-(4-amino-2,6-dichlorophenoxy)-2-(pyridin-2-ylmethyl)-3,4-dihydroisoquinolin-1(2*H*)-one **46b** (2.1 g, 5.1 mmol) in acetic acid (20 mL) was added a solution of sodium nitrite (0.45 g, 6.5 mmol) in water (5 mL) at 0°C, then *N*-cyanoacetylurethane (0.95 g, 6.1 mmol) was added. The mixture was reacted for 2 hours. Water (50 mL) was added to quench the reaction. The mixture was stirred for 10 minutes, filtered, and the filter cake was collected and dried to obtain a yellow solid **46c** (2.8 g, yield 95%).

### Step 4: Synthesis of

### 2-(3,5-dichloro-4-((2-(pyridin-2-ylmethyl)-1-oxo-1,2,3,4-tetrahydroisoquinolin-6-yl)oxy)phenyl)-3,5-dioxo-2,3,4, 5-tetrahydro-1,2,4-triazine-6-carbonitrile 46d

To a solution of ethyl (2-cyano-2-(2-(3,5-dichloro-4-((2-(pyridin-2-ylmethyl)-1-oxo-1,2,3,4-tetrahydroisoquinolin-6-yl)oxy)phenyl)hydr azono)acetyl)carbamate **46c** (2.8 g, 4.8 mmol) in *N*,*N*-dimethylformamide (30 mL) was added sodium acetate (0.43 g, 5.2 mmol), and the mixture was reacted at 120°C for 6 hours. The reaction solution was cooled to room temperature, then water (50 mL) was added to quench the reaction. The reaction mixture was stirred for 15 minutes, and filtered. The filter cake was collected and dried, then recrystallized with ethyl acetate/petroleum ether (2/1, 30 mL) at 85 °C to give a white solid **46d** (1.3 g, yield 50%, HPLC purity: 91.60%).
MS (ESI, pos. ion) *m*/*z*: 535.9 [M+H]⁺;
¹H NMR (400 MHz, DMSO-*d₆*) δ (ppm) 13.11 (s, 1H), 8.51 (d, *J* = 4.8 Hz, 1H), 8.16-7.81 (m, 3H), 7.76 (t, J = 7.8 Hz, 1H), 7.51-7.11 (m, 2H), 7.05- 6.72 (m, 2H), 4.78 (s, 2H), 3.61 (t, *J =* 6.6 Hz, 2H), 3.01 (t, *J =* 6.5 Hz, 2H).

### Step 5: Synthesis of

### 2-(3,5-dichloro-4-((2-(pyridin-2-ylmethyl)-1-oxo-1,2,3,4-tetrahydroisoquinolin-6-yl)oxy)phenyl)-3,5-dioxo-2,3,4, 5-tetrahydro-1,2,4-triazine-6-carboxylic acid 46e

2-(3,5-Dichloro-4-((2-(pyridin-2-ylmethyl)-1-oxo-1,2,3,4-tetrahydroisoquinolin-6-yl)oxy)phenyl)-3,5-dioxo-2,3,4,5-tetrahydro-1,2,4-triazine-6-carbonitrile **46d** (1.2 g, 2.2 mmol) was dissolved in acetic acid (12 mL), then concentrated hydrochloric acid (6.0 mL) was added. The mixture was reacted at 120°C for 12 hours. The reaction solution was cooled to room temperature, and concentrated *in vacuo* to obtain a yellow solid **46e** (1.2 g, yield 97%).

### Step 6: Synthesis of

### 2-(3,5-dichloro-4-((2-(pyridin-2-ylmethyl)-1-oxo-1,2,3,4-tetrahydroisoquinolin-6-yl)oxy)phenyl)-1,2,4-triazine-3, 5(2H,4H)-dione 46

2-(3,5-Dichloro-4-((2-(pyridin-2-ylmethyl)-1-oxo-1,2,3,4-tetrahydroisoquinolin-6-yl)oxy)phenyl)-3,5-dioxo-2,3,4,5-tetrahydro-1,2,4-triazine-6-carboxylic acid **46e** (1.0 g, 1.8 mmol) was dissolved in thioglycolic acid (3 mL). The mixture was reacted at 140°C for 12 hours. The reaction solution was cooled to room temperature and the reaction solution was purified by silica gel column chromatography (petroleum ether/ethyl acetate=7/3) to obtain a white solid **46** (0.43 g, yield 47%, HPLC purity: 92.83%).
MS (ESI, neg. ion) *m*/*z:* 511.0 [M+H]⁺;
¹H NMR (400 MHz, DMSO-*d₆*) δ (ppm) 12.52 (s, 1H), 8.51 (d, *J* = 4.8 Hz, 1H), 8.02-7.83 (m, 3H), 7.83-7.63 (m, 2H), 7.38-7.23 (m, 2H), 6.93-6.76 (m, 2H), 4.78 (s, 2H), 3.61 (t, *J* = 6.6 Hz, 2H), 3.01 (t, *J* = 6.6 Hz, 2H).

### Example 47

### 2-(3,5-dichloro-4-((1-oxo-2-(pyridin-2-yl)-1,2,3,4-tetrahydroisoquinolin-6-yl)oxy)phenyl)-3,5-dioxo-2,3,4,5-t etrahydro-1,2,4-triazine-3,5(2H,4H)-dione 47

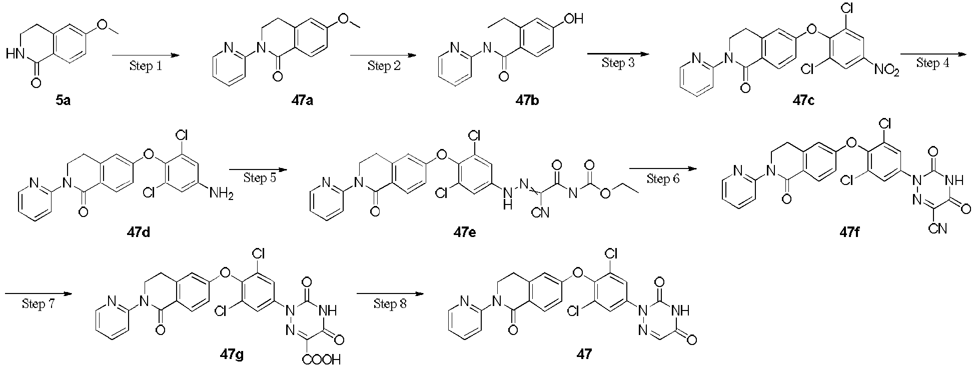

### Step 1: Synthesis of 6-methoxy-2-(pyridin-2-yl)-3,4-dihydroisoquinolin-1(2H)-one 47a

6-Methoxy-3,4-dihydroisoquinolin-1(2*H*)-one **5a** (1.50 g, 8.5 mmol), ketone iodide (0.48 g, 2.5 mmol), 2-iodopyridine (3.5 g, 17 mmol) and potassium carbonate (2.3 g, 17 mmol) were dissolved in N,N-dimethylformamide (40 mL), and the mixture was reacted at 150°C for 11 hours. The reaction solution was cooled to room temperature, and water (40 mL) was added to quench the reaction. The mixture was extracted with ethyl acetate (60 mL × 2). The combined organic layers were washed with saturated sodium chloride (20 mL), dried over ahydrous sodium sulfate and concentrated by suction filtration. The residue was purified by silica gel column chromatography (petroleum ether/ethyl acetate = 2/1) to give a white solid **47a** (1.66 g, yield 77%).

MS (ESI, pos. ion) *m*/*z*: 255.1 [M+H]⁺.

### Step 2: Synthesis of 6-hydroxy-2-(pyridin-2-yl)-3,4-dihydroisoquinolin-1(2H)-one 47a

At 0°C, to a solution of 6-methoxy-2-(pyridin-2-yl)-3,4-dihydroisoquinolin-1(2*H*)-one **47a** (1.67 g, 6.54 mmol) in dichloromethane (40 mL) was added boron tribromide (1.60 mL, 16.6 mmol) dropwise. Then the mixture was reacted at room temperature for 16 hours. The reaction solution was quenched by pouring into ice water (30 mL). The mixture was stirred for 10 minutes, filtered, and the filter cake was washed with water (10 mL), and the filter cake was collected and dried to obtain a white solid **47b** (1.06 g, yield 68%).

MS (ESI, pos. ion) *m*/*z*: 258.2 [M+H]⁺.

### Step 3: Synthesis of 6-(2,6-dichloro-4-nitrophenoxy)-2-(pyridin-2-yl)-3,4-dihydroisoquinolin-1(2H)-one 47c

To a solution of 6-hydroxy-2-(pyridin-2-yl)-3,4-dihydroisoquinolin-1(2*H*)-one **47b** (1.06 g, 4.41 mmol) and 1,2,3-trichloro-5-nitrobenzene (1.10 g, 4.86 mmol) in *N,N*-dimethylformamide (6 mL) was added potassium carbonate (1.22 g, 8.83 mmol), and the mixture was reacted at 40°C for 5.5 hours. The reaction solution was cooled to room temperature, then water (30 mL) was added. The reaction mixture was stirred for 10 minutes, filtered, and rinsed with water (10 mL). The filter cake was collected and dried to obtain a white solid **47c** (1.75 g, yield 92%).

### Step 4: Synthesis of 6-(4-amino-2,6-dichlorophenoxy)-2-(pyridin-2-yl)-3,4-dihydroisoquinolin-1(2H)-one 47d

To a solution of 6-(2,6-dichloro-4-nitrophenoxy)-2-(pyridin-2-yl)-3,4-dihydroisoquinolin-1(2*H*)-one **47c** (1.75 g, 4.07 mmol) in acetic acid (30 mL) was added iron powder (0.91 g, 16.3 mmol), and the mixture was reacted at 55°C for 8 hours. The reaction solution was cooled to room temperature, iron powder was removed, and water (50 mL) was added. The mixture was extracted with ethyl acetate (10 mL × 2). The combined organic layers were washed with saturated sodium chloride (20 mL × 3), dried over anhydrous sodium sulfate, and concentrated by suction filtration to give a white solid **47d** (1.45 g, yield 89%).

### Step 5: Synthesis of ethyl

### (2-cyano-2-(2-(3,5-dichloro-4-((1-oxo-2-(pyridin-2-yl)-1,2,3,4-tetrahydroisoquinolin-6-yl)oxy)phenyl)hydrazono) acetyl)carbamate 47e

To a solution of 6-(4-amino-2,6-dichlorophenoxy)-2-(pyridin-2-yl)-3,4-dihydroisoquinolin-1(2*H*)-one **47d** (0.40 g, 1.0 mmol) in acetic acid (8 mL) was added a solution of sodium nitrite (0.14 g, 2.0 mmol) in water (4 mL) dropwise at 0°C. After stirring for 15 minutes, *N*-cyanoacetylurethane (0.16 g, 1.0 mmol) was added, and the mixture was reacted for 3 hours. Water (20 mL) was added to the reaction solution. The mixture was stirred for 10 minutes, filtered, washed with water (10 mL × 2), and the filter cake was collected and dried to obtain a yellow solid **47e** (0.53 g, yield 94%).

### Step 6: Synthesis of

### 2-(3,5-dichloro-4-((1-oxo-2-(pyridin-2-yl)-1,2,3,4-tetrahydroisoquinolin-6-yl)oxy)phenyl)-3,5-dioxo-2,3,4,5-tetra hydro-1,2,4-triazine-6-carbonitrile 47f

To a solution of ethyl (2-cyano-2-(2-(3,5-dichloro-4-((1-oxo-2-(pyridin-2-yl)-1,2,3,4-tetrahydroisoquinolin-6-yl)oxy)phenyl)hydrazono) acetyl)carbamate **47e** (0.53 g, 0.93 mmol) in *N*,*N* dimethylformamide (11 mL) was added sodium acetate (0.17 g, 2.1 mmol), and the mixture was reacted at 120°C for 8 hours. The reaction solution was cooled to room temperature, then water (80 mL) was added to quench the reaction. The reaction mixture was stirred for 10 minutes, filtered, and rinsed with water (10 mL × 2). The filter cake was collected and dried to obtain a yellow solid **47f** (0.38 g, yield 78%, HPLC purity: 99.44%).
MS (ESI, neg. ion) *m*/*z*: 518.9 [M-H]⁻;
¹H NMR (400 MHz, DMSO-*d₆*) δ (ppm) 13.31 (s, 1H), 8.46 (d, *J* = 4.9 Hz, 1H), 8.02 (d, *J* = 8.6 Hz, 1H), 7.92-7.78 (m, 4H), 7.21 (t, *J* = 6.1 Hz, 1H), 6.97 (s, 1H), 6.91 (dd, *J* = 8.7, 2.5 Hz, 1H), 4.19 (t, *J* = 6.5 Hz, 2H), 3.10 (t, *J* = 6.4 Hz, 2H).

### Step 7: Synthesis of

### 2-(3,5-dichloro-4-((1-oxo-2-(pyridin-2-yl)-1,2,3,4-tetrahydroisoquinolin-6-yl)oxy)phenyl)-3,5-dioxo-2,3,4,5-tetra hydro-1,2,4-triazine-6-carboxylic acid 47g

2-(3,5-Dichloro-4-((1-oxo-2-(pyridin-2-yl)-1,2,3,4-tetrahydroisoquinolin-6-yl)oxy)phenyl)-3,5-dio xo-2,3,4,5-tetrahydro-1,2,4-triazine-6-carbonitrile **47f** (0.38 g, 0.73 mmol) was dissolved in acetic acid (6 mL), then concentrated hydrochloric acid (3 mL) was added. The mixture was reacted at 100°C for 10 hours. The reaction solution was cooled to room temperature, then water (20 mL) was added. The reaction mixture was stirred for 10 minutes, filtered, and rinsed with water (10 mL × 3). The filter cake was collected and dried to obtain a yellow solid **47g** (0.39 g, yield 96%).

### Step 8: Synthesis of

### 2-(3,5-dichloro-4-((1-oxo-2-(pyridin-2-yl)-1,2,3,4-tetrahydroisoquinolin-6-yl)oxy)phenyl)-1,2,4-triazine-3,5(2H,4 H)-dione 47

2-(3,5-Dichloro-4-((1-oxo-2-(pyridin-2-yl)-1,2,3,4-tetrahydroisoquinolin-6-yl)oxy)phenyl)-3,5-dio xo-2,3,4,5-tetrahydro-1,2,4-triazine-6-carboxylic acid **47g** (0.39 g, 0.72 mmol) was dissolved in thioglycolic acid (2 mL). The mixture was reacted at 130°C for 7 hours. The reaction solution was cooled to room temperature, and ethyl acetate (30 mL) was added. The mixture was washed successively with water (10 mL) and saturated sodium chloride solution (10 mL), dried over anhydrous sodium sulfate and concentrated by suction filtration. The residue was purified by silica gel column chromatography (petroleum ether/ethyl acetate = 3/1), and the obtained solid was recrystallized at 85°C (petroleum ether/ethyl acetate = 1/1, 20 mL) to give a white solid **47** (0.11 g, yield 31%, HPLC purity: 93.27%).
MS (ESI, neg. ion) *m*/*z*: 494.0 [M-H]⁻;
¹H NMR (400 MHz, DMSO-*d₆*) δ (ppm) 12.54 (s, 1H), 8.46 (dd, *J* = 5.0, 1.9 Hz, 1H), 8.03 (d, *J* = 8.6 Hz, 1H), 7.92-7.77 (m, 4H), 7.74 (s, 1H), 7.25-7.18 (m, 1H), 6.95 (d, *J* = 2.6 Hz, 1H), 6.88 (dd, *J* = 8.7, 2.6 Hz, 1H), 4.19 (t, *J* = 6.3 Hz, 2H), 3.11 (t, *J* = 6.4 Hz, 2H).

### Example 48

### 2-(3,5-dichloro-4-((1-oxo-2-(pyridin-4-yl)-1,2,3,4-tetrahydroisoquinolin-6-yl)oxy)phenyl)-3,5-dioxo-2,3,4,5-t etrahydro-1,2,4-triazine-3,5(2H,4H)-dione 48

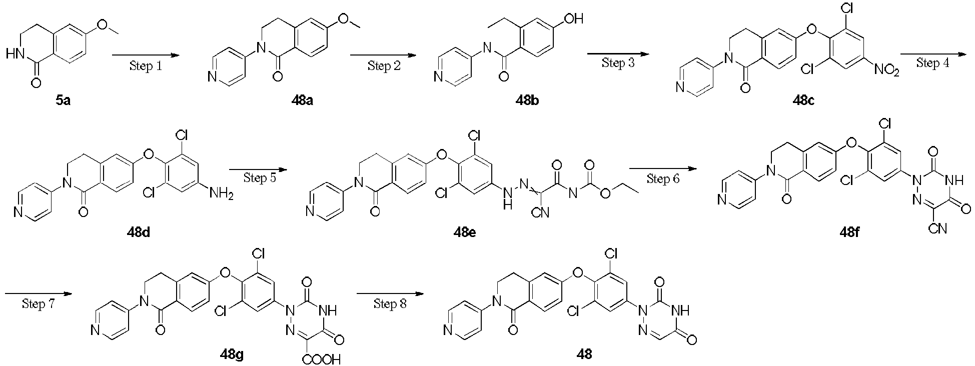

### Step 1: Synthesis of 6-methoxy-2-(pyridin-4-yl)-3,4-dihydroisoquinolin-1(2H)-one 48a

6-Methoxy-3,4-dihydroisoquinolin-1(2*H*)-one **5a** (1.50 g, 8.5 mmol), ketone iodide (0.32 g, 1.7 mmol), 4-iodopyridine (3.2 g, 17 mmol) and potassium carbonate (2.3 g, 17 mmol) were dissolved in toluene (20 mL), and the mixture was reacted at 120°C for 99 hours. The reaction solution was cooled to room temperature, and water (40 mL) was added to quench the reaction. The mixture was extracted with ethyl acetate (60 mL × 2). The combined organic layers were washed with saturated sodium chloride (20 mL), dried over ahydrous sodium sulfate and concentrated by suction filtration. The residue was purified by silica gel column chromatography (petroleum ether/ethyl acetate = 1/1) to give a white solid **48a** (1.85 g, yield 86%).

### Step 2: Synthesis of 6-hydroxy-2-(pyridin-4-yl)-3,4-dihydroisoquinolin-1(2H)-one 48b

At 0°C, to a solution of 6-methoxy-2-(pyridin-4-yl)-3,4-dihydroisoquinolin-1(2*H*)-one **48a** (1.83 g, 7.20 mmol) in dichloromethane (30 mL) was added boron tribromide (1.73 mL, 18.0 mmol) dropwise. Then the mixture was reacted at room temperature for 16 hours. The reaction solution was quenched by pouring into ice water (30 mL). The mixture was stirred for 10 minutes, filtered, and the filter cake was washed with water (10 mL), and the filter cake was collected and dried to obtain a white solid **48b** (1.80 g, yield 100%).

### Step 3: Synthesis of 6-(2,6-dichloro-4-nitrophenoxy)-2-(pyridin-4-yl)-3,4-dihydroisoquinolin-1(2H)-one 48c

To a solution of 6-hydroxy-2-(pyridin-4-yl)-3,4-dihydroisoquinolin-1(2*H*)-one **48b** (1.73 g, 7.20 mmol) and 1,2,3-trichloro-5-nitrobenzene (1.79 g, 7.90 mmol) in *N,N*-dimethylformamide (20 mL) was added potassium carbonate (3.98 g, 28.8 mmol), and the mixture was reacted at 40°C for 5.5 hours. The reaction solution was cooled to room temperature, then water (30 mL) was added. The reaction mixture was stirred for 10 minutes, filtered, and rinsed with water (10 mL). The filter cake was collected and dried to obtain a white solid **48c** (2.56 g, yield 83%).

### Step 4: Synthesis of 6-(4-amino-2,6-dichlorophenoxy)-2-(pyridin-4-yl)-3,4-dihydroisoquinolin-1(2H)-one 48d

To a solution of 6-(2,6-dichloro-4-nitrophenoxy)-2-(pyridin-4-yl)-3,4-dihydroisoquinolin-1(2*H*)-one **48c** (2.16 g, 5.02 mmol) in acetic acid (30 mL) was added iron powder (1.07 g, 19.2 mmol), and the mixture was reacted at 55°C for 4 hours. The reaction solution was cooled to room temperature, iron powder was removed, and water (100 mL) was added. The mixture was extracted with ethyl acetate (20 mL × 2). The combined organic layers were washed with saturated sodium chloride (20 mL × 3), dried over anhydrous sodium sulfate, and concentrated by suction filtration to give a white solid **48d** (1.98 g, yield 98%).

### Step 5: Synthesis of ethyl

### (2-cyano-2-(2-(3,5-dichloro-4-((1-oxo-2-(pyridin-4-yl)-1,2,3,4-tetrahydroisoquinolin-6-yl)oxy)phenyl)hydrazono) acetyl)carbamate 48e

To a solution of 6-(4-amino-2,6-dichlorophenoxy)-2-(pyridin-4-yl)-3,4-dihydroisoquinolin-1(2*H*)-one **48d** (0.50 g, 1.25 mmol) in acetic acid (10 mL) was added a solution of sodium nitrite (0.17 g, 2.49 mmol) in water (5 mL) dropwise at 0°C. After stirring for 15 minutes, *N*-cyanoacetylurethane (0.23 g, 1.5 mmol) was added, and the mixture was reacted for 3 hours. Water (20 mL) was added to the reaction solution. The mixture was stirred for 30 minutes, filtered, washed with water (10 mL × 2), and the filter cake was collected and dried to obtain a yellow solid **48e** (0.70 g, yield 99%).

### Step 6: Synthesis of

### 2-(3,5-dichloro-4-((1-oxo-2-(pyridin-4-yl)-1,2,3,4-tetrahydroisoquinolin-6-yl)oxy)phenyl)-3,5-dioxo-2,3,4,5-tetra hydro-1,2,4-triazine-6-carbonitrile 48f

To a solution of ethyl (2-cyano-2-(2-(3,5-dichloro-4-((1-oxo-2-(pyridin-4-yl)-1,2,3,4-tetrahydroisoquinolin-6-yl)oxy)phenyl)hydrazono) acetyl)carbamate **48e** (0.70 g, 1.23 mmol) in *N,N*-dimethylformamide (15 mL) was added sodium acetate (0.25 g, 3.07 mmol), and the mixture was reacted at 120°C for 8 hours. The reaction solution was cooled to room temperature, then water (80 mL) was added to quench the reaction. The reaction mixture was stirred for 10 minutes, filtered, and rinsed with water (10 mL × 2). The filter cake was collected and dried to obtain a yellow solid **48f** (0.62 g, yield 96%, HPLC purity: 97.11%).
MS (ESI, neg. ion) *m*/*z*: 519.0 [M-H]⁻;
¹H NMR (400 MHz, DMSO-*d₆*) δ (ppm) 8.58 (s, 2H), 8.00 (d, *J* = 8.6 Hz, 1H), 7.86 (s, 2H), 7.52 (s, 2H), 6.97 (d, *J* = 2.6 Hz, 1H), 6.91 (dd, *J* = 8.7, 2.7 Hz, 1H), 4.04 (t, *J* = 6.5 Hz, 2H), 3.14 (t, *J* = 6.3 Hz, 2H).

### Step 7: Synthesis of

### 2-(3,5-dichloro-4-((1-oxo-2-(pyridin-4-yl)-1,2,3,4-tetrahydroisoquinolin-6-yl)oxy)phenyl)-3,5-dioxo-2,3,4,5-tetra hydro-1,2,4-triazine-6-carboxylic acid 48g

2-(3,5-Dichloro-4-((1-oxo-2-(pyridin-4-yl)-1,2,3,4-tetrahydroisoquinolin-6-yl)oxy)phenyl)-3,5-dio xo-2,3,4,5-tetrahydro-1,2,4-triazine-6-carbonitrile **48f** (0.52 g, 1.00 mmol) was dissolved in acetic acid (4 mL), then concentrated hydrochloric acid (2 mL) was added. The mixture was reacted at 100°C for 12 hours. The reaction solution was cooled to room temperature, then water (20 mL) was added. The reaction mixture was stirred for 10 minutes, filtered, and rinsed with water (10 mL × 3). The filter cake was collected and dried to obtain a yellow solid **48g** (0.51 g, yield 95%).

### Step 8: Synthesis of

### 2-(3,5-dichloro-4-((1-oxo-2-(pyridin-4-yl)-1,2,3,4-tetrahydroisoquinolin-6-yl)oxy)phenyl)-1,2,4-triazine-3,5(2H,4 H)-dione 48

2-(3,5-Dichloro-4-((1-oxo-2-(pyridin-4-yl)-1,2,3,4-tetrahydroisoquinolin-6-yl)oxy)phenyl)-3,5-dio xo-2,3,4,5-tetrahydro-1,2,4-triazine-6-carboxylic acid **48g** (0.51 g, 0.94 mmol) was dissolved in thioglycolic acid (2 mL). The mixture was reacted at 120°C for 7 hours. The reaction solution was cooled to room temperature, and ethyl acetate (30 mL) was added. The mixture was washed successively with water (10 mL) and saturated sodium chloride solution (10 mL), then dried over anhydrous sodium sulfate and concentrated by suction filtration. The residue was purified by silica gel column chromatography (petroleum ether/ethyl acetate = 1/1), and the obtained solid was recrystallized at 85°C (petroleum ether/ethyl acetate = 1/1, 50 mL) to give a white solid **48** (99 mg, yield 21%, HPLC purity: 90.75%).
MS (ESI, neg. ion) *m*/*z:* 494.0 [M-H]⁻;
¹H NMR (400 MHz, DMSO-*d₆*) δ (ppm) 12.54 (s, 1H), 8.83 (d, *J* = 6.5 Hz, 2H), 8.08 (d, *J* = 6.7 Hz, 2H), 8.06 (d, *J* = 8.7 Hz, 1H), 7.89 (s, 2H), 7.75 (s, 1H), 6.99 (d, *J* = 2.6 Hz, 1H), 6.94 (dd, *J* = 8.7, 2.6 Hz, 1H), 4.20 (t, *J* = 6.3 Hz, 2H), 3.19 (t, *J* = 6.3 Hz, 2H).

### Example 49 2-(3,5-dichloro-4-((1-oxo-2-(pyridin-3-yl)-1,2,3,4-tetrahydroisoquinolin-6-yl)oxy)phenyl)-3,5-dioxo-2,3,4,5-t etrahydro-1,2,4-triazine-3,5(2H,4H)-dione 49

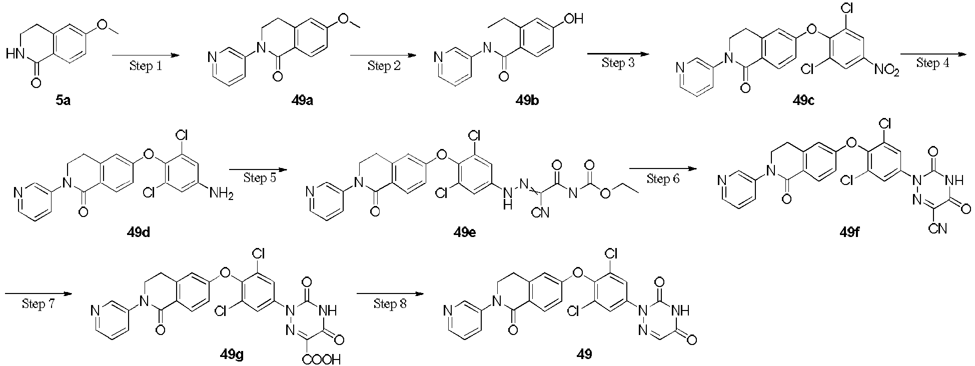

### Step 1: Synthesis of 6-methoxy-2-(pyridin-3-yl)-3,4-dihydroisoquinolin-1(2H)-one 49a

6-Methoxy-3,4-dihydroisoquinolin-1(2*H*)-one **5a** (1.50 g, 8.5 mmol), ketone iodide (0.32 g, 1.7 mmol), 3-iodopyridine (2.60 g, 12.7 mmol) and potassium carbonate (2.34 g, 17 mmol) were dissolved in toluene (30 mL), and the mixture was reacted at 120°C for 90 hours. The reaction solution was cooled to room temperature, and water (40 mL) was added to quench the reaction. The mixture was extracted with ethyl acetate (60 mL × 2). The combined organic layers were washed with saturated sodium chloride (20 mL), dried over ahydrous sodium sulfate and concentrated by suction filtration. The residue was purified by silica gel column chromatography (petroleum ether/ethyl acetate = 2/1) to give a white solid **49a** (2.15 g, yield 100%).

### Step 2: Synthesis of 6-hydroxy-2-(pyridin-3-yl)-3,4-dihydroisoquinolin-1(2H)-one 49b

At 0°C, to a solution of 6-methoxy-2-(pyridin-3-yl)-3,4-dihydroisoquinolin-1(2*H*)-one **49a** (2.15 g, 8.45 mmol) in dichloromethane (40 mL) was added boron tribromide (2.04 mL, 21.2 mmol) dropwise. Then the mixture was reacted at room temperature for 16 hours. The reaction solution was quenched by pouring into ice water (30 mL). The mixture was stirred for 10 minutes, filtered, and the filter cake was washed with water (10 mL), and the filter cake was collected and dried to obtain a white solid **49b** (2.03 g, yield 100%).

### Step 3: Synthesis of 6-(2,6-dichloro-4-nitrophenoxy)-2-(pyridin-3-yl)-3,4-dihydroisoquinolin-1(2H)-one 49c

To a solution of 6-hydroxy-2-(pyridin-3-yl)-3,4-dihydroisoquinolin-1(2*H*)-one **49b** (2.03 g, 8.45 mmol) and 1,2,3-trichloro-5-nitrobenzene (2.10 g, 9.27 mmol) in *N,N*-dimethylformamide (40 mL) was added potassium carbonate (9.34 g, 67.6 mmol), and the mixture was reacted at 70°C for 5.5 hours. The reaction solution was cooled to room temperature, then water (30 mL) was added. The reaction mixture was stirred for 10 minutes, filtered, and rinsed with water (10 mL). The filter cake was collected and dried to obtain a white solid **49c** (2.34 g, yield 65%).

### Step 4: Synthesis of 6-(4-amino-2,6-dichlorophenoxy)-2-(pyridin-3-yl)-3,4-dihydroisoquinolin-1(2H)-one 49d

To a solution of 6-(2,6-dichloro-4-nitrophenoxy)-2-(pyridin-3-yl)-3,4-dihydroisoquinolin-1(2*H*)-one **49c** (2.34 g, 5.45 mmol) in acetic acid (30 mL) was added iron powder (1.22 g, 21.8 mmol), and the mixture was reacted at 55°C for 5 hours. The reaction solution was cooled to room temperature, iron powder was removed, and water (50 mL) was added. The mixture was extracted with ethyl acetate (20 mL × 2). The combined organic layers were washed with saturated sodium chloride (20 mL × 3), dried over anhydrous sodium sulfate, and concentrated by suction filtration to give a white solid **49d** (1.75 g, yield 80%).

### Step 5: Synthesis of ethyl

### (2-cyano-2-(2-(3,5-dichloro-4-((1-oxo-2-(pyridin-3-yl)-1,2,3,4-tetrahydroisoquinolin-6-yl)oxy)phenyl)hydrazono) acetyl)carbamate 49e

To a solution of 6-(4-amino-2,6-dichlorophenoxy)-2-(pyridin-3-yl)-3,4-dihydroisoquinolin-1(2*H*)-one **49d** (0.50 g, 1.25 mmol) in acetic acid (10 mL) was added a solution of sodium nitrite (0.17 g, 2.49 mmol) in water (5 mL) dropwise at 0°C. After stirring for 15 minutes, *N*-cyanoacetylurethane (0.23 g, 1.5 mmol) was added, and the mixture was reacted for 3 hours. Water (20 mL) was added to the reaction solution. The mixture was stirred for 30 minutes, filtered, washed with water (10 mL × 2), and the filter cake was collected and dried to obtain a yellow solid **49e** (0.71 g, yield 100%).

### Step 6: Synthesis of

### 2-(3,5-dichloro-4-((1-oxo-2-(pyridin-3-yl)-1,2,3,4-tetrahydroisoquinolin-6-yl)oxy)phenyl)-3,5-dioxo-2,3,4,5-tetra hydro-1,2,4-triazine-6-carbonitrile 49f

To a solution of ethyl (2-cyano-2-(2-(3,5-dichloro-4-((1-oxo-2-(pyridin-3-yl)-1,2,3,4-tetrahydroisoquinolin-6-yl)oxy)phenyl)hydrazono) acetyl)carbamate **48e** (0.71 g, 1.25 mmol) in N,Ndimethylformamide (11 mL) was added sodium acetate (0.25 g, 3.07 mmol), and the mixture was reacted at 120°C for 8 hours. The reaction solution was cooled to room temperature, then water (80 mL) was added to quench the reaction. The reaction mixture was stirred for 10 minutes, filtered, and rinsed with water (10 mL × 2). The filter cake was collected and dried to obtain a yellow solid **49f** (0.62 g, yield 96%, HPLC purity: 97.94%).
MS (ESI, neg. ion) *m*/*z*: 518.9 [M-H]⁻;
¹H NMR (400 MHz, DMSO-*d₆*) δ (ppm) 8.80 (s, 1H), 8.53 (d, *J* = 5.0 Hz, 1H), 8.07 (d, *J* = 8.5 Hz, 1H), 7.98 (d, *J* = 8.6 Hz, 1H), 7.86 (s, 2H), 7.65 (dd, *J* = 8.3, 4.9 Hz, 1H), 6.98 (d, *J* = 2.6 Hz, 1H), 6.91 (dd, *J =* 8.6, 2.6 Hz, 1H), 4.04 (t, *J* = 6.4 Hz, 2H), 3.17 (t, *J* = 6.2 Hz, 2H).

### Step 7: Synthesis of

### 2-(3,5-dichloro-4-((1-oxo-2-(pyridin-3-yl)-1,2,3,4-tetrahydroisoquinolin-6-yl)oxy)phhenyl)-3,5-dioxo-2,3,4,5-tetra hydro-1,2,4-triazine-6-carboxylic acid 49g

2-(3,5-Dichloro-4-((1-oxo-2-(pyridin-3-yl)-1,2,3,4-tetrahydroisoquinolin-6-yl)oxy)phenyl)-3,5-dio xo-2,3,4,5-tetrahydro-1,2,4-triazine-6-carbonitrile **49f** (0.68 g, 1.30 mmol) was dissolved in acetic acid (8 mL), then concentrated hydrochloric acid (4 mL) was added. The mixture was reacted at 100°C for 10 hours. The reaction solution was cooled to room temperature, then water (20 mL) was added. The reaction mixture was stirred for 10 minutes, filtered, and rinsed with water (10 mL × 3). The filter cake was collected and dried to obtain a yellow solid **49g** (0.53 g, yield 75%).

### Step 8: Synthesis of

### 2-(3,5-dichloro-4-((1-oxo-2-(pyridin-3-yl)-1,2,3,4-tetrahydroisoquinolin-6-yl)oxy)phenyl)-1,2,4-triazine-3,5(2H,4 H)-dione 49

2-(3,5-Dichloro-4-((1-oxo-2-(pyridin-3-yl)-1,2,3,4-tetrahydroisoquinolin-6-yl)oxy)phenyl)-3,5-dio xo-2,3,4,5-tetrahydro-1,2,4-triazine-6-carboxylic acid **49g** (0.53 g, 0.97 mmol) was dissolved in thioglycolic acid (2 mL). The mixture was reacted at 130°C for 4 hours. The reaction solution was cooled to room temperature, and ethyl acetate (30 mL) was added. The mixture was washed successively with water (10 mL) and saturated sodium chloride solution (10 mL), dried over anhydrous sodium sulfate and concentrated by suction filtration. The residue was purified by silica gel column chromatography (petroleum ether/ethyl acetate = 3/1), and the obtained solid was recrystallized at 85°C (petroleum ether/ethyl acetate = 1/1, 20 mL) to give a white solid **49** (0.26 g, yield 53%, HPLC purity: 92.52%).
MS (ESI, neg. ion) *m*/*z*: 494.0 [M-H]⁻;
¹H NMR (400 MHz, DMSO-*d₆*) δ (ppm) 12.54 (s, 1H), 9.01 (dt, *J* = 8.2, 2.5 Hz, 1H), 8.69 (d, *J* = 5.5 Hz, 1H), 8.47 (t, *J* = 9.2 Hz, 1H), 8.00 (d, *J* = 8.6 Hz, 1H), 7.97 (d, *J* = 2.3 Hz, 1H), 7.89 (s, 2H), 7.74 (d, *J =* 1.5 Hz, 1H), 6.97 (d, *J* = 2.6 Hz, 1H), 6.91 (dd, *J* = 8.6, 2.7 Hz, 1H), 4.11 (t, *J* = 6.2 Hz, 2H), 3.18 (t, *J* = 6.4 Hz, 2H).

### Example 50 2-(3,5-dichloro-4-((1-oxo-2-(pyrimidin-2-yl)-1,2,3,4-tetrahydroisoquinolin-6-yl)oxy)phenyl)-3,5-dioxo-2,3,4, 5-tetrahydro-1,2,4-triazine-3,5(2H,4H)-dione 50

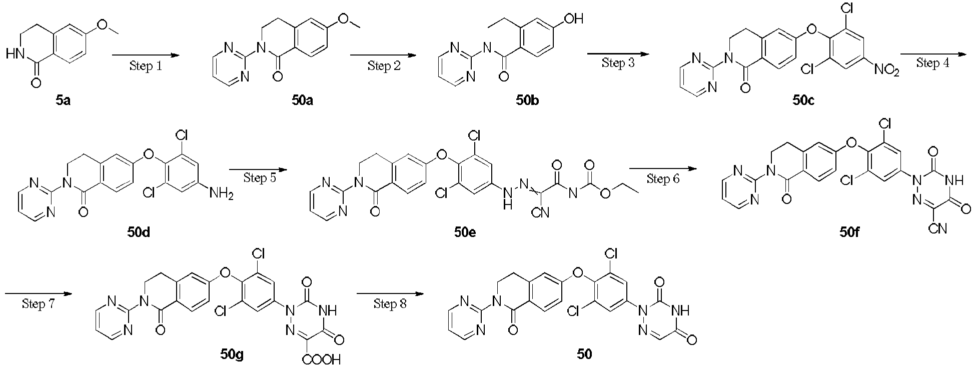

### Step 1: Synthesis of 6-methoxy-2-(pyrimidin-2-yl)-3,4-dihydroisoquinolin-1(2H)-one 50a

6-Methoxy-3,4-dihydroisoquinolin-1(2*H*)-one **5a** (1.50 g, 8.5 mmol), 4,5-bisdiphenylphosphine-9,9-dimethylxanthene (0.49 g, 0.85 mmol), cesium carbonate (3.68 g, 17.0 mmol) and 2-iodopyrimidine (3.5 g, 17.0 mmol) were dissolved in 1,4-dioxane (20 mL), and the mixture was reacted at 120°C for 17 hours. The reaction solution was cooled to room temperature, and filtered through a celite pad. The filter cake was washed with dichloromethane (50 mL) and ethanol (10 mL), the filtrate was concentrated, and the residue was purified by silica gel column chromatography (petroleum ether/ethyl acetate = 1/ 2) to give a tan solid **50a** (1.29 g, yield 90%).

### Step 2: Synthesis of 6-hydroxy-2-(pyrimidin-2-yl)-3,4-dihydroisoquinolin-1(2H)-one 50b

At 0°C, to a solution of 6-methoxy-2-(pyrimidin-2-yl)-3,4-dihydroisoquinolin-1(2*H*)-one **50a** (1.29 g, 5.05 mmol) in dichloromethane (30 mL) was added boron tribromide (0.80 mL, 8.30 mmol) dropwise. Then the mixture was reacted at room temperature for 3 hours. The reaction solution was quenched by pouring into ice water (30 mL). The mixture was stirred for 10 minutes, filtered, and the filter cake was washed with water (10 mL), and the filter cake was collected and dried to obtain a white solid **50b** (1.22 g, yield 100%).

### Step 3: Synthesis of 6-(2,6-dichloro-4-nitrophenoxy)-2-(pyrimidin-2-yl)-3,4-dihydroisoquinolin-1(2H)-one 50c

To a solution of 6-hydroxy-2-(pyrimidin-2-yl)-3,4-dihydroisoquinolin-1(2*H*)-one **50b** (1.22 g, 5.06 mmol) and 1,2,3-trichloro-5-nitrobenzene (1.26 g, 5.56 mmol) in *N,N*-dimethylformamide (25 mL) was added potassium carbonate (1.40 g, 10.1 mmol), and the mixture was reacted at 70°C for 6 hours. The reaction solution was cooled to room temperature, then water (30 mL) was added. The reaction mixture was stirred for 10 minutes, filtered, and rinsed with water (10 mL). The filter cake was collected and dried to obtain a red solid **50c** (1.85 g, yield 85%).

### Step 4: Synthesis of 6-(4-amino-2,6-dichlorophenoxy)-2-(pyrimidin-2-yl)-3,4-dihydroisoquinolin-1(2H)-one 50d

To a solution of 6-(2,6-dichloro-4-nitrophenoxy)-2-(pyrimidin-2-yl)-3,4-dihydroisoquinolin-1(2*H*)-one **50c** (1.85 g, 4.29 mmol) in acetic acid (30 mL) was added iron powder (0.96 g, 17.1 mmol), and the mixture was reacted at 55°C for 10 hours. The reaction solution was cooled to room temperature, iron powder was removed, and water (50 mL) was added. The mixture was extracted with ethyl acetate (10 mL × 2). The combined organic layers were washed with saturated sodium chloride (20 mL × 3), dried over anhydrous sodium sulfate, and concentrated by suction filtration to give a white solid **50d** (1.32 g, yield 77%).

### Step 5: Synthesis of ethyl

### (2-cyano-2-(2-(3,5-dichloro-4-((1-oxo-2-(pyrimidin-2-yl)-1,2,3,4-tetrahydroisoquinolin-6-yl)oxy)phenyl)hydrazo no)acetyl)carbamate 50e

To a solution of 6-(4-amino-2,6-dichlorophenoxy)-2-(pyrimidin-2-yl)-3,4-dihydroisoquinolin-1(2*H*)-one **50d** (0.50 g, 1.25 mmol) in acetic acid (10 mL) was added a solution of sodium nitrite (0.17 g, 2.49 mmol) in water (5 mL) dropwise at 0°C. After stirring for 15 minutes, *N*-cyanoacetylurethane (0.23 g, 1.50 mmol) was added, and the mixture was reacted for 3.5 hours. Water (20 mL) was added to the reaction solution. The mixture was stirred for 30 minutes, filtered, washed with water (10 mL × 2), and the filter cake was collected and dried to obtain a yellow solid **50e** (0.70 g, yield 99%).

### Step 6: Synthesis of

### 2-(3,5-dichloro-4-((1-oxo-2-(pyrimidin-2-yl)-1,2,3,4-tetrahydroisoquinolin-6-yl)oxy)phenyl)-3,5-dioxo-2,3,4,5-tet rahydro-1,2,4-triazine-6-carbonitrile 50f

To a solution of ethyl (2-cyano-2-(2-(3,5-dichloro-4-((1-oxo-2-(pyrimidin-2-yl)-1,2,3,4-tetrahydroisoquinolin-6-yl)oxy)phenyl)hydrazo no)acetyl)carbamate **50e** (0.70 g, 1.23 mmol) in *N,N*-dimethylformamide (15 mL) was added sodium acetate (0.25 g, 3.1 mmol), and the mixture was reacted at 120°C for 10 hours. The reaction solution was cooled to room temperature, then water (40 mL) was added to quench the reaction. The reaction mixture was stirred for 10 minutes, filtered, and rinsed with water (10 mL × 2). The filter cake was collected and dried, and the obtained brown solid was recrystallized (ethyl acetate/methanol/petroleum ether = 2/1/2, 50 mL) at 85°C to give a yellow solid **50f** (0.46 g, yield 71%, HPLC purity: 97.56%).
MS (ESI, neg. ion) *m*/*z*: 520.6 [M-H]⁻;
¹H NMR (400 MHz, DMSO-*d₆*) δ (ppm) 8.81 (d, *J* = 4.8 Hz, 2H), 8.02 (d, *J* = 8.6 Hz, 1H), 7.87 (s, 2H), 7.35 (t, *J* = 4.9 Hz, 1H), 6.93 (s, 1H), 6.90 (d, *J* = 8.2 Hz, 1H), 4.10 (t, *J* = 6.2 Hz, 2H), 3.12 (t, *J* = 6.5 Hz, 2H).

### Step 7: Synthesis of

### 2-(3,5-dichloro-4-((1-oxo-2-(pyrimidin-2-yl)-1,2,3,4-tetrahydroisoquinolin-6-yl)oxy)phenyl)-3,5-dioxo-2,3,4,5-tet rahydro-1,2,4-triazine-6-carboxylic acid 50g

2-(3,5-Dichloro-4-((1-oxo-2-(pyrimidin-2-yl)-1,2,3,4-tetrahydroisoquinolin-6-yl)oxy)phenyl)-3,5-d ioxo-2,3,4,5-tetrahydro-1,2,4-triazine-6-carbonitrile **50f** (0.30 g, 0.57 mmol) was dissolved in acetic acid (4 mL), then concentrated hydrochloric acid (2 mL) was added. The mixture was reacted at 100°C for 12 hours. The reaction solution was cooled to room temperature, then water (20 mL) was added. The reaction mixture was stirred for 10 minutes, filtered, and rinsed with water (10 mL × 3). The filter cake was collected and dried to obtain a yellow solid **50g** (0.30 g, yield 96%).

### Step 8: Synthesis of

### 2-(3,5-dichloro-4-((1-oxo-2-(pyrimidin-2-yl)-1,2,3,4-tetrahydroisoquinolin-6-yl)oxy)phenyl)-1,2,4-triazine-3,5(2 H,4H)-dione 50

2-(3,5-Dichloro-4-((1-oxo-2-(pyrimidin-2-yl)-1,2,3,4-tetrahydroisoquinolin-6-yl)oxy)phenyl)-3,5-d ioxo-2,3,4,5-tetrahydro-1,2,4-triazine-6-carboxylic acid **50g** (0.30 g, 0.55 mmol) was dissolved in thioglycolic acid (1 mL). The mixture was reacted at 90°C for 5 hours. The reaction solution was cooled to room temperature, and ethyl acetate (30 mL) was added. The mixture was washed successively with water (10 mL) and saturated sodium chloride solution (10 mL), then dried over anhydrous sodium sulfate and concentrated by suction filtration. The residue was separated and purified by pre-HPLC [45%ACN / 55%H₂O (0.1%TFA), Phenomenes ACE specification: C18 10µm×50mm×250mm, flow rate: 100 mL/min] to obtain a white solid **50** (60 mg, yield 22%, HPLC purity: 99.63%).
MS (ESI, neg. ion) *m*/*z:* 495.1 [M-H]⁻;
¹H NMR (400 MHz, DMSO-*d₆*) δ (ppm) 12.53 (s, 1H), 8.82 (d, *J* = 4.9 Hz, 2H), 8.03 (d, *J* = 8.7 Hz, 1H), 7.88 (s, 2H), 7.74 (s, 1H), 7.36 (t, *J* = 4.8 Hz, 1H), 6.95 (s, 1H), 6.89 (d, *J* = 8.7 Hz, 1H), 4.11 (t, *J* = 6.2 Hz, 2H), 3.13 (t, *J* = 6.2 Hz, 2H).

### Example 51

### 2-(3,5-dichloro-4-((2-formyl-1-oxo-1,2,3,4-tetrahydroisoquinolin-6-yl)oxy)phenyl)-3,5-dioxo-2,3,4,5-tetrahy dro-1,2,4-triazine-6-carbonitrile 51

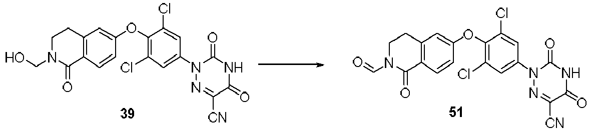

2-(3,5-Dichloro-4-((2-(hydroxymethyl)-1-oxo-1,2,3,4-tetrahydroisoquinolin-6-yl)oxy)phenyl)-3,5-dioxo-2,3,4,5-tetrahydro-1,2,4-triazine-6-carbonitrile **39** (30 mg, 0.063 mmol) and pyridinium dichromate (60 mg, 0.16 mmol) were dissolved in dichloromethane (6 mL). The mixture was reacted at room temperature for 15 hours. The reaction solution was added with ethyl acetate (10 mL), and ultrasonically oscillated for 10 minutes. The mixture was filtered and the filtrate was concentrated. The resulting residue was purified by silica gel column chromatography (petroleum ether/ethyl acetate = 1/3) to obtain a white solid **51** (21 mg, yield 70%, HPLC purity: 96.92%).
MS (ESI, neg. ion) *m*/*z:* 470.4 [M-H]⁻;
¹H NMR (400 MHz, DMSO-*d₆*) δ (ppm) 13.30 (s, 1H), 9.44 (s, 1H), 8.04 (d, *J =* 8.7 Hz, 1H), 7.86 (s, 2H), 7.01 (d, *J* = 2.1 Hz, 1H), 6.96 (dd, *J* = 8.6, 2.4 Hz, 1H), 3.88 (t, *J* = 6.2 Hz, 2H), 3.04 (t, *J* = 6.1 Hz, 2H).

### Example 52

### 2-(3,5-dichloro-4-((1-oxo-2-(thiazol-2-yl)-1,2,3,4-tetrahydroisoquinolin-6-yl)oxy)phenyl)-3,5-dioxo-2,3,4,5-te trahydro-1,2,4-triazine-3,5(2H,4H)-dione 52

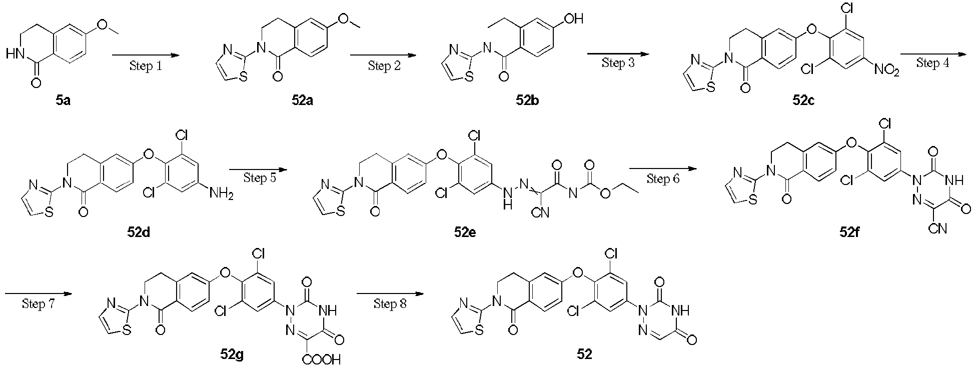

### Step 1: Synthesis of 6-methoxy-2-(thiazol-2-yl)-3,4-dihydroisoquinolin-1(2H)-one 52a

6-Methoxy-3,4-dihydroisoquinolin-1(2H)-one 5a (1.50 g, 8.5 mmol), ketone iodide (0.48 g, 2.5 mmol), 2-bromothiazole (2.80 g, 17 mmol) and potassium carbonate (2.3 g, 17 mmol) were dissolved in N,N-dimethylformamide (15 mL), and the mixture was reacted at 150°C for 41 hours. The reaction solution was cooled to room temperature, and water (40 mL) was added to quench the reaction. The mixture was extracted with ethyl acetate (60 mL × 2). The combined organic layers were washed with saturated sodium chloride (20 mL), dried over ahydrous sodium sulfate and concentrated by suction filtration. The residue was purified by silica gel column chromatography (petroleum ether/ethyl acetate = 8/1) to give a white solid **52a** (0.86 g, yield 39%).

### Step 2: Synthesis of 6-hydroxy-2-(thiazol-2-yl)-3,4-dihydroisoquinolin-1(2H)-one 52b

At 0°C, to a solution of 6-methoxy-2-(thiazol-2-yl)-3,4-dihydroisoquinolin-1(2*H*)-one **50a** (0.86 g, 3.30 mmol) in dichloromethane (20 mL) was added boron tribromide (0.80 mL, 8.30 mmol) dropwise. Then the mixture was reacted at room temperature for 10 hours. The reaction solution was quenched by pouring into ice water (30 mL). The mixture was stirred for 10 minutes, filtered, and the filter cake was washed with water (10 mL), and the filter cake was collected and dried to obtain a white solid **52b** (0.80 g, yield 98%).

### Step 3: Synthesis of 6-(2,6-dichloro-4-nitrophenoxy)-2-(thiazol-2-yl)-3,4-dihydroisoquinolin-1(2H)-one 52c

To a solution of 6-hydroxy-2-(thiazol-2-yl)-3,4-dihydroisoquinolin-1(2*H*)-one **52b** (0.80 g, 3.25 mmol) and 1,2,3-trichloro-5-nitrobenzene (0.81 g, 3.57 mmol) in *N,N*-dimethylformamide (16 mL) was added potassium carbonate (0.90 g, 6.50 mmol), and the mixture was reacted at 40°C for 17 hours. The reaction solution was cooled to room temperature, then water (30 mL) was added. The reaction mixture was stirred for 10 minutes, filtered, and rinsed with water (10 mL). The filter cake was collected and dried to obtain a white solid **52c** (1.06 g, yield 75%).

### Step 4: Synthesis of 6-(4-amino-2,6-dichlorophenoxy)-2-(thiazol-2-yl)-3,4-dihydroisoquinolin-1(2H)-one 52d

To a solution of 6-(2,6-dichloro-4-nitrophenoxy)-2-(thiazol-2-yl)-3,4-dihydroisoquinolin-1(2*H*)-one **52c** (1.06 g, 2.42 mmol) in acetic acid (20 mL) was added iron powder (0.54 g, 9.69 mmol), and the mixture was reacted at 55°C for 8 hours. The reaction solution was cooled to room temperature, iron powder was removed, and water (50 mL) was added. The mixture was extracted with ethyl acetate (10 mL × 2). The combined organic layers were washed with saturated sodium chloride (20 mL × 3), dried over anhydrous sodium sulfate, and concentrated by suction filtration to give a white solid **52d** (0.74 g, yield 75%).

### Step 5: Synthesis of ethyl

### (2-cyano-2-(2-(3,5-dichloro-4-((1-oxo-2-(thiazol-2-yl)-1,2,3,4-tetrahydroisoquinolin-6-yl)oxy)phenl)hydrazono) acetyl)carbamate 52e

To a solution of 6-(4-amino-2,6-dichlorophenoxy)-2-(thiazol-2-yl)-3,4-dihydroisoquinolin-1(2*H*)-one **52d** (0.40 g, 1.00 mmol) in acetic acid (8 mL) was added a solution of sodium nitrite (0.14 g, 2.00 mmol) in water (4 mL) dropwise at 0°C. After stirring for 15 minutes, *N*-cyanoacetylurethane (0.19 g, 1.19 mmol) was added, and the mixture was reacted for 3 hours. Water (20 mL) was added to the reaction solution. The mixture was stirred for 30 minutes, filtered, washed with water (10 mL × 2), and the filter cake was collected and dried to obtain a yellow solid **52e** (0.56 g, yield 100%).

### Step 6: Synthesis of

### 2-(3,5-dichloro-4-((1-oxo-2-(thiazol-2-yl)-1,2,3,4-tetrahydroisoquinolin-6-yl)oxy)phenyl)-3,5-dioxo-2,3,4,5-tetrah ydro-1,2,4-triazine-6-carbonitrile 52f

To a solution of ethyl (2-cyano-2-(2-(3,5-dichloro-4-((1-oxo-2-(thiazol-2-yl)-1,2,3,4-tetrahydroisoquinolin-6-yl)oxy)phenyl)hydrazono) acetyl)carbamate **52e** (0.56 g, 0.98 mmol) in *N,N*-dimethylformamide (12 mL) was added sodium acetate (0.18 g, 2.20 mmol), and the mixture was reacted at 120°C for 10 hours. The reaction solution was cooled to room temperature, then water (80 mL) was added to quench the reaction. The reaction mixture was stirred for 10 minutes, filtered, and rinsed with water (10 mL × 2). The filter cake was collected and dried, and the obtained brown solid was recrystallized (ethyl acetate/petroleum ether = 1/1, 100 mL) at 85°C to give a yellow solid **52f** (0.45 g, yield 87%, HPLC purity: 89.52%).
MS (ESI, neg. ion) *m*/*z*: 525.4 [M-H]⁻;
¹H NMR (400 MHz, DMSO-*d₆*) δ (ppm) 8.05 (d, *J* = 8.6 Hz, 1H), 7.87 (s, 2H), 7.58 (s, 1H), 7.35 (s, 1H), 7.01 (s, 1H), 6.95 (d, *J* = 8.8 Hz, 1H), 4.51 (t, *J* = 6.5 Hz, 2H), 3.18 (t, *J=* 6.4 Hz, 2H).

### Step 7: Synthesis of

### 2-(3,5-dichloro-4-((1-oxo-2-(thiazol-2-yl)-1,2,3,4-tetrahydroisoquinolin-6-yl)oxy)phenyl)-3,5-dioxo-2,3,4,5-tetrah ydro-1,2,4-triazine-6-carboxylic acid 52g

2-(3,5-Dichloro-4-((1-oxo-2-(thiazol-2-yl)-1,2,3,4-tetrahydroisoquinolin-6-yl)oxy)phenyl)-3,5-diox o-2,3,4,5-tetrahydro-1,2,4-triazine-6-carbonitrile **52f** (0.40 g, 0.76 mmol) was dissolved in acetic acid (4 mL), then concentrated hydrochloric acid (2 mL) was added. The mixture was reacted at 100°C for 12 hours. The reaction solution was cooled to room temperature, then water (20 mL) was added. The reaction mixture was stirred for 10 minutes, filtered, and rinsed with water (10 mL × 3). The filter cake was collected and dried to obtain a yellow solid **52g** (0.41 g, yield 100%).

### Step 8: Synthesis of

### 2-(3,5-dichloro-4-((1-oxo-2-(thiazol-2-yl)-1,2,3,4-tetrahydroisoquinolin-6-yl)oxy)phenyl)-1,2,4-triazine-3,5(2H,4 H)-dione 52

2-(3,5-Dichloro-4-((1-oxo-2-(thiazol-2-yl)-1,2,3,4-tetrahydroisoquinolin-6-yl)oxy)phenyl)-3,5-diox o-2,3,4,5-tetrahydro-1,2,4-triazine-6-carboxylic acid **52g** (0.54 g, 0.99 mmol) was dissolved in thioglycolic acid (2 mL). The mixture was reacted at 130°C for 9 hours. The reaction solution was cooled to room temperature, and ethyl acetate (30 mL) was added. The mixture was washed successively with water (10 mL) and saturated sodium chloride solution (10 mL), then dried over anhydrous sodium sulfate and concentrated by suction filtration. The residue was purified by silica gel column chromatography (petroleum ether/ethyl acetate = 3/1), and the obtained solid was recrystallized at 85°C (ethyl acetate/petroleum ether = 1/1, 20 mL) to give a white solid **52** (0.11 g, yield 46%, HPLC purity: 98.09%).
MS (ESI, neg. ion) *m*/*z:* 500.7 [M-H]⁻;
¹H NMR (400 MHz, DMSO-*d₆*) δ (ppm) 12.53 (s, 1H), 8.05 (d, *J =* 8.7 Hz, 1H), 7.89 (s, 2H), 7.74 (s, 1H), 7.58 (d, *J* = 3.5 Hz, 1H), 7.35 (d, *J* = 3.6 Hz, 1H), 7.00 (s, 1H), 6.92 (d, *J* = 8.6 Hz, 1H), 4.51 (t, *J* = 6.6 Hz, 2H), 3.18 (t, *J* = 6.4 Hz, 2H).

### Example 53 2-(5-dichloro-6-((2-(4-fluorobenzyl)-1-oxo-1,2,3,4-tetrahydroisoquinolin-6-yl)oxy)pyridin-3-yl)-1,2,4-triazin e-3,5(2H,4H)-dione 53

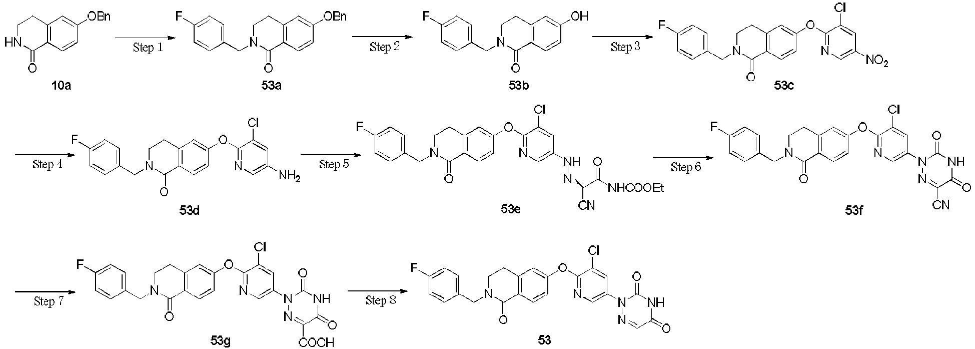

### Step 1: Synthesis of 6-(benzyloxy)-2-(4-fluorobenzyl)-3,4-dihydroisoquinolin-1(2H)-one 53a

To a solution of 6-(benzyloxy)-3,4-dihydroisoquinolin-1(2*H*)-one **10a** (10.0 g, 61.3 mmol) in *N,N*-dimethylformamide (80 mL) was added potassium carbonate (15.0 g, 151 mmol). 4-fluorobenzyl bromide (8.8 mL, 74.0 mmol) was added dropwise to the mixture with stirring, then mixture was reacted at 40°C for 10 minutes. The reaction solution was cooled to room temperature, then water (250 mL) was added to quench the reaction. The reaction mixture was filtered. The filter cake was collected and dried, then slurried with (ethyl acetate/petroleum ether = 1/7, 80 mL) to give a white solid **53a** (10.6 g, yield 68%)

### Step 2: Synthesis of 2-(4-fluorobenzyl)-6-hydroxy-3,4-dihydroisoquinolin-1(2H)-one 53b

6-(Benzyloxy)-2-(4-fluorobenzyl)-3,4-dihydroisoquinolin-1(2*H*)-one **53a** (4.0 g, 11.0 mmol) was dissolved in ethanol (40 mL), and 10% palladium carbon (0.40 g) was added. The reaction mixture was degassed and refilled with hydrogen, and the mixture was hydrogenated (hydrogen balloon) at room temperature for 2 hours. The reaction solution was filtered, and the filtrate was collected and concentrated to obtain a white solid **53b** (2.9 g, yield 97%).

### Step 3: Synthesis of

### 6-((3-chloro-5-nitropyridin-2-yl)oxy)-2-(4-fluorobenzyl)-3,4-dihydroisoquinolin-1(2H)-one 53c

To a solution of 2-(4-fluorobenzyl)-6-hydroxy-3,4-dihydroisoquinolin-1(2*H*)-one **53b** (2.9 g, 11 mmol) and 2,3-dichloro-5-nitropyridine (2.5 g, 13 mmol) in *N,N*-dimethylformamide (30 mL) was added potassium carbonate (1.6 g, 16 mmol), and the mixture was reacted at 80°C for 3 hours. The reaction solution was cooled to room temperature, then water (60 mL) was added to quench the reaction. The reaction mixture was stirred for 10 minutes, filtered, and the filter cake was collected and dried to obtain a gray solid **53c** (4.5 g, yield 98%).

### Step 4: Synthesis of

### 6-((5-amino-3-chloropyridin-2-yl)oxy)-2-(4-fluorobenzyl)-3,4-dihydroisoquinolin-1(2H)-one 53d

To a solution of 6-((3-chloro-5-nitropyridin-2-yl)oxy)-2-(4-fluorobenzyl)-3,4-dihydroisoquinolin-1(2*H*)-one **53c** (4.5 g, 11 mmol) in acetic acid (40 mL) was added iron powder (1.5 g, 27 mmol), and the mixture was reacted at 60°C for 5 hours. The reaction solution was cooled to room temperature, then water (100 mL) was added to quench the reaction. The mixture was extracted with ethyl acetate (120 mL × 2). The combined organic layers were washed with saturated sodium chloride (20 mL × 3), dried over anhydrous sodium sulfate, and concentrated by suction filtration to give black **oil 53d** (4.0 g, yield 96%).

MS (ESI, pos. ion) *m*/*z*: 431.0 [M+H]⁺.

### Step 5: Synthesis of ethyl

### (2-(2-(5-chloro-6-((2-(4-fluorobenzyl)-1-oxo-1,2,3,4-tetrahydroisoquinolin-6-yl)oxy)pyridin-3-yl)hydrazono)-2-c yanoacetyl)carbamate 53e

To a solution of 6-((5-amino-3-chloropyridin-2-yl)oxy)-2-(4-fluorobenzyl)-3,4-dihydroisoquinolin-1(2*H*)-one **53d** (1.50 g, 3.77 mmol) in acetic acid (25 mL) was added a solution of sodium nitrite (0.52 g, 7.54 mmol) in water (10 mL) at 0°C, then *N*-cyanoacetylurethane (0.88 g, 5.66 mmol) was added. The mixture was reacted for 2 hours. Water (50 mL) was added to quench the reaction. The mixture was stirred for 20 minutes, filtered, and the filter cake was washed with water (10 mL). Then the filter cake was collected and dried to obtain a light yellow solid **53e** (2.00 g, yield 96%).

### Step 6: Synthesis of

### 2-(5-chloro-6-((2-(4-fluorobenzyl)-1-oxo-1,2,3,4-tetrahydroisoquinolin-6-yl)oxy)pyridin-3-yl)-3,5-dioxo-2,3,4,5-t etrahydro-1,2,4-triazine-6-carbonitrile 53f

To a solution of ethyl (2-(2-(5-chloro-6-((2-(4-fluorobenzyl)-1-oxo-1,2,3,4-tetrahydroisoquinolin-6-yl)oxy)pyridin-3-yl)hydrazono)-2-c yanoacetyl)carbamate **53e** (2.00 g, 3.62 mmol) in *N,N*-dimethylformamide (25 mL) was added sodium acetate (0.36 g, 4.36 mmol), and the mixture was reacted at 120°C for 6 hours. The reaction solution was cooled to room temperature, and water (20 mL) was added to quench the reaction. The mixture was extracted with ethyl acetate (120 mL × 2). The combined organic layers were washed with saturated sodium chloride solution (20 mL × 3), dried over anhydrous sodium sulfate, filtered and concentrated. The resulting residue was recrystallized at 80°C (ethanol/ethyl acetate/petroleum ether=1/5/8, 35 mL) to obtain a white solid **53f** (0.90 g, yield 48%, HPLC purity: 98.11%).
MS (ESI, neg. ion) *m*/*z*: 517.9 [M-H]⁻;
¹H NMR (400 MHz, DMSO-*d₆*) δ (ppm) 13.29 (s, 1H), 8.25 (s, 2H), 8.00 (d, *J* = 8.4 Hz, 1H), 7.38 (dd, *J* = 8.4, 5.5 Hz, 2H), 7.30-6.95 (m, 4H), 4.70 (s, 2H), 3.51 (t, *J* = 6.6 Hz, 2H), 2.98 (t, *J* = 6.6 Hz, 2H).

### Step 7: Synthesis of

### 2-(5-chloro-6-((2-(4-fluorobenzyl)-1-oxo-1,2,3,4-tetrahydroisoquinolin-6-yl)oxy)pyridin-3-yl)-3,5-dioxo-2,3,4,5-etrahydro-1,2,4-triazine-6-carboxylic acid 53g

2-(5-chloro-6-((2-(4-fluorobenzyl)-1-oxo-1,2,3,4-tetrahydroisoquinolin-6-yl)oxy)pyridin-3-yl)-3,5-dioxo-2,3,4,5-tetrahydro-1,2,4-triazine-6-carbonitrile **53f** (0.90 g, 1.73 mmol) was dissolved in acetic acid (16 mL), then concentrated hydrochloric acid (4 mL) was added. The mixture was reacted at 120°C for 12 hours. The reaction solution was cooled to room temperature, then water (45 mL) was added. The reaction mixture was stirred for 10 minutes, filtered, and rinsed with water (10 mL × 2). The filter cake was collected and dried to obtain a yellow solid **53g** (0.80 g, yield 86%).

### Step 8: Synthesis of

### 2-(5-dichloro-6-((2-(4-fluorobenzyl)-1-oxo-1,2,3,4-tetrahydroisoquinolin-6-yl)oxy)pyridin-3-yl)-1,2,4-triazine-3,5 (2H,4H)-dione 53

2-(5-Chloro-6-((2-(4-fluorobenzyl)-1-oxo-1,2,3,4-tetrahydroisoquinolin-6-yl)oxy)pyridin-3-yl)-3,5 -dioxo-2,3,4,5-tetrahydro-1,2,4-triazine-6-carboxylic acid **53g** (0.80 g, 1.49 mmol) was dissolved in acetic acid (6 mL), then thiourea (0.57 g, 7.45 mmol) was added. The mixture was reacted at 120°C for 24 hours. The reaction solution was cooled to room temperature, and water (25 mL) was added to quench the reaction. The mixture was extracted with ethyl acetate (40 mL × 2). The combined organic layers were washed successively with water (15 mL × 3) and saturated sodium chloride solution (20 mL × 3), dried over anhydrous sodium sulfate and concentrated by suction filtration. The residue was purified by silica gel column chromatography (petroleum ether/ethyl acetate = 1/1) to give a white solid **53** (0.42 g, yield 57%, HPLC purity: 99.39%).
MS (ESI, pos. ion) *m*/*z*: 494.0 [M+H]⁺;
¹H NMR (400 MHz, DMSO-*d₆*) δ (ppm) 12.49 (s, 1H), 8.28 (q, *J* = 2.4 Hz, 2H), 7.99 (d, *J* = 8.4 Hz, 1H), 7.70 (s, 1H), 7.38 (dd, *J* = 8.4, 5.5 Hz, 2H), 7.18 (ddd, *J* = 9.2, 6.8, 2.6 Hz, 4H), 4.70 (s, 2H), 3.51 (t, *J* = 6.6 Hz, 2H), 2.98 (t, *J* = 6.6 Hz, 2H).

### Example of activity test

### 1. Detection of the agonistic activity of the compound of the present invention to TRα or TRβ in the dual luciferase reporter gene experiment

Test materials:
HEK293 cells, purchased from ATCC, Cat No. CRL-1573;
Fugene HD transfection reaagent, purchased from Promega, Cat No. E231A;
DMEM, purchased from Gibco, Cat No. 11995;
FBS, purchased from Biosera, Cat No. FB-1280/500;
0.25% Trypsin-EDTA, purchased from Gibco, Cat No. 25200-072;
Dual-Luciferase Reporter Assay System, purchased from Promega, Cat No. E1960;
96-well plate (round bottom), purchased from Corning, Cat No. 3365.

### Test method:

HEK293 cells were cultured in a full medium of 10% FBS+DMEM. pBind-TRα or pBind-TRβ (100 ng/µl), pG5Luc (100 ng/µl), FuGENE HD and Opti-MEM were mixed thoroughly and incubated at room temperature for 15 min, meanwhile HEK293 cells were digested with 0.25% Trypsin-EDTA and resuspended with full medium. The cell density was calculated and adjusted to 500,000 cells/ml, and then the transcription mixture was added to mix with the cell suspension, plated in a 96-well plate (100 µL/well), and incubated at 37°C for 24 h. After 24 h, the test compound was dissolved in DMSO and diluted 3 times to a total of 10 concentrations, and then the compound was diluted with DMEM to a solution containing 10% DMSO. 5 µL of the compound was placed in a 96-well plate. The final concentration of DMSO was 0.5%, and the compound was co-cultured with the cells for 18 h. After 18 h, the fluorescence signals of firefly and renilla were detected by Dual-Luciferase Reporter Assay System. The firefly fluorescence signal (F) was divided by the renilla fluorescence signal (R) to calculate the F/R ratio, and the Graph Pad Prism software was used to draw the curve and calculate the EC₅₀ value.

The test results show that the compound of the present invention has obvious agonistic activity and selectivity to TPβ.

### 2. Detection of the binding activity of the compound of the present invention to TRα or TRβ in vitro

Test materials
LanthaScreen TR-FRET Thyroid Receptor beta Coactivator Assay kit was purchased from Invitrogen, Cat. No. PV4686;
LanthaScreen TR-FRET Thyroid Receptor alfa Coactivator Assay kit was purchased from Invitrogen, Cat. No. PV4687.

Test method:
The method was experimented with LanthaScreen TR-FRET Thyroid Receptor beta/alfa Coactivator Assay kit. The test compound was dissolved in DMSO and diluted 3 times to a total of 10 concentrations, and then diluted with TR-FRET Coregulator Buffer C in the kit to form a solution containing 2% DMSO. 10 µL of the solution containing 2% DMSO was taken into a 384-well plate, then 5 µL of 4 × TR beta/alfa-LBD, 5 µL of a mixture containing 0.4 µM fluorescein-SRC2-2 and 8 nM Tb anti-GST antibody were added into each well, mixed thoroughly, and incubated at room temperature for 1 h in the dark. After 1 h, BMG LABTECH's PHERAstar FSX microplate reader was used to read the fluorescence value (RFU) at excitation 520 nm and emission 495 nm. The TR-FRET ratio was calculated by dividing the emission signal at 520 nm by the emission signal at 495 nm. Graph Pad Prism 5 software was used to draw the curve and calculate the EC₅₀ value. The *in vitro* binding activity test results of some of the compounds of the examples of the present invention are shown in Table 1 below.

**Table 1 The in vitro binding activity test results of some of the compounds of the examples of the present invention**

| **Example No.** | **EC₅₀ (µM)** | | **Example No.** | **EC₅₀ (µM)** | |
|---|---|---|---|---|---|
| | **TRα** | **TRβ** | | **TRα** | **TRβ** |
| 1 | > 100 | 0.26 | 31d | >100 | 0.034 |
| 2 | 1.47 | 0.10 | 31 | 0.107 | <0.0046 |
| 3 | 0.29 | 0.026 | 32d | >100 | 0.054 |
| 4 | 0.21 | 0.018 | 32 | 0.181 | 0.0046 |
| 5 | 0.074 | 0.014 | 33d | >100 | 0.11 |
| 7 | 0.95 | 0.092 | 33 | 0.156 | 0.017 |
| 8 | > 100 | >10 | 34 | >100 | 0.021 |
| 9 | > 100 | >10 | 35d | >100 | 0.080 |
| 10 | 0.206 | 0.049 | 35 | 0.092 | <0.0046 |
| 12 | 0.451 | 0.021 | 36 | 0.079 | <0.0046 |
| 13 | 0.073 | 0.013 | 37 | >100 | 0.043 |
| 14 | 0.226 | 0.030 | 38 | <0.046 | <0.0046 |
| 15 | 0.176 | 0.024 | 39 | >100 | 0.472 |
| 16 | 0.211 | 0.022 | 40d | >100 | 0.035 |
| 17 | 0.311 | 0.034 | 40 | 0.179 | <0.0046 |
| 18 | >100 | 0.192 | 42 | >100 | 0.016 |
| 19 | >100 | 0.501 | 43 | >100 | 0.031 |
| 20d | >100 | 0.534 | 44 | >100 | 0.0097 |
| 20 | >100 | 0.012 | 45 | >100 | 0.061 |
| 21d | >100 | 0.368 | 46 | >100 | 0.035 |
| 21 | >100 | 0.019 | 47f | >100 | 0.214 |
| 22 | 0.156 | 0.011 | 47 | 0.161 | 0.039 |
| 23 | 0.386 | 0.013 | 48 | 0.558 | 0.065 |
| 24 | >100 | 0.075 | 49f | >100 | 0.769 |
| 25 | >100 | 0.241 | 49 | >100 | 0.107 |
| 26 | 7.037 | 0.476 | 50f | 2.620 | 0.213 |
| 27 | >100 | 0.14 | 50 | 0.274 | 0.12 |
| 28g | >100 | 0.128 | 51 | >100 | 0.904 |
| 28 | >100 | 0.017 | 52 | 0.23 | 0.016 |
| 29 | >100 | 0.091 | | | |
| 30d | >100 | 0.065 | | | |
| 30 | 0.093 | 0.0062 | | | |

The test results show that the compound of the present invention has strong binding affinity and selectivity to TRβ.

### 3. The pharmacokinetic determination of the compound of the present invention

Test purposes: The following method was used to determine the pharmacokinetics of the compound of the present invention.

Test materials:
Experimental reagents and test samples used: Propranolol (internal standard), methanol, ammonium acetate, K₂EDTA (potassium ethylenediaminetetraacetic acid), formic acid, acetonitrile, MTBE (methyl tert-butyl ether), KolliphorHS15 (macrogol 12 hydroxystearate), DMSO (dimethyl sulfoxide) are commercially available;
SD rats: male, 180-220 g, 7-8 weeks old, purchased from Hunan Slack Experimental Animal Co., Ltd.

Test method:
1. Preparation of the test sample
Each test sample prepared was completely dissolved in a mixture of 5% DMSO + 5% KolliphorHS 15 + 90% Saline according solubility property thereof.
2. Design of animal experiment

| | |
|---|---|
| Test substance | Example compounds of the present invention |
| Animal grouping | Intravenous injection/i.v.: n = 3; blood collection time (hours/h): 0.083, 0.25, 0.5, 1, 2, 5, 7, 24 |
| | Oral gavage/i.g.: n = 3; blood collection time (hour/h): 0.083, 0.25, 0.5, 1, 2, 5, 7, 24 |
| Drug-delivery way | Intravenous: intravenous administration of hind limbs; Oral: intragastric administration. |
| Blood collection method | Tail vein blood sampling |
| Blood volume | 200 ~ 400 µL/time point |
| Anticoagulant | K₂EDTA |
| Plasma preparation | All samples were centrifuged at 10,000 rpm, 4°C for 2 min within 60 min to separate the plasma. Samples were stored at -80°C until assayed. Backup samples are stored for 1 month after analysis. |
| Fasting situation | Fasted for 15 h before administration, free access to water. Eat 4 h after administration. |
| Stock solution | Test substance: 20% DMSO; Internal standard: Propranolol aqueous solution (100 ng/mL) |
| Data processing | Pharmacokinetic parameters were calculated using a noncompartmental method by WinNonLin 6.1 software. |

3. Animal dosage form

| Group | Gender | Number | Dosage | Dosing concentration | Dosing volume |
|---|---|---|---|---|---|
| Intravenous injection i.v. | Male | 3 | 1 mg/kg | 1 mg/mL | 1 mL/kg |
| Gavage i.g. | Male | 3 | 5 mg/kg | 1 mg/mL | 5 mL/kg |

4. Solution preparation
(1) Configuration of the stock solution of the test sample: an appropriate amount of the test product was accurately weighed, dissolved in DMSO, diluted to 1 mg/mL with acetonitrile, and shaken well to obtain the stock solution of the test sample, which was stored at -20°C for use.
(2) Preparation of internal standard solution: a certain amount of 1 mg/mL Propranolol stock solution was precisely pipetted and diluted to 100 ng/mL with water.

5. Sample analysis
Samples were processed by liquid-liquid extraction, chromatographically separated, quantitatively analyzed by multiple reaction ion monitoring (MRM) on a triple quadrupole tandem mass spectrometer, and the concentration of the results was calculated by instrument quantitative software.
6. Plasma sample pretreatment
30 µL of plasma sample was precisely pipetted, added with 250 µL of internal standard, and vortexed to mix thoroughly. Plasma samples was extracted once with 1 mL of MTBE, centrifuged at 13,000 rpm for 2 min at 4°C. 800 µL of the supernatant was sucked out, evaporated to dryness in a 96-well nitrogen blower, and the residue was reconstituted with 150 µL of methanol/water (v/v = 50/50), vortexed to mix, and injected with an injection volume of 8 µL.
7. Preparation of standard sample
An appropriate amount of compound stock solution was precisely pipetted, diluted with acetonitrile to make a series of standard solutions. 20 µL of each of the above series of standard solutions was precisely pipetted, added with 180 µL of blank plasma, vortexed and mixed thoroughly, and prepare plasma samples equivalent to plasma concentrations of 3, 5, 10, 30, 100, 300, 1,000, 3,000, 5,000, and 10,000 ng/mL. The plasma samples were all operated according to the "plasma sample pretreatment", and double-sample analysis was performed for each concentration to establish a standard curve.
8. Analysis method
LC/MS/MS method was used to determine the content of the test compound in rat plasma after administration of different compounds.
9. Data processing

Pharmacokinetic parameters were calculated using a noncompartmental method by WinNonLin 6.1 software.

The pharmacokinetic test results of some of the compounds of the examples of the present invention are shown in Table 2 below.

**Table 2 The pharmacokinetic test results of some of the compounds of the examples of the present invention**

| Example No. | Route | Dosage (mg/kg) | Cmax (ng/ml) | AUCₗₐₛₜ (h*ng/ml) | AUC_{INF} (h*ng/ml) | T_{1/2} (h) | Cl (ml/min/ kg) | Vss (L/kg) | F (%) |
|---|---|---|---|---|---|---|---|---|---|
| 20 | iv | 1 | 3990 | 16300 | 16400 | 3.16 | 1.01 | 0.251 | - |
| | ig | 5 | 10400 | 93700 | 94400 | 3.57 | -- | -- | 115.1 |
| 21 | iv | 1 | 2760 | 1020 | 1020 | 1.02 | 16.3 | 0.308 | -- |
| | ig | 5 | 4730 | 4160 | 4180 | 1.44 | - | -- | 81.9 |
| 24 | iv | 1 | 4020 | 3720 | 3790 | 1.16 | 4.39 | 0.337 | -- |
| | ig | 5 | 7790 | 21900 | 223000 | 2.65 | - | - | 117.7 |
| 28 | iv | 1 | 8400 | 8730 | 9640 | 2.91 | 1.73 | 0.246 | -- |
| | ig | 5 | 15500 | 47000 | 47000 | 2.29 | - | -- | 97.6 |
| 29 | iv | 1 | 4270 | 48700 | 56000 | 8.47 | 0.297 | 0.194 | -- |
| | ig | 5 | 10300 | 159000 | 236000 | 14.1 | - | -- | 66.2 |
| 34 | iv | 1 | 3020 | 18800 | 22400 | 9.87 | 0.744 | 0.531 | -- |
| | ig | 5 | 8460 | 110000 | 124000 | 7.67 | -- | -- | 111 |
| 37 | iv | 1 | 4200 | 20900 | 21100 | 3.61 | 0.789 | 0.212 | - |
| | ig | 5 | 12300 | 121000 | 122000 | 3.5 | - | -- | 116.3 |
| 42 | iv | 1 | 4340 | 1860 | 1960 | 2.97 | 8.49 | 0.658 | -- |
| | ig | 5 | 7700 | 12700 | 13200 | 2.53 | - | -- | 134.8 |
| 43 | iv | 1 | 3410 | 39800 | 47700 | 9.52 | 0.349 | 0.26 | -- |
| | ig | 5 | 8120 | 134000 | 212000 | 15.7 | - | - | 68.4 |
| 44 | iv | 1 | 5430 | 2110 | 2150 | 2.11 | 7.74 | 0.4 | -- |
| | ig | 5 | 9030 | 13900 | 14300 | 1.64 | - | -- | 133.3 |
| 45 | iv | 1 | 1780 | 2460 | 2510 | 1.15 | 6.65 | 0.57 | -- |
| | ig | 5 | 5320 | 20300 | 20600 | 4.14 | -- | - | 163.9 |
| 46 | iv | 1 | 4160 | 9810 | 10000 | 4.24 | 1.66 | 0.424 | -- |
| | ig | 5 | 6410 | 61900 | 633000 | 5.93 | - | -- | 126.5 |
| 49 | iv | 1 | 5300 | 3580 | 3730 | 1.14 | 4.46 | 0.306 | -- |
| | ig | 5 | 9680 | 25300 | 25800 | 3.63 | - | -- | 138.3 |

The test results show that the compounds of the examples of the present invention exhibit excellent pharmacokinetic properties when administered intravenously or orally orally.

### 4. The pharmacodynamic evaluation of the compound of the present invention

Test materials:
Western diet: purchased from Research diet, item number: D12079B;
MCD diet: purchased from Nantong Trofe Feed Technology Co., Ltd., item number: TP3006R;
ALT, AST, ALP, TG, CHO, HDL, LDL and GLU: purchased from Roche, item numbers were: 20764957322, 20764949322, 03333701190, 20767107322, 03039773190, 04399803190, 03038866322 and 0440448319, respectively.

8-Week-old male OB/OB mice: purchased from Jiangsu Jicui Yaokang Biotechnology Co., Ltd.; 8-week-old male db/db mice: purchased from Jiangsu Jicui Yaokang Biotechnology Co., Ltd.

### A. Pharmacodynamic evaluation of compound in Western diet-induced non-alcoholic steatohepatitis (NASH) model of OB/OB mouse

OB/OB mice are leptin gene-deficient mice, and the NASH model of OB/OB mice induced by Western diet is a commonly used NASH *in vivo* drug efficacy evaluation model. Animals were acclimated to the experiment for 1 week. OB/OB mice were fed with Western diet feed, and the feed was changed three times a week (Monday, Wednesday, Friday). The mice were given drugs at the fifth week after feeding, and administered orally once a day for 6 weeks. The entire experimental period was 10 weeks. During the experiment, the basic situation of the animals was monitored every day, and the body weight of the mice was recorded once a week. After the experiment, the mice were fasted overnight, and the mice were anesthetized, and the whole blood was collected from the orbit, centrifuged at 4,000 rpm for 10 min at 4°C to obtain serum, which was stored at -80°C. Serum was used for detection of ALT, AST, ALP, TG, CHO, HDL, LDL and GLU. Mice were dissected and livers were removed and weighed. The middle lobe of the liver was stored in an EP tube at -80°C for the determination of the contents of TG and CHO in the liver. The left lobe of the liver was fixed in 10% formalin, stained with HE, and scored with NAS.

### B. Pharmacodynamic evaluation of compound in MCD diet-induced non-alcoholic steatohepatitis (NASH) model of db/db mouse

db/db mice are leptin gene-deficient mice, and the NASH model of db/db mice induced by MCD diet is a commonly used NASH *in vivo* drug efficacy evaluation model. Animals were acclimated to the experiment for 1 week. db/db mice were fed with MCD diet feed, and the feed was changed three times a week (Monday, Wednesday, Friday). The mice were experimented with drugs while building a model, and administered orally once a day for 8 weeks. The entire experimental period was 8 weeks. During the experiment, the basic situation of the animals was monitored every day, and the body weight of the mice was recorded once a week. After the experiment, the mice were fasted overnight, and the mice were anesthetized, and the whole blood was collected from the orbit, centrifuged at 4,000 rpm for 10 min at 4°C to obtain serum, which was stored at -80°C. Serum was used for detection of ALT, AST, ALP, TG, CHO, HDL, LDL and GLU. Mice were dissected and livers were removed and weighed. The middle lobe of the liver was stored in an EP tube at -80°C for the determination of the contents of TG and CHO in the liver. The left lobe of the liver was fixed in 10% formalin, stained with HE, and scored with NAS.

The test results show that the compound of the invention can effectively reduce fat accumulation in the liver, relieve inflammation, and improve liver fibrosis.

Reference throughout this specification to "an embodiment", "some embodiments", "one embodiment", "another example", "an example", "a specific example", or "some examples" means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", "in an example", "in a specific example", or "in some examples" in various places throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A compound having Formula (I) or a stereoisomer, a geometric isomer, a tautomer, an N-oxide, a solvate, a metabolite, a pharmaceutically acceptable salt or a prodrug thereof, 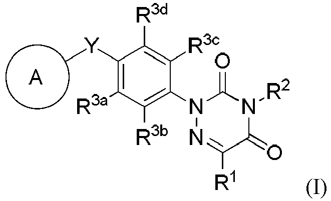 wherein,
Y is -O-, -S-, -NR⁰-, -C(=O)-, C₁₋₆ alkylene, C₂₋₆ alkenylene, C₂₋₆ alkynylene, -NR°C(=O)- or -C(=O)NR⁰-; wherein the Y is optionally substituted with 1, 2 or 3 R^{x};
R⁰ is H, deuterium, C₁₋₆ alkyl, C₁₋₆ haloalkyl, hydroxy C₁₋₆ alkyl, amino C₁₋₆ alkyl or cyano C₁₋₆ alkyl;
each of R^{3a}, R^{3b}, R^{3c} and R^{3d} is independently H, deuterium, F, Cl, Br, I, -CN, -NO₂, -COOH, -OH, -NH₂, -SH, C₁₋₆ alkyl, C₁₋₆ alkoxy, C₁₋₆ alkylthio, C₁₋₆ alkylamino, C₁₋₆ haloalkyl, C₁₋₆ haloalkoxy, hydroxy C₁₋₆ alkyl, amino C₁₋₆ alkyl or cyano C₁₋₆ alkyl;
R¹ is H, deuterium, F, Cl, Br, I, -CN, -NO₂, -COOH, -OH, -NH₂, -SH, C₁₋₆ alkyl, C₂₋₆ alkenyl, C₂₋₆ alkynyl, -C(=O)-C₁₋₆ alkoxy, -C(=O)-C₁₋₆ alkyl, -C(=O)-C₁₋₆ alkylamino, -C(=O)NH₂, -S (=O)₂-C₁₋₆ alkyl, -S(=O)₂-C₁₋₆ alkylamino, -S(=O)₂NH₂, C₁₋₆ alkylamino, C₁₋₆ alkoxy, C₁₋₆ haloalkyl, C₁₋₆ haloalkoxy, hydroxy C₁₋₆ alkyl, amino C₁₋₆ alkyl, carboxy C₁₋₆ alkyl or cyano C₁₋₆ alkyl;
R² is H, deuterium, C₁₋₆ alkyl, C₂₋₆ alkenyl, C₂₋₆ alkynyl, C₃₋₆ cycloalkyl, heterocyclyl consisting of 5-6 atoms, C₆₋₁₀ aryl or heteroaryl consisting of 5-6 atoms;
ring A is 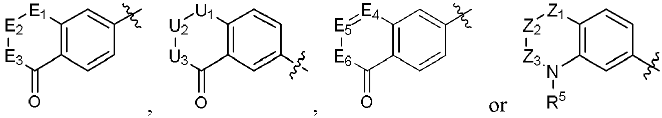 ; wherein, ring A is optionally substituted with 1, 2 or 3 R^{y};
each of E₁, U₁ and Z₁ is independently -(CR^{4a}R^{4b})_{q}-, -C(=O)-, -O-, -S-, -S(=O)-, -S(=O)₂- or -NR^{a}-;
each of E₂, U₂ and Z₂ is independently -CR^{4c}R^{4d}-, -C(=O)-, -O-, -S-, -S(=O)-, -S(=O)₂- or -NR^{b}-;
each of E₃, E₆, U₃ and Z₃ is independently -CR^{4e}R^{4f}-, -C(=O)-, -O-, -S-, -S(=O)-, -S(=O)₂- or -NR^{c}-;
E₄ is -CR^{4g}= or -N=; E₅ is -CR^{4h}= or -N=;
q is 0, 1, 2 or 3;
each R^{a}, R^{b}, R^{c} and R⁵ is independently H, deuterium, C₁₋₆ alkyl, C₂₋₆ alkenyl, C₂₋₆ alkynyl, C₁₋₆ haloalkyl, C₃₋₆ cycloalkyl, heterocyclyl consisting of 5-6 atoms, C₆₋₁₀ aryl or heteroaryl consisting of 5-6 atoms, wherein each R^{a}, R^{b}, R^{c} and R⁵ is independently and optionally substituted with 1, 2 or 3 R^{y1};
each R^{4a}, R^{4b}, R^{4c}, R^{4d}, R^{4e}, R^{4f}, R^{4g} and R^{4h} is independently H, deuterium, F, Cl, Br, I, -CN, -NO₂, -COOH, -OH, -NH₂, -SH, C₁₋₆ alkyl, C₂₋₆ alkenyl, C₂₋₆ alkynyl, C₁₋₆ alkoxy, C₁₋₆ alkylamino, C₁₋₆ haloalkyl, C₁₋₆ haloalkoxy, C₃₋₆ cycloalkyl, C₃₋₆ cycloalkyl-C₁₋₄ alkylene, heterocyclyl consisting of 5-6 atoms, (heterocyclyl consisting of 5-6 atoms)-C₁₋₄ alkylene, C₆₋₁₀ aryl, C₆₋₁₀ aryl-C₁₋₄ alkylene, heteroaryl consisting of 5-6 atoms or (heteroaryl consisting of 5-6 atoms)-C₁₋₄ alkylene, wherein each R^{4a}, R^{4b}, R^{4c}, R^{4d}, R^{4e}, R^{4f}, R^{4g} and R^{4h} is independently and optionally substituted with 1, 2 or 3 R^{y2};
or R^{4a} and R^{4b}, together with the carbon atoms to which they are attached, form a C₃₋₈ carbon ring or a heterocyclyl consisting of 5-6 atoms, or R^{4c} and R^{4d}, together with the carbon atoms to which they are attached, form a C₃₋₈ carbon ring or a heterocyclyl consisting of 5-6 atoms, or R^{4e} and R^{4f}, together with the carbon atoms to which they are attached, form a C₃₋₈ carbon ring or a heterocyclyl consisting of 5-6 atoms, wherein each C₃₋₈ carbon ring and heterocyclyl consisting of 5-6 atoms is independently unsubstituted or substituted with 1, 2 or 3 R^{y3};
each R^{x} is independently deuterium, F, Cl, Br, I, -CN, -OH, -NH₂, C₁₋₆ alkyl, C₁₋₆ haloalkyl, C₁₋₆ haloalkoxy, C₁₋₆ alkoxy or C₁₋₆ alkylamino;
each R^{y} is independently deuterium, F, Cl, Br, I, -CN, -OH, -NH₂, C₁₋₆ alkyl, C₁₋₆ haloalkyl, C₁₋₆ haloalkoxy, C₁₋₆ alkoxy or C₁₋₆ alkylamino; or two R^{y} linked on adjacent atoms, together with the atoms to which they are attached, form a C₃₋₈ carbon ring or a heterocyclyl consisting of 5-6 atoms, wherein each C₃₋₈ carbon ring and heterocyclyl consisting of 5-6 atoms is independently unsubstituted or substituted with 1, 2 or 3 R^{y4};
each R^{y1} is independently deuterium, F, Cl, Br, I, -CN, -OH, -NH₂, -SH, oxo, -OC(=O)-C₁₋₆ alkyl, -C(=O)-C₁₋₆ alkoxy, -C(=O)-C₁₋₆ alkyl, -C(=O)-C₁₋₆ alkylamino, -C(=O)NH₂, -S(=O)₂-C₁₋₆ alkyl, -S(=O)₂-C₁₋₆ alkylamino, -S(=O)₂NH₂, C₁₋₆ alkyl, C₁₋₆ haloalkyl, C₁₋₆ haloalkoxy, C₁₋₆ alkoxy, C₁₋₆ alkylthio, C₁₋₆ alkylamino, C₃₋₆ cycloalkyl, heterocyclyl consisting of 5-6 atoms, C₆₋₁₀ aryl or heteroaryl consisting of 5-6 atoms, wherein each R^{y1} is independently and optionally substituted with 1, 2 or 3 R^{z};
each R^{z}, R^{y2}, R^{y3} and R^{y4} is independently deuterium, F, Cl, Br, I, -CN, -OH, -NH₂, -COOH, C₁₋₆ alkyl, C₁₋₆ haloalkyl, C₁₋₆ haloalkoxy, C₁₋₆ alkoxy, C₁₋₆ alkylthio or C₁₋₆ alkylamino.

2. The compound of claim 1, wherein each of R^{3a}, R^{3b}, R^{3c} and R^{3d} is independently H, deuterium, F, Cl, Br, I, -CN, -NO₂, -COOH, -OH, -NH₂, -SH, methyl, ethyl, n-propyl, isopropyl, methoxy, ethoxy, methylthio, methylamino, -CF₃, -CHF₂, -CH₂F, -CH₂CF₃, -CH₂CHF₂, -OCF₃, -OCHF₂, -OCH₂F, hydroxymethyl, aminomethyl or cyanomethyl.

3. The compound of claim 1 or 2, wherein the R¹ is H, deuterium, F, Cl, Br, I, -CN, -NO₂, -COOH, -OH, -NH₂, -SH, methyl, ethyl, n-propyl, isopropyl, -CH=CH₂, -CH₂CH=CH₂, -CH=CHCH₃, -C≡CH, -C(=O)-OCH₃, -C(=O)-OCH₂CH₃, -C(=O)-OCH(CH₃)₂, -C(=O)-OCH₂CH₂CH₃, -C(=O)-O(CH₂)₃CH₃, -C(=O)-OCH₂CH(CH₃)₂, -C(=O)-CH₃, -C(=O)-CH₂CH₃, -C(=O)-NHCH₃, -C(=O)-N(CH₃)₂, -C(=O)NH₂, -S(=O)₂-CH₃, -S(=O)₂-CH₂CH₃, -S(=O)₂-NHCH₃, -S(=O)₂NH₂, methylamino, ethylamino, methoxy, ethoxy, -CF₃, -CHF₂, -CH₂F, -CH₂CF₃, -CH₂CHF₂, -OCF₃, -OCHF₂, -OCH₂F, hydroxymethyl, aminomethyl, carboxymethyl or cyanomethyl.

4. The compound of any one of claims 1-3, wherein each R^{a}, R^{b}, R^{c} and R⁵ is independently H, deuterium, C₁₋₄ alkyl, C₂₋₄ alkenyl, C₂₋₄ alkynyl, C₁₋₄ haloalkyl, C₃₋₆ cycloalkyl, heterocyclyl consisting of 5-6 atoms, C₆₋₁₀ aryl or heteroaryl consisting of 5-6 atoms, wherein each R^{a}, R^{b}, R^{c} and R⁵ is independently and optionally substituted with 1, 2 or 3 R^{y1}.

5. The compound of any one of claims 1-4, wherein each R^{a}, R^{b}, R^{c} and R⁵ is independently H, deuterium, methyl, ethyl, *n*-propyl, isopropyl, *n*-butyl, *tert*-butyl, -CH=CH₂, -CH₂CH=CH₂, -CH=CHCH₃, -C≡CH, -CF₃, -CHF₂, -CH₂F, -CH₂CF₃, -CH₂CHF₂, cyclopropyl, cyclobutyl, cyclopentyl, cyclohexyl, pyrrolidinyl, pyrazolidinyl, tetrahydrofuranyl, tetrahydrothiophenyl, tetrahydropyranyl, piperidinyl, morpholinyl, thiomorpholinyl, piperazinyl, phenyl, furanyl, thienyl, imidazolyl, pyrimidinyl, pyridyl, pyrrolyl, pyrazinyl, thiazolyl or oxazolyl, wherein each R^{a}, R^{b}, R^{c} and R⁵ is independently and optionally substituted with 1, 2 or 3 R^{y1}.

6. The compound of any one of claims 1-5, wherein each R^{y1} is independently deuterium, F, Cl, Br, I, -CN, -OH, -NH₂, -SH, oxo, -OC(=O)-C₁₋₄ alkyl, -C(=O)-C₁₋₄ alkoxy, -C(=O)-C₁₋₄ alkyl, -C(=O)-C₁₋₄ alkylamino, -C(=O)NH₂, -S(=O)₂-C₁₋₄ alkyl, -S(=O)₂-C₁₋₄ alkylamino, -S(=O)₂NH₂, C₁₋₄ alkyl, C₁₋₄ haloalkyl, C₁₋₄ haloalkoxy, C₁₋₄ alkoxy, C₁₋₄ alkylthio, C₁₋₄ alkylamino, C₃₋₆ cycloalkyl, heterocyclyl consisting of 5-6 atoms, C₆₋₁₀ aryl or heteroaryl consisting of 5-6 atoms, wherein each R^{y1} is independently and optionally substituted with 1, 2 or 3 R^{z}.

7. The compound of any one of claims 1-6, wherein each R^{y1} is independently deuterium, F, Cl, Br, I, -CN, -OH, -NH₂, -SH, oxo, -OC(=O)-methyl, -OC(=O)-ethyl, -OC(=O)-n-propyl, -OC(=O)-isopropyl, -OC(=O)-*n*-butyl, -OC(=O)-*tert*-butyl, -OC(=O)-isobutyl, -C(=O)O-methyl, -C(=O)O-ethyl, -C(=O)O-*n*-propyl, -C(=O)O-isopropyl, -C(=O)O-*n*-butyl, -C(=O)O-*tert*-butyl, -C(=O)O-isobutyl, -C(=O)-methyl, -C(=O)-ethyl, -C(=O)-*n*-propyl, -C(=O)-isopropyl, -C (=O)-*n*-butyl, -C(=O)-*tert*-butyl, -C(=O)-isobutyl, -C(=O)-methylamino, -C(=O)-ethylamino, -C(=O)NH₂, -S(=O)₂-C₁₋₃ alkyl, -S(=O)₂-C₁₋₃ alkylamino, -S(=O)₂NH₂, methyl, ethyl, *n*-propyl, isopropyl, *tert*-butyl, -CF₃, -CHF₂, -CH₂F, -CH₂CF₃, -CH₂CHF₂, -OCF₃, -OCHF₂, -OCH₂F, -OCH₂CF₃, -OCH₂CHF₂, -OCHFCH₃, methoxy, ethoxy, *n*-propoxy, isopropoxy, methylthio, ethylthio, methylamino, ethylamino, cyclopropyl, cyclobutyl, cyclopentyl, cyclohexyl, pyrrolidinyl, pyrazolidinyl, tetrahydrofuryl, tetrahydrothiophenyl, tetrahydropyranyl, piperidinyl, morpholinyl, thiomorpholinyl, piperazinyl, phenyl, furyl, thienyl, imidazolyl, pyrimidinyl, pyridinyl, pyrrolyl, pyridazinyl, pyrazinyl, thiazolyl or oxazolyl, wherein each R^{y1} is independently and optionally substituted with 1, 2 or 3 R^{z}.

8. The compound of any one of claims 1-7, wherein each R^{4a}, R^{4b}, R^{4c}, R^{4d}, R^{4e}, R^{4f}, R^{4g} and R^{4h} is independently H, deuterium, F, Cl, Br, I, -CN, -NO₂, -COOH, -OH, -NH₂, -SH, methyl, ethyl, n-propyl, isopropyl, n-butyl, tert-butyl, -CH=CH₂, -CH₂CH=CH₂, -CH=CHCH₃, -C≡CH, methoxy, ethoxy, methylamino, ethylamino, -CF₃, -CHF₂, -CH₂F, -CH₂CF₃, -CH₂CHF₂, -OCF₃, -OCHF₂, -OCH₂F, cyclopropyl, cyclobutyl, cyclopentyl, cyclohexyl, cyclopropyl-CH₂-, cyclobutyl-CH₂-, cyclopentyl-CH₂-, cyclohexyl-CH₂-, pyrrolidinyl, pyrazolidinyl, tetrahydrofuranyl, tetrahydrothienyl, piperidinyl, morpholinyl, thiomorpholinyl, piperazinyl, pyrrolidinyl-CH₂-, pyrazolidinyl-CH₂-, tetrahydrofuranyl-CH₂-, tetrahydrothiophenyl-CH₂-, piperidinyl-CH₂-, morpholinyl-CH₂-, thiomorpholinyl-CH₂-, piperazinyl-CH₂-, phenyl, phenyl-CH₂-, phenyl-CH₂CH₂-, furanyl, thienyl, imidazolyl, pyrimidinyl, pyridyl, pyrrolyl, pyridazinyl, pyrazinyl, thiazolyl, oxazolyl, furyl-CH₂-, thienyl-CH₂-, imidazolyl-CH₂-, pyrimidinyl-CH₂-, pyridyl-CH₂- or pyrrolyl-CH₂-, wherein each R^{4a}, R^{4b}, R^{4c}, R^{4a}, R^{4e}, R^{4f}, R^{4g} and R^{4h} is independently and optionally substituted with 1, 2 or 3 R^{y2};
or R^{4a} and R^{4b}, together with the carbon atoms to which they are attached, form a C₃₋₆ carbon ring or a heterocyclyl consisting of 5-6 atoms, or R^{4c} and R^{4d}, together with the carbon atoms to which they are attached, form a C₃₋₆ carbon ring or a heterocyclyl consisting of 5-6 atoms, or R^{4e} and R^{4f}, together with the carbon atoms to which they are attached, form a C₃₋₆ carbon ring or a heterocyclyl consisting of 5-6 atoms, wherein each C₃₋₆ carbon ring and heterocyclyl consisting of 5-6 atoms is independently unsubstituted or substituted with 1, 2 or 3 RY3.

9. The compound of any one of claims 1-8, wherein each R^{z}, R^{y2}, R^{y3} and R^{y4} is independently deuterium, F, Cl, Br, I, -CN, -OH, -NH₂, - COOH, methyl, ethyl, n-propyl, isopropyl, -CF₃, -CHF₂, -CH₂F, -OCF₃, -OCHF₂, -OCH₂F, methoxy, ethoxy or methylamino.

10. The compound of any one of claims 1-10 having one of the following structures: 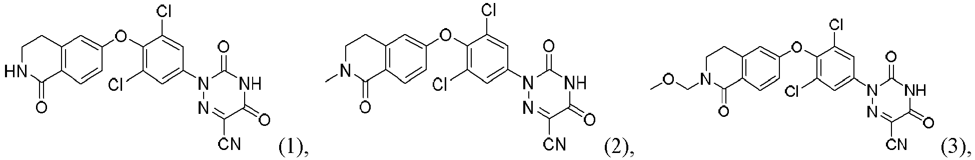 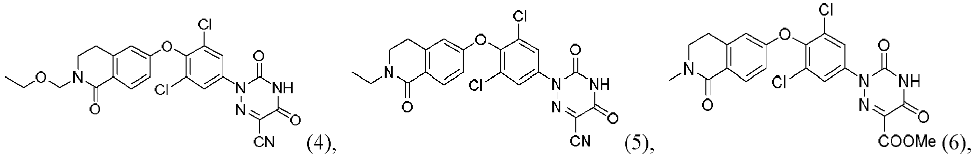 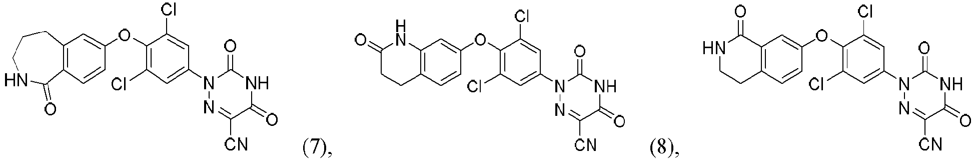 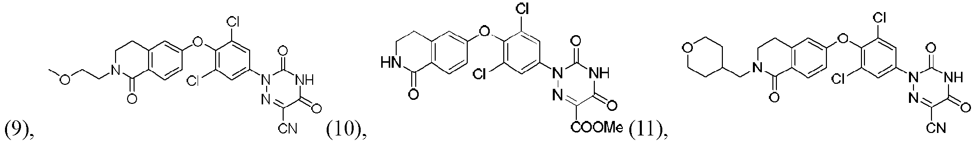 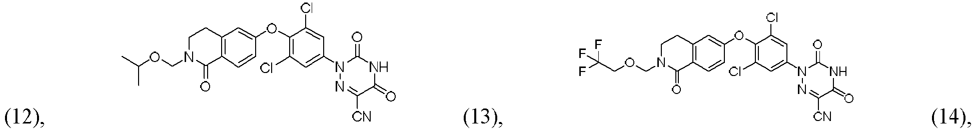 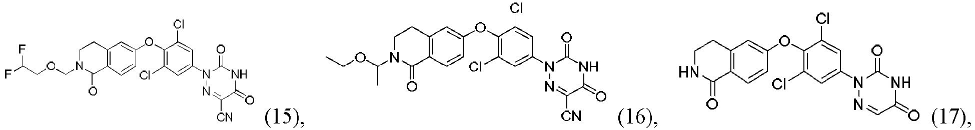 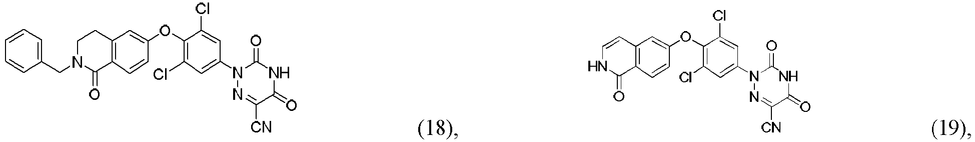 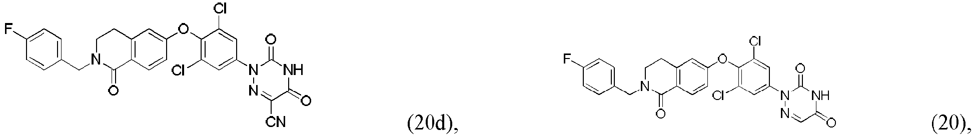 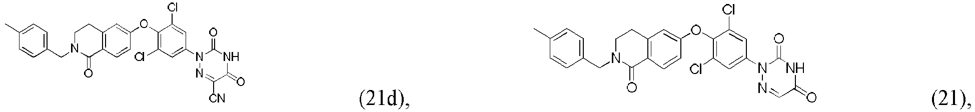 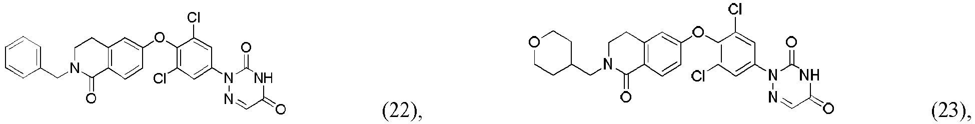 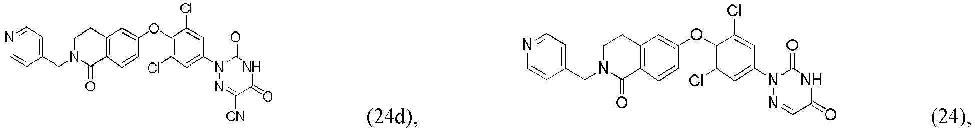 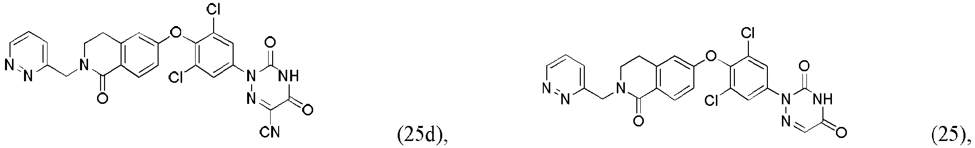 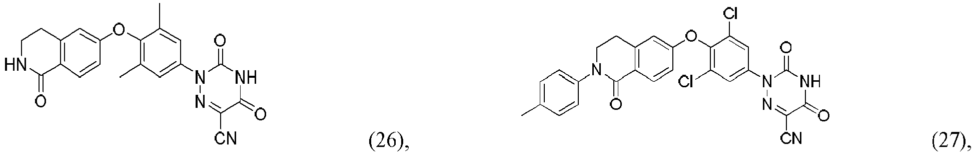 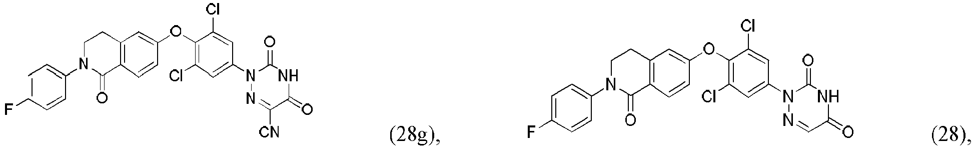 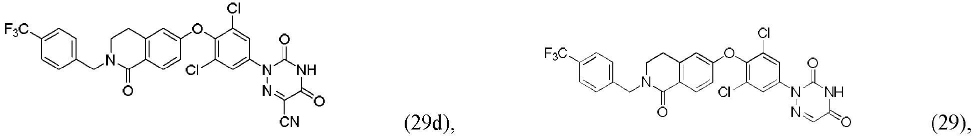 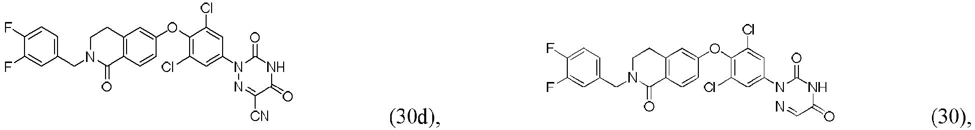 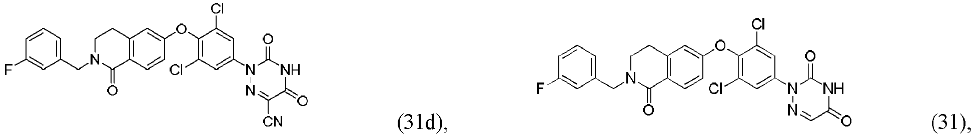 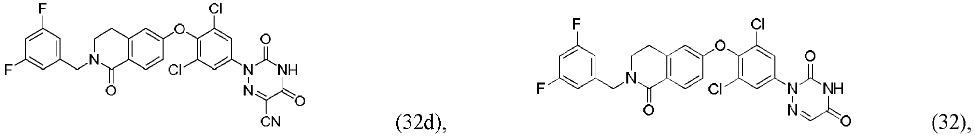 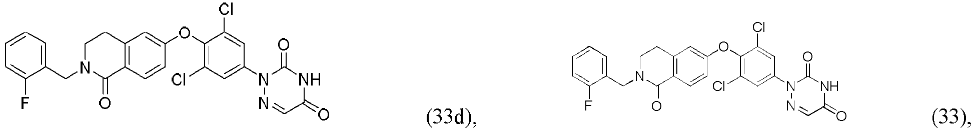 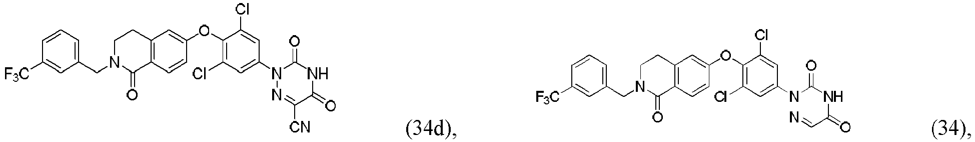 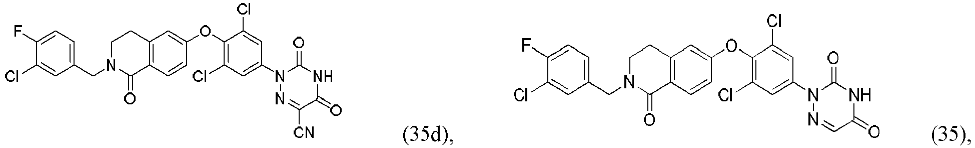 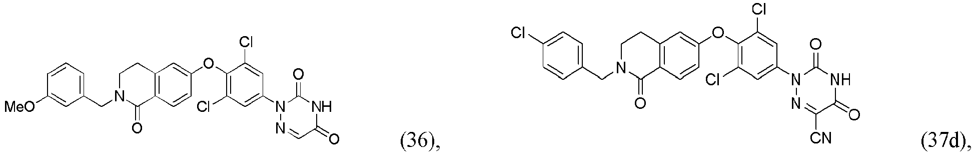 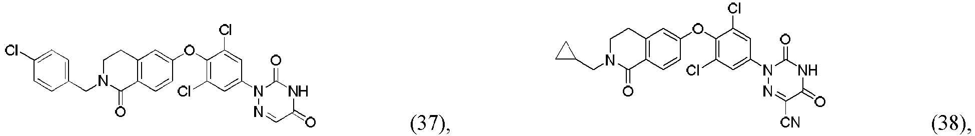 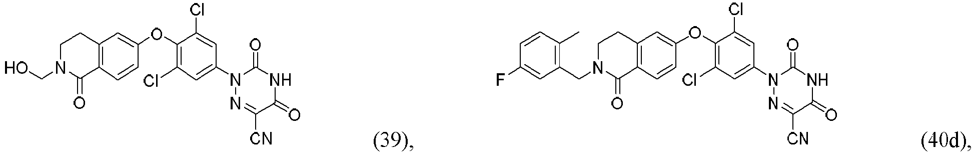 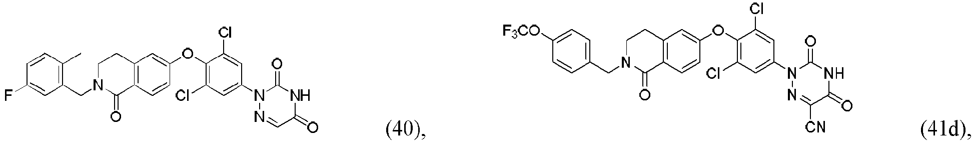 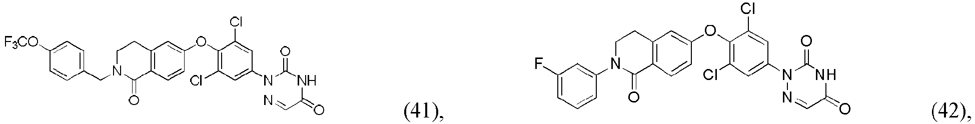 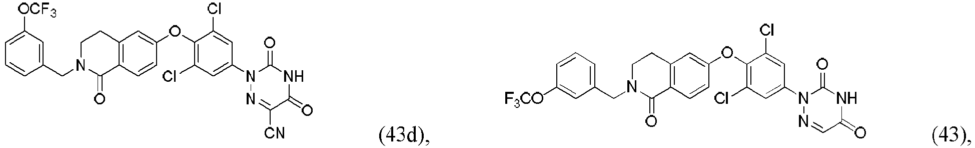 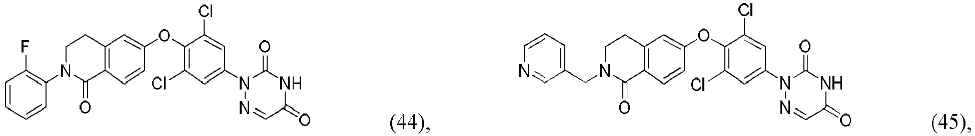 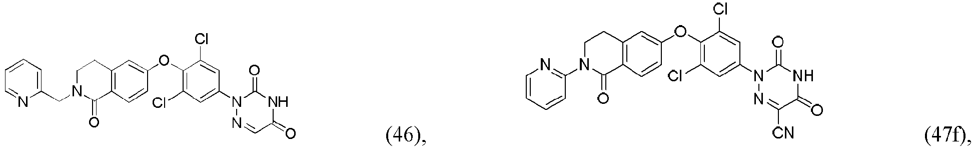 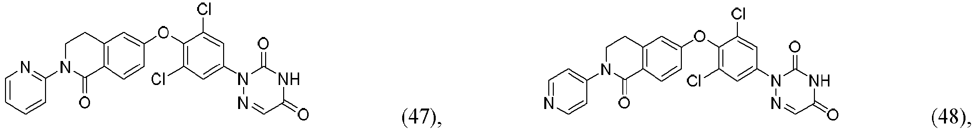 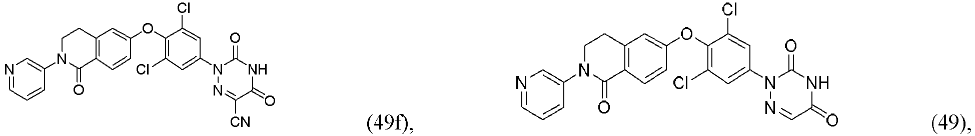 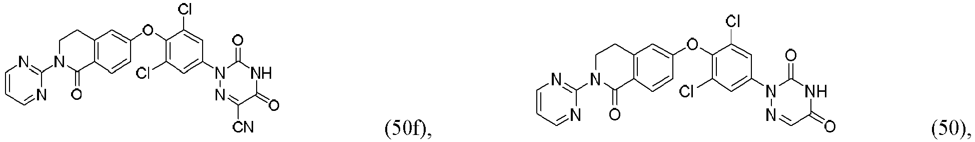 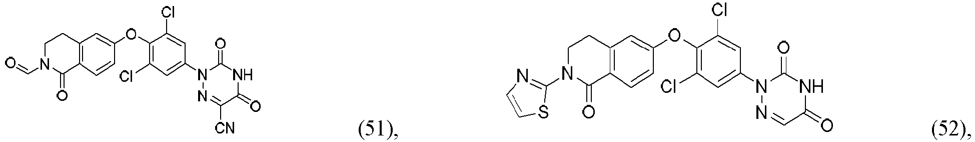 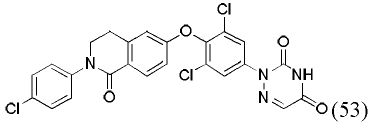 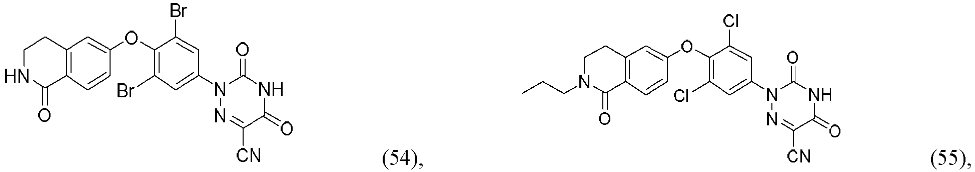 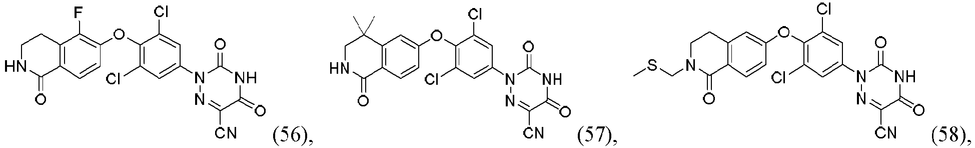 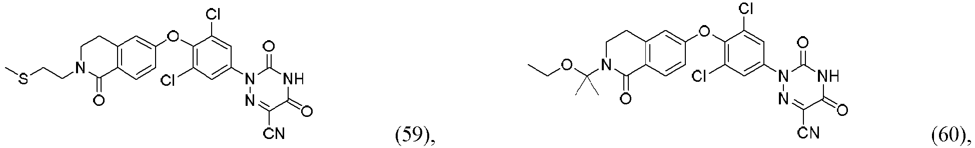 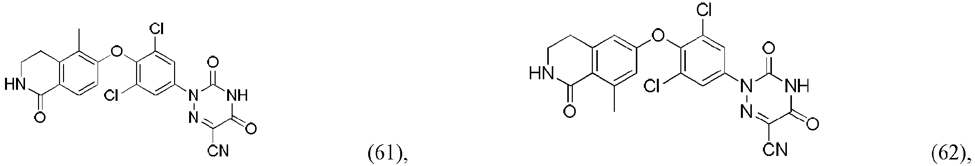 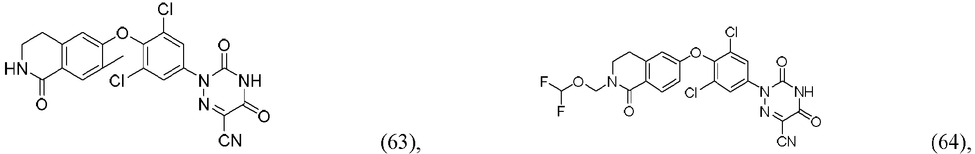 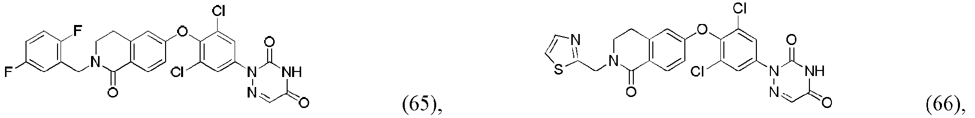 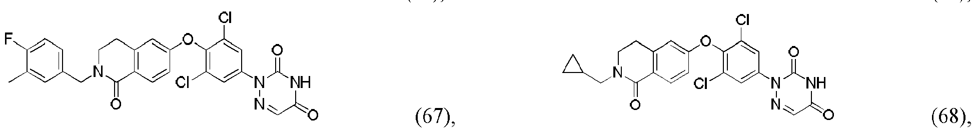 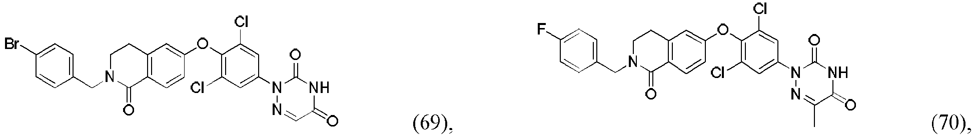 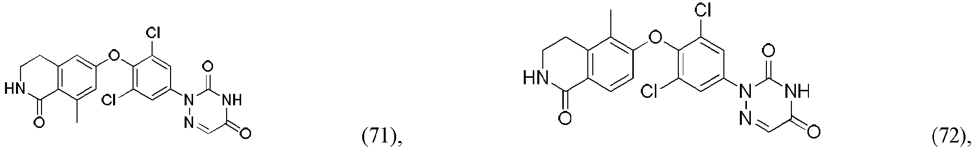 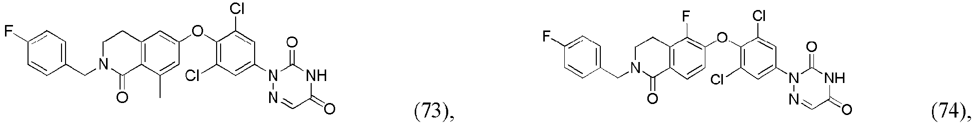 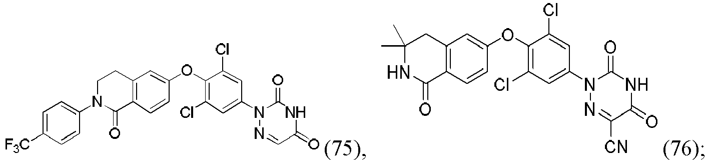 or a stereoisomer, a geometric isomer, a tautomer, an N-oxide, a solvate, a metabolite, a pharmaceutically acceptable salt or a prodrug thereof.

11. A pharmaceutical composition comprising the compound of any one of claims 1 to 10, optionally, further comprising any one of pharmaceutically acceptable carriers, excipients, adjuvants, and vehicles or any combination thereof.

12. Use of the compound of any one of claims 1-10 or the pharmaceutical composition of claim 11 in the manufacture of a medicament for stimulating thyroid hormone receptors; or for preventing, treating or alleviating diseases regulated by thyroid hormone receptors.

13. The use of claim 12, wherein the thyroid hormone receptor is a thyroid hormone β receptor.

14. The use of claim 12, wherein the diseases regulated by thyroid hormone receptors are neurodegenerative diseases, nonalcoholic fatty liver diseases, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer.

15. Use of the compound of any one of claims 1-10 or the pharmaceutical composition of claim 11 in the manufacture of a medicament for preventing, treating or alleviating the following diseases: neurodegenerative diseases, nonalcoholic fatty liver diseases, liver fibrosis, idiopathic pulmonary fibrosis, atherosclerosis, coronary heart diseases, hypertension, hypercholesterolemia, hyperlipidemia, hypertriglyceridemia, dyslipidemia, obesity, diabetes mellitus, metabolic disorder, lipid metabolism disorder, glycogen storage disease type 1A, hypothyroidism or thyroid cancer.

16. The use of claim 14 or 15, wherein the nonalcoholic fatty liver disease is nonalcoholic simple fatty liver, nonalcoholic steatohepatitis, cryptogenic cirrhosis associated with nonalcoholic fatty liver disease or primary liver cancer;
the neurodegenerative disease is demyelinating disease, chronic demyelinating disease, leukodystrophy, dementia, ischemic stroke, lacunar stroke, multiple sclerosis, MCT8 deficiency, X-linked adrenal dystrophy, amyotrophic lateral sclerosis or Alzheimer's disease.
